# EUROPEAN PATENT APPLICATION

(11) **EP 4 835 086 A1**
(43) Date of publication of application: **04.11.2026**
(21) Application number: 24913117.8
(22) Date of filing: 27.12.2024
(51) Int. Cl.: C12N 15/85, A61K 31/711, A61K 47/08, C07C 219/06, C07C 219/16

(54) **METHOD FOR DELIVERING NUCLEIC ACID TO IMMUNE CELLS, AGENT FOR DELIVERING NUCLEIC ACID TO IMMUNE CELLS, AND USE THEREOF**

(30) Priority: 28.12.2023 JP 2023222203; 10.07.2024 JP 2024110673
(71) Applicant: FUJIFILM Corporation, Tokyo 106-8620 (JP)
(72) Inventor: UMETANI Sayako, Ashigarakami-gun, Kanagawa 258-8577 (JP); NAKAMURA Naoto, Ashigarakami-gun, Kanagawa 258-8577 (JP); NITABARU Tatsuya, Ashigarakami-gun, Kanagawa 258-8577 (JP); FUKUNAGA Hirofumi, Ashigarakami-gun, Kanagawa 258-8577 (JP)
(74) Representative: Klunker IP Patentanwälte PartG mbB
(86) International application number: PCT/JP2024/046425
(87) International publication number: WO 2025/143231

(57) **Abstract**

An object of the present invention is to provide a method for delivering nucleic acid to an immune cell and a nucleic acid delivery agent for an immune cell, the method and the agent being capable of delivering nucleic acid to both an immune cell subjected to activation treatment and an immune cells not subjected to activation treatment; and to provide a method of delivering a method for delivering nucleic acid to an immune cell and a nucleic acid delivery agent for an immune cell, the method and the agent being capable of encapsulating nucleic acid in a simple manner without a dedicated device. According to the present invention, there is provided a method for delivering nucleic acid to an immune cell (excluding an in vivo delivery method) including a step A of preparing lipid particles not containing nucleic acid using an ionizable lipid, a non-ionizable lipid, and a lipid having a nonionic polymer, a step B of preparing nucleic acid-containing lipid particles by mixing the lipid particles not containing nucleic acid with nucleic acid, and a step C of bringing the nucleic acid-containing lipid particles into contact with an immune cell.

## Description

### Technical Field

The present invention relates to a method for delivering nucleic acid to an immune cell, a nucleic acid delivery agent for an immune cell, and a use thereof.

### Background Art

In immune cell therapy including chimeric antigen receptor (CAR) T cell therapy, immune cells that are genetically modified to express a therapeutic exogenous gene such as CAR or to modulate the expression of an endogenous gene are used. In the genetic modification of immune cells, a virus vector method is generally used, but there are problems such as safety concerns due to viruses and high cost. In addition, as a non-viral method, an electroporation method has been used in the related art, but there is a problem that cytotoxicity and DNA damage due to electroporation occur, which leads to proliferation delay and chromosomal abnormalities.

Regarding nucleic acid delivery to an immune cell using lipid particles, Patent Document 1 describes that Cas9 mRNA and gRNA are encapsulated in separate LNP to perform genome editing at a plurality of sites, and used for genome editing of a T cell. In addition, Patent Document 2 describes that nucleic acid (mRNA) is delivered to a T cell using an LNP encapsulating the nucleic acid. Patent Document 3 describes that a freeze-dried LNP is rehydrated, mixed with a main hydrate solution and nucleic acid, and subjected to a heat treatment at 95°C or the like, thereby encapsulating the nucleic acid in the LNP without using a dedicated device. Patent Document 4 describes that a purified empty LNP (LNP not containing nucleic acid) and nucleic acid are mixed by a pump system to obtain a nucleic acid-encapsulating LNP.

### Prior Art Documents

### Patent Documents

Patent Document 1: WO2021/222287A
Patent Document 2: WO2020/210901A
Patent Document 3: WO2021/060440A
Patent Document 4: WO2018/089801A

### Summary of Invention

### Object to be solved by the invention

In the lipid nanoparticles described in Patent Documents 1 and 2, a treatment of sufficiently activating an immune cell is required, and there is a limitation on the culturing method that can be used. Since it is known that the activation treatment of the immune cell reduces the efficacy of the treatment of the immune cell, it is desirable not to perform the activation treatment. In addition, in Patent Documents 1 and 2, there is a problem that a high-priced dedicated device such as a high-speed microfluidic device is required for encapsulating nucleic acid in a lipid nanoparticle, and in addition, a complicated step such as ultrafiltration is required each time the nucleic acid is encapsulated. In Patent Document 3, there is a problem that a complicated step of rehydrating a freeze-dried product and encapsulating nucleic acid is required. In addition, in Patent Document 4, there is a problem that heating is required.

In view of such circumstances, an object of the present invention is to provide a method for delivering nucleic acid to an immune cell and a nucleic acid delivery agent for an immune cell, the method and the agent being capable of delivering nucleic acid to both an immune cell subjected to activation treatment and an immune cell not subjected to activation treatment. Another object of the present invention is to provide a method of delivering a method for delivering nucleic acid to an immune cell and a nucleic acid delivery agent for an immune cell, the method and the agent being capable of encapsulating nucleic acid in a simple manner without a dedicated device.

### Means for solving the object

According to an aspect of the present invention, the following inventions are provided.
<1> A method for delivering nucleic acid to an immune cell (excluding an in vivo delivery method), the method comprising:
   a step A of preparing lipid particles not containing nucleic acid using an ionizable lipid, a non-ionizable lipid, and a lipid having a nonionic polymer;
   a step B of preparing nucleic acid-containing lipid particles by mixing the lipid particles not containing nucleic acid with nucleic acid; and
   a step C of bringing the nucleic acid-containing lipid particles into contact with an immune cell.
<2> The method according to <1>, further comprising:
   freezing and storing the lipid particles not containing nucleic acid and thawing the lipid particles not containing nucleic acid that have been frozen and stored before mixing with the nucleic acid.
<3> The method according to <1> or <2>, in which the step B includes a step of incubating the lipid particles not containing nucleic acid and an aqueous solution containing the nucleic acid at 0°C to 30°C for 0.1 to 120 minutes, and a step of adjusting a pH of the resulting mixture to 6.5 to 8.5.
<4> The method according to any one of <1> to <3>, in which, in the step B, the mixing of the lipid particles not containing nucleic acid with the nucleic acid is performed by any one selected from mixing in which a liquid is moved back and forth in a reciprocating direction in a container, pipette mixing, stirrer mixing in a batch container, mixing in which a liquid content is agitated by rotating a container, or flask agitation.
<5> The method according to any one of <1> to <4>, further comprising:
   a step of adding an apolipoprotein to a culture solution containing the immune cell before bringing the nucleic acid-containing lipid particles into contact with the immune cell in the step C.
<6> The method according to any one of <1> to <5>, in which the ionizable lipid is a compound represented by Formula (1) or a salt thereof, 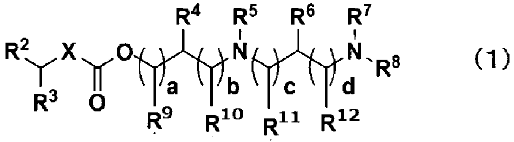 in the formula, X represents -NR¹- or -O-,
   R¹ represents a hydrogen atom, a hydrocarbon group having 6 to 24 carbon atoms, or a group represented by R²¹-L¹-R²²-, where R²¹ represents a hydrocarbon group having 1 to 24 carbon atoms, L¹ represents -O(CO)O-, -O(CO)-, -(CO)O-, -O-, or 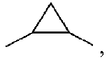 and R²² represents a divalent hydrocarbon linking group having 1 to 18 carbon atoms,
   R² and R³ each independently represent a hydrogen atom, a hydrocarbon group having 3 to 24 carbon atoms, or a group represented by R³¹-L²-R³²-, where R³¹ represents a hydrocarbon group having 1 to 24 carbon atoms, L² represents -O(CO)O-, -O(CO)-, -(CO)O-, -O-, or 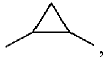 and R³² represents a divalent hydrocarbon linking group having 1 to 18 carbon atoms,
   R⁴, R⁵, R⁶, R⁷, R⁸, R¹⁰, R¹¹, and R¹² each independently represent a hydrogen atom or an alkyl group having 1 to 18 carbon atoms which may be substituted,
   any one or more of the pairs of R⁴ and R⁵, R¹⁰ and R⁵, R⁵ and R¹², R⁴ and R⁶, R⁵ and R⁶, R⁶ and R⁷, R⁶ and R¹⁰, R¹² and R⁷, and R⁷ and R⁸ may be linked to each other to form a 4-to 7-membered ring which may contain an O atom,
   a substituent on the alkyl group having 1 to 18 carbon atoms which may be substituted is a hydroxyl group, a carboxyl group, an amino group represented by -NR⁴⁵R⁴⁶, a substituted or unsubstituted aryl group, a substituted or unsubstituted heteroaryl group, or a group represented by -O(CO)O-R⁴¹, -O(CO)-R⁴², -(CO)O-R⁴³, or -O-R⁴⁴, where R⁴¹, R⁴², R⁴³, R⁴⁴, R⁴⁵, and R⁴⁶ each independently represent a hydrocarbon group having 1 to 18 carbon atoms,
   a substituent on the substituted or unsubstituted aryl group and on the substituted or unsubstituted heteroaryl group is an alkyl group having 1 to 18 carbon atoms, a hydroxyl group, a carboxyl group, an amino group represented by -NR⁴⁵R⁴⁶, or a group represented by -O(CO)O-R⁴¹, -O(CO)-R⁴², -(CO)O-R⁴³, or -O-R⁴⁴, where R⁴¹, R⁴², R⁴³, R⁴⁴, R⁴⁵, and R⁴⁶ each independently represent a hydrocarbon group having 1 to 18 carbon atoms, and
   a, b, c, and d each independently represent an integer of 0 to 3, provided that a + b is 1 or more, and c + d is 1 or more.
<7> The method according to any one of <1> to <5>, in which the ionizable lipid is a compound represented by Formula (2) or a salt thereof, 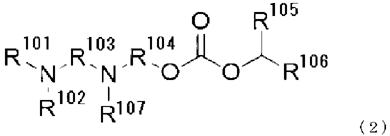 in the formula,
   R¹⁰¹ and R¹⁰² each independently represent a hydrocarbon group having 1 to 18 carbon atoms, and R¹⁰³ represents a hydrocarbon group having 2 to 8 carbon atoms, where the hydrocarbon groups represented by R¹⁰¹, R¹⁰², and R¹⁰³ may be substituted with one or more substituents selected from -OH, -COOH, -NR¹⁵¹R¹⁵², -OC(O)O-R¹⁵³, -C(O)O-R¹⁵⁴, -OC(O)-R¹⁵⁵, and -O-R¹⁵⁶, and R¹⁰² and R¹⁰³ may be linked to each other to form a 4- to 7-membered ring,
   R¹⁰⁴ represents a hydrocarbon group having 1 to 8 carbon atoms,
   R¹⁰⁵ and R¹⁰⁶ each independently represent a hydrocarbon group having 1 to 8 carbon atoms or -R¹⁰⁸-L¹⁰¹-R¹⁰⁹, excluding a case where both R¹⁰⁵ and R¹⁰⁶ are hydrocarbon groups having 1 to 8 carbon atoms,
   R¹⁰⁷ represents -R¹¹⁰-L¹⁰²-R¹¹¹-L¹⁰³-R¹¹²,
   R¹⁵¹ and R¹⁵² each independently represent a hydrocarbon group having 1 to 8 carbon atoms,
   R¹⁵³, R¹⁵⁴, R¹⁵⁵, and R¹⁵⁶ each independently represent a hydrocarbon group having 1 to 24 carbon atoms,
   the hydrocarbon groups represented by R¹⁵³, R¹⁵⁴, R¹⁵⁵, and R¹⁵⁶ may be substituted with an aryl group having 6 to 20 carbon atoms or -S-R¹⁵⁸,
   the aryl group having 6 to 20 carbon atoms may be substituted with -OH, -COOH, -NR¹⁵¹R¹⁵², -OC(O)O-R¹⁵³, -C(O)O-R¹⁵⁴, -OC(O)-R¹⁵⁵, -O-R¹⁵⁶, or -(hydrocarbon group having 1 to 12 carbon atoms)-R¹⁵⁷,
   R¹⁵⁸ represents a hydrocarbon group having 1 to 12 carbon atoms,
   R¹⁵⁷ represents -OH, -COOH, -NR¹⁶¹R¹⁶², -OC(O)O-R¹⁶³, -C(O)O-R¹⁶⁴, -OC(O)-R¹⁶⁵, or -O-R¹⁶⁶,
   R¹⁶¹ and R¹⁶² each independently represent a hydrocarbon group having 1 to 8 carbon atoms,
   R¹⁶³, R¹⁶⁴, R¹⁶⁵, and R¹⁶⁶ each independently represent a hydrocarbon group having 1 to 24 carbon atoms,
   the hydrocarbon groups represented by R¹⁶³, R¹⁶⁴, R¹⁶⁵, and R¹⁶⁶ may be substituted with an aryl group having 6 to 20 carbon atoms or -S-R¹⁶⁸,
   the aryl group having 6 to 20 carbon atoms may be substituted with -OH, -COOH, -NR¹⁶¹R¹⁶², -OC(O)O-R¹⁶³, -C(O)O-R¹⁶⁴, -OC(O)-R¹⁶⁵, -O-R¹⁶⁶, or a hydrocarbon group having 1 to 12 carbon atoms,
   R¹⁶⁸ represents a hydrocarbon group having 1 to 12 carbon atoms,
   L¹⁰¹, L¹⁰², and L¹⁰³ each independently represent -OC(O)O-, -C(O)O-, -OC(O)-, or -O-,
   R¹⁰⁸ represents a hydrocarbon group having 1 to 12 carbon atoms,
   R¹⁰⁹ represents a hydrocarbon group having 1 to 24 carbon atoms,
   R¹¹⁰ represents a hydrocarbon group having 1 to 8 carbon atoms,
   R¹¹¹ represents a hydrocarbon group having 1 to 24 carbon atoms,
   R¹¹² represents a hydrocarbon group having 1 to 24 carbon atoms,
   the hydrocarbon groups represented by R¹⁰⁹ and R¹¹² may be substituted with an aryl group, -OC(O)O-R¹⁵³, -C(O)O-R¹⁵⁴, -OC(O)-R¹⁵⁵, or -S-R¹⁵⁸, where definitions of R¹⁵³, R¹⁵⁴, R¹⁵⁵, and R¹⁵⁸ are as described above, and
   the hydrocarbon group represented by R¹¹¹ may be substituted with -OC(O)O-R¹⁵³, -C(O)O-R¹⁵⁴, or -OC(O)-R¹⁵⁵, where the definitions of R¹⁵³, R¹⁵⁴, and R¹⁵⁵ are as described above.
<8> The method according to any one of <1> to <7>, in which the non-ionizable lipid contains a sterol or a derivative thereof, and a phospholipid.
<9> The method according to <8>, in which a molar ratio of the sterol to total lipids in a lipid composition is 30 to 70 mol%.
<10> The method according to any one of <1> to <9>, in which a pH of a lipid composition is 3.0 to 6.5.
<11> The method according to any one of <1> to <10>, in which, in the step B, a mass ratio of a lipid concentration to a nucleic acid concentration in a solution after the mixing is 5:1 to 1,000:1.
<12> The method according to any one of <1> to <11>, in which the immune cell are an activated cell or a non-activated cell.
<13> The method according to any one of <1> to <12>, in which the immune cells are derived from a primary cell or a stem cell.
<14> The method according to any one of <1> to <13>, in which the nucleic acid is
   mRNA, or
   a nucleic acid for gene editing that contains an mRNA encoding a Cas nuclease and a guide RNA.
<15> A nucleic acid delivery agent for an immune cell, comprising: a lipid composition containing an ionizable lipid that is a compound represented by Formula (1) or Formula (2) or a salt thereof, a non-ionizable lipid, a lipid having a nonionic polymer, and a nucleic acid, 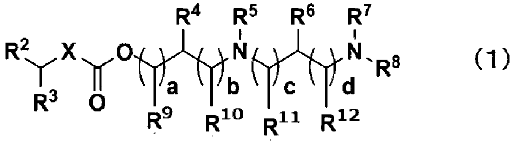 in the formula, X represents -NR¹- or -O-,
   R¹ represents a hydrogen atom, a hydrocarbon group having 6 to 24 carbon atoms, or a group represented by R²¹-L¹-R²²-, where R²¹ represents a hydrocarbon group having 1 to 24 carbon atoms, L¹ represents -O(CO)O-, -O(CO)-, -(CO)O-, -O-, or 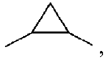 and R³² represents a divalent hydrocarbon linking group having 1 to 18 carbon atoms,
   R² and R³ each independently represent a hydrogen atom, a hydrocarbon group having 3 to 24 carbon atoms, or a group represented by R³¹-L²-R³²-, where R³¹ represents a hydrocarbon group having 1 to 24 carbon atoms, L² represents -O(CO)O-, -O(CO)-, -(CO)O-, -O-, or 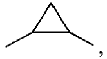 and R³² represents a divalent hydrocarbon linking group having 1 to 18 carbon atoms,
   R⁴, R⁵, R⁶, R⁷, R⁸, R¹⁰, R¹¹, and R¹² each independently represent a hydrogen atom or an alkyl group having 1 to 18 carbon atoms which may be substituted,
   any one or more of the pairs of R⁴ and R⁵, R¹⁰ and R⁵, R⁵ and R¹², R⁴ and R⁶, R⁵ and R⁶, R⁶ and R⁷, R⁶ and R¹⁰, R¹² and R⁷, and R⁷ and R⁸ may be linked to each other to form a 4-to 7-membered ring which may contain an O atom,
   a substituent on the alkyl group having 1 to 18 carbon atoms which may be substituted is a hydroxyl group, a carboxyl group, an amino group represented by -NR⁴⁵R⁴⁶, a substituted or unsubstituted aryl group, a substituted or unsubstituted heteroaryl group, or a group represented by -O(CO)O-R⁴¹, -O(CO)-R⁴², -(CO)O-R⁴³, or -O-R⁴⁴, where R⁴¹, R⁴², R⁴³, R⁴⁴, R⁴⁵, and R⁴⁶ each independently represent a hydrocarbon group having 1 to 18 carbon atoms,
   a substituent on the substituted or unsubstituted aryl group and on the substituted or unsubstituted heteroaryl group is an alkyl group having 1 to 18 carbon atoms, a hydroxyl group, a carboxyl group, an amino group represented by -NR⁴⁵R⁴⁶, or a group represented by -O(CO)O-R⁴¹, -O(CO)-R⁴², -(CO)O-R⁴³, or -O-R⁴⁴, where R⁴¹, R⁴², R⁴³, R⁴⁴, R⁴⁵, and R⁴⁶ each independently represent a hydrocarbon group having 1 to 18 carbon atoms, and
   a, b, c, and d each independently represent an integer of 0 to 3, provided that a + b is 1 or more, and c + d is 1 or more, 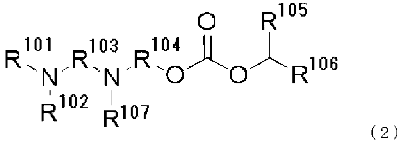
   in the formula,
   R¹⁰¹ and R¹⁰² each independently represent a hydrocarbon group having 1 to 18 carbon atoms, and R¹⁰³ represents a hydrocarbon group having 2 to 8 carbon atoms, where the hydrocarbon groups represented by R¹⁰¹, R¹⁰², and R¹⁰³ may be substituted with one or more substituents selected from -OH, -COOH, -NR¹⁵¹R¹⁵², -OC(O)O-R¹⁵³, -C(O)O-R¹⁵⁴, -OC(O)-R¹⁵⁵, and -O-R¹⁵⁶, and R¹⁰² and R¹⁰³ may be linked to each other to form a 4- to 7-membered ring,
   R¹⁰⁴ represents a hydrocarbon group having 1 to 8 carbon atoms,
   R¹⁰⁵ and R¹⁰⁶ each independently represent a hydrocarbon group having 1 to 8 carbon atoms or -R¹⁰⁸-L¹⁰¹-R¹⁰⁹, excluding a case where both R¹⁰⁵ and R¹⁰⁶ are hydrocarbon groups having 1 to 8 carbon atoms,
   R¹⁰⁷ represents -R¹¹⁰-L¹⁰²-R¹¹¹-L¹⁰³-R¹¹²,
   R¹⁵¹ and R¹⁵² each independently represent a hydrocarbon group having 1 to 8 carbon atoms,
   R¹⁵³, R¹⁵⁴, R¹⁵⁵, and R¹⁵⁶ each independently represent a hydrocarbon group having 1 to 24 carbon atoms,
   the hydrocarbon groups represented by R¹⁵³, R¹⁵⁴, R¹⁵⁵, and R¹⁵⁶ may be substituted with an aryl group having 6 to 20 carbon atoms or -S-R¹⁵⁸,
   the aryl group having 6 to 20 carbon atoms may be substituted with -OH, -COOH, -NR¹⁵¹R¹⁵², -OC(O)O-R¹⁵³, -C(O)O-R¹⁵⁴, -OC(O)-R¹⁵⁵, -O-R¹⁵⁶, or -(hydrocarbon group having 1 to 12 carbon atoms)-R¹⁵⁷,
   R¹⁵⁸ represents a hydrocarbon group having 1 to 12 carbon atoms,
   R¹⁵⁷ represents -OH, -COOH, -NR¹⁶¹R¹⁶², -OC(O)O-R¹⁶³, -C(O)O-R¹⁶⁴, -OC(O)-R¹⁶⁵, or -O-R¹⁶⁶,
   R¹⁶¹ and R¹⁶² each independently represent a hydrocarbon group having 1 to 8 carbon atoms,
   R¹⁶³, R¹⁶⁴, R¹⁶⁵, and R¹⁶⁶ each independently represent a hydrocarbon group having 1 to 24 carbon atoms,
   the hydrocarbon groups represented by R¹⁶³, R¹⁶⁴, R¹⁶⁵, and R¹⁶⁶ may be substituted with an aryl group having 6 to 20 carbon atoms or -S-R¹⁶⁸,
   the aryl group having 6 to 20 carbon atoms may be substituted with -OH, -COOH, -NR¹⁶¹R¹⁶², -OC(O)O-R¹⁶³, -C(O)O-R¹⁶⁴, -OC(O)-R¹⁶⁵, -O-R¹⁶⁶, or a hydrocarbon group having 1 to 12 carbon atoms,
   R¹⁶⁸ represents a hydrocarbon group having 1 to 12 carbon atoms,
   L¹⁰¹, L¹⁰², and L¹⁰³ each independently represent -OC(O)O-, -C(O)O-, -OC(O)-, or -O-,
   R¹⁰⁸ represents a hydrocarbon group having 1 to 12 carbon atoms,
   R¹⁰⁹ represents a hydrocarbon group having 1 to 24 carbon atoms,
   R¹¹⁰ represents a hydrocarbon group having 1 to 8 carbon atoms,
   R¹¹¹ represents a hydrocarbon group having 1 to 24 carbon atoms,
   R¹¹² represents a hydrocarbon group having 1 to 24 carbon atoms,
   the hydrocarbon groups represented by R¹⁰⁹ and R¹¹² may be substituted with an aryl group, -OC(O)O-R¹⁵³, -C(O)O-R¹⁵⁴, -OC(O)-R¹⁵⁵, or -S-R¹⁵⁸, where definitions of R⁵³, R⁵⁴, R⁵⁵, and R⁵⁸ are as described above, and
   the hydrocarbon group represented by R¹¹¹ may be substituted with -OCCO)O-R¹⁵³, -C(O)O-R¹⁵⁴, or -OCCO)-R¹⁵⁵, where the definitions of R¹⁵³, R¹⁵⁴, and R¹⁵⁵ are as described above.
<16> The nucleic acid delivery agent for an immune cell according to <15>, in which the non-ionizable lipid contains a sterol or a derivative thereof, and a phospholipid.
<17> The nucleic acid delivery agent for an immune cell according to <16>, in which a molar ratio of the sterol to total lipids in a lipid composition is 30 to 70 mol%.
<18> The nucleic acid delivery agent for an immune cell according to any one of <15> to <17>, in which the nucleic acid is
   mRNA, or
   a nucleic acid for gene editing that contains an mRNA encoding a Cas nuclease and a guide RNA.
<19> A nucleic acid delivery agent for an immune cell, comprising:
   a lipid composition containing an ionizable lipid that is a compound represented by Formula (1) or Formula (2) or a salt thereof, a non-ionizable lipid, and a lipid having a nonionic polymer, 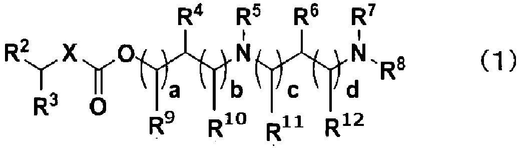
   in the formula, X represents -NR¹- or -O-,
   R¹ represents a hydrogen atom, a hydrocarbon group having 10 to 24 carbon atoms, or a group represented by R²¹-L¹-R²²-, where R²¹ represents a hydrocarbon group having 1 to 24 carbon atoms, L¹ represents -O(CO)O-, -O(CO)-, -(CO)O-, -O-, or 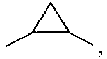 and R³² represents a divalent hydrocarbon linking group having 1 to 18 carbon atoms,
   R² and R³ each independently represent a hydrogen atom, a hydrocarbon group having 3 to 24 carbon atoms, or a group represented by R³¹-L²-R³²-, where R³¹ represents a hydrocarbon group having 1 to 24 carbon atoms, L² represents -O(CO)O-, -O(CO)-, -(CO)O-, -O-, or 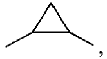 and R³² represents a divalent hydrocarbon linking group having 1 to 18 carbon atoms,
   R⁴, R⁵, R⁶, R⁷, R⁸, R⁹, R¹⁰, R¹¹, and R¹² each independently represent a hydrogen atom or an alkyl group having 1 to 18 carbon atoms which may be substituted,
   any one or more of the pairs of R⁴ and R⁵, R¹⁰ and R⁵, R⁵ and R¹², R⁴ and R⁶, R⁵ and R⁶, R⁶ and R⁷, R⁶ and R¹⁰, R¹² and R⁷, and R⁷ and R⁸ may be linked to each other to form a 4-to 7-membered ring which may contain an O atom,
   a substituent on the alkyl group having 1 to 18 carbon atoms which may be substituted is a hydroxyl group, a carboxyl group, an amino group represented by -NR⁴⁵R⁴⁶, a substituted or unsubstituted aryl group, a substituted or unsubstituted heteroaryl group, or a group represented by -O(CO)O-R⁴¹, -O(CO)-R⁴², -(CO)O-R⁴³, or -O-R⁴⁴, where R⁴¹, R⁴², R⁴³, R⁴⁴, R⁴⁵, and R⁴⁶ each independently represent a hydrocarbon group having 1 to 18 carbon atoms,
   a substituent on the substituted or unsubstituted aryl group and on the substituted or unsubstituted heteroaryl group is an alkyl group having 1 to 18 carbon atoms, a hydroxyl group, a carboxyl group, an amino group represented by -NR⁴⁵R⁴⁶, or a group represented by -O(CO)O-R⁴¹, -O(CO)-R⁴², -(CO)O-R⁴³, or -O-R⁴⁴, where R⁴¹, R⁴², R⁴³, R⁴⁴, R⁴⁵, and R⁴⁶ each independently represent a hydrocarbon group having 1 to 18 carbon atoms, and
   a, b, c, and d each independently represent an integer of 0 to 3, provided that a + b is 1 or more, and c + d is 1 or more, 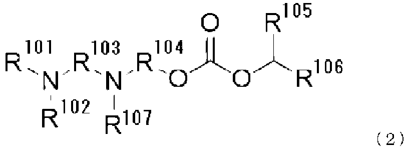
   in the formula,
   R¹⁰¹ and R¹⁰² each independently represent a hydrocarbon group having 1 to 18 carbon atoms, and R¹⁰³ represents a hydrocarbon group having 2 to 8 carbon atoms, where the hydrocarbon groups represented by R¹⁰¹, R¹⁰², and R¹⁰³ may be substituted with one or more substituents selected from -OH, -COOH, -NR¹⁵¹R¹⁵², -OC(O)O-R¹⁵³, -C(O)O-R¹⁵⁴, -OC(O)-R¹⁵⁵, and -O-R¹⁵⁶, and R¹⁰² and R¹⁰³ may be linked to each other to form a 4- to 7-membered ring,
   R¹⁰⁴ represents a hydrocarbon group having 1 to 8 carbon atoms,
   R¹⁰⁵ and R¹⁰⁶ each independently represent a hydrocarbon group having 1 to 8 carbon atoms or -R¹⁰⁸-L¹⁰¹-R¹⁰⁹, excluding a case where both R¹⁰⁵ and R¹⁰⁶ are hydrocarbon groups having 1 to 8 carbon atoms,
   R¹⁰⁷ represents -R¹¹⁰-L¹⁰²-R¹¹¹-L¹⁰³-R¹¹²,
   R¹⁵¹ and R¹⁵² each independently represent a hydrocarbon group having 1 to 8 carbon atoms,
   R¹⁵³, R¹⁵⁴, R¹⁵⁵, and R¹⁵⁶ each independently represent a hydrocarbon group having 1 to 24 carbon atoms,
   the hydrocarbon groups represented by R¹⁵³, R¹⁵⁴, R¹⁵⁵, and R¹⁵⁶ may be substituted with an aryl group having 6 to 20 carbon atoms or -S-R¹⁵⁸,
   the aryl group having 6 to 20 carbon atoms may be substituted with -OH, -COOH, -NR¹⁵¹R¹⁵², -OC(O)O-R¹⁵³, -C(O)O-R¹⁵⁴, -OC(O)-R¹⁵⁵, -O-R¹⁵⁶, or -(hydrocarbon group having 1 to 12 carbon atoms)-R¹⁵⁷,
   R¹⁵⁸ represents a hydrocarbon group having 1 to 12 carbon atoms,
   R¹⁵⁷ represents -OH, -COOH, -NR¹⁶¹R¹⁶², -OC(O)O-R¹⁶³, -C(O)O-R¹⁶⁴, -OC(O)-R¹⁶⁵, or -O-R¹⁶⁶,
   R¹⁶¹ and R¹⁶² each independently represent a hydrocarbon group having 1 to 8 carbon atoms,
   R¹⁶³, R¹⁶⁴, R¹⁶⁵, and R¹⁶⁶ each independently represent a hydrocarbon group having 1 to 24 carbon atoms,
   the hydrocarbon groups represented by R¹⁶³, R¹⁶⁴, R¹⁶⁵, and R¹⁶⁶ may be substituted with an aryl group having 6 to 20 carbon atoms or -S-R¹⁶⁸,
   the aryl group having 6 to 20 carbon atoms may be substituted with -OH, -COOH, -NR¹⁶¹R¹⁶², -OC(O)O-R¹⁶³, -C(O)O-R¹⁶⁴, -OC(O)-R¹⁶⁵, -O-R¹⁶⁶, or a hydrocarbon group having 1 to 12 carbon atoms,
   R¹⁶⁸ represents a hydrocarbon group having 1 to 12 carbon atoms,
   L¹⁰¹, L¹⁰², and L¹⁰³ each independently represent -OC(O)O-, -C(O)O-, -OC(O)-, or -O-,
   R¹⁰⁸ represents a hydrocarbon group having 1 to 12 carbon atoms,
   R¹⁰⁹ represents a hydrocarbon group having 1 to 24 carbon atoms,
   R¹¹⁰ represents a hydrocarbon group having 1 to 8 carbon atoms,
   R¹¹¹ represents a hydrocarbon group having 1 to 24 carbon atoms,
   R¹¹² represents a hydrocarbon group having 1 to 24 carbon atoms,
   the hydrocarbon groups represented by R¹⁰⁹ and R¹¹² may be substituted with an aryl group, -OC(O)O-R¹⁵³, -C(O)O-R¹⁵⁴, -OC(O)-R¹⁵⁵, or -S-R¹⁵⁸, where definitions of R⁵³, R⁵⁴, R⁵⁵, and R⁵⁸ are as described above, and
   the hydrocarbon group represented by R¹¹¹ may be substituted with -OCCO)O-R¹⁵³, -C(O)O-R¹⁵⁴, or -OCCO)-R¹⁵⁵, where the definitions of R¹⁵³, R¹⁵⁴, and R¹⁵⁵ are as described above.
<20> The nucleic acid delivery agent for an immune cell according to <19>, in which the non-ionizable lipid contains a sterol or a derivative thereof, and a phospholipid.
<21> The nucleic acid delivery agent for an immune cell according to <19>, in which a molar ratio of the sterol to total lipids in a lipid composition is 30 to 70 mol%.

A kit for delivering nucleic acid to a cell, the kit comprising: the following reagents (A) to (C),
(A) a lipid composition containing an ionizable lipid that is a compound represented by Formula (1) or Formula (2) or a salt thereof, a non-ionizable lipid, and a lipid having a nonionic polymer;
(B) a pH adjusting agent; and
(C) an apolipoprotein, 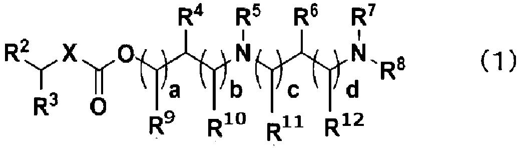 in the formula, X represents -NR¹- or -O-,
   R¹ represents a hydrogen atom, a hydrocarbon group having 14 to 24 carbon atoms, or a group represented by R²¹-L¹-R²²-, where R²¹ represents a hydrocarbon group having 1 to 24 carbon atoms, L¹ represents -O(CO)O-, -O(CO)-, -(CO)O-, -O-, or 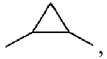 and R³² represents a divalent hydrocarbon linking group having 1 to 18 carbon atoms,
   R² and R³ each independently represent a hydrogen atom, a hydrocarbon group having 3 to 24 carbon atoms, or a group represented by R³¹-L²-R³²-, where R³¹ represents a hydrocarbon group having 1 to 24 carbon atoms, L² represents -O(CO)O-, -O(CO)-, -(CO)O-, -O-, or 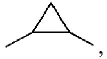 and R³² represents a divalent hydrocarbon linking group having 1 to 18 carbon atoms,
   R⁴, R⁵, R⁶, R⁷, R⁸, R¹⁰, R¹¹, and R¹² each independently represent a hydrogen atom or an alkyl group having 1 to 18 carbon atoms which may be substituted,
   any one or more of the pairs of R⁴ and R⁵, R¹⁰ and R⁵, R⁵ and R¹², R⁴ and R⁶, R⁵ and R⁶, R⁶ and R⁷, R⁶ and R¹⁰, R¹² and R⁷, and R⁷ and R⁸ may be linked to each other to form a 4-to 7-membered ring which may contain an O atom,
   a substituent on the alkyl group having 1 to 18 carbon atoms which may be substituted is a hydroxyl group, a carboxyl group, an amino group represented by -NR⁴⁵R⁴⁶, a substituted or unsubstituted aryl group, a substituted or unsubstituted heteroaryl group, or a group represented by -O(CO)O-R⁴¹, -O(CO)-R⁴², -(CO)O-R⁴³, or -O-R⁴⁴, where R⁴¹, R⁴², R⁴³, R⁴⁴, R⁴⁵, and R⁴⁶ each independently represent a hydrocarbon group having 1 to 18 carbon atoms,
   a substituent on the substituted or unsubstituted aryl group and on the substituted or unsubstituted heteroaryl group is an alkyl group having 1 to 18 carbon atoms, a hydroxyl group, a carboxyl group, an amino group represented by -NR⁴⁵R⁴⁶, or a group represented by -O(CO)O-R⁴¹, -O(CO)-R⁴², -(CO)O-R⁴³, or -O-R⁴⁴, where R⁴¹, R⁴², R⁴³, R⁴⁴, R⁴⁵, and R⁴⁶ each independently represent a hydrocarbon group having 1 to 18 carbon atoms, and
   a, b, c, and d each independently represent an integer of 0 to 3, provided that a + b is 1 or more, and c + d is 1 or more, 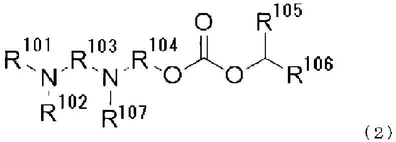
   in the formula,
   R¹⁰¹ and R¹⁰² each independently represent a hydrocarbon group having 1 to 18 carbon atoms, and R¹⁰³ represents a hydrocarbon group having 2 to 8 carbon atoms, where the hydrocarbon groups represented by R¹⁰¹, R¹⁰², and R¹⁰³ may be substituted with one or more substituents selected from -OH, -COOH, -NR¹⁵¹R¹⁵², -OC(O)O-R¹⁵³, -C(O)O-R¹⁵⁴, -OC(O)-R¹⁵⁵, and -O-R¹⁵⁶, and R¹⁰² and R¹⁰³ may be linked to each other to form a 4- to 7-membered ring,
   R¹⁰⁴ represents a hydrocarbon group having 1 to 8 carbon atoms,
   R¹⁰⁵ and R¹⁰⁶ each independently represent a hydrocarbon group having 1 to 8 carbon atoms or -R¹⁰⁸-L¹⁰¹-R¹⁰⁹, excluding a case where both R¹⁰⁵ and R¹⁰⁶ are hydrocarbon groups having 1 to 8 carbon atoms,
   R¹⁰⁷ represents -R¹¹⁰-L¹⁰²-R¹¹¹-L¹⁰³-R¹¹²,
   R¹⁵¹ and R¹⁵² each independently represent a hydrocarbon group having 1 to 8 carbon atoms,
   R¹⁵³, R¹⁵⁴, R¹⁵⁵, and R¹⁵⁶ each independently represent a hydrocarbon group having 1 to 24 carbon atoms,
   the hydrocarbon groups represented by R¹⁵³, R¹⁵⁴, R¹⁵⁵, and R¹⁵⁶ may be substituted with an aryl group having 6 to 20 carbon atoms or -S-R¹⁵⁸,
   the aryl group having 6 to 20 carbon atoms may be substituted with -OH, -COOH, -NR¹⁵¹R¹⁵², -OC(O)O-R¹⁵³, -C(O)O-R¹⁵⁴, -OC(O)-R¹⁵⁵, -O-R¹⁵⁶, or -(hydrocarbon group having 1 to 12 carbon atoms)-R¹⁵⁷,
   R¹⁵⁸ represents a hydrocarbon group having 1 to 12 carbon atoms,
   R¹⁵⁷ represents -OH, -COOH, -NR¹⁶¹R¹⁶², -OC(O)O-R¹⁶³, -C(O)O-R¹⁶⁴, -OC(O)-R¹⁶⁵, or -O-R¹⁶⁶,
   R¹⁶¹ and R¹⁶² each independently represent a hydrocarbon group having 1 to 8 carbon atoms,
   R¹⁶³, R¹⁶⁴, R¹⁶⁵, and R¹⁶⁶ each independently represent a hydrocarbon group having 1 to 24 carbon atoms,
   the hydrocarbon groups represented by R¹⁶³, R¹⁶⁴, R¹⁶⁵, and R¹⁶⁶ may be substituted with an aryl group having 6 to 20 carbon atoms or -S-R¹⁶⁸,
   the aryl group having 6 to 20 carbon atoms may be substituted with -OH, -COOH, -NR¹⁶¹R¹⁶², -OC(O)O-R¹⁶³, -C(O)O-R¹⁶⁴, -OC(O)-R¹⁶⁵, -O-R¹⁶⁶, or a hydrocarbon group having 1 to 12 carbon atoms,
   R¹⁶⁸ represents a hydrocarbon group having 1 to 12 carbon atoms,
   L¹⁰¹, L¹⁰², and L¹⁰³ each independently represent -OC(O)O-, -C(O)O-, -OC(O)-, or -O-,
   R¹⁰⁸ represents a hydrocarbon group having 1 to 12 carbon atoms,
   R¹⁰⁹ represents a hydrocarbon group having 1 to 24 carbon atoms,
   R¹¹⁰ represents a hydrocarbon group having 1 to 8 carbon atoms,
   R¹¹¹ represents a hydrocarbon group having 1 to 24 carbon atoms,
   R¹¹² represents a hydrocarbon group having 1 to 24 carbon atoms,
   the hydrocarbon groups represented by R¹⁰⁹ and R¹¹² may be substituted with an aryl group, -OC(O)O-R¹⁵³, -C(O)O-R¹⁵⁴, -OC(O)-R¹⁵⁵, or -S-R¹⁵⁸, where definitions of R⁵³, R⁵⁴, R⁵⁵, and R⁵⁸ are as described above, and
   the hydrocarbon group represented by R¹¹¹ may be substituted with -OC(O)O-R¹⁵³, -C(O)O-R¹⁵⁴, or -OC(O)-R¹⁵⁵, where the definitions of R¹⁵³, R¹⁵⁴, and R¹⁵⁵ are as described above.

<23> A method for delivering nucleic acid to a cell using the kit according <22>, the method comprising: the following steps (1) to (4),
(1) a step of mixing the reagent (A) with a nucleic acid,
(2) a step of adjusting a pH of the mixture obtained in the step (1) with the reagent (B),
(3) a step of adding the reagent (C) to a culture medium containing a cell, and
(4) a step of adding the mixture obtained in the step (2) to the culture medium obtained in the step (3).

<24> The method for delivering a nucleic acid to a cell according to <23>, in which the cell is an immune cell.

<25> At least one compound selected from the group consisting of the following compounds, or a salt thereof, 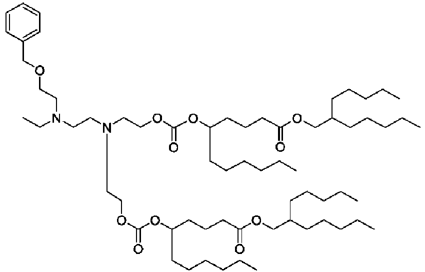 bis(2-pentylheptyl) 11-(2-((2-(benzyloxy)ethyl)(ethyl)amino)ethyl)-5,17-dihexyl-7,15-dioxo-6,8,14,16-tetraoxa-11 -azahenicosanedioate, 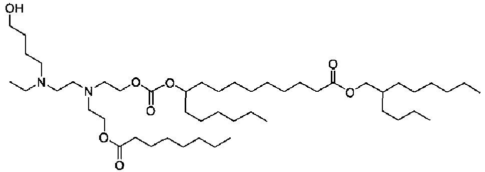 2-butyloctyl 5-ethyl-14-hexyl-1-hydroxy-8-(2-(octanoyloxy)ethyl)-12-oxo-11,13-dioxa-5,8-diazatricosan-2 3-oate, 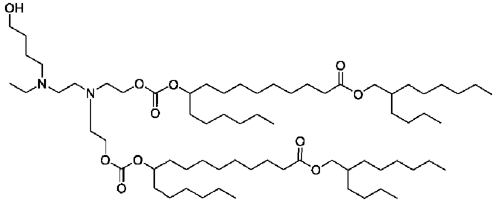 bis(2-butyloctyl) 16-(2-(ethyl(4-hydroxybutyl)amino)ethyl)-10,22-dihexyl-12,20-dioxo-11,13,19,21-tetraoxa-16 -azahentriacontanedioate, 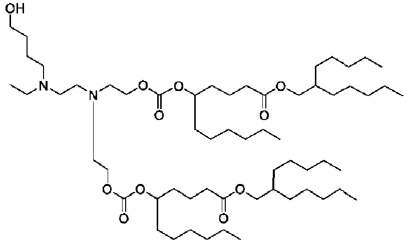 bis(2-pentylheptyl) 11-(2-(ethyl(4-hydroxybutyl)amino)ethyl)-5,17-dihexyl-7,15-dioxo-6,8,14,16-tetraoxa-11-aza henicosanedioate, 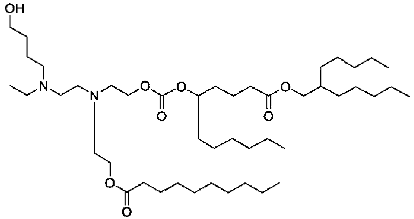 2-pentylheptyl 8-(2-(decanoyloxy)ethyl)-5-ethyl-14-hexyl-1-hydroxy-12-oxo-11,13-dioxa-5,8-diazaoctadecan -18-oate, 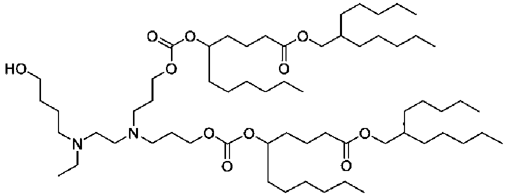 bis(2-pentylheptyl) 12-(2-(ethyl(4-hydroxybutyl)amino)ethyl)-5,19-dihexyl-7,17-dioxo-6,8,16,18-tetraoxa-12-azat ricosanedioate, 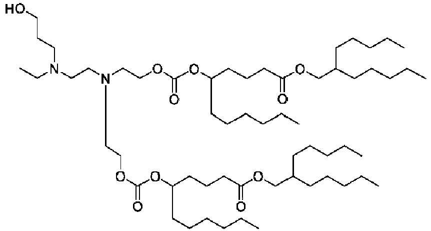 bis(2-pentylheptyl) 11-(2-(ethyl(3-hydroxypropyl)amino)ethyl)-5,17-dihexyl-7,15-dioxo-6,8,14,16-tetraoxa-11-az ahenicosanedioate, 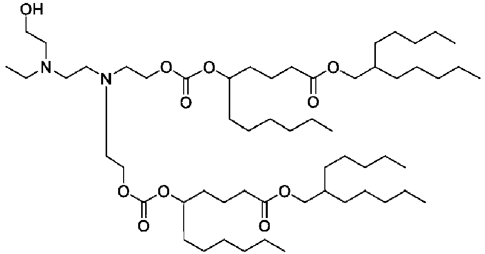 bis(2-pentylheptyl) 11-(2-(ethyl(2-hydroxyethyl)amino)ethyl)-5,17-dihexyl-7,15-dioxo-6,8,14,16-tetraoxa-11-azah enicosanedioate, 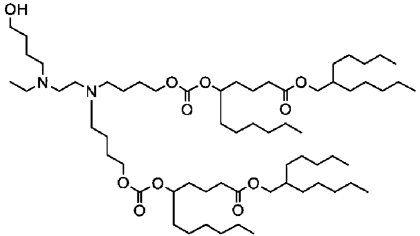 bis(2-pentylheptyl) 13-(2-(ethyl(4-hydroxybutyl)amino)ethyl)-5,21-dihexyl-7,19-dioxo-6,8,18,20-tetraoxa-13-aza pentacosanedioate, 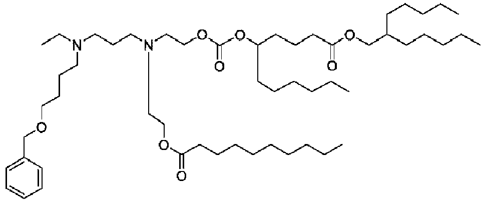 2-pentylheptyl 11-(2-(decanoyloxy)ethyl)-7-ethyl-17-hexyl-15-oxo-1-phenyl-2,14,16-trioxa-7,11-diazahenico san-21-oate, 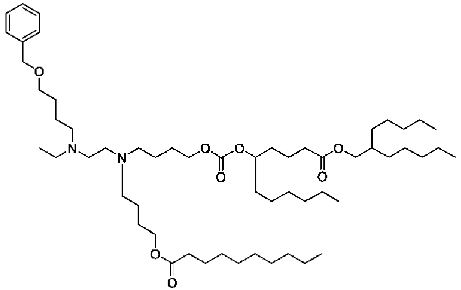 2-pentylheptyl 10-(4-(decanoyloxy)butyl)-7-ethyl-18-hexyl-16-oxo-1-phenyl-2,15,17-trioxa-7,10-diazadocos an-22-oate, 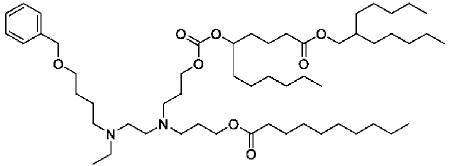 2-pentylheptyl 10-(3-(decanoyloxy)propyl)-7-ethyl-17-hexyl-15-oxo-1-phenyl-2,14,16-trioxa-7,10-diazahenic osan-21-oate, and 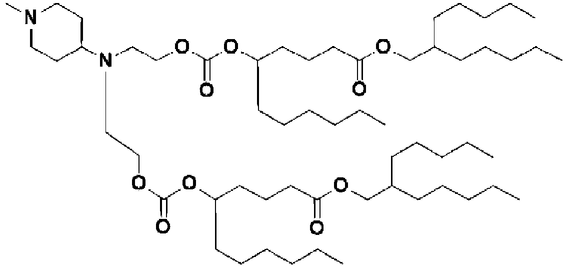 bis(2-pentylheptyl) 5,17-dihexyl-11-(1-methylpiperidin-4-yl)-7,15-dioxo-6,8,14,16-tetraoxa-11-azahenicosanedioa te.

### Effect of the invention

According to the present invention, it is possible to deliver nucleic acid to both an immune cell subjected to activation treatment and an immune cell not subjected to activation treatment. In addition, according to the present invention, it is possible to easily encapsulate nucleic acid in lipid particles not containing nucleic acid without using a dedicated device.

### BRIEF DESCRIPTION OF THE DRAWINGS

[FIG. 1] FIG. 1 shows the frequency (%) of TCR-negative cells among T cells.
[FIG. 2] FIG. 2 shows cell viability.
[FIG. 3] FIG. 3 shows a cell proliferation rate.
[FIG. 4] FIG. 4 shows the frequency (%) of TCR-negative cells among T cells.
[FIG. 5] FIG. 5 shows the frequency (%) of TCR-negative cells among T cells.
[FIG. 6] FIG. 6 shows cell viability.
[FIG. 7] FIG. 7 shows the frequency (%) of TCR-negative cells among T cells.
[FIG. 8] FIG. 8 shows cell viability.
[FIG. 9] FIG. 9 shows a GFP-positive cell ratio of T cells.
[FIG. 10] FIG. 10 shows a GFP-positive cell ratio of T cells.
[FIG. 11] FIG. 11 shows the frequency (%) of TCR-negative cells among T cells.
[FIG. 12] FIG. 12 shows the frequency (%) of TCR-negative cells among T cells.
[FIG. 13] FIG. 13 shows the frequency (%) of TCR-negative cells among T cells.
[FIG. 14] FIG. 14 shows a relationship between a cholesterol ratio and TCR KO efficiency.
[FIG. 15] FIG. 15 shows a GFP-positive cell ratio of T cells.
[FIG. 16] FIG. 16 shows the frequency (%) of TCR-negative cells among T cells.
[FIG. 17] FIG. 17 shows the frequency (%) of TCR-negative cells among T cells.
[FIG. 18] FIG. 18 shows the frequency (%) of B2M-negative cells among T cells.
[FIG. 19] FIG. 19 shows the frequency (%) of TCR-negative cells and B2M-negative cells among T cells.
[FIG. 20] FIG. 20 shows the frequency (%) of TCR-negative cells among T cells.
[FIG. 21] FIG. 21 shows the frequency (%) of TCR-negative cells and B2M-negative cells among T cells.
[FIG. 22] FIG. 22 shows translocation frequencies at a B2M cleavage region and a TCR cleavage region in T cells.
[FIG. 23] FIG. 23 shows a GFP-positive cell ratio of T cells.
[FIG. 24] FIG. 24 shows a GFP-positive cell ratio of NK cells.
[FIG. 25] FIG. 25 shows a GFP-positive cell ratio of NKT cells.
[FIG. 26] FIG. 26 shows a GFP-positive cell ratio of B cells.
[FIG. 27] FIG. 27 shows a GFP-positive cell ratio of THP-1 cells.
[FIG. 28] FIG. 28 shows fluorescence microscopic images (bright field and GFP fluorescence) of THP-1 cells.
[FIG. 29] FIG. 29 shows a GFP-positive cell ratio of BM-MSCs.
[FIG. 30] FIG. 30 shows fluorescence microscopic images (bright field and GFP fluorescence) of BM-MSCs.
[FIG. 31] FIG. 31 shows a GFP-positive cell ratio of iPS cell-derived nerve cells.
[FIG. 32] FIG. 32 shows fluorescence microscopic images (bright field and GFP fluorescence) of iPS cell-derived nerve cells.
[FIG. 33] FIG. 33 shows a GFP-positive cell ratio of T cells.

### Embodiments for carrying out the invention

Hereinafter, the present invention will be described in detail.

In the present specification, "to" shows a range including numerical values described before and after "to" as a minimum value and a maximum value, respectively.

According to the present invention, there is provided a method for delivering nucleic acid to an immune cell, the method comprising:
a step A of preparing lipid particles not containing nucleic acid using an ionizable lipid, a non-ionizable lipid, and a lipid having a nonionic polymer;
a step B of preparing nucleic acid-containing lipid particles by mixing the lipid particles not containing nucleic acid with nucleic acid; and
a step C of bringing the nucleic acid-containing lipid particles into contact with an immune cell. However, a delivery method in vivo may be excluded.

According to the present invention, there is further provided a nucleic acid delivery agent for an immune cell, the nucleic acid delivery agent including a lipid composition containing an ionizable lipid which is a compound represented by Formula (1) or Formula (2) or a salt thereof, a non-ionized lipid, a lipid having a nonionic polymer, and a nucleic acid.

According to the present invention, there is further provided a nucleic acid delivery agent for an immune cell, the nucleic acid delivery agent including a lipid composition in a frozen state, the lipid composition containing an ionizable lipid which is a compound represented by Formula (1) or Formula (2) or a salt thereof, a non-ionized lipid, and a lipid having a nonionic polymer.

The present invention can be used for producing an immune cell in which gene expression is modified, and provides a lipid composition that efficiently delivers a nucleic acid to an immune cell and a method for easily encapsulating a nucleic acid in lipid particles, which leads to an increase in general-purpose properties and a reduction in cost of producing an immune cell in which gene expression is modified.

### <Compound represented by Formula (1) or salt thereof>

The ionizable lipid is preferably a compound represented by Formula (1) or a salt thereof.

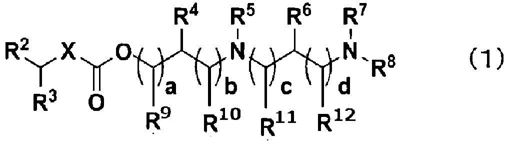

In the formula, X represents -NR¹- or -O-,
R¹ represents a hydrogen atom, a hydrocarbon group having 6 to 24 carbon atoms, or a group represented by R²¹-L¹-R²²-, where R²¹ represents a hydrocarbon group having 1 to 24 carbon atoms, L¹ represents -O(CO)O-, -O(CO)-, -(CO)O-, -O-, or 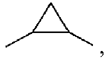 and R²² represents a divalent hydrocarbon linking group having 1 to 18 carbon atoms,
R² and R³ each independently represent a hydrogen atom, a hydrocarbon group having 3 to 24 carbon atoms, or a group represented by R³¹-L²-R³²-, where R³¹ represents a hydrocarbon group having 1 to 24 carbon atoms, L² represents -O(CO)O-, -O(CO)-, -(CO)O-, -O-, or 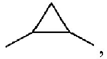 and R³² represents a divalent hydrocarbon linking group having 1 to 18 carbon atoms,
R⁴, R⁵, R⁶, R⁷, R⁸, R⁹, R¹⁰, R¹¹, and R¹² each independently represent a hydrogen atom or an alkyl group having 1 to 18 carbon atoms which may be substituted,
any one or more of the pairs of R⁴ and R⁵, R¹⁰ and R⁵, R⁵ and R¹², R⁴ and R⁶, R⁵ and R⁶, R⁶ and R⁷, R⁶ and R¹⁰, R¹² and R⁷, and R⁷ and R⁸ may be linked to each other to form a 4-to 7-membered ring which may contain an O atom,
a substituent on the alkyl group having 1 to 18 carbon atoms which may be substituted is a hydroxyl group, a carboxyl group, an amino group represented by -NR⁴⁵R⁴⁶, a substituted or unsubstituted aryl group, a substituted or unsubstituted heteroaryl group, or a group represented by -O(CO)O-R⁴¹, -O(CO)-R⁴², -(CO)O-R⁴³, or -O-R⁴⁴, where R⁴¹, R⁴², R⁴³, R⁴⁴, R⁴⁵, and R⁴⁶ each independently represent a hydrocarbon group having 1 to 18 carbon atoms,
a substituent on the substituted or unsubstituted aryl group and on the substituted or unsubstituted heteroaryl group is an alkyl group having 1 to 18 carbon atoms, a hydroxyl group, a carboxyl group, an amino group represented by -NR⁴⁵R⁴⁶, or a group represented by -O(CO)O-R⁴¹, -O(CO)-R⁴², -(CO)O-R⁴³, or -O-R⁴⁴, where R⁴¹, R⁴², R⁴³, R⁴⁴, R⁴⁵, and R⁴⁶ each independently represent a hydrocarbon group having 1 to 18 carbon atoms, and
a, b, c, and d each independently represent an integer of 0 to 3, provided that a + b is 1 or more, and c + d is 1 or more.

As the hydrocarbon group having 6 to 24 carbon atoms that is represented by R¹ and the hydrocarbon group having 3 to 24 carbon atoms that is represented by R² and R³, an alkyl group, an alkenyl group, or an alkynyl group is preferable, and an alkyl group or an alkenyl group is more preferable. The alkyl group having 6 to 24 carbon atoms and the alkyl group having 3 to 24 carbon atoms may be linear or branched or may be chain-like or cyclic. The alkyl group having 6 to 24 carbon atoms is preferably an alkyl group having 6 to 20 carbon atoms, and the alkyl group having 3 to 24 carbon atoms is more preferably an alkyl group having 6 to 20 carbon atoms. Specifically, examples thereof include a hexyl group, a heptyl group, an octyl group, a nonyl group, a decyl group, an undecyl group, a dodecyl group, a tridecyl group, a trimethyldodecyl group (preferably a 3,7,11-trimethyldodecyl group), a tetradecyl group, a pentadecyl group, a hexadecyl group, a tetramethylhexadecyl group (preferably a 3,7,11,15-tetramethylhexadecyl group), a heptadecyl group, an octadecyl group, a nonadecyl group, and an icosyl group. The alkenyl group having 6 to 24 carbon atoms and the alkenyl group having 3 to 24 carbon atoms may be linear or branched or may be chain-like or cyclic. The alkenyl group having 6 to 24 carbon atoms is preferably an alkenyl group having 6 to 20 carbon atoms, and the alkenyl group having 3 to 24 carbon atoms is more preferably an alkenyl group having 6 to 20 carbon atoms. Specifically, examples thereof include a hexenyl group, a heptenyl group, an octenyl group, a nonenyl group, a decenyl group, an undecenyl group, a dodecenyl group, a dodecadienyl group, a tridecenyl group, a tetradecenyl group, a pentadecenyl group, a hexadecenyl group (preferably a (Z)-hexadec-9-enyl group), a hexadecadienyl group, a heptadecenyl group (preferably a (Z)-heptadec-8-enyl group), a heptadecadienyl group (preferably an (8Z,11Z)-heptadeca-8,11-dienyl group), an octadecenyl group (preferably a (Z)-octadec-9-enyl group), an octadecadienyl group (preferably a (9Z,12Z)-octadeca-9,12-dienyl group), a nonadecenyl group, an icosenyl group (preferably a (Z)-icos-11-enyl group), and an icosadienyl group (preferably an (11Z,14Z)-icosa-11,14-dienyl group). The alkynyl group having 6 to 24 carbon atoms is preferably an alkynyl group having 6 to 20 carbon atoms, and the alkynyl group having 3 to 24 carbon atoms is more preferably an alkynyl group having 6 to 20 carbon atoms. Specifically, examples thereof include a hexynyl group, a heptynyl group, an octynyl group, a nonynyl group, a decynyl group, an undecynyl group, a dodecynyl group, a tetradecynyl group, a pentadecynyl group, a hexadecynyl group, a heptadecynyl group, and an octadecynyl group. All of the above alkenyl groups preferably have one double bond or two double bonds. All of the above alkynyl groups preferably have one triple bond or two triple bonds.

As hydrocarbon group having 1 to 24 carbon atoms that is represented by R²¹ and R³¹, an alkyl group having 10 to 24 carbon atoms, an alkenyl group having 10 to 24 carbon atoms, or an alkynyl group having 10 to 24 carbon atoms is preferable. The alkyl group having 10 to 24 carbon atoms may be linear or branched or may be chain-like or cyclic. The alkyl group having 10 to 24 carbon atoms is preferably an alkyl group having 12 to 24 carbon atoms. Specifically, examples thereof include a decyl group, an undecyl group, a dodecyl group, a tridecyl group, a trimethyldodecyl group (preferably a 3,7,11-trimethyldodecyl group), a tetradecyl group, a pentadecyl group, a hexadecyl group, a tetramethylhexadecyl group (preferably a 3,7,11,15-tetramethylhexadecyl group), a heptadecyl group, an octadecyl group, a 2-butylhexyl group, a 2-butyloctyl group, a 1-pentylhexyl group, a 2-pentylheptyl group, a 3-pentyloctyl group, a 1-hexylheptyl group, a 1-hexylnonyl group, a 2-hexyloctyl group, a 2-hexyldecyl group, a 3-hexylnonyl group, a 1-heptyloctyl group, a 2-heptylnonyl group, a 2-heptylundecyl group, a 3-heptyldecyl group, a 1-octylnonyl group, a 2-octyldecyl group, a 2-octyldodecyl group, a 3-octylundecyl group, a 2-nonylundecyl group, a 3-nonyldodecyl group, a 2-decyldodecyl group, a 2-decyltetradecyl group, a 3-decyltridecyl group, and a 2-(4,4-dimethylpentan-2-yl)-5,7,7-trimethyloctyl group. The alkenyl group having 10 to 24 carbon atoms may be linear or branched or may be chain-like or cyclic. Specifically, examples thereof include a decenyl group, an undecenyl group, a dodecenyl group, a dodecadienyl group, a tridecenyl group (preferably a (Z)-tridec-8-enyl group), a tetradecenyl group (preferably a tetradec-9-enyl group), a pentadecenyl group (preferably a (Z)-pentadec-8-enyl group), a hexadecenyl group (preferably a (Z)-hexadec-9-enyl group), a hexadecadienyl group, a heptadecenyl group (preferably a (Z)-heptadec-8-enyl group), a heptadecadienyl group (preferably a (8Z,11Z)-heptadeca-8,11-dienyl group), an octadecenyl group (preferably a (Z)-octadec-9-enyl group), and an octadecadienyl group (preferably a (9Z,12Z)-octadeca-9,12-dienyl group). The alkynyl group having 10 to 24 carbon atoms may be linear or branched or may be chain-like or cyclic. Specifically, examples thereof include a decynyl group, an undecynyl group, a dodecynyl group, a tetradecynyl group, a pentadecynyl group, a hexadecynyl group, a heptadecynyl group, and an octadecynyl group. All of the above alkenyl groups preferably have one double bond or two double bonds. All of the above alkynyl groups preferably have one triple bond or two triple bonds.

As the divalent hydrocarbon linking group having 1 to 18 carbon atoms that is represented by R²² and R³², an alkylene group having 1 to 18 carbon atoms or an alkenylene group having 2 to 18 carbon atoms is preferable. The alkylene group having 1 to 18 carbon atoms may be linear or branched or may be chain-like or cyclic. The number of carbon atoms in the alkylene group is preferably 1 to 12, more preferably 1 to 10, and even more preferably 2 to 10. Specifically, examples thereof include a methylene group, an ethylene group, a trimethylene group, a tetramethylene group, a pentamethylene group, a hexamethylene group, a heptamethylene group, an octamethylene group, a nonamethylene group, a decamethylene group, an undecamethylene group, and a dodecamethylene group. The alkenylene group having 2 to 18 carbon atoms may be linear or branched or may be chain-like or cyclic. The number of carbon atoms in the alkenylene group is preferably 1 to 12, and more preferably 2 to 10.

As a preferred range of L¹, -O(CO)O-, -O(CO)-, or -(CO)O- is preferable and -O(CO)-or -(CO)O- is more preferable.

As a preferred range of L², -O(CO)O-, -O(CO)-, or -(CO)O- is preferable and -O(CO)- or -(CO)O- is more preferable.

The alkyl group having 1 to 18 carbon atoms which may be substituted and which is represented by R⁴, R⁶, R⁹, R¹⁰, R¹¹, and R¹² may be linear or branched or may be chain-like or cyclic. The number of carbon atoms in the alkyl group having 1 to 18 carbon atoms is preferably 1 to 12. Specifically, examples thereof include a methyl group, an ethyl group, a propyl group, an isopropyl group, a cyclopropyl group, a butyl group, an isobutyl group, a tert-butyl group, a cyclobutyl group, a pentyl group, a cyclopentyl group, a hexyl group, a cyclohexyl group, a heptyl group, an octyl group, a nonyl group, a decyl group, an undecyl group, and a dodecyl group. In a case where the alkyl group has a substituent, as the substituent, a hydroxyl group, a carboxyl group, or a group represented by -O(CO)O-R⁴¹, -O(CO)-R⁴², -(CO)O-R⁴³, or -O-R⁴⁴ is preferable, and a group represented by -O(CO)-R⁴² or -(CO)O-R⁴³ is more preferable.

The alkyl group having 1 to 18 carbon atoms which may be substituted and which is represented by R⁵, R⁷, and R⁸ may be linear or branched or may be chain-like or cyclic. The number of carbon atoms in the alkyl group is preferably 1 to 12, and more preferably 1 to 8. Specifically, examples thereof include a methyl group, an ethyl group, a propyl group, an isopropyl group, a cyclopropyl group, a butyl group, an isobutyl group, a tert-butyl group, a cyclobutyl group, a pentyl group, a cyclopentyl group, a hexyl group, a cyclohexyl group, a heptyl group, an octyl group, a nonyl group, a decyl group, an undecyl group, and a dodecyl group. In a case where the alkyl group has a substituent, as the substituent, a hydroxyl group, a carboxyl group, or a group represented by -O(CO)O-R⁴¹, -O(CO)-R⁴², -(CO)O-R⁴³, or -O-R⁴⁴ is preferable, and a group represented by -O(CO)-R⁴² or -(CO)O-R⁴³ is more preferable.

Examples of the 4- to 7-membered ring which may contain an O atom include an azetidine ring, a pyrrolidine ring, a piperidine ring, a morpholine ring, and an azepane ring. The 4- to 7-membered ring is preferably a 6-membered ring and is preferably a piperidine ring or a morpholine ring.

In a case where the alkyl group which is represented by R⁴, R⁵, R⁶, R⁷, R⁸, R⁹, R¹⁰, R¹¹, and R¹² and has 1 to 18 carbon atoms that may be substituted has a substituted or unsubstituted aryl group as a substituent, the number of carbon atoms in the aryl group is preferably 6 to 22, more preferably 6 to 18, and even more preferably 6 to 10. Specifically, examples of the aryl group include a phenyl group, a naphthyl group, an anthracenyl group, and a phenanthrenyl group. As the substituent on the aryl group, an alkyl group having 1 to 18 carbon atoms, a hydroxyl group, a carboxyl group, an amino group represented by -NR⁴⁵R⁴⁶, or a group represented by -O(CO)O-R⁴¹, -O(CO)-R⁴², -(CO)O-R⁴³, or -O-R⁴⁴ is preferable, and a hydroxyl group or a carboxyl group is more preferable. Specifically, examples of the substituted aryl group include a hydroxyphenyl group, and a carboxyphenyl group.

In a case where the alkyl group which is represented by R⁴, R⁵, R⁶, R⁷, R⁸, R⁹, R¹⁰, R¹¹, and R¹² and has 1 to 18 carbon atoms that may be substituted has a substituted or unsubstituted heteroaryl group as a substituent, the number of carbon atoms in the heteroaryl group is preferably 1 to 12, and more preferably 1 to 6. Specifically, examples of the heteroaryl group include a pyridyl group, a pyrazolyl group, an imidazolyl group, a benzimidazolyl group, a thiazolyl group, and an oxazolyl group. As the substituent on the heteroaryl group, an alkyl group having 1 to 18 carbon atoms, a hydroxyl group, a carboxyl group, an amino group represented by -NR⁴⁵R⁴⁶, or a group represented by -O(CO)O-R⁴¹, -O(CO)-R⁴², -(CO)O-R⁴³, or -O-R⁴⁴ is preferable, and a hydroxyl group or a carboxyl group is more preferable. Specifically, examples of the substituted or unsubstituted heteroaryl group include a hydroxypyridyl group, a carboxypyridyl group, and a pyridonyl group.

As hydrocarbon group having 1 to 18 carbon atoms that is represented by R⁴¹, R⁴², R⁴³, R⁴⁴, R⁴⁵, and R⁴⁶, an alkyl group having 1 to 18 carbon atoms, an alkenyl group having 2 to 18 carbon atoms, or an alkynyl group having 2 to 18 carbon atoms is preferable, and an alkyl group having 1 to 18 carbon atoms or an alkenyl group having 2 to 18 carbon atoms is more preferable. The alkyl group having 1 to 18 carbon atoms may be linear or branched or may be chain-like or cyclic. The number of carbon atoms in the alkyl group having 1 to 18 carbon atoms is preferably 3 to 18, and more preferably 5 to 18. Specifically, examples thereof include a propyl group, an isopropyl group, a cyclopropyl group, a butyl group, an isobutyl group, a tert-butyl group, a cyclobutyl group, a pentyl group, a cyclopentyl group, a hexyl group, a cyclohexyl group, a heptyl group, an octyl group, a nonyl group, a decyl group, an undecyl group, a dodecyl group, a tridecyl group, a trimethyldodecyl group (preferably a 3,7,11-trimethyldodecyl group), a tetradecyl group, a pentadecyl group, a hexadecyl group, a heptadecyl group, an octadecyl group, and a benzyl group. The alkenyl group having 2 to 18 carbon atoms may be linear or branched or may be chain-like or cyclic. The number of carbon atoms in the alkenyl group having 2 to 18 carbon atoms is preferably 3 to 18, and more preferably 5 to 18. Specifically, examples thereof include an allyl group, a prenyl group, a pentenyl group, a hexenyl group, a heptenyl group, an octenyl group, a nonenyl group (preferably a (Z)-2-nonenyl group or an (E)-2-nonenyl group), a decenyl group, an undecenyl group, a dodecenyl group, a dodecadienyl group, a tridecenyl group (preferably a (Z)-tridec-8-enyl group), a tetradecenyl group (preferably a tetradec-9-enyl group), a pentadecenyl group (preferably a (Z)-pentadec-8-enyl group), a hexadecenyl group (preferably a (Z)-hexadec-9-enyl group), a hexadecadienyl group, a heptadecenyl group (preferably a (Z)-heptadec-8-enyl group), a heptadecadienyl group (preferably a (8Z,11Z)-heptadeca-8,11-dienyl group), an octadecenyl group (preferably a (Z)-octadec-9-enyl group), and an octadecadienyl group (preferably a (9Z,12Z)-octadeca-9,12-dienyl group). The alkynyl group having 2 to 18 carbon atoms may be linear or branched or may be chain-like or cyclic. The number of carbon atoms in the alkynyl group is preferably 3 to 18, and more preferably 5 to 18. Specifically, examples thereof include a propargyl group, a butynyl group, a pentynyl group, a hexynyl group, a heptynyl group, an octynyl group, a nonynyl group, a decynyl group, an undecynyl group, a dodecynyl group, a tetradecynyl group, a pentadecynyl group, a hexadecynyl group, a heptadecynyl group, and an octadecynyl group.

In a case where X represents -NR¹-, R¹ preferably represents a hydrocarbon group having 6 to 24 carbon atoms or a group represented by R²¹-L¹-R²²-. In this case, it is preferable that one of R² and R³ represents a hydrogen atom and the other represents a hydrocarbon group having 6 to 24 carbon atoms or a group represented by R³¹-L²-R³²-.

In a case where X represents -O-, it is preferable that R² and R³ each independently represent a hydrocarbon group having 6 to 24 carbon atoms or a group represented by R³¹-L²-R³²-.

R⁴, R⁶, R⁹, R¹⁰, R¹¹, and R¹² are preferably a hydrogen atom.

It is preferable that R⁵ is a hydrogen atom, an alkyl group having 1 to 18 carbon atoms, an alkyl group having 1 to 18 carbon atoms which may be substituted with -O(CO)-R⁴² or -(CO)O-R⁴³, an alkyl group having 1 to 18 carbon atoms which may be substituted with an aryl group, or an alkyl group having 1 to 18 carbon atoms which may be substituted with a hydroxyl group. In a case where R⁵ is an alkyl group, R⁵ may be linked to R⁴, R⁶, R¹⁰, and R¹² to form a ring which may contain an O atom. Particularly, it is preferable that R⁵ is an alkyl group having 1 to 18 carbon atoms, an alkyl group having 1 to 18 carbon atoms which may be substituted with -O(CO)-R⁴² or -(CO)O-R⁴³, an alkyl group having 1 to 12 carbon atoms which may be substituted with an aryl group, or an alkyl group having 1 to 8 carbon atoms which may be substituted with a hydroxyl group, and more preferable that R⁵ is an alkyl group having 1 to 18 carbon atoms or an alkyl group having 1 to 18 carbon atoms which may be substituted with -O(CO)-R⁴² or -(CO)O-R⁴³.

It is preferable that R⁷ and R⁸ are each independently a hydrogen atom, a hydrocarbon group having 1 to 18 carbon atoms, an alkyl group having 1 to 18 carbon atoms which may be substituted with -O(CO)-R⁴² or -(CO)O-R⁴³, an alkyl group having 1 to 8 carbon atoms which may be substituted with an aryl group, or an alkyl group having 1 to 8 carbon atoms which may be substituted with a hydroxyl group, or alternatively, R⁷ and R⁸ are linked to each other to form a 4- to 7-membered ring which may contain an O atom.

R⁵ is not linked to R⁷ or R⁸ and does not form a ring with R⁷ or R⁸.

a + b is preferably 1 or 2 and more preferably 1, and c + d is preferably 1 or 2 and more preferably 1.

The compound represented by Formula (1) is preferably a compound represented by Formula (1-1).

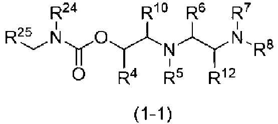

R²⁴ represents a hydrogen atom, a hydrocarbon group having 6 to 24 carbon atoms, or a group represented by R²¹-L¹-R²²-, where R²¹ represents a hydrocarbon group having 1 to 24 carbon atoms, L¹ represents -O(CO)O-, -O(CO)-, -(CO)O-, -O-, or 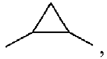 and R²² represents a divalent hydrocarbon linking group having 1 to 18 carbon atoms.
R²⁵ represents a hydrogen atom, a hydrocarbon group having 3 to 24 carbon atoms, or a group represented by R³¹-L²-R³²-, where R³¹ represents a hydrocarbon group having 1 to 24 carbon atoms, L² represents -O(CO)O-, -O(CO)-, -(CO)O-, -O-, or 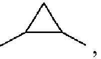 and R³² represents a divalent hydrocarbon linking group having 1 to 18 carbon atoms.
R⁴, R⁵, R⁶, R⁷, R⁸, R¹⁰, and R ¹² each independently represent a hydrogen atom or an alkyl group having 1 to 18 carbon atoms that may be substituted, and
any one or more of the pairs of R⁴ and R⁵, R¹⁰ and R⁵, R⁵ and R¹², R⁴ and R⁶, R⁵ and R⁶, R⁶ and R⁷, R⁶ and R¹⁰, R¹² and R⁷, and R⁷ and R⁸ may be linked to each other to form a 4-to 7-membered ring which may contain an O atom. However, it is preferable that R⁵ is not linked to R⁷ or R⁸ and does not form a ring with R⁷ or R⁸.

The substituent on the alkyl group having 1 to 18 carbon atoms which may be substituted is a hydroxyl group, a carboxyl group, an amino group represented by -NR⁴⁵R⁴⁶, a substituted or unsubstituted aryl group, a substituted or unsubstituted heteroaryl group, or a group represented by -O(CO)O-R⁴¹, -O(CO)-R⁴², -(CO)O-R⁴³, or -O-R⁴⁴, where R⁴¹, R⁴², R⁴³, R⁴⁴, R⁴⁵, and R⁴⁶ each independently represent a hydrocarbon group having 1 to 18 carbon atoms,
a substituent on the substituted or unsubstituted aryl group and on the substituted or unsubstituted heteroaryl group is an alkyl group having 1 to 18 carbon atoms, a hydroxyl group, a carboxyl group, an amino group represented by -NR⁴⁵R⁴⁶, or a group represented by -O(CO)O-R⁴¹, -O(CO)-R⁴², -(CO)O-R⁴³, or -O-R⁴⁴, where R⁴¹, R⁴², R⁴³, R⁴⁴, R⁴⁵, and R⁴⁶ each independently represent a hydrocarbon group having 1 to 18 carbon atoms.

The definitions and preferred ranges of R⁴, R⁵, R⁶, R⁷, R⁸, R¹⁰, and R¹² in Formula (1-1) are the same as those of R⁴, R⁵, R⁶, R⁷, R⁸, R¹⁰, and R¹² in Formula (1).

R²⁴ in Formula (1-1) is preferably an alkyl group or an alkenyl group having 6 to 24 carbon atoms. The alkyl group having 6 to 24 carbon atoms may be linear or branched or may be chainlike or cyclic. The alkyl group having 6 to 24 carbon atoms is preferably an alkyl group having 8 to 20 carbon atoms. Specifically, examples thereof include an octyl group, a nonyl group, a decyl group, an undecyl group, a dodecyl group, a tridecyl group, a trimethyldodecyl group (preferably a 3,7,11-trimethyldodecyl group), a tetradecyl group, a pentadecyl group, a hexadecyl group, a tetramethylhexadecyl group (preferably a 3,7,11,15-tetramethylhexadecyl group), a heptadecyl group, an octadecyl group, a nonadecyl group, an icosyl group, and the like. The alkenyl group having 6 to 24 carbon atoms may be linear or branched or may be chainlike or cyclic. The alkenyl group having 6 to 24 carbon atoms is preferably an alkenyl group having 8 to 20 carbon atoms. Specifically, examples thereof include an octenyl group, a nonenyl group, a decenyl group, an undecenyl group, a dodecenyl group, a dodecadienyl group, a tridecenyl group, a tetradecenyl group, a pentadecenyl group, a hexadecenyl group (preferably a (Z)-hexadec-9-enyl group), a hexadecadienyl group, a heptadecenyl group (preferably a (Z)-heptadec-8-enyl group), a heptadecadienyl group (preferably a (8Z,11Z)-heptadeca-8,11 -dienyl group), an octadecenyl group (preferably a (Z)-octadec-9-enyl group), an octadecadienyl group (preferably a (9Z,12Z)-octadeca-9,12-dienyl group), a nonadecenyl group, an icosenyl group (preferably a (Z)-icos-11-enyl group), an icosadienyl group (preferably a (11Z,14Z)-icosa-11,14-dienyl group), and the like.

It is preferable that all of the above alkenyl groups have one double bond or two double bonds.

R²⁵ in Formula (1-1) is preferably an alkyl group or an alkenyl group having 6 to 24 carbon atoms. The alkyl group having 6 to 24 carbon atoms may be linear or branched or may be chainlike or cyclic. The alkyl group having 6 to 24 carbon atoms is preferably an alkyl group having 7 to 20 carbon atoms. Specifically, examples thereof include a hexyl group, a heptyl group, an octyl group, a nonyl group, a decyl group, an undecyl group, a dodecyl group, a tridecyl group, a trimethyldodecyl group (preferably a 3,7,11-trimethyldodecyl group), a tetradecyl group, a pentadecyl group, a hexadecyl group, a tetramethylhexadecyl group (preferably a 3,7,11,15-tetramethylhexadecyl group), a heptadecyl group, an octadecyl group, and the like. The alkenyl group having 6 to 24 carbon atoms may be linear or branched or may be chainlike or cyclic. The alkenyl group having 6 to 24 carbon atoms is preferably an alkenyl group having 8 to 20 carbon atoms. Specifically, examples thereof include an octenyl group, a nonenyl group, a decenyl group, an undecenyl group, a dodecenyl group, a dodecadienyl group, a tridecenyl group, a tetradecenyl group, a pentadecenyl group, a hexadecenyl group (preferably a (Z)-hexadec-9-enyl group), a hexadecadienyl group, a heptadecenyl group (preferably a (Z)-heptadec-8-enyl group), a heptadecadienyl group (preferably a (8Z,11Z)-heptadeca-8,11 -dienyl group), an octadecenyl group (preferably a (Z)-octadec-9-enyl group), an octadecadienyl group (preferably a (9Z,12Z)-octadeca-9,12-dienyl group), a nonadecenyl group, an icosenyl group (preferably a (Z)-icos-11-enyl group), an icosadienyl group (preferably a (11Z,14Z)-icosa-11,14-dienyl group), and the like.

It is preferable that all of the above alkenyl groups have one double bond or two double bonds.

In a preferred embodiment,
X represents -O-;
R², R³, R³¹, L², and R³² have the same definitions as R², R³, R³¹, L², and R³² in Formula (1),
R⁴, R⁵, R⁶, R⁷, R⁸, R⁹, R¹⁰, R¹¹, and R¹² each independently represent a hydrogen atom or an alkyl group having 1 to 18 carbon atoms which may be substituted,
the substituent on the alkyl group having 1 to 18 carbon atoms that may be substituted and the substituent on the substituted or unsubstituted aryl group and on the substituted or unsubstituted heteroaryl group have the same definitions as those in Formula (1), a + b is 1, and c + d is 1 or 2.

In a more preferred embodiment, the compound represented by Formula (1) is a compound represented by Formula (1-2).

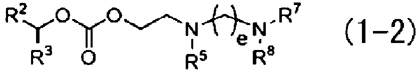

In the formula, R² and R³ each independently represent a hydrogen atom, a hydrocarbon group having 3 to 24 carbon atoms, or a group represented by R³¹-L²-R³²-,
R³¹ represents a hydrocarbon group having 1 to 24 carbon atoms,
L² represents -O(CO)O-, -O(CO)-, -(CO)O-, -O-, or 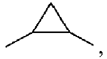
R³² represents a divalent hydrocarbon linking group having 1 to 18 carbon atoms,
R⁵ represents a hydrogen atom or an alkyl group having 1 to 18 carbon atoms which may be substituted,
R⁷ and R⁸ each independently represent a hydrogen atom or an alkyl group having 1 to 18 carbon atoms which may be substituted,
a substituent on the alkyl group having 1 to 18 carbon atoms which may be substituted is a hydroxyl group, a carboxyl group, an amino group represented by -NR⁴⁵R⁴⁶, a substituted or unsubstituted aryl group, a substituted or unsubstituted heteroaryl group, or a group represented by -O(CO)O-R⁴¹, -O(CO)-R⁴², -(CO)O-R⁴³, or -O-R⁴⁴, where R⁴¹, R⁴², R⁴³, R⁴⁴, R⁴⁵, and R⁴⁶ each independently represent a hydrocarbon group having 1 to 18 carbon atoms,
a substituent on the substituted or unsubstituted aryl group and on the substituted or unsubstituted heteroaryl group is an alkyl group having 1 to 18 carbon atoms, a hydroxyl group, a carboxyl group, an amino group represented by -NR⁴⁵R⁴⁶, or a group represented by -O(CO)O-R⁴¹, -O(CO)-R⁴², -(CO)O-R⁴³, or -O-R⁴⁴, where R⁴¹, R⁴², R⁴³, R⁴⁴, R⁴⁵, and R⁴⁶ each independently represent a hydrocarbon group having 1 to 18 carbon atoms, and
e represents 2 or 3.
R², R³, R⁵, R⁷, and R⁸ have the same definitions as R², R³, R⁵, R⁷, and R⁸ in Formula (1).

Formula (1-2) preferably represents a compound in which R⁷ and R⁸ each independently represent a hydrogen atom or an alkyl group having 1 to 18 carbon atoms, the substituent on the alkyl group which is represented by R⁵ and has 1 to 18 carbon atoms that may be substituted is a hydroxyl group, a substituted or unsubstituted aryl group, or a group represented by -O(CO)O-R⁴¹, -O(CO)-R⁴², -(CO)O-R⁴³, or -O-R⁴⁴, R⁴¹, R⁴², R⁴³, R⁴⁴, R⁴⁵, and R⁴⁶ each independently represent a hydrocarbon group having 1 to 18 carbon atoms, the substituent on the substituted or unsubstituted aryl group is an alkyl group having 1 to 18 carbon atoms, a hydroxyl group, a carboxyl group, an amino group represented by -NR⁴⁵R⁴⁶, or a group represented by -O(CO)O-R⁴¹, -O(CO)-R⁴², -(CO)O-R⁴³, or -O-R⁴⁴, and R⁴¹, R⁴², R⁴³, R⁴⁴, R⁴⁵, and R⁴⁶ each independently represent a hydrocarbon group having 1 to 18 carbon atoms.

Formula (1-2) more preferably represents a compound in which R² and R³ each independently represent a hydrocarbon group having 3 to 24 carbon atoms or a group represented by R³¹-L²-R³²-, L² represents -O(CO)- or -(CO)O-, R⁷ and R⁸ each independently represent a hydrogen atom or an alkyl group having 1 to 18 carbon atoms, the substituent on the alkyl group which is represented by R⁵ and has 1 to 18 carbon atoms that may be substituted is an unsubstituted aryl group, -O(CO)-R⁴², or -(CO)O-R⁴³, and R⁴² and R⁴³ each independently represent a hydrocarbon group having 1 to 18 carbon atoms.

Formula (1-2) even more preferably represents a compound in which R² and R³ each independently represent a hydrogen atom or a hydrocarbon group having 3 to 24 carbon atoms, R⁷ and R⁸ each independently represent a hydrogen atom or an alkyl group having 1 to 18 carbon atoms, the substituent on the alkyl group which is represented by R⁵ and has 1 to 18 carbon atoms that may be substituted is an unsubstituted aryl group or a group represented by -O(CO)-R⁴² or -(CO)O-R⁴³, and R⁴² and R⁴³ each independently represent a hydrocarbon group having 1 to 18 carbon atoms.

Formula (1-2) preferably represents a compound in which at least one of R² or R³ represents a group represented by R³¹-L²-R³²-, L² represents -O(CO)- or -(CO)O-, R⁷ and R⁸ each independently represent a hydrogen atom or an alkyl group having 1 to 18 carbon atoms, the substituent on the alkyl group which is represented by R⁵ and has 1 to 18 carbon atoms that may be substituted is an unsubstituted aryl group or a group represented by -O(CO)-R⁴² or -(CO)O-R⁴³, and R⁴² and R⁴³ each independently represent a hydrocarbon group having 1 to 18 carbon atoms.

Formula (1-2) more preferably represents a compound in which R² and R³ each independently represent a group represented by R³¹-L²-R³²-, L² represents -O(CO)- or -(CO)O-, R⁷ and R⁸ each independently represent a hydrogen atom or an alkyl group having 1 to 18 carbon atoms, the substituent on the alkyl group which is represented by R⁵ and has 1 to 18 carbon atoms that may be substituted is an unsubstituted aryl group or a group represented by -O(CO)-R⁴² or -(CO)O-R⁴³, and R⁴² and R⁴³ each independently represent a hydrocarbon group having 1 to 18 carbon atoms.

Formula (1-2) preferably represents a compound in which one of R² and R³ represents a group represented by R³¹-L²-R³²- and the other represents a hydrocarbon group having 3 to 24 carbon atoms,L² represents -O(CO)- or -(CO)O-, R⁷ and R⁸ each independently represent a hydrogen atom or an alkyl group having 1 to 18 carbon atoms, the substituent on the alkyl group which is represented by R⁵ and has 1 to 18 carbon atoms that may be substituted is an unsubstituted aryl group or a group represented by -O(CO)-R⁴² or -(CO)O-R⁴³, and R⁴² and R⁴³ each independently represent a hydrocarbon group having 1 to 18 carbon atoms.

Formula (1-2) more preferably represents a compound in which one of R² and R³ represents a group represented by R³¹-L²-R³²- and the other represents a hydrocarbon group having 6 carbon atoms, L² represents -O(CO)- or -(CO)O-, R⁷ and R⁸ each independently represent a hydrogen atom or an alkyl group having 1 to 18 carbon atoms, the substituent on the alkyl group which is represented by R⁵ and has 1 to 18 carbon atoms that may be substituted is a group represented by -O(CO)-R⁴² or -(CO)O-R⁴³, and R⁴² and R⁴³ each independently represent a hydrocarbon group having 1 to 18 carbon atoms.

Formula (1-2) even more preferably represents a compound in which one of R² and R³ represents a group represented by R³¹-L²-R³²- and the other represents a hydrocarbon group having 6 carbon atoms, L² represents -O(CO)- or -(CO)O-, R⁵ represents a hydrogen atom or an alkyl group having 1 to 18 carbon atoms, and R⁷ and R⁸ each independently represent a hydrogen atom or an alkyl group having 1 to 18 carbon atoms.

Formula (1-2) still more preferably represents a compound in which one of R² and R³ represents a group represented by R³¹-L²-R³²- and the other represents a hydrocarbon group having 6 carbon atoms, L² represents -O(CO)- or -(CO)O-, R⁵ represents a hydrogen atom or an alkyl group having 1 to 18 carbon atoms, R⁷ and R⁸ each independently represent a hydrogen atom or an alkyl group having 1 to 18 carbon atoms, and e represents 2.

Formula (1-2) yet more preferably represents a compound in which one of R² and R³ represents a group represented by R³¹-L²-R³²- and the other represents a hydrocarbon group having 3 to 5 carbon atoms, L² represents -O(CO)- or -(CO)O-, R⁵ represents a hydrogen atom or an alkyl group having 1 to 18 carbon atoms, and R⁷ and R⁸ each independently represent a hydrogen atom or an alkyl group having 1 to 18 carbon atoms.

Formula (1-2) more preferably represents a compound in which one of R² and R³ represents a group represented by R³¹-L²-R³²- and the other represents a hydrocarbon group having 3 to 5 carbon atoms, L² represents -O(CO)- or -(CO)O-, R⁵ represents a hydrogen atom or an alkyl group having 1 to 18 carbon atoms, R⁷ and R⁸ each independently represent a hydrogen atom or an alkyl group having 1 to 18 carbon atoms, and e represents 2.

Formula (1-2) still more preferably represents a compound in which one of R² and R³ represents a group represented by R³¹-L²-R³²- and the other represents a hydrocarbon group having 6 carbon atoms, L² represents -O(CO)- or -(CO)O-, R⁵ represents a hydrogen atom or a substituted alkyl group having 1 to 18 carbon atoms, R⁷ and R⁸ each independently represent a hydrogen atom or an alkyl group having 1 to 18 carbon atoms, the substituent on the substituted alkyl group having 1 to 18 carbon atoms is a hydroxy group, or a group represented by -O(CO)-R⁴² or -(CO)O-R⁴³, and R⁴² and R⁴³ each independently represent a hydrocarbon group having 1 to 18 carbon atoms.

Formula (1-2) still more preferably represents a compound in which one of R² and R³ represents a group represented by R³¹-L²-R³²- and the other represents a hydrocarbon group having 6 carbon atoms, L² represents -O(CO)- or -(CO)O-, R⁵ represents a hydrogen atom or a substituted alkyl group having 1 to 18 carbon atoms, R⁷ and R⁸ each independently represent a hydrogen atom or an alkyl group having 1 to 18 carbon atoms, the substituent on the substituted alkyl group having 1 to 18 carbon atoms is a group represented by -O(CO)-R⁴² or -(CO)O-R⁴³, R⁴² and R⁴³ each independently represent a hydrocarbon group having 1 to 18 carbon atoms, and e represents 2.

The compound may form a salt.

Examples of the salt in a basic group include salts with mineral acids such as hydrochloric acid, hydrobromic acid, nitric acid, and sulfuric acid; salts with organic carboxylic acids such as formic acid, acetic acid, citric acid, oxalic acid, fumaric acid, maleic acid, succinic acid, malic acid, tartaric acid, aspartic acid, trichloroacetic acid, and trifluoroacetic acid; and salts with sulfonic acids such as methanesulfonic acid, benzenesulfonic acid, p-toluenesulfonic acid, mesitylenesulfonic acid, and naphthalenesulfonic acid.

Examples of the salt in an acidic group include salts with alkali metals such as sodium and potassium; salts with alkaline earth metals such as calcium and magnesium; ammonium salts; and salts with nitrogen-containing organic bases such as trimethylamine, triethylamine, tributylamine, pyridine, N,N-dimethylaniline, N-methylpiperidine, N-methylmorpholine, diethylamine, dicyclohexylamine, procaine, dibenzylamine, N-benzyl-β-phenethylamine, 1-ephenamine, and N,N'-dibenzylethylenediamine.

Among the above salts, preferred examples of the salt include pharmacologically acceptable salts.

Preferred specific examples of the compound represented by Formula (1) include the compounds described in Examples described later, but the present invention is not construed as being limited thereto.

### <Compound represented by Formula (2) or salt thereof>

The ionizable lipid is preferably a compound represented by Formula (2) or a salt thereof.

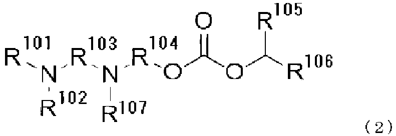

In the formula,
R¹⁰¹ and R¹⁰² each independently represent a hydrocarbon group having 1 to 18 carbon atoms, and R¹⁰³ represents a hydrocarbon group having 2 to 8 carbon atoms, where the hydrocarbon groups represented by R¹⁰¹, R¹⁰², and R¹⁰³ may be substituted with one or more substituents selected from -OH, -COOH, -NR¹⁵¹R¹⁵², -OC(O)O-R¹⁵³, -C(O)O-R¹⁵⁴, -OC(O)-R¹⁵⁵, and -O-R¹⁵⁶, and R¹⁰² and R¹⁰³ may be linked to each other to form a 4- to 7-membered ring,
R¹⁰⁴ represents a hydrocarbon group having 1 to 8 carbon atoms,
R¹⁰⁵ and R¹⁰⁶ each independently represent a hydrocarbon group having 1 to 8 carbon atoms or -R¹⁰⁸-L¹⁰¹-R¹⁰⁹, excluding a case where both R¹⁰⁵ and R¹⁰⁶ are hydrocarbon groups having 1 to 8 carbon atoms,
R¹⁰⁷ represents -R¹¹⁰-L¹⁰²-R¹¹¹-L¹⁰³-R¹¹²,
R¹⁵¹ and R¹⁵² each independently represent a hydrocarbon group having 1 to 8 carbon atoms,
R¹⁵³, R¹⁵⁴, R¹⁵⁵, and R¹⁵⁶ each independently represent a hydrocarbon group having 1 to 24 carbon atoms,
the hydrocarbon groups represented by R¹⁵³, R¹⁵⁴, R¹⁵⁵, and R¹⁵⁶ may be substituted with an aryl group having 6 to 20 carbon atoms or -S-R¹⁵⁸,
the aryl group having 6 to 20 carbon atoms may be substituted with -OH, -COOH, -NR¹⁵¹R¹⁵², -OC(O)O-R¹⁵³, -C(O)O-R¹⁵⁴, -OC(O)-R¹⁵⁵, -O-R¹⁵⁶, or -(hydrocarbon group having 1 to 12 carbon atoms)-R¹⁵⁷,
R¹⁵⁸ represents a hydrocarbon group having 1 to 12 carbon atoms, and
R¹⁵⁷ represents -OH, -COOH, -NR¹⁶¹R¹⁶², -OC(O)O-R¹⁶³, -C(O)O-R¹⁶⁴, -OC(O)-R¹⁶⁵, or -O-R¹⁶⁶.
R¹⁶¹ and R¹⁶² each independently represent a hydrocarbon group having 1 to 8 carbon atoms,
R¹⁶³, R¹⁶⁴, R¹⁶⁵, and R¹⁶⁶ each independently represent a hydrocarbon group having 1 to 24 carbon atoms,
the hydrocarbon groups represented by R¹⁶³, R¹⁶⁴, R¹⁶⁵, and R¹⁶⁶ may be substituted with an aryl group having 6 to 20 carbon atoms or -S-R¹⁶⁸,
the aryl group having 6 to 20 carbon atoms may be substituted with -OH, -COOH, -NR¹⁶¹R¹⁶², -OC(O)O-R¹⁶³, -C(O)O-R¹⁶⁴, -OC(O)-R¹⁶⁵, -O-R¹⁶⁶, or a hydrocarbon group having 1 to 12 carbon atoms,
R¹⁶⁸ represents a hydrocarbon group having 1 to 12 carbon atoms, and
L¹⁰¹, L¹⁰², and L¹⁰³ each independently represent -OC(O)O-, -C(O)O-, -OC(O)-, or -O-.
R¹⁰⁸ represents a hydrocarbon group having 1 to 12 carbon atoms,
R¹⁰⁹ represents a hydrocarbon group having 1 to 24 carbon atoms,
R¹¹⁰ represents a hydrocarbon group having 1 to 8 carbon atoms,
R¹¹¹ represents a hydrocarbon group having 1 to 24 carbon atoms,
R¹¹² represents a hydrocarbon group having 1 to 24 carbon atoms,
the hydrocarbon groups represented by R¹⁰⁹ and R¹¹² may be substituted with an aryl group, -OC(O)O-R¹⁵³, -C(O)O-R¹⁵⁴, -OC(O)-R¹⁵⁵, or -S-R¹⁵⁸, where definitions of R⁵³, R⁵⁴, R⁵⁵, and R⁵⁸ are as described above, and
the hydrocarbon group represented by R¹¹¹ may be substituted with -OC(O)O-R¹⁵³, -C(O)O-R¹⁵⁴, or -OC(O)-R¹⁵⁵, where the definitions of R¹⁵³, R¹⁵⁴, and R¹⁵⁵ are as described above.

A hydrocarbon group having 1 to 24 carbon atoms, a hydrocarbon group having 1 to 18 carbon atoms, a hydrocarbon group having 1 to 12 carbon atoms, a hydrocarbon group having 2 to 8 carbon atoms, and a hydrocarbon group having 1 to 8 carbon atoms are each preferably an alkyl group, an alkenyl group, or an alkynyl group.

The alkyl group may be linear or branched, or may be chainlike or cyclic. Specifically, examples of the alkyl group include a methyl group, an ethyl group, a propyl group, an isopropyl group, a cyclopropyl group, a butyl group, an isobutyl group, a tert-butyl group, a cyclobutyl group, a pentyl group, a cyclopentyl group, a hexyl group, a cyclohexyl group, a heptyl group, an octyl group, a nonyl group, a decyl group, an undecyl group, a dodecyl group, a tridecyl group, a trimethyldodecyl group (preferably a 3,7,11-trimethyldodecyl group), a tetradecyl group, a pentadecyl group, a hexadecyl group, a tetramethylhexadecyl group (preferably a 3,7,11,15-tetramethylhexadecyl group), a heptadecyl group, an octadecyl group, a 2-butylhexyl group, a 2-butyloctyl group, a 1-pentylhexyl group, a 2-pentylheptyl group, a 3-pentyloctyl group, a 1-hexylheptyl group, a 1-hexylnonyl group, a 2-hexyloctyl group, a 2-hexyldecyl group, a 3-hexylnonyl group, a 1-heptyloctyl group, a 2-heptylnonyl group, a 2-heptylundecyl group, a 3-heptyldecyl group, a 1-octylnonyl group, a 2-octyldecyl group, a 2-octyldodecyl group, a 3-octylundecyl group, a 2-nonylundecyl group, a 3-nonyldodecyl group, a 2-decyldodecyl group, a 2-decyltetradecyl group, a 3-decyltridecyl group, a 2-(4,4-dimethylpentan-2-yl)-5,7,7-trimethyloctyl group, and the like.

The alkenyl group may be linear or branched, or may be chainlike or cyclic. Specifically, examples thereof include an allyl group, a prenyl group, a pentenyl group, a hexenyl group, a heptenyl group, an octenyl group, a nonenyl group (preferably a (Z)-2-nonenyl group or an (E)-2-nonenyl group), a decenyl group, an undecenyl group, a dodecenyl group, a dodecadienyl group, a tridecenyl group (preferably a (Z)-tridec-8-enyl group), a tetradecenyl group (preferably a tetradec-9-enyl group), a pentadecenyl group (preferably a (Z)-pentadec-8-enyl group), a hexadecenyl group (preferably a (Z)-hexadec-9-enyl group), a hexadecadienyl group, a heptadecenyl group (preferably a (Z)-heptadec-8-enyl group), a heptadecadienyl group (preferably a (8Z,11Z)-heptadeca-8,11-dienyl group), an octadecenyl group (preferably a (Z)-octadec-9-enyl group), and an octadecadienyl group (preferably a (9Z,12Z)-octadeca-9,12-dienyl group).

The alkynyl group may be linear or branched, or may be chainlike or cyclic. Specifically, examples thereof include a propargyl group, a butynyl group, a pentynyl group, a hexynyl group, a heptynyl group, an octynyl group, a nonynyl group, a decynyl group, an undecynyl group, a dodecynyl group, a tetradecynyl group, a pentadecynyl group, a hexadecynyl group, a heptadecynyl group, and an octadecynyl group.

All of the above alkenyl groups preferably have one double bond or two double bonds. All of the above alkynyl groups preferably have one triple bond or two triple bonds.

The hydrocarbon group having 1 to 12 carbon atoms is preferably an alkylene group having 1 to 12 carbon atoms or an alkenylene group having 2 to 12 carbon atoms. The alkylene group having 1 to 12 carbon atoms and the alkenylene group having 2 to 12 carbon atoms may be linear or branched, or may be chainlike or cyclic.

Specifically, examples thereof include a methylene group, an ethylene group, a trimethylene group, a tetramethylene group, a pentamethylene group, a hexamethylene group, a heptamethylene group, an octamethylene group, a nonamethylene group, a decamethylene group, an undecamethylene group, and the like.

The aryl group preferably has 6 to 20 carbon atoms, more preferably has 6 to 18 carbon atoms, and even more preferably 6 to 10 carbon atoms. Specifically, examples of the aryl group include a phenyl group, a naphthyl group, an anthracenyl group, and a phenanthrenyl group.

R¹⁰¹ and R¹⁰² each independently preferably represent a hydrocarbon group having 1 to 12 carbon atoms, more preferably represent a hydrocarbon group having 1 to 6 carbon atoms, and even more preferably represent a hydrocarbon group having 1 to 3 carbon atoms.

R¹⁰³ preferably represents a hydrocarbon group having 2 to 6 carbon atoms and more preferably represents a hydrocarbon group having 2 to 4 carbon atoms.

The hydrocarbon groups represented by R¹⁰¹, R¹⁰², and R¹⁰³ may be preferably substituted with -OH.

L¹⁰¹ and L¹⁰³ each independently preferably represent -C(O)O- or -OC(O)-.

L¹⁰² preferably represents -OC(O)O-, -C(O)O-, or -OC(O)-.

R¹⁰⁸ preferably represents a hydrocarbon group having 1 to 10 carbon atoms and more preferably represents a hydrocarbon group having 1 to 8 carbon atoms.

R¹⁰⁹ preferably represents a hydrocarbon group having 1 to 20 carbon atoms and more preferably represents a hydrocarbon group having 1 to 16 carbon atoms.

R¹¹¹ preferably represents a hydrocarbon group having 1 to 16 carbon atoms and more preferably represents a hydrocarbon group having 1 to 9 carbon atoms.

R¹¹² preferably represents a hydrocarbon group having 1 to 20 carbon atoms and more preferably represents a hydrocarbon group having 1 to 16 carbon atoms.

The hydrocarbon groups represented by R¹⁰⁹ and R¹¹² may be preferably substituted with an aryl group or -S-R¹⁵⁸. Here, R¹⁵⁸ preferably represents a hydrocarbon group having 1 to 8 carbon atoms.

The hydrocarbon group represented by R¹¹¹ may be preferably substituted with -C(O)O-R¹⁵⁵ or -OC(O)-R¹⁵⁶, where R¹⁵⁵ and R¹⁵⁶ each independently represent a hydrocarbon group having 1 to 16 carbon atoms.

The hydrocarbon groups represented by R¹⁵⁵ and R¹⁵⁶ may be preferably substituted with an aryl group having 6 to 20 carbon atoms or -S-R¹⁵⁸, and the definition of R¹⁵⁸ is as described above.

The compound represented by Formula (2) is preferably a compound represented by Formula (2-1) as a first example.

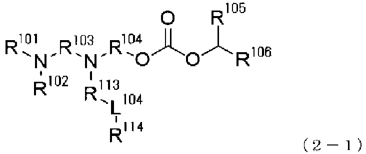

In the formula,
R¹⁰¹ and R¹⁰² each independently represent a hydrocarbon group having 1 to 18 carbon atoms, and R¹⁰³ represents a hydrocarbon group having 2 to 8 carbon atoms, where the hydrocarbon groups represented by R¹⁰¹, R¹⁰², and R¹⁰³ may be substituted with -OH, -COOH, -NR¹⁵¹R¹⁵², -OC(O)O-R¹⁵³, -C(O)O-R¹⁵⁴, -OC(O)-R¹⁵⁵, or -O-R¹⁵⁶, and R¹⁰² and R¹⁰³ may be linked to each other to form a 4- to 7-membered ring,
R¹⁰⁴ represents a hydrocarbon group having 1 to 8 carbon atoms,
R¹⁰⁵ and R¹⁰⁶ each independently represent a hydrocarbon group having 1 to 8 carbon atoms or -R¹⁰⁸-L¹⁰¹-R¹⁰⁹, excluding a case where both R¹⁰⁵ and R¹⁰⁶ are hydrocarbon groups having 1 to 8 carbon atoms,
L¹⁰¹ represents -OC(O)O-, -C(O)O-, -OC(O)-, or -O-,
R¹⁰⁸ represents a hydrocarbon group having 1 to 12 carbon atoms,
R¹⁰⁹ represents a hydrocarbon group having 1 to 24 carbon atoms, where the hydrocarbon group represented by R¹⁰⁹ may be substituted with an aryl group, -OC(O)O-R¹⁵³, -C(O)O-R¹⁵⁴, -OC(O)-R¹⁵⁵, or -S-R¹⁵⁸,
R¹⁵¹ and R¹⁵² each independently represent a hydrocarbon group having 1 to 8 carbon atoms,
R¹⁵³, R¹⁵⁴, R¹⁵⁵, and R¹⁵⁶ each independently represent a hydrocarbon group having 1 to 24 carbon atoms,
the hydrocarbon groups represented by R¹⁵³, R¹⁵⁴, R¹⁵⁵, and R¹⁵⁶ may be substituted with an aryl group having 6 to 20 carbon atoms or -S-R¹⁵⁸,
the aryl group having 6 to 20 carbon atoms may be substituted with -OH, -COOH, -NR¹⁵¹R¹⁵², -OC(O)O-R¹⁵³, -C(O)O-R¹⁵⁴, -OC(O)-R¹⁵⁵, -O-R¹⁵⁶, or -(hydrocarbon group having 1 to 12 carbon atoms)-R¹⁵⁷,
R¹⁵⁸ represents a hydrocarbon group having 1 to 12 carbon atoms, and
R¹⁵⁷ represents -OH, -COOH, -NR¹⁵¹R¹⁵², -OC(O)O-R¹⁵³, -C(O)O-R¹⁵⁴, -OC(O)-R¹⁵⁵, or -O-R¹⁵⁶.
R¹¹³ represents a hydrocarbon group having 1 to 8 carbon atoms,
R¹¹⁴ represents -R¹¹⁵-L¹⁰⁵-R¹¹⁶, where R¹¹⁵ represents a hydrocarbon group having 1 to 24 carbon atoms, L¹⁰⁵ represents -OC(O)O-, -C(O)O-, -OC(O)-, or -O-, and R¹¹⁶ represents a hydrocarbon group having 1 to 24 carbon atoms,
the hydrocarbon group having 1 to 24 carbon atoms represented by R¹¹⁵ may be substituted with -OC(O)O-R¹⁵³, -C(O)O-R¹⁵⁴, or -OC(O)-R¹⁵⁵, where definitions of R¹⁵³, R¹⁵⁴, and R¹⁵⁵ are as described above, and
the hydrocarbon groups having 1 to 24 carbon atoms represented by R¹¹⁶ may be substituted with an aryl group having 6 to 20 carbon atoms, -OC(O)O-R¹⁵³, -C(O)O-R¹⁵⁴, -OC(O)-R¹⁵⁵, or -S-R¹⁵⁸, where definitions of R⁵³, R⁵⁴, R⁵⁵, and R⁵⁸ are as described above.

In Formula (1-1), R¹⁰¹ and R¹⁰² each independently preferably represent a hydrocarbon group having 1 to 12 carbon atoms, more preferably represent a hydrocarbon group having 1 to 6 carbon atoms, and even more preferably represent a hydrocarbon group having 1 to 3 carbon atoms.

R¹⁰³ preferably represents a hydrocarbon group having 2 to 6 carbon atoms and more preferably represents a hydrocarbon group having 2 to 4 carbon atoms.

The hydrocarbon groups represented by R¹⁰¹, R¹⁰², and R¹⁰³ may be preferably substituted with -OH or an -O-benzyl group.

L¹⁰¹ and L¹⁰⁴ each independently preferably represent -C(O)O- or -OC(O)-.
R¹⁰⁸ preferably represents a hydrocarbon group having 1 to 10 carbon atoms and more preferably represents a hydrocarbon group having 1 to 8 carbon atoms.

R¹⁰⁹ preferably represents a hydrocarbon group having 1 to 18 carbon atoms, and the hydrocarbon group represented by R¹⁰⁹ may be substituted with an aryl group having 6 to 20 carbon atoms or -S-R¹⁵⁸.

R¹¹⁴ preferably represents -R¹¹⁵-L¹⁰⁵-R¹¹⁶, where R¹¹⁵ represents a hydrocarbon group having 1 to 18 carbon atoms, L¹⁰⁵ represents -OC(O)O-, and R¹¹⁶ represents a hydrocarbon group having 1 to 18 carbon atoms.

The hydrocarbon group having 1 to 18 carbon atoms represented by R¹¹⁵ may be preferably substituted with -C(O)O-R¹⁵⁵ or -OC(O)-R¹⁵⁶. R¹⁵⁵ and R¹⁵⁶ each independently represent a hydrocarbon group having 1 to 16 carbon atoms, and the hydrocarbon groups represented by R¹⁵⁵ and R¹⁵⁶ may be substituted with an aryl group having 6 to 20 carbon atoms or -S-R¹⁵⁸, where the definition of R¹⁵⁸ is as described above.

The hydrocarbon group having 1 to 18 carbon atoms represented by R¹¹⁶ may be preferably substituted with an aryl group or -S-R¹⁵⁸, where the definition of R¹⁵⁸ is as described above.

The compound represented by Formula (2) is preferably a compound represented by Formula (2-2) as a second example.

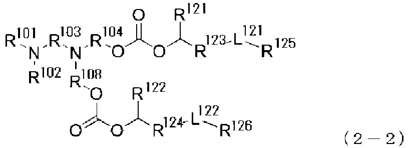

In the formula,
R¹⁰¹ and R¹⁰² each independently represent a hydrocarbon group having 1 to 18 carbon atoms, and R¹⁰³ represents a hydrocarbon group having 2 to 8 carbon atoms, where the hydrocarbon groups represented by R¹⁰¹, R¹⁰², and R¹⁰³ may be substituted with -OH, -COOH, -NR¹⁵¹R¹⁵², -OC(O)O-R¹⁵³, -C(O)O-R¹⁵⁴, -OC(O)-R¹⁵⁵, or -O-R¹⁵⁶, and R¹⁰² and R¹⁰³ may be linked to each other to form a 4- to 7-membered ring,
R¹⁰⁴ and R¹⁰⁸ each independently represent a hydrocarbon group having 1 to 8 carbon atoms,
R¹²¹ and R¹²² each independently represent a hydrocarbon group having 1 to 18 carbon atoms,
R¹²³ and R¹²⁴ each independently represent a hydrocarbon group having 1 to 12 carbon atoms,
R¹²⁵ and R¹²⁶ each independently represent a hydrocarbon group having 1 to 24 carbon atoms,
L¹²¹ and L¹²² each independently represent -OC(O)O-, -C(O)O-, -OC(O)-, or -O-,
the hydrocarbon groups represented by R¹²⁵ and R¹²⁶ may be substituted with an aryl group having 6 to 20 carbon atoms, -OC(O)O-R¹⁵³, -C(O)O-R¹⁵⁴, -OC(O)-R¹⁵⁵, or -S-R¹⁵⁸,
R¹⁵¹ and R¹⁵² each independently represent a hydrocarbon group having 1 to 8 carbon atoms,
R¹⁵³, R¹⁵⁴, R¹⁵⁵, and R¹⁵⁶ each independently represent a hydrocarbon group having 1 to 18 carbon atoms,
the aryl group having 6 to 20 carbon atoms may be substituted with -OH, -COOH, -NR¹⁵¹R¹⁵², -OC(O)O-R¹⁵³, -C(O)O-R¹⁵⁴, -OC(O)-R¹⁵⁵, -O-R¹⁵⁶, or -(hydrocarbon group having 1 to 12 carbon atoms)-R¹⁵⁷, where R¹⁵⁷ represents -OH, -COOH, -NR¹⁵¹R¹⁵², -OC(O)O-R¹⁵³, -C(O)O-R¹⁵⁴, -OC(O)-R¹⁵⁵, or -O-R¹⁵⁶.
R¹⁵⁸ represents a hydrocarbon group having 1 to 12 carbon atoms.

In Formula (2-2), R¹⁰¹ and R¹⁰² each independently preferably represent a hydrocarbon group having 1 to 12 carbon atoms, more preferably represent a hydrocarbon group having 1 to 6 carbon atoms, and even more preferably represent a hydrocarbon group having 1 to 3 carbon atoms. The hydrocarbon groups represented by R¹⁰¹ and R¹⁰² may be preferably substituted with -OH.

R¹⁰³ preferably represents a hydrocarbon group having 2 to 6 carbon atoms and more preferably represents a hydrocarbon group having 2 to 4 carbon atoms.

R¹²¹ and R¹²² each independently represent preferably a hydrocarbon group having 1 to 12 carbon atoms, more preferably represents a hydrocarbon group having 1 to 8 carbon atoms and more preferably represents a hydrocarbon group having 1 to 6 carbon atoms.

R¹²³ and R¹²⁴ each independently preferably represent a hydrocarbon group having 1 to 10 carbon atoms and more preferably represent a hydrocarbon group having 1 to 8 carbon atoms.

R¹²⁵ and R¹²⁶ each independently preferably represent a hydrocarbon group having 1 to 20 carbon atoms, more preferably represent a hydrocarbon group having 1 to 16 carbon atoms, and even more preferably represent a hydrocarbon group having 1 to 12 carbon atoms.

L¹²¹ and L¹²² each independently preferably represent -C(O)O- or -OC(O)-.

The compound represented by Formula (2) is preferably a compound represented by Formula (2-3) as a third example.

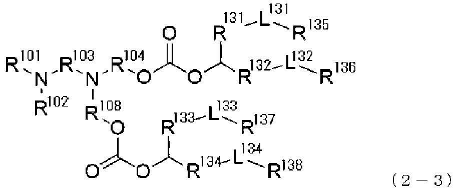

In the formula,
R¹⁰¹ and R¹⁰² each independently represent a hydrocarbon group having 1 to 18 carbon atoms, and R¹⁰³ represents a hydrocarbon group having 2 to 8 carbon atoms, where the hydrocarbon groups represented by R¹⁰¹, R¹⁰², and R¹⁰³ may be substituted with -OH, -COOH, -NR¹⁵¹R¹⁵², -OC(O)O-R¹⁵³, -C(O)O-R¹⁵⁴, -OC(O)-R¹⁵⁵, or -O-R¹⁵⁶, and R¹⁰² and R¹⁰³ may be linked to each other to form a 4- to 7-membered ring,
R¹⁰⁴ and R¹⁰⁸ each independently represent a hydrocarbon group having 1 to 8 carbon atoms,
R¹³¹, R¹³², R¹³³, and R¹³⁴ each independently represent a hydrocarbon group having 1 to 12 carbon atoms,
R¹³⁵, R¹³⁶, R¹³⁷, and R¹³⁸ each independently represent a hydrocarbon group having 1 to 24 carbon atoms,
L¹³¹, L¹³², L¹³³ and L¹³⁴ each independently represent -OC(O)O-, -C(O)O-, -OC(O)-, or -O-,
the hydrocarbon groups represented by R¹³⁵, R¹³⁶, R¹³⁷, and R¹³⁸ may be substituted with an aryl group having 6 to 20 carbon atoms, -OC(O)O-R¹⁵³, -C(O)O-R¹⁵⁴, -OC(O)-R¹⁵⁵, or -S-R¹⁵⁸,
R¹⁵¹ and R¹⁵² each independently represent a hydrocarbon group having 1 to 8 carbon atoms,
R¹⁵³, R¹⁵⁴, R¹⁵⁵, and R¹⁵⁶ each independently represent a hydrocarbon group having 1 to 18 carbon atoms,
the aryl group having 6 to 20 carbon atoms may be substituted with -OH, -COOH, -NR¹⁵¹R¹⁵², -OC(O)O-R¹⁵³, -C(O)O-R¹⁵⁴, -OC(O)-R¹⁵⁵, -O-R¹⁵⁶, or -(hydrocarbon group having 1 to 12 carbon atoms)-R¹⁵⁷, where R¹⁵⁷ represents -OH, -COOH, -NR¹⁵¹R¹⁵², -OC(O)O-R¹⁵³, -C(O)O-R¹⁵⁴, -OC(O)-R¹⁵⁵, or -O-R¹⁵⁶.
R¹⁵⁸ represents a hydrocarbon group having 1 to 12 carbon atoms.

In Formula (2-3), R¹⁰¹ and R¹⁰² each independently preferably represent a hydrocarbon group having 1 to 12 carbon atoms, more preferably represent a hydrocarbon group having 1 to 6 carbon atoms, and even more preferably represent a hydrocarbon group having 1 to 3 carbon atoms. The hydrocarbon groups represented by R¹⁰¹ and R¹⁰² may be preferably substituted with -OH.

R¹⁰³ preferably represents a hydrocarbon group having 2 to 6 carbon atoms and more preferably represents a hydrocarbon group having 2 to 4 carbon atoms.

R¹³¹, R¹³², R¹³³, and R¹³⁴ each independently preferably represent a hydrocarbon group having 1 to 10 carbon atoms, more preferably represent a hydrocarbon group having 1 to 8 carbon atoms, and even more preferably represent a hydrocarbon group having 1 to 3 carbon atoms.

R¹³⁵, R¹³⁶, R¹³⁷, and R¹³⁸ each independently preferably represent a hydrocarbon group having 1 to 20 carbon atoms, more preferably represent a hydrocarbon group having 1 to 16 carbon atoms, and even more preferably represent a hydrocarbon group having 1 to 12 carbon atoms. The hydrocarbon groups represented by R¹³⁵, R¹³⁶, R¹³⁷, and R¹³⁸ may be preferably substituted with an aryl group having 6 to 20 carbon atoms or S-R¹⁵⁸. More preferably, these may be substituted with -S-R¹⁵⁸.

R¹³⁵, R¹³⁶, R¹³⁷, and R¹³⁸ each independently particularly preferably represent a hydrocarbon group having 1 to 12 carbon atoms substituted with -S-R¹⁵⁸, or a hydrocarbon group having 1 to 12 carbon atoms.

L¹³¹, L¹³², L¹³³, and L¹³⁴ each independently preferably represent -C(O)O-, or -OC(O)-.

R¹⁵⁸ preferably represents a hydrocarbon group having 1 to 10 carbon atoms and more preferably represents a hydrocarbon group having 1 to 8 carbon atoms.

The compound may form a salt.

Examples of the salt in a basic group include salts with mineral acids such as hydrochloric acid, hydrobromic acid, nitric acid, and sulfuric acid; salts with organic carboxylic acids such as formic acid, acetic acid, citric acid, oxalic acid, fumaric acid, maleic acid, succinic acid, malic acid, tartaric acid, aspartic acid, trichloroacetic acid, and trifluoroacetic acid; and salts with sulfonic acids such as methanesulfonic acid, benzenesulfonic acid, p-toluenesulfonic acid, mesitylenesulfonic acid, and naphthalenesulfonic acid.

Examples of the salt in an acidic group include salts with alkali metals such as sodium and potassium; salts with alkaline earth metals such as calcium and magnesium; ammonium salts; salts with nitrogen-containing organic bases such as trimethylamine, triethylamine, tributylamine, pyridine, N,N-dimethylaniline, N-methylpiperidine, N-methylmorpholine, diethylamine, dicyclohexylamine, procaine, dibenzylamine, N-benzyl-β-phenethylamine, 1-ephenamine, and N,N'-dibenzylethylenediamine; and the like.

Among the above salts, preferred examples of the salt include pharmacologically acceptable salts.

Specific preferred examples of the compound represented by Formula (2) include the compounds described in Examples later, but the present invention is not construed as being limited thereto.

The compounds of Synthesis Examples 33 to 44 and 55 in Examples later are novel compounds. According to the present invention, there is provided the compound of Synthesis Examples 33 to 44 and 55 in Examples later.

### <Manufacturing method>

The compound represented by Formula (1) or Formula (2) is described in WO2019/235635A and WO2022/230964A, and can be manufactured according to the manufacturing methods described in WO2019/235635A and WO2022/230964A.

### <Sterol>

The non-ionized lipid preferably contains a sterol or a derivative thereof. In a case where the lipid composition according to the embodiment of the present invention contain a sterol, the fluidity of the membrane can be reduced, and hence the lipid composition can be effectively stabilized.

The sterol is not particularly limited, and examples thereof can include cholesterol, phytosterol (sitosterol, stigmasterol, fucosterol, spinasterol, brassicasterol, and the like), ergosterol, cholestanone, cholestenone, coprostanol, cholesteryl-2'-hydroxyethyl ether, and cholesteryl-4'-hydroxybutyl ether. Among these, cholesterol is preferable.

In the lipid composition, the blending amount of the sterol or the derivative thereof is preferably 30% to 70% by mole, more preferably 30% by mole to 65% by mole, and still more preferably 30% by mole to 60% by mole in terms of a molar ratio with respect to the total lipids in the lipid composition.

### [Phospholipid]

The non-ionized lipid may contain a phospholipid. The phospholipid is not particularly limited, and examples thereof include phosphatidylcholine, phosphatidylethanolamine, sphingomyelin, ceramide, and the like. Among these, phosphatidylcholine is preferable. In addition, the phospholipid may be used alone or in combination with a plurality of different neutral lipids.

The phosphatidylcholine is not particularly limited, and examples thereof include soybean lecithin (SPC), hydrogenated soybean lecithin (HSPC), egg yolk lecithin (EPC), hydrogenated egg yolk lecithin (HEPC), dimyristoylphosphatidylcholine (DMPC), dipalmitoylphosphatidylcholine (DPPC), distearoylphosphatidylcholine (DSPC), 1-palmitoyl-2-oleoylphosphatidylcholine (POPC), dioleoylphosphatidylcholine (DOPC), and the like.

The phosphatidylethanolamine is not particularly limited, and examples thereof include dimyristoylphosphatidylethanolamine (DMPE), dipalmitoylphosphatidylethanolamine (DPPE), distearoylphosphatidylethanolamine (DSPE), dioleoylphosphatidylethanolamine (DOPE), dilinoleoylphosphatidylethanolamine (DLoPE), diphytanoylphosphatidylethanolamine (D(Phy)PE), 1-palmitoyl-2-oleoylphosphatidylethanolamine (POPE), ditetradecylphosphatidylethanolamine, dihexadecylphosphatidylethanolamine, dioctadecylphosphatidylethanolamine and diphytanylphosphatidylethanolamine.

The sphingomyelin is not particularly limited, and examples thereof include egg yolk-derived sphingomyelin, milk-derived sphingomyelin, and the like.

The ceramide is not particularly limited, and examples thereof include egg yolk-derived ceramide, milk-derived ceramide, and the like.

The phospholipid is preferably selected from the group consisting of distearoylphosphatidylcholine, dioleoylphosphatidylcholine, and dioleoylphosphatidylethanolamine.

In the lipid composition, the blending amount of the phospholipid is preferably 1% to 30% by mole and more preferably 1% to 20% by mole in terms of the molar ratio to the total amount of the lipids in the lipid composition.

### <Lipid having nonionic hydrophilic polymer>

The lipid composition in the present invention may contain a lipid having a nonionic hydrophilic polymer. In the present invention, by containing the lipid having a nonionic hydrophilic polymer, it is possible to obtain the dispersion stabilizing effect of the lipid composition.

The nonionic hydrophilic polymer is not particularly limited, and examples thereof include a nonionic vinyl-based polymer, a nonionic polyamino acid, a nonionic polyester, a nonionic polyether, a nonionic natural polymer, a nonionic modified natural polymer, and a block polymer or a graft copolymer having two or more types of these polymers as constitutional units.

Among these nonionic hydrophilic polymers, a nonionic polyether, a nonionic polyester, a nonionic polyamino acid, or a nonionic synthetic polypeptide is preferable, a nonionic polyether or a nonionic polyester is more preferable, a nonionic polyether or a nonionic monoalkoxy polyether is even more preferable, and polyethylene glycol (hereinafter, polyethylene glycol will be also called PEG) is particularly preferable. That is, the lipid having a nonionic hydrophilic polymer is preferably a lipid having a polyethylene glycol chain.

The lipid having a nonionic hydrophilic polymer is not particularly limited, and examples thereof include PEG-modified phosphoethanolamine, a diacylglycerol PEG derivative, a monoacylglycerol PEG derivative, a dialkylglycerol PEG derivative, a cholesterol PEG derivative, a ceramide PEG derivative, and the like. Among these, monoacylglycerol PEG derivative or diacylglycerol PEG derivative is preferable.

The lipid having a polyethylene glycol chain is particularly preferably selected from dimyristoyl-rac-glycerol polyethylene glycol, distearoyl-rac-glycerol polyethylene glycol, and distearoylphosphatidylethanolamine polyethylene glycol.

The weight-average molecular weight of the PEG chain of the nonionic hydrophilic polymer derivative is preferably 500 to 5,000 and more preferably 750 to 3,000.

The nonionic hydrophilic polymer chain may be branched or may have a substituent such as a hydroxymethyl group.

In the lipid composition, the blending amount of the lipid having a nonionic hydrophilic polymer chain is preferably 0.1 to 3 mol%, more preferably 0.3 to 3 mol%, and still more preferably 0.5 to 3 mol% with respect to the total lipids in the lipid composition in terms of molar ratio.

In the present invention, a step A of preparing lipid particles not containing nucleic acid is first performed using an ionizable lipid, a non-ionizable lipid, and a lipid having a nonionic polymer.

The lipid particles not containing a nucleic acid can be manufactured by dissolving all or some of the components of the lipid particles not containing a nucleic acid, which are oil-soluble components, in an organic solvent or the like to form an oil phase, and mixing the oil phase with a water phase. A micromixer may be used for mixing, or an emulsification using an emulsifying machine such as a homogenizer, an ultrasonic emulsifying machine, a high-pressure injection emulsifying machine, or the like may be performed.

Alternatively, the lipid particles can also be manufactured by a method in which a lipid-containing solution is subjected to evaporation to dryness using an evaporator under reduced pressure or subjected to spray drying using a spray drier such that a dried mixture containing a lipid is prepared, and the mixture is added to an aqueous solvent and further emulsified using the aforementioned emulsifying machine or the like.

One of the examples of the method for manufacturing the lipid particles containing a nucleic acid is a method including
a step (a) of dissolving the constituent components of the lipid particles containing the compound in an organic solvent to obtain an oil phase,
a step (b) of mixing the oil phase obtained in the step (a) with a water phase to obtain a dispersion liquid of lipid particles,
a step (c) of diluting the dispersion liquid obtained in the step (b),
a step (d) of removing the organic solvent from the dispersion liquid of the lipid particles, and a step (e) of adjusting the concentration of the dispersion liquid of lipid particles.

In the step (a), the components of the lipid particles not containing a nucleic acid are dissolved in an organic solvent (an alcohol such as ethanol or an ester). The total lipid concentration is not particularly limited, but is generally 1 mmol/L to 100 mmol/L, preferably 5 mmol/L to 80 mmol/L, and more preferably 10 mmol/L to 70 mmol/L.

In the step (b), the oil phase and the water phase may be mixed by any method, and a batch type or an in-line method using a channel device may be used. As the in-line method, a microchannel device is preferably used, and as the microchannel device to be used, a Y-shaped mixer, a T-shaped mixer, a herringbone mixer, a ring micromixer, an impingement jet mixer, or the like can be used. The mixing ratio (volume ratio) of water phase:oil phase is preferably 5:1 to 1:1 and more preferably 4:1 to 2:1.

A component such as a buffer component or an antioxidant for pH adjustment can be added as necessary. The pH of the water phase is preferably 2.0 to 7.0 and more preferably 3.0 to 6.0. Acetic acid, citric acid, malic acid, phosphoric acid, MES, HEPES, or the like is preferably used as a buffer component for adjusting the pH, and as necessary, salts such as sodium chloride and potassium chloride may be added for the purpose of adjusting a salt strength, or sugars or sugar alcohols, such as sucrose, trehalose, and mannitol may be added for the purpose of adjusting an osmotic pressure.

In the step (c), by mixing the dispersion liquid of lipid particles with the dilution solution, the content of the organic solvent can be reduced and the lipid particles can be stabilized. The dilution solution may be water, but the pH or salt strength may be adjusted with the dilution solution. The component contained in the dilution solution can be optionally selected depending on the purpose. For example, for the purpose of adjusting the pH, a buffer solution (for example, a citrate buffer solution, a citrate buffered physiological saline, an acetate buffer solution, an acetate buffered physiological saline, a phosphate buffered physiological saline, a Tris buffer solution, an MES buffer solution, a HEPES buffer solution, or the like) may be used. In addition, sodium chloride, potassium chloride, sucrose, trehalose, fructose, mannitol, or the like may be contained for the purpose of adjusting the salt strength or the osmotic pressure, and a buffer solution to which these additives are further added can also be used.

The dispersion liquid of lipid particles and the dilution solution may be mixed by any method, and a batch type or an in-line method using a channel device may be used. As the channel device used at the time of mixing, a Y-shaped mixer, a T-shaped mixer, or the like can be used. In addition, the time from mixing the oil phase with the water phase to mixing the dilution solution therewith is not particularly limited, but the dilution is preferably performed within 30 seconds and more preferably performed within 10 seconds after mixing the oil phase with the water phase.

The mixing ratio (liquid amount ratio) of the dispersion liquid of lipid particles to the dilution solution is preferably 1:0.5 to 1:10 and more preferably 1:1 to 1:5.

In some embodiments, in the step (c), the dispersion liquid of lipid particles may be mixed with the dilution solution a plurality of times depending on the purpose. In addition, the dilution solutions to be used may be the same as or different from each other. In the dispersion liquid of lipid particles, the particle diameter of the lipid particles may change depending on the pH, and thus the adjustment of the pH of the dispersion liquid is important. Therefore, for example, to adjust the pH of the dispersion liquid of lipid particles after mixing with the dilution solution, a buffer solution having a concentration and a pH suitable for the adjustment or a buffer solution further containing other components may be used.

Furthermore, the plurality of dilution steps may be continuously performed, and the interval between the dilution step and the next dilution step can be optionally set, and for example, the interval may be 10 seconds, 30 seconds, 1 minute, 5 minutes, 10 minutes, 30 minutes, 1 hour, 2 hours, 3 hours, 4 hours, 6 hours, 12 hours, or 24 hours.

In addition, the pH of the dispersion liquid of lipid particles after performing the step (c) is preferably 3.0 to 10.0, more preferably 3.5 to 9.0, and particularly preferably 4.0 to 8.5.

The lipid particles can be sized as necessary. The method of sizing is not particularly limited, and the particle size can be reduced by using an extruder or the like.

In addition, the dispersion liquid containing the lipid composition can be frozen or freeze-dried by a general method.

In the step (d), a method of removing the organic solvent from the dispersion liquid of lipid particles is not particularly limited, and a general method can be used. For example, a pH buffer solution such as phosphate buffered physiological saline or a Tris buffer solution can be used as the dialysate, and additives such as any salt or sugar can be added as necessary for the purpose of adjusting the osmotic pressure or protecting the dialysate from freezing.

In the step (e), the concentration of the dispersion liquid of lipid particles obtained in the step (d) can be adjusted. In a case of diluting the dispersion liquid, the dispersion liquid can be diluted to an appropriate concentration by using a solution such as phosphate buffered physiological saline, physiological saline, a Tris buffer solution, or a sucrose-containing Tris buffer solution as a dilution solution. In a case of concentrating the dispersion liquid, the dispersion liquid obtained in the step (d) can be concentrated by ultrafiltration using an ultrafiltration membrane, or the like. It is preferable to use the concentrated dispersion as it is. Alternatively, it is preferable to adjust the concentrated dispersion liquid to a desired concentration by using the aforementioned diluent after the concentration.

In addition, in some embodiments, the organic solvent removal step (step (d)) and the concentration adjustment step (step (e)) can be continuously performed using tangential flow filtration (TFF). In the present step, the organic solvent removal step and the concentration adjustment step may be performed in any order. The organic solvent removal step and the concentration adjustment step may be each performed a plurality of times as necessary.

As the solution that can be used in the dialysis in the step (d) or the dilution in the step (e), an excipient, a freeze protective agent, a buffering agent, or an antioxidant may be added. The excipient or the freeze protective agent is not particularly limited, and examples thereof include sugars and sugar alcohols. Examples of the sugars include sucrose, trehalose, maltose, glucose, lactose, fructose, and the like, and examples of the sugar alcohols include mannitol, sorbitol, inositol, xylitol, and the like. The buffering agent is not particularly limited, and examples thereof include ACES, BES, bicine, CAPS, CHES, DIPSO, EPPS, HEPES, HEPPSO, MES, MOPS, MOPSO, TAPS, TAPSO, TES, tricine, tris, phosphoric acid, acetic acid, citric acid, and the like. Examples of the antioxidant include EDTA, ascorbic acid, tocopherol, and the like.

The dispersion liquid of the lipid particles may be subjected to sterile filtration. As a filtration method, a hollow fiber membrane, a reverse osmosis membrane, a membrane filter, or the like can be used to remove insoluble substances from the dispersion liquid of lipid particles. In the present invention, the filtration method is not particularly limited, but it is preferable to filter the dispersion liquid through a filter having a pore diameter capable of sterilization (preferably a filtration sterilization filter with a pore diameter of 0.2 µm). In addition, it is preferable to perform the sterile filtration after the step (d) or the step (e).

Furthermore, as necessary, the dispersion liquid of the lipid particles not containing a nucleic acid can be subjected to freezing or freeze-drying. The dispersion liquid of the lipid particles can be frozen or freeze-dried by a general method, and the method is not particularly limited.

Preferably, in the present invention, the lipid particles not containing a nucleic acid can be cryopreserved, and the lipid particles not containing a nucleic acid cryopreserved can be thawed before mixing with the nucleic acid.

In the present invention, the step B of preparing nucleic acid-containing lipid particles by mixing the lipid particles not containing nucleic acid, prepared as described above, with nucleic acid is performed.

Examples of the nucleic acid include a circular double-stranded DNA (plasmid DNA, a small circular double-stranded DNA without a drug resistance gene, or the like), a single-stranded DNA, a double-stranded DNA, a small interfering RNA (siRNA), a micro RNA (miRNA), an mRNA, an antisense oligonucleotide (also referred to as an ASO), a ribozyme, an aptamer, a saRNA, and a sgRNA, and any of these may be contained. Two or more types of nucleic acids may be used. In addition, the lipid composition may contain a modified nucleic acid. The nucleic acid is particularly preferably a circular double-stranded DNA or an RNA, and most preferably a plasmid DNA or an mRNA. The number of bases is preferably 5 to 20,000 bases.

The nucleic acid may be a sequence for gene editing, and may be an mRNA encoding a DNA nuclease.

The nucleic acid may be a sequence for gene editing, and may be a guide RNA.

The nucleic acid may be a nucleic acid mixture which is a sequence for gene editing and contains an mRNA encoding a Cas nuclease and a guide RNA. That is, the nucleic acid may be a nucleic acid for gene editing that contains an mRNA encoding a Cas nuclease and a guide RNA. The above-described mixture may further contain any donor DNA.

The nucleic acid may be a nucleic acid mixture which is a sequence for single-base editing and contains an mRNA encoding a deaminase and a mutant Cas nuclease, and a guide RNA.

The nucleic acid may be a nucleic acid mixture which is a sequence for substituting a target DNA nucleotide and contains an mRNA encoding a fusion protein of an artificial reverse transcriptase and a Cas9 endonuclease, and a prime editing guide RNA.

The nucleic acid may be a nucleic acid mixture which is a sequence for guide RNA-dependent gene transcription activation (CRISPR activator system or the like) and contains an mRNA encoding a fusion protein of an activator protein (VP64, p65, or Rta) and a mutant Cas nuclease, and a guide RNA. Alternatively, the nucleic acid may be a nucleic acid mixture which contains an mRNA encoding an activator protein (MS2, p65, or HSF1), an mRNA encoding a fusion protein of VP64 and a mutant Cas nuclease, and a guide RNA.

The nucleic acid may be a nucleic acid mixture containing a sequence for guide RNA-dependent gene transcription suppression (CRISPR interference system or the like), an mRNA encoding a fusion protein of a transcriptional suppression factor (KRAB or the like) and a mutant Cas nuclease, and a guide RNA.

The nucleic acid may be an mRNA or DNA encoding a DNA recombinase, and may be a nucleic acid mixture which further contains any donor DNA. The DNA recombinase is not particularly limited, and examples thereof include a transposase (for example, Sleeping beauty transposase, piggyBac transposase, Tol2, or the like), a Cre recombinase, and a serine integrase.

The nucleic acid may be an mRNA encoding a reverse transcriptase, which is a sequence for inserting an exogenous gene into a host cell genome, and may be a nucleic acid mixture which further contains any RNA as a donor.

The nucleic acid may be an mRNA encoding a sequence for expressing an exogenous gene, or a DNA containing the above-described exogenous gene, a promoter sequence, and a terminal sequence.

The nucleic acid may be a sequence for gene editing or gene transcription suppression of a gene (target gene) present in an immune cell. The target gene is not particularly limited, and examples thereof include a T cell receptor gene (TRAC or TRBC), MHC-I (or HLA-I), MHC-II, B2M, a gene related to a self-antigen, a gene related to an inhibitory receptor or a ligand thereof (PDCD1, CD274 (or PD-L1), PDCD1LG2, LAG3, or CTLA4), a gene related to cytotoxicity (TGFBR2, PGE2, EP2, EP4, FAS, or FASLG), a gene related to cell exhaustion or differentiation (SOCS1, ZC3H12A, NR4A1, NR4A2, NR4A3, PRDM1, BLIMP1, or TIM3), a gene related to cell death (CASP3, CASP6, or CASP7), a gene related to an inflammation response (CGAS, STING, TBK1, or the like), a methylated gene (TET1, TET2, or DNMT3A), a gene related to immune escape (CD47 or NKG2A), and others (DGK, EZH2, CSF2, PAX5, LDLR, MAP4K1, CISH, CD5, CD52, ADORA2A, CD39, CD73, CD5, MCM3AP, EIF3D, CAD, HGS, RPL19, MAK16, PDGFRA, NRF1, EP400, CBLB, RPS7, CPSF4, IL2RG, RPL38, IL2RB, JAK3, MCM2, SNRPC, PSMD4, MAP4K1, BRD9, RNF20, RNF40, NFKB2, NMT1, MYB, TSC1, EIF3K, RPL19, TBX21, PRDM1, SUV39H1, or ARID1A).

The nucleic acid may be a sequence for expressing an exogenous gene that enhances the function of an immune cell, or a sequence for transcriptionally activating a gene (target gene) present in an immune cell. The exogenous gene or the target gene for transcriptional activation is not particularly limited, and examples thereof include a cytokine (IFNG, IL2, IL7, IL12, IL15, IL18, IL19, or IL21), a cytokine receptor (IL2RA, IL2RB, IL2RG, IL12B1, or IL12B2), a costimulatory factor (CD28 or TNFRSF9), a gene involved in immune evasion (CD47 or HLA-E), a metabolism-related gene (GLUT1 or PPARGC1A), a gene related to exhaustion suppression (FOXO1, TCF7, or LEF1), a gene related to cell survival (BCL2), others (a dominant negative form TGFBR2, AKT1, LTBR, AHCY, DUPD1, AKR1C4, ATF6B, ITM2A, AHNAK, BATF, GPD1, CDK2, CDK1, GPN3, MRPL51, DBI, CALML2, IL12B, IFNL2, CLIC1, HOMER1, ADA, CYP27A1, MRPL18, RAN, SLC10A7, CRLF2, VAV1, TRIM21, LHX6, FOXO4, IRX4, FOXQ1, OTUD7B, LCP2, FOSB, RAC2, FOSL1, APOBEC3D, RIPK3, EMP1, ANXA2R, CDKN2C, OTUD7A, CD2, LAT, LCP2, TBX21, or EOMES), and a sequence encoding a secreted protein that induces inflammation.

The nucleic acid may be a sequence for expressing a chimeric antigen receptor gene, a T cell receptor gene, an antibody, a bispecific antibody, a multispecific antibody, a single chain Fv (scFv), a nanobody, or a bispecific T cell engager (BiTE).

The nucleic acid may be a sequence for expressing an exogenous gene involved in cell initialization (or reprogramming). The exogenous gene is not particularly limited, and examples thereof include Oct3/4, Sox2, Klf4, C-Myc, Nanog, and Lin28.

In the step B, a mass ratio of the lipid concentration to the nucleic acid concentration in the solution after the mixing is preferably 5:1 to 1,000:1, more preferably 5:1 to 500:1, still more preferably 7:1 to 200:1, and particularly preferably 7:1 to 100:1.

The step B may preferably include a step of incubating the lipid particles not containing a nucleic acid and the aqueous solution containing a nucleic acid at 0°C to 30°C for 0.1 to 120 minutes, and a step of adjusting the pH of the resulting mixture to 6.5 to 8.5.

In the step B, the mixing of the lipid particles not containing a nucleic acid and the nucleic acid can be performed by any of a method of mixing liquids using a flow channel, mixing in which a liquid is made to travel in a reciprocating direction in a container, pipette mixing, stirrer mixing in a batch container, mixing in which a container is rotated to stir a content liquid, or flask stirring.

The aqueous solution containing a nucleic acid can be obtained by dissolving a nucleic acid in water or a buffer solution. The concentration of the nucleic acid is not particularly limited, but is preferably 1 to 2,000 µg/mL and more preferably 10 to 1,000 µg/mL. A component such as a buffer component or an antioxidant for pH adjustment can be added as necessary.

The lipid particles containing a nucleic acid, which are obtained as a result of the step B, may be used for contact with immune cells immediately, or may be used for contact with immune cells after being stored in a refrigerated state or frozen state as necessary.

### <Lipid particles and lipid composition>

The lipid composition may be a lipid particle. The lipid particles mean particles composed of lipids, and include a composition having any structure selected from a lipid aggregate in which lipids are aggregated, a micelle, a liposome, a lipid nanoparticle (LNP), or a lipoplex. The liposome has a lipid bilayer structure and has a water phase in the inside, and includes a liposome which has a single bilayer membrane, and a multilayer liposome which has multiple layers stacked together. The present invention may include any of these liposomes. As the lipid particle, a lipid nanoparticle (LNP) is preferable.

The form of the lipid particles can be checked by electron microscopy, structural analysis using X-rays, and the like. For example, by a method using Cryo transmission electron microscopy (CryoTEM method), it is possible to check, for example, whether or not a lipid particle is, such as a liposome, a bimolecular lipid membrane structure (lamella structure) and a structure having an inner water layer, and whether or not a lipid particle has a structure having an inner core with a high electron density and packed with constituent components including a lipid. The X-ray small angle scattering (SAXS) analysis also makes it possible to check whether or not a lipid particle has a bimolecular lipid membrane structure (lamella structure).

The particle size of the lipid particles is not particularly limited, and is preferably 10 to 1,000 nm, more preferably 30 to 500 nm, and even more preferably 50 to 250 nm. The particle size of the lipid particles can be measured by a general method (for example, a dynamic light scattering method, a laser diffraction method, or the like).

The pH of the lipid composition is preferably 3.0 to 6.5 and more preferably 4.0 to 6.5.

In the present invention, after the step B, a step C of bringing the nucleic acid-containing lipid particles obtained in the step B into contact with immune cells is performed.

Preferably, before bringing the nucleic acid-containing lipid particles into contact with the immune cells in the step C, a step of adding an apolipoprotein to a culture solution containing the immune cells may be performed.

The apolipoprotein is a group of proteins that binds to a lipoprotein and acts as an activator of an enzyme group involved in the recognition of the lipoprotein or lipid metabolism, or as a coenzyme. The apolipoproteins are roughly classified into five types from apolipoprotein A to E depending on the structure and function, and some of them are classified into subclasses such as apolipoprotein A-I and C-II. In the present invention, for example, apolipoprotein E, particularly apolipoprotein E3 may be used. The origin of the apolipoprotein is not particularly limited, and an apolipoprotein of a mammal such as a human can be used.

The step C of bringing the nucleic acid-containing lipid particles into contact with the immune cells can be performed by transfecting the nucleic acid-containing lipid particles into the immune cells ex vivo, in vitro, or in vivo.

The immune cell may be any of an activated cell or an inactivated cell, and can be optionally selected depending on a method of producing the cell.

The activation treatment refers to, for example, in a case of a T cell, performing a main stimulation signal through a TCR/CD3 complex using an anti-CD3 antibody, an anti-CD28 antibody, or the like, a co-stimulation signal through CD28, or stimulation through a lectin pathway. This activation treatment is usually performed in the presence of a cytokine signal such as IL-2, IL-7, or IL-15, and these activation treatments induce the expression of a cytokine receptor such as IL-2R or active cell proliferation. For example, the activated T cells mean T cells that have been subjected to such an activation treatment.

In addition, the inactivated cell refers to, for example, in a case of a T cell, culturing in the presence of a cytokine signal such as IL-2, IL-7, or IL-15 without performing the above-described activation treatment. However, the method is not limited to the method described here.

Examples of the step of transfecting the immune cells using the nucleic acid-containing lipid particles include the following.
(i) Step of seeding immune cells
(ii) Step of bringing nucleic acid-containing lipid particles into contact with immune cells (step C)
(iii) Step of culturing immune cells

In a case where activated cells are used as the immune cells, the activation treatment may be performed in the step (i). The activation treatment can be performed by the above-described method.

In the step (i), examples of the culture medium used in a case of seeding the immune cells include serum-free culture media (RPMI 1640 culture medium, PRIME-XV T Cell Expansion XSFM (FUJIFILM Irvine Scientific), PRIME-XV T Cell CDM (FUJIFILM Irvine Scientific), TexMACS^{™} Medium (Miltenyi Biotec), CTS Optimizer Pro Serum Free Medium (Gibco), CTS^{™} Optimizer^{™} T Cell Expansion SFM (Gibco), KBM501/KBM502/KBM550/KBM551 (Kohjin Biotech Co., Ltd.), and Alys505/705 (Cell Science & Technology Institute, Inc.)).

Additives can be added to the culture medium. Examples of the additives include serum (fetal calf serum, human serum, and the like), serum substitutes (StemSure serum substitute (FUJIFILM Wako Pure Chemical Corporation), OpTimizer^{™} T-Cell Expansion Supplement (Gibco), and the like), and growth factors.

The culture medium can also be replaced with another culture medium the day before or on the day of the step (ii).

In a case where the above-described additives are added to the culture medium, it is preferable to replace the culture medium with a serum-free culture medium in which serum or a serum substitute is not added the day before or on the day of the step (ii).

The culture medium used in the step (ii) is the same as the above-described culture medium, and additives can be further added thereto. Examples of the additives include the above-described apolipoprotein and the like.

The step (ii) is the same as the above-described step C, and is as described above.

On the day of the step (ii), the culture solution may be replaced with a newly prepared culture medium using a method such as centrifugation or perfusion culture.

The step (ii) can be performed at any time point from the same day of the step (i) to the end of the step (iii). In a case where activated cells are used as the immune cells, the step (ii) is preferably performed 1 day to 7 days after the activation treatment, and more preferably 2 days or 3 days after the activation treatment. In addition, the activation treatment can be performed a plurality of times, and the step (ii) may be performed each time.

In the step (iii), the culture medium described in the step (i) and additives can be used as the culture medium in a case of culturing the immune cells. In the step (iii), the serum or the serum substitute may be added or not added under any conditions.

In the step (iii), it is preferable to add a culture medium in an amount of 0.5 times or more the amount of the culture solution the day after the step (ii). Alternatively, it is preferable to remove a part of the culture supernatant (not containing cells) or to remove the entire amount or a part of the culture supernatant using a method such as centrifugation or perfusion culture, and to add a newly prepared culture medium to replace the entire amount or a part of the culture medium in the culture solution.

Examples of the culture container used in the steps (i) to (iii) include a culture plate (for example, an untreated plate, a low-adsorption plate, a plate for adhesion culture, or the like), a culture flask, a culture container (for example, G-Rex (Wilson Wolf) or the like) having a gas-permeable membrane on the bottom surface, a culture bag, a culture tube, and a culture reactor.

The immune cell is preferably a mammal-derived cell and more preferably a human-derived cell.

The immune cell is not particularly limited, and can be selected from, for example, a lymphocyte (for example, a T cell, a B cell, a natural killer cell (NK cell), an NKT cell, or an iNKT cell), a monocyte, a macrophage, a mast cell, a dendritic cell, a granulocyte (for example, a neutrophil, an eosinophil, or a basophil), a hematopoietic stem/progenitor cell, a primary immune cell, a CD3⁺ cell, a CD4⁺ cell, a CD8⁺ T cell, a regulatory T cell (Treg), a B cell, an NK cell, a natural lymphocyte, or a dendritic cell (DC).

The immune cells may be preferably selected from peripheral blood mononuclear cells (PBMC), lymphocytes, tumor-infiltrating lymphocytes (TIL), T cells, CD4⁺ cells, CD8⁺ cells, memory T cells, naive T cells, or stem cell memory T cells.

The immune cells may be primary cells or cells derived from stem cells (preferably, pluripotent stem cells).

### <Nucleic acid delivery agent to cells other than immune cells>

The present invention can be delivered to cells other than immune cells. Examples of the cells other than immune cells include cells derived from mammals, and the cells are preferably cells derived from humans. The cells other than immune cells are not particularly limited, but it is preferable to select the cells from mesenchymal stem cells, nerve cells, iPS cell-derived nerve cells, HEK293 cells, and CHO cells. Specific examples thereof include the following nucleic acid delivery agents.

As the delivery agent to mesenchymal stem cells, a nucleic acid delivery agent to mesenchymal cells, which contains a lipid composition containing an ionizable lipid which is a compound represented by Formula (1) or Formula (2) or a salt thereof, a non-ionizable lipid, a lipid having a nonionic polymer, and a nucleic acid, is preferable. Formula (1) and Formula (2) are the same as the above-described definitions.

The nucleic acid contained in the nucleic acid delivery agent to mesenchymal stem cells may be a sequence for gene editing or gene transcription suppression of a gene (target gene) present in the mesenchymal stem cells. The target gene is not particularly limited, but examples thereof include MHC-I (or HLA-I), MHC-II, B2M, a gene related to a self-antigen, and a cell death-related gene (CASP3, CASP6, CASP7, and UCHL1).

The nucleic acid contained in the nucleic acid delivery agent to mesenchymal stem cells may be a sequence for expressing an exogenous gene that enhances the function of the mesenchymal stem cells, a sequence for transcriptionally activating a gene (target gene) present in the mesenchymal stem cells, or a sequence for regulating the function and proliferation of cells around the mesenchymal stem cells. The exogenous gene or the target gene for transcriptional activation is not particularly limited, but examples thereof include a growth factor (bFGF, EGF, NGF, BDNF, sonic hedgehog (SHH), neurotrophic tyrosine kinase receptor 1 (NTRK1)), a transcription factor (STAT3, SOX9, nuclear receptor-related factor 1 (Nurr1), neurotrophic tyrosine kinase receptor 1 (NTRK1), achaete-scute family bHLH transcription factor 1 (ASCL1), conserved dopamine neurotrophic factor (CDNF), neurogenin 1, Txnip, Vcam1, AABR07054614.1, Aldh1a3, and Cox4i2), a nutrient factor (BDNF, GDNF, NGF, NT-3, HGF, and VEGF), a gene related to cell proliferation and survival factors (a gene related to Notch signal transduction, Wnt/β-catenin signal transduction, and BMP signal transduction, for example, TCF1-4, Wnt1, Wnt2, Wnt-3a, Wnt-5a, NT-3, Ngn1, and Ngn2), a factor related to immune regulation (cytokines such as IL-1Ra, IL-10, prostaglandin E2 (PGE2), TSG-6, monocyte chemoattractant protein-1 (MCP-1/CCL2), and TGF-β, indoleamine pyrrole 2,3-dioxygenase (IDO), human leukocyte antigen (HLA)-G5, and IFNγ), an anti-apoptotic gene (mitochondrial rho GTPase 1, H2AX (Y142F), superoxide dismutase 2 (SOD2), Bcl-2, adrenal cortex medullin (ADM), SOD-1, SOD-3, and glutathione peroxidase-1 (GPx)), and others (a sequence encoding TrkC, Snail, Shh, Nice4, as-miR-383, miR-381, BRD4, WNT5A, CNTF, miR-214, miR-21, miR-145-5p, GIT1, KCC2, miR-146a-5p, miR-31, CXCR4, miR-138-5p, ERK1/2, HGF, NGR1, Zeb2, Axin2, TSP4, ITGA4, HIF-1α, CCR2, CCL2, EGFL7, PSP, miR-145, ZFAS1, miR-455-5p, VIP, VEGF-A, Sirtuin 1, Netrin 1, KLF7, CDNF, GDNF, Persephin, Nurr1, FGF8, NTRK1, α-syn, miR-188-3p, as-miR-937, FOXQ-1, and Lin28B).

As the delivery agent to nerve cells, a nucleic acid delivery agent to nerve cells, which contains a lipid composition containing an ionizable lipid which is a compound represented by Formula (1) or Formula (2) or a salt thereof, a non-ionizable lipid, a lipid having a nonionic polymer, and a nucleic acid, is preferable. Formula (1) and Formula (2) are the same as the above-described definitions.

The nucleic acid contained in the nucleic acid delivery agent to nerve cells may be a sequence for gene editing or gene expression suppression of a gene (target gene) present in the nerve cells. The target gene is not particularly limited, but examples thereof include a central nervous system disease-related gene (BACE1-AS, APP, Tau, VDAC1, BACE1, presenilin 1 (PS1), ROCK-II, mutant presenilin 1 (L392V PS-1), 12 PP-2A, ACAT-1, Nogo receptor, α-synuclein (SNCA), Htt, GFAP, Vimentin, EphB3, iNOS, Nischarin, RhoA, T-bet, Notch1, LINGO-1, NR4A2, TRIF, caspase-2, and CaMKII), MHC-I (or HLA-I), MHC-II, B2M, a gene related to a self-antigen, and a cell death-related gene (CASP3, CASP6, CASP7, and UCHL1).

The nucleic acid contained in the nucleic acid delivery agent to nerve cells may be a sequence for expressing an exogenous gene that enhances the function of the nerve cells, or a sequence for transcriptionally activating a gene (endogenous gene) present in the nerve cells. The exogenous gene or the target gene for transcriptional activation is not particularly limited, but examples thereof include a survival promoting factor (Hif1a, Akt1, Bcl-2, Bcl-xl, and the like) and a sequence encoding the above-described central nervous system disease-related gene.

### <Use>

According to the present invention, the nucleic acid delivery agent for an immune cell according to the embodiment of the present invention can be used for a pharmaceutical use. In a case of being used for a pharmaceutical use, the nucleic acid delivery agent according to the embodiment of the present invention can be administered to a living body alone or in a mixture with a pharmaceutically acceptable carrier. In addition, in a case of being used for a pharmaceutical use, a route of administration in a case of administering the nucleic acid delivery agent is not particularly limited, and the nucleic acid delivery agent can be administered by any method.

In order to further deliver the nucleic acid delivery agent according to the embodiment of the present invention to the immune cell, a molecule (hereinafter, also referred to as a target molecule) targeting the immune cell may be bonded to the surface of the lipid composition. The target molecule is not particularly limited, and a low-molecular-weight molecule, a peptide, a nucleic acid, or an antibody can be used.

In addition, in a case where the target molecule is bonded to the surface of the lipid composition, the lipid composition may contain a lipid having a modification group for chemical or electrical bonding between the lipid composition and the target molecule. Examples of the lipid having the modification group include a lipid having a maleimide group and a polyethylene glycol chain.

### <Kits and uses thereof>

According to the present invention, there is provided a kit for delivering a nucleic acid to a cell, the kit including the following reagents (A) to (C).
(A) A lipid composition containing an ionizable lipid that is a compound represented by Formula (1) or Formula (2) or a salt thereof, a non-ionizable lipid, and a lipid having a nonionic polymer;
(B) a pH adjusting agent; and
(C) an apolipoprotein.

The description and the preferred aspects of the ionizable lipid which is a compound represented by Formula (1) or Formula (2) or a salt thereof, the non-ionized lipid, and the lipid having a nonionic polymer are as described above.

As the pH adjusting agent, a buffer solution (for example, a citrate buffer solution, a citrate buffer physiological saline, an acetate buffer solution, an acetate buffer physiological saline, a phosphate buffer physiological saline, a tris buffer solution, an MES buffer solution, or an HEPES buffer solution) may be used. In addition, for the purpose of adjusting the salt strength or the osmotic pressure, sodium chloride, potassium chloride, sucrose, trehalose, fructose, mannitol, or the like may be contained, and a buffer solution obtained by further adding these additives to the above-described buffer solution can be used.

The apolipoprotein is as described above.

According to the present invention, there is further provided a method for delivering a nucleic acid to a cell, using the above-described kit of the present invention, the method including the following steps (1) to (4).
(1) A step of mixing the reagent (A) with a nucleic acid;
(2) a step of adjusting a pH of the mixture obtained in the step (1) with the reagent (B),
(3) a step of adding the reagent (C) to a culture medium containing a cell, and
(4) a step of adding the mixture obtained in the step (2) to the culture medium obtained in the step (3).

The cell is preferably an immune cell, and the preferred immune cell is as described above.

Next, the present invention will be described based on examples, but the present invention is not limited thereto.

### Examples

Unless otherwise specified, for the purification by column chromatography, an automatic purification device ISOLERA (Biotage), a medium pressure separation and purification device Purif-espoir-2 (Shoko Science Co., Ltd.), or a medium pressure liquid chromatograph YFLC W-prep 2XY (Yamazen Corporation) was used.

Unless otherwise specified, as a carrier for silica gel column chromatography, Chromatorex Q-Pack SI 50 (FUJI SILYSIA CHEMICAL LTD.) or HIGH FLASH COLUMN WO01, WO02, WO03, WO04, or WO05 (Yamazen Corporation) was used.

As an NH silica gel, Chromatorex Q-Pack NH 60 (FUJI SILYSIA CHEMICAL LTD.) was used.

NMR spectra were measured using a Bruker AVNEO400 (manufactured by Bruker Corporation) and using tetramethylsilane as an internal standard, and all δ values were shown in ppm.

MS spectra were measured using an ACQUITY SQD LC/MS System (manufactured by WATERS).

### [Synthesis Example 1]

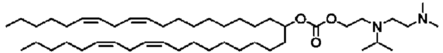

2-((2-(Dimethylamino)ethyl)(isopropyl)amino)ethyl((6Z,9Z,28Z,31Z)-heptatriaconta-6,9,28,3 1-tetraen-19-yl)carbonate (compound 1) was synthesized according to Examples described in WO2019/235635A.

### [Synthesis Example 2]

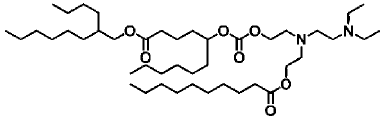

2-Butyloctyl 6-(2-(decanoyloxy)ethyl)-3-ethyl-12-hexyl-10-oxo-9,11-dioxa-3,6-diazahexadecan-16-oate (compound 2) was synthesized according to Examples described in WO2019/235635A.

### [Synthesis Example 3]

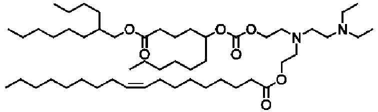

2-Butyloctyl 3-ethyl-12-hexyl-6-(2-(oleoyloxy)ethyl)-10-oxo-9,11-dioxa-3,6-diazahexadecan-16-oate (compound 3) was synthesized according to Examples described in WO2019/235635A.

### [Synthesis Example 4]

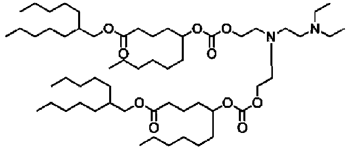

Bis(2-pentylheptyl) 11-(2-(diethylamino)ethyl)-5,17-dihexyl-7,15-dioxo-6,8,14,16-tetraoxa-11-azahenicosanedioat e (compound 4) was synthesized according to Examples described in WO2022/230964A.

### [Synthesis Example 5]

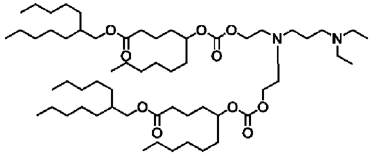

Bis(2-pentylheptyl) 11-(3-(diethylamino)propyl)-5,17-dihexyl-7,15-dioxo-6,8,14,16-tetraoxa-11-azahenicosanedio ate (compound 5) was synthesized according to Examples described in WO2022/230964A.

### [Synthesis Example 6]

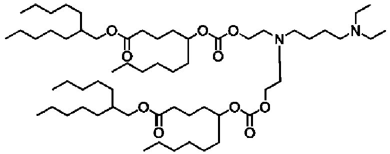

Bis(2-pentylheptyl) 11-(4-(diethylamino)butyl)-5,17-dihexyl-7,15-dioxo-6,8,14,16-tetraoxa-11-azahenicosanedioat e (compound 6) was synthesized according to Examples described in WO2022/230964A.

### [Synthesis Example 7]

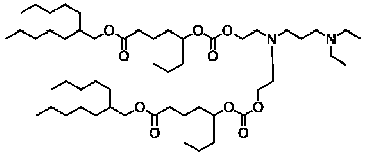

Bis(2-pentylheptyl) 11-(3-(diethylamino)propyl)-7,15-dioxo-5,17-dipropyl-6,8,14,16-tetraoxa-11-azahenicosanedi oate (compound 7) was synthesized according to Examples described in WO2022/230964A.

### [Synthesis Example 8]

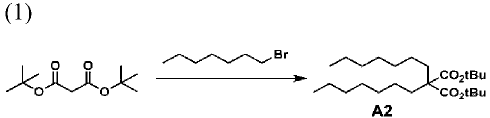

6.6 g of powdered potassium hydroxide was added to a mixture of 50 mL of dimethyl sulfoxide and 30 mL of toluene, 10 mL of a toluene solution of 10 g of di-tert-butyl malonate and 10 mL of a toluene solution of 19.9 g of 1-bromoheptane were added thereto under ice cooling, and the mixture was stirred overnight at 30°C or lower. The reaction mixture was stirred in an ice bath, hydrochloric acid was added thereto for neutralization, and then the organic layer was isolated. The obtained organic layer was washed with water and saturated saline, anhydrous sodium sulfate was added thereto for drying, and the solvent was distilled off under reduced pressure, thereby obtaining 22.9 g of di-tert-butyl 2,2-diheptylmalonate (A2) as a light yellow oily substance.

¹H-NMR (CDCl₃) δ: 1.79-1.74 (4H, m), 1.44 (18H, s), 1.32-1.10 (20H, m), 0.87 (6H, t, J = 6.8 Hz).

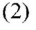

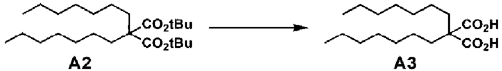

45 mL of trifluoroacetic acid was added to a mixture of 22.9 g of di-tert-butyl 2,2-diheptylmalonate (A2), 23 mL of toluene, and 2.3 mL of water, the mixture was stirred at room temperature for 2 hours, and the solvent was distilled off under reduced pressure. 23 mL of hexane was added to the residue, the mixture was stirred under ice cooling, and then the resulting solid was collected by filtration. The obtained solid matter was dried to obtain 11.7 g of 2,2-diheptylmalonic acid (A3) as a white solid.

¹H-NMR (CDCl₃) δ: 1.97-1.92 (4H, m), 1.32-1.17 (20H, m), 0.87 (6H, t, J = 7.3 Hz).

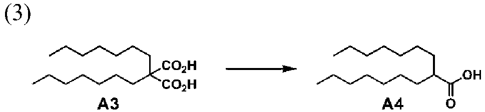

11.7 g of 2,2-diheptylmalonic acid (A3) was stirred at 165°C for 3 hours to obtain 11.7 g of 2-heptylnonanoic acid (A4) as a light yellow oily substance.

¹H-NMR (CDCl₃) δ: 2.39-2.32 (1H, m), 1.96-1.93 (1H, m), 1.68-1.41 (4H, m), 1.36-1.16 (20H, m), 0.89-0.85 (6H, m).

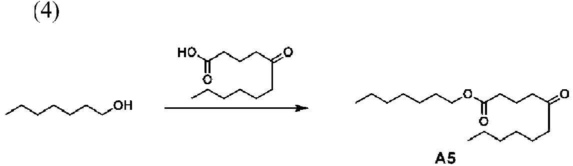

2.9 mL of 1-heptanol and 170 mg of 4-toluenesulfonic acid monohydrate were added to a 40 mL toluene solution of 4.0 g of 5-oxoundecanoic acid synthesized according to the method described in WO2019/235635A, and the mixture was stirred under heating and reflux for 5 hours. The solvent of the reaction mixture was distilled off under reduced pressure, and the residue was purified by NH silica gel column chromatography (hexane-ethyl acetate) to obtain 5.8 g of heptyl 5-oxoundecanoate (A5) as a light yellow oily substance.

¹H-NMR (CDCl₃) δ: 4.05 (2H, t, J = 6.7 Hz), 2.46 (2H, t, J = 7.2 Hz), 2.38 (2H, t, J = 7.5 Hz), 2.32 (2H, t, J = 7.2 Hz), 1.93-1.83 (2H, m), 1.66-1.49 (4H, m), 1.37-1.20 (14H, m), 0.91-0.84 (6H, m).

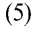

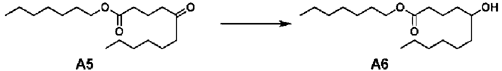

0.60 g of sodium borohydride was added to a mixed solution of 15 mL of tetrahydrofuran and 15 mL of methanol of 3.0 g of heptyl 5-oxoundecanoate (A5) under ice cooling, and the mixture was stirred under ice cooling for 2 hours. The reaction mixture was added dropwise to ice water, and a 1N hydrochloric acid aqueous solution was added thereto until the solution became acidic. After adding ethyl acetate thereto, the organic layer was isolated. The organic layer was washed with saturated saline, and anhydrous sodium sulfate was then added thereto to dry the organic layer, and the solvent was distilled off under reduced pressure. The obtained residue was purified by silica gel column chromatography (hexane-ethyl acetate) to obtain 3.0 g of heptyl 5-hydroxyundecanoate (A6) as a colorless oily substance.

¹H-NMR (CDCl₃) δ: 4.06 (2H, t, J = 6.7 Hz), 3.63-3.54 (1H, m), 2.33 (2H, t, J = 7.2 Hz), 1.84-1.21 (24H, m), 0.93-0.84 (6H, m).

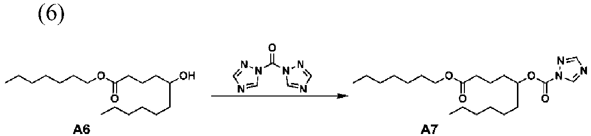

2.5 g of 1,1'-carbonyldi(1,2,4-triazole) was added to a 30 mL tetrahydrofuran solution of 3.0 g of heptyl 5-hydroxyundecanoate (A6), and the mixture was stirred at room temperature overnight. Ethyl acetate and water were added to the reaction mixture, and the organic layer was isolated. The organic layer was washed with saturated saline, and anhydrous sodium sulfate was then added thereto to dry the organic layer, and the solvent was distilled off under reduced pressure. The obtained residue was purified by silica gel column chromatography (hexane-ethyl acetate) to obtain 4.0 g of 1-(heptyloxy)-1-oxaundecan-5-yl 1H-1,2,4-triazole-1-carboxylate (A7) as a colorless oily substance.

¹H-NMR (CDCl₃) δ: 8.82 (1H, s), 8.07 (1H, s), 5.22-5.13 (1H, m), 4.05 (2H, t, J = 6.7 Hz), 2.35 (2H, t, J = 6.9 Hz), 1.87-1.67 (6H, m), 1.65-1.50 (2H, m), 1.43-1.19 (16H, m), 0.93-0.79 (6H, m).

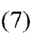

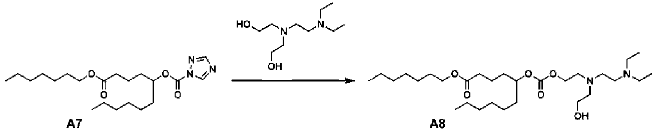

3.9 g of 2,2'-((2-(diethylamino)ethyl)azanediyl)bis(ethan-1-ol) and 3.4 mL of 1,8-diazabicyclo[5.4.0]-7-undecene were added to a 30 mL acetonitrile solution of 3.0 g of 1-(heptyloxy)-1-oxaundecan-5-yl 1H-1,2,4-triazole-1-carboxylate (A7), and the mixture was stirred at 50°C for 3 hours. Ethyl acetate and water were added to the reaction mixture, and the organic layer was isolated. The organic layer was washed with saturated saline, and anhydrous sodium sulfate was then added thereto to dry the organic layer, and the solvent was distilled off under reduced pressure. The obtained residue was purified by silica gel column chromatography (methanol-ethyl acetate), and then purified by NH silica gel column chromatography (hexane-ethyl acetate) to obtain 2.6 g of heptyl 3-ethyl-12-hexyl-6-(2-hydroxyethyl)-10-oxo-9,11-dioxa-3,6-diazahexadecan-16-oate (A8) as a colorless oily substance.

¹H-NMR (CDCl₃) δ: 4.74-4.65 (1H, m), 4.23-4.16 (2H, m), 4.05 (2H, t, J = 6.7 Hz), 3.57-3.50 (2H, m), 2.88 (2H, t, J = 6.1 Hz), 2.73-2.62 (4H, m), 2.59-2.50 (4H, m), 2.47 (2H, t, J = 5.9 Hz), 2.31 (2H, t, J = 7.0 Hz), 1.72-1.47 (8H, m), 1.38-1.20 (16H, m), 1.02 (6H, t, J = 7.1 Hz), 0.92-0.82 (6H, m).

MS m/z (M+H): 532.

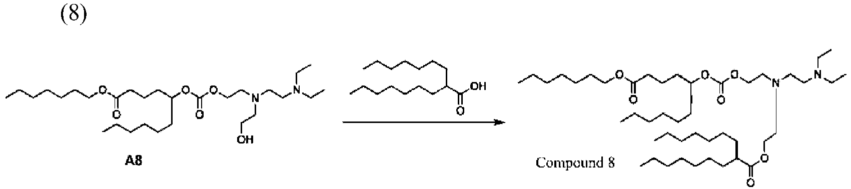

145 mg of 2-heptylnonanoic acid (A4), 147 mg of 1-ethyl-3-(3-dimethylaminopropyl)carbodiimide hydrochloride, 138 mg of 4-dimethylaminopyridine, and 0.3 mL of triethylamine were added to a 2 mL dichloromethane solution of 200 mg of heptyl 3-ethyl-12-hexyl-6-(2-hydroxyethyl)-10-oxo-9,11-dioxa-3,6-diazahexadecan-16-oate (A8), and the mixture was stirred at room temperature overnight. The solvent was distilled off from the reaction mixture under reduced pressure, ethyl acetate and water were added thereto, and the organic layer was isolated. The organic layer was washed with saturated saline, and anhydrous sodium sulfate was then added thereto to dry the organic layer, and the solvent was distilled off under reduced pressure. The obtained residue was purified by silica gel column chromatography (methanol-ethyl acetate), and then purified by NH silica gel column chromatography (hexane-ethyl acetate) to obtain 224 mg of heptyl 3-ethyl-6-(2-((2-heptylnonanoyl)oxy)ethyl)-12-hexyl-10-oxo-9,11-dioxa-3,6-diazahexadecan-16-oate (compound 8) as a colorless oily substance.

¹H-NMR (CDCl₃) δ:4.72-4.64 (1H, m), 4.20-4.10 (4H, m), 4.05 (2H, t, J = 6.7 Hz), 2.88-2.77 (4H, m), 2.72-2.62 (2H, m), 2.58-2.46 (6H, m), 2.36-2.25 (3H, m), 1.73-1.18 (48H, m), 1.01 (6H, t, J = 7.1 Hz), 0.92-0.82 (12H, m).

MS m/z (M + H): 770.

### [Synthesis Example 9]

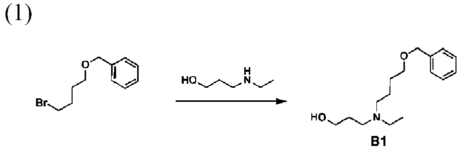

5.5 g of ((4-bromobutoxy)methyl)benzene and 8.0 g of potassium carbonate were added to a 40 mL ethanol solution of 2.0 g of 3-(ethylamino)propan-1-ol, and the mixture was stirred at room temperature for 15 minutes and then stirred under heating and reflux for 6 hours. The insoluble matter of the reaction mixture was filtered off, and the filtrate was distilled off under reduced pressure. The obtained residue was purified by NH silica gel column chromatography (hexane-ethyl acetate) to obtain 3.6 g of 3-((4-(benzyloxy)butyl)(ethyl)amino)propan-1-ol (B1) as a colorless oily substance.

MS m/z (M + H): 266.

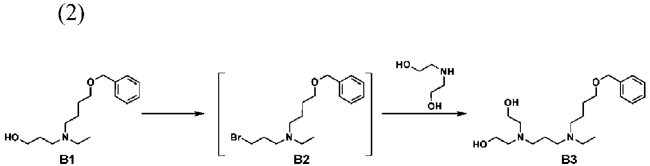

2.5 g of carbon tetrabromide was added to a 18 mL tetrahydrofuran solution of 1.0 g of 3-((4-(benzyloxy)butyl)(ethyl)amino)propan-1-ol (B1), a 2 mL tetrahydrofuran solution of 2.0 g of triphenylphosphine was added dropwise thereto under ice cooling, and the mixture was stirred at room temperature for 2 hours to obtain a mixture of 4-(benzyloxy)-N-(3-bromopropyl)-N-ethylbutan-1-amine (B2). 50 mL of ethanol, 0.80 g of diethanolamine, and 1.6 g of potassium carbonate were added to the obtained reaction mixture, and the mixture was stirred at room temperature for 15 minutes and then stirred under heating and reflux for 3 hours. The insoluble matter of the reaction mixture was filtered off, and the filtrate was distilled off under reduced pressure. The obtained residue was purified by NH silica gel column chromatography (hexane-ethyl acetate) to obtain 0.41 g of 2,2'-((3-((4-(benzyloxy)butyl)(ethyl)amino)propyl)azanediyl)bis(ethan-1-ol) (B3) as a light yellow oily substance.

MS m/z (M + H): 353.

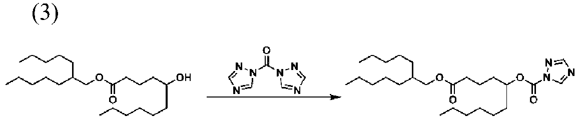

2.1 g of 1,1'-carbonyldi(1,2,4-triazole) was added to a 30 mL tetrahydrofuran solution of 3.0 g of 2-pentylheptyl 5-hydroxyundecanoate described in WO2021/095876A, and the mixture was stirred at room temperature overnight. Water and ethyl acetate were added to the reaction mixture, and the organic layer was separated. The organic layer was dried over anhydrous sodium sulfate, and the solvent was distilled off under reduced pressure. The residue was purified by silica gel column chromatography (hexane-ethyl acetate) to obtain 4.3 g of 1-oxo-1-((2-pentylheptyl)oxy)undecan-5-yl 1H-1,2,4-triazole-1-carboxylate as a colorless oily substance.

¹H-NMR (CDCl₃) δ: 8.82 (1H, s), 8.07 (1H, s), 5.21-5.14 (1H, m), 3.97 (2H, d, J = 5.6 Hz), 2.36 (2H, t, J = 7.2 Hz), 1.88-1.55 (7H, m), 1.40-1.22 (24H, m), 0.91-0.85 (9H, m).

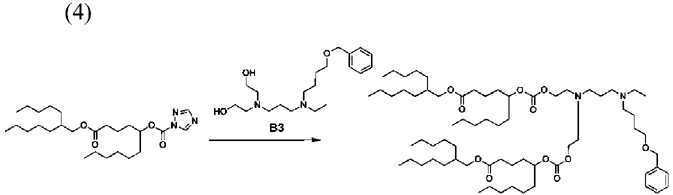

6 mL of acetonitrile, 0.41 g of 2,2'-((3-((4-(benzyloxy)butyl)(ethyl)amino)propyl)azanediyl)bis(ethan-1-ol) (B3), and 0.54 g of 1,8-diazabicyclo[5.4.0]-7-undecene were added to 0.55 g of 1-oxo-1-((2-pentylheptyl)oxy)undecan-5-yl 1H-1,2,4-triazole-1-carboxylate, and the mixture was stirred at 55°C for 4 hours. The reaction mixture was cooled to 30°C, ethyl acetate and water were added thereto, and the organic layer was separated. The organic layer was dried over anhydrous sodium sulfate, and then the solvent was distilled off under reduced pressure. The obtained residue was purified by silica gel column chromatography (ethyl acetate-methanol-triethylamine) and then purified by NH silica gel column chromatography (hexane-ethyl acetate) to obtain 0.25 g of bis(2-pentylheptyl) 11-((3-((4-(benzyloxy)butyl)(ethyl)amino)propyl)-5,17-dihexyl-7,15-dioxo-6,8,14,16-tetraoxa -11-azahenicosanedioate as a colorless oily substance.

MS m/z (M + H): 1146.

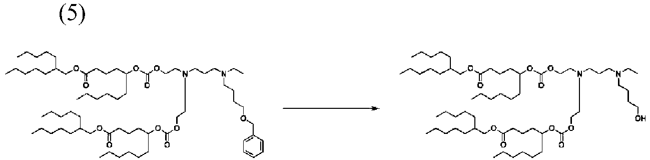

57 mg of 10% palladium on carbon (55% water-wet product) and 105 mg of ammonium formate were added to a 4 mL methanol solution of 0.25 g of bis(2-pentylheptyl) 11-((3-((4-(benzyloxy)butyl)(ethyl)amino)propyl)-5,17-dihexyl-7,15-dioxo-6,8,14,16-tetraoxa -11-azahenicosanedioate, and the mixture was stirred under reflux for 15 hours. The insoluble matter was removed by filtration through Celite, and the filtrate was distilled off under reduced pressure. The obtained residue was purified by NH silica gel column chromatography (hexane-ethyl acetate) to obtain 95 mg of bis(2-pentylheptyl) 11-((3-(ethyl(4-hydroxybutyl)amino)propyl)-5,17-dihexyl-7,15-dioxo-6,8,14,16-tetraoxa-11-a zahenicosanedioate (compound 9) as a colorless oily substance.

¹H-NMR (CDCl₃) δ:4.74-4.63 (2H, m), 4.22-4.08 (4H, m), 3.97 (4H, d, J = 5.8 Hz), 3.60-3.48 (2H, m), 2.80 (4H, t, J = 6.4 Hz), 2.60-2.45 (6H, m), 2.44-2.37 (2H, m), 2.32 (4H, t, J = 7.5 Hz), 1.74-1.47 (21H, m), 1.38-1.19 (48H, m), 1.10-1.00 (3H, m), 0.93-0.80 (18H, m).

MS m/z (M + H): 1056.

### [Synthesis Example 10]

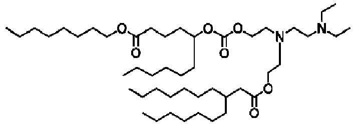

Octyl 3-ethyl-6-(2-((3-heptyldecanoyl)oxy)ethyl)-12-hexyl-10-oxo-9,11-dioxa-3,6-diazahexadecane-16-oate (compound 10) was synthesized by the same method as in Synthesis Examples 8 (4) to (8), except that 1-octanol was used instead of 1-heptanol in Synthesis Example 8 (4) and 3-heptyldecanoic acid described in WO2019/235635 was used instead of 2-heptylnonanoic acid (A4) in Synthesis Example 8 (8).

¹H-NMR (CDCl₃) δ:4.73-4.64 (1H, m), 4.20-4.09 (4H, m), 4.05 (2H, t, J = 6.7 Hz), 2.89-2.77 (4H, m), 2.73-2.42 (8H, m), 2.31 (2H, t, J = 7.0 Hz), 2.22 (2H, d, J = 6.8 Hz), 1.88-1.77 (1H, m), 1.71-1.50 (8H, m), 1.38-1.19 (42H, m), 1.10-0.96 (6H, m), 0.92-0.83 (12H, m).

S m/z (M+H): 798.

### [Synthesis Example 11]

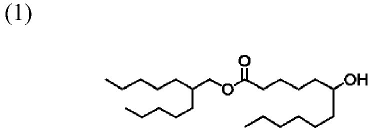

2-Pentylheptyl 6-hydroxydodecanoate was obtained in the same manner as in Example 84 (1) described in WO2019/235635, except that adipic acid monomethyl was used instead of 10-methoxy-10-oxodecanoic acid and 2-pentylheptan-1-ol was used instead of 2-butyl-1-octanol.

¹H-NMR (CDCl₃) δ:3.97 (2H, d, J = 5.8 Hz), 3.59 (1H, m), 2.32 (2H, t, J = 7.4 Hz), 1.79-1.19 (34H, m), 0.92-0.83 (9H, m).

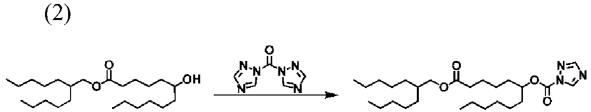

0.73 g of 1,1'-carbonyldi(1,2,4-triazole) was added to a solution of 1.1 g of 2-pentylheptyl 6-hydroxydodecanoate in 11 mL of tetrahydrofuran, and the mixture was stirred at 30°C for 4 hours. Ethyl acetate and water were added to the reaction mixture, and the organic layer was isolated. The organic layer was washed with saturated saline, and anhydrous sodium sulfate was then added thereto to dry the organic layer, and the solvent was distilled off under reduced pressure. The obtained residue was purified by silica gel column chromatography (hexane-ethyl acetate), thereby obtaining 1.2 g of 1-oxo-1-((2-pentylheptyl)oxy)dodecan-6-yl 1H-1,2,4-triazole-1-carboxylate as a colorless oily substance.

¹H-NMR (CDCl₃) δ: 8.81 (1H,s), 8.07 (1H, s), 5.20-5.12 (1H, m), 3.95 (2H, d, J = 5.8 Hz), 2.31 (2H, t, J = 7.42 Hz), 1.87-1.52 (7H, m), 1.48-1.19 (26H, m), 0.92-0.83 (9H, m).

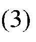

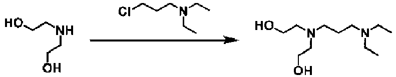

4.6 g of 3-chloro-N,N-diethylpropan-1-amine and 4.1 g of potassium carbonate were added to a solution of 2.5 g of diethanolamine in 25 mL of ethanol, and the mixture was stirred at room temperature for 15 minutes and then stirred under heating and reflux for 2 hours. 1 g of 3-chloro-N,N-diethylpropan-1-amine was added thereto, and the mixture was stirred under heating and reflux for 2 hours again. The insoluble matter of the reaction mixture was filtered off, and the filtrate was distilled off under reduced pressure. The obtained residue was purified by NH silica gel column chromatography (hexane-ethyl acetate-methanol), thereby obtaining 3.0 g of 2,2'-((3-(diethylamino)propyl)azanediyl)bis(ethan-1-ol) as a colorless oily substance.

¹H-NMR (CDCl₃) δ: 3.62 (4H, t, J = 5.2 Hz), 2.26 (2H, t, J = 6.0 Hz), 2.61-2.49 (10H, m), 1.68-1.60 (2H, m), 1.04 (6H, t, J = 7.2 Hz).

MS m/z (M + H): 219.

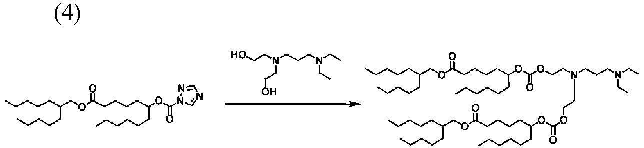

208 mL of acetonitrile, 24.4 g of 1-oxo-1-((2-pentylheptyl)oxy)dodecan-6-yl 1H-1,2,4-triazole-1-carboxylate, and 10.9 g of 1,8-diazabicyclo[5.4.0]-7-undecene were added to 5.2 g of 2,2'-((3-(diethylamino)propyl)azanediyl)bis(ethan-1-ol), and the mixture was stirred at 50°C for 2 hours. The reaction mixture was cooled to room temperature, ethyl acetate and water were added thereto, and the organic layer was separated. The organic layer was dried over anhydrous sodium sulfate, and then the solvent was distilled off under reduced pressure. The obtained residue was purified by silica gel column chromatography (ethyl acetate-methanol) and then purified by NH silica gel column chromatography (hexane-ethyl acetate), thereby obtaining 20.3 g of bis(2-pentylheptyl) 12-(3-(diethylamino)propyl)-8,16-dioxo-6,18-dipentyl-7,9,15,17-tetraoxa-12-azatricosanedioat e (compound 11) as a colorless oily substance.

¹H-NMR (CDCl₃) δ: 4.71-4.62 (2H, m), 4.22-4.08 (4H, m), 3.96 (4H, d, J = 5.8 Hz), 2.80 (4H, t, J = 6.4 Hz), 2.60-2.45 (6H, m), 2.44-2.37 (2H, m), 2.30 (4H, t, J = 7.5 Hz), 1.69-1.49 (16H, m), 1.44-1.18 (52H, m), 1.00 (6H, t, J = 7.1 Hz), 0.93-0.80 (18H, m).

MS m/z (M + H): 1040.

### [Synthesis Example 12]

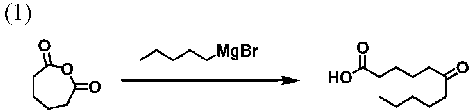

7.8 mL of a 1 mol/L pentyl magnesium bromide-tetrahydrofuran solution was added dropwise to a 50 mL tetrahydrofuran suspension of 1.0 g of adipic acid anhydride under ice cooling, and the mixture was stirred at the same temperature for 2 hours. 20 mL of a 1 mol/L hydrochloric acid aqueous solution was added to the reaction mixture under ice cooling, ethyl acetate was added thereto, and the organic layer was separated. The obtained organic layer was washed with water and a saturated sodium chloride aqueous solution, dried over anhydrous magnesium sulfate, and the solvent was distilled off under reduced pressure. The obtained residue was purified by silica gel column chromatography (hexane-ethyl acetate), thereby obtaining 0.44 g of a mixture of 6-oxoundecanoic acids.

¹H-NMR (CDCl₃) δ: 2.49-2.32 (6H, m), 1.67-1.52 (6H, m), 1.37-1.20 (4H, m), 0.89 (3H, t, J = 7.1 Hz).

MS m/z (M + H): 201.

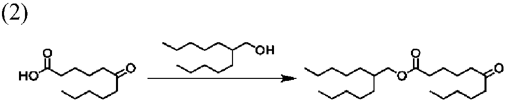

6-Oxoundecanoic acid 2-pentylheptyl as a light yellow oily substance was obtained by the same method as in Synthesis Example 8 (4), except that 6-oxoundecanoic acid was used instead of 5-oxoundecanoic acid and 2-pentylheptan-1-ol was used instead of 1-heptanol.

(3)

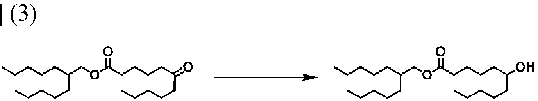

6-Hydroxyundecanoic acid 2-pentylheptyl as a colorless oily substance was obtained by the same method as in Synthesis Example 8 (5), except that 6-oxoundecanoic acid 2-pentylheptyl was used instead of 5-oxoundecanoic acid heptyl.

¹H-NMR (CDCl₃) δ: 3.97 (2H, d, J = 5.8 Hz), 3.59 (1H, m), 2.32 (2H, t, J = 7.44 Hz), 1.79-1.19 (32H, m), 0.92-0.83 (9H, m).

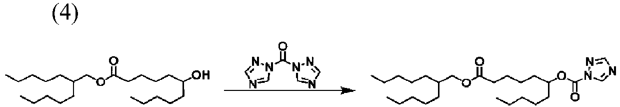

1-Oxo-1-((2-pentylheptyl)oxy)undecan-6-yl 1H-1,2,4-triazole-1-carboxylate was obtained by the same method as in Synthesis Example 11 (2).

¹H-NMR (CDCl₃) δ: 8.81 (1H, s), 8.07 (1H, s), 5.20-5.13 (1H, m), 3.95 (2H, d, J = 5.81), 2.31 (2H, t, J = 7.4 Hz), 1.90-1.51 (7H, m), 1.50-1.19 (24H, m), 0.95-0.82 (9H, m).

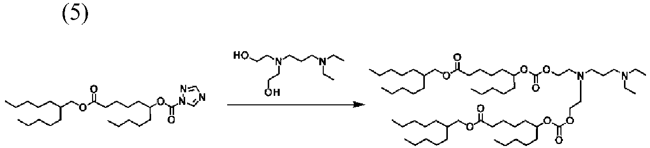

Bis(2-pentylheptyl) 12-(3-(diethylamino)propyl)-8,16-dioxo-6,18-dipentyl-7,9,15,17-tetraoxa-12-azatricosanedioat e (Compound 12) was obtained by the same method as in Synthesis Example 11 (4).

¹H-NMR (CDCl₃) δ: 4.72-4.61 (2H, m), 4.22-4.07 (4H, m), 3.96 (4H, d, J = 5.8 Hz), 2.80 (4H, t, J = 6.4 Hz), 2.59-2.45 (6H, m), 2.44-2.36 (2H, m), 2.30 (4H, t, J = 7.5 Hz), 1.70-1.48 (16H, m), 1.44-1.19 (48H, m), 1.00 (6H, t, J = 7.1 Hz), 0.92-0.81 (18H, m).

MS m/z (M + H): 1012.

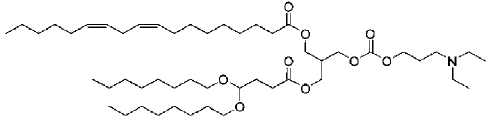

(9Z,12Z)-3-((4,4-bis(octyloxy)butanoyl)oxy)-2-((((3-(diethylamino)propoxy)carbonyl)oxy)me thyl)propyloctadeca-9,12-dienoate (Compound 13) was obtained according to Preparation of Example 13 of WO2015/095340.

### [Synthesis Example 14]

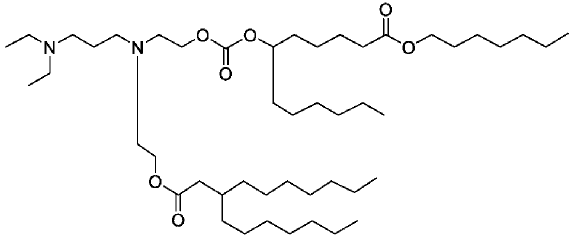

According to Examples described in WO2024/190817, heptyl 3-ethyl-7-(2-((3-heptyldecanoyl)oxy)ethyl)-13-hexyl-11-oxo-10,12-dioxa-3,7-diazaoctadecan-18-oate (Compound 14) was synthesized.

### [Synthesis Example 15]

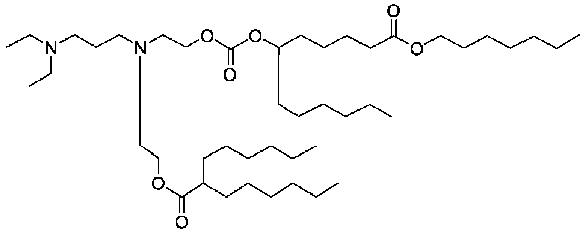

According to Examples described in WO2024/190817, heptyl 3-ethyl-13-hexyl-7-(2-((2-hexyloctanoyl)oxy)ethyl)-11-oxo-10,12-dioxa-3,7-diazaoctadecan-1 8-oate (Compound 15) was synthesized.

### [Synthesis Example 16]

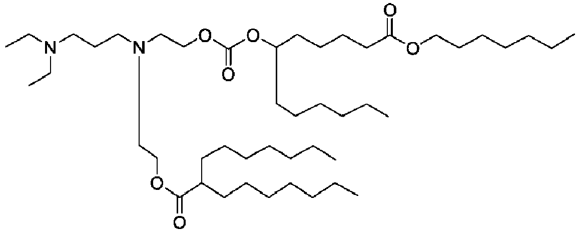

According to Examples described in WO2024/190817, heptyl 3-ethyl-7-(2-((2-heptylnonanoyl)oxy)ethyl)-13-hexyl-11-oxo-10,12-dioxa-3,7-diazaoctadecan-18-oate (Compound 16) was synthesized.

### [Synthesis Example 17]

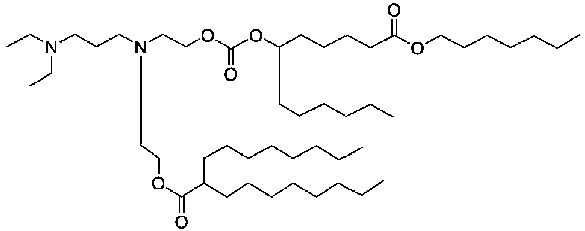

According to Examples described in WO2024/190817, heptyl 3-ethyl-13-hexyl-7-(2-((2-octyldodecanoyl)oxy)ethyl)-11-oxo-10,12-dioxa-3,7-diazaoctadecan -18-oate (Compound 17) was synthesized.

### [Synthesis Example 18]

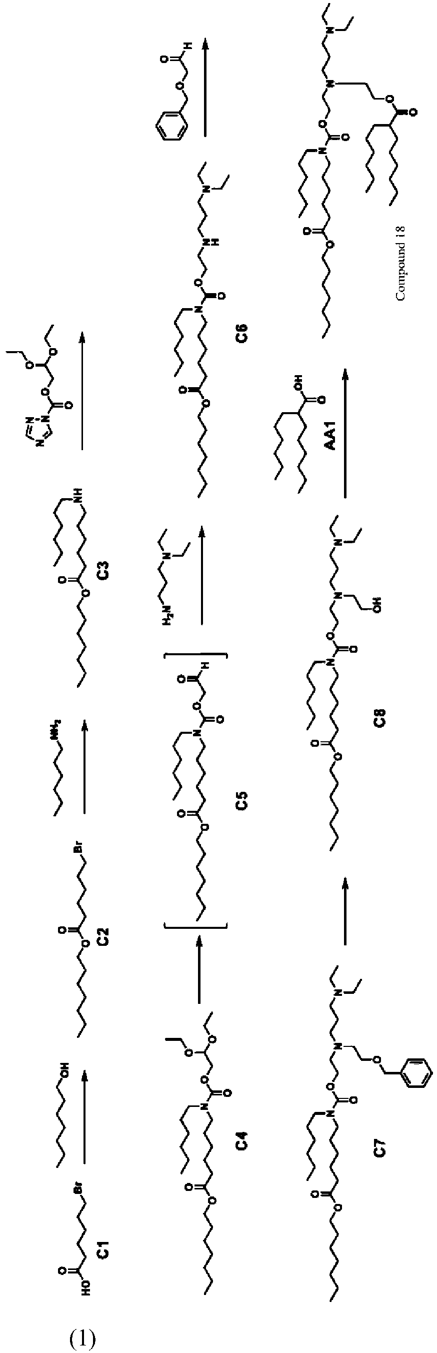

6-bromohexanoic acid (8.8 g) and 4-toluenesulfonic acid monohydrate (370 mg) were added to a toluene (50 mL) solution of 1-heptanol (5.0 g), and the mixture was stirred under heating and reflux for 6 hours. The solvent of the reaction mixture was distilled off under reduced pressure, and the residue was purified by NH silica gel column chromatography (hexane-ethyl acetate), thereby obtaining heptyl 6-bromohexanoate (C2) (12.5 g) as a light yellow oily substance.

¹H-NMR (CDCl₃) δ: 4.06 (2H, t, J = 6.7 Hz), 4.06 (2H, t, J = 6.7 Hz), 2.32 (2H, t, J = 7.4 Hz), 1.92-1.83 (2H, m), 1.72-1.56 (4H, m), 1.55-1.41 (4H, m), 1.40-1.20 (6H, m), 0.93-0.83 (3H, m).

(2)
Potassium carbonate (4.2 g) was added to a mixture of heptyl 6-bromohexanoate (C2) (3.0 g), n-hexylamine (4.1 mL), and acetonitrile (30 mL), and the mixture was stirred at 60°C for 4 hours. The reaction mixture was cooled to room temperature, and then the solvent was distilled off under reduced pressure. Ethyl acetate and water were added to the residue, and the organic layer was separated. The organic layer was washed with saturated saline, and anhydrous sodium sulfate was then added thereto to dry the organic layer, and the solvent was distilled off under reduced pressure. The obtained residue was purified by silica gel column chromatography (methanol-ethyl acetate), and then purified by NH silica gel column chromatography (ethyl acetate-hexane), thereby obtaining heptyl 6-(hexylamino)hexanoate (C3) (2.5 g) as a colorless oily substance.

¹H-NMR (CDCl₃) δ: 4.05 (2H, t, J = 6.7 Hz), 2.63-2.54 (4H, m), 2.30 (2H, t, J = 7.5 Hz), 1.70-1.56 (4H, m), 1.55-1.41 (4H, m), 1.40-1.20 (17H, m), 0.93-0.84 (6H, m).

(3)
A mixture of heptyl 6-(hexylamino)hexanoate (C3) (2.5 g), 2,2-diethoxyethyl 1H-1,2,4-triazole-1-carboxylate (2.2 g), acetonitrile (25 mL), and triethylamine (2.2 mL) was stirred at 50°C for 4 hours. The mixture was cooled to room temperature, and then the solvent was distilled off under reduced pressure. Ethyl acetate (20 mL) and water (20 mL) were added to the residue, and the organic layer was separated. The obtained organic layer was washed with saturated saline, anhydrous sodium sulfate was added thereto to dry the organic layer, and the solvent was distilled off under reduced pressure. The obtained residue was purified by silica gel column chromatography (ethyl acetate-hexane), thereby obtaining heptyl 6-(((2,2-diethoxyethoxy)carbonyl)(hexyl)amino)hexanoate (C4) (3.9 g) as a colorless oily substance.

¹H-NMR (CDCl₃) δ: 4.72-4.67 (1H, m), 4.17-4.02 (4H, m), 3.77-3.51 (4H, m), 3.26-3.12 (4H, m), 2.30 (2H, t, J = 7.5 Hz), 1.70-1.45 (10H, m), 1.37-1.17 (20H, m), 0.93-0.84 (6H, m).

(4)
A mixture of heptyl 6-(((2,2-diethoxyethoxy)carbonyl)(hexyl)amino)hexanoate (C4) (2.0 g), formic acid (8 mL), and water (2 mL) was stirred at 50°C for 2 hours, and then toluene was added thereto, followed by distillation under reduced pressure. The operation of adding toluene again and distilling off under reduced pressure was repeated twice to obtain heptyl 6-(hexyl((2-oxoethoxy)carbonyl)amino)hexanoate (C5) (1.67 g) as a crude product.

¹H-NMR (CDCl₃) δ: 9.63 (1H, s), 4.61 (2H, s), 4.06 (2H, t, J = 6.7 Hz), 3.31-3.11 (4H, m), 2.31 (2H, t, J = 7.3 Hz), 1.72-1.42 (8H, m), 1.40-1.20 (14H, m), 0.94-0.83 (6H, m).

(5)
N,N-Diethyl-1,3-diaminopropane (1.3 mL), acetic acid (0.1 mL), and sodium triacetoxyborohydride (2.5 g) were added to a solution of heptyl 6-hexyl((2-oxoethoxy)carbonyl)amino)hexanoate (C5) (1.6 g) in ethyl acetate (16 mL) at room temperature, and the mixture was stirred at room temperature for 4 hours. A saturated aqueous solution of sodium hydrogen carbonate was added to the reaction mixture, and then the organic layer was separated and washed with saturated saline. Anhydrous sodium sulfate was added thereto for drying, and the solvent was distilled off under reduced pressure. The obtained residue was purified by NH silica gel column chromatography (ethyl acetate-hexane), thereby obtaining heptyl 3-ethyl-12-hexyl-11-oxo-10-oxa-3,7,12-triazaoctadecane-18-oate (C6) (1.26 g) as a colorless oily substance.

¹H-NMR (CDCl₃) δ: 4.17 (2H, t, J = 5.6 Hz), 4.05 (2H, t, J = 6.8 Hz), 3.27-3.09 (4H, m), 2.85 (2H, t, J = 5.6 Hz), 2.67 (2H, t, J = 7.0 Hz), 2.55-2.43 (6H, m), 2.30 (2H, t, J = 7.5 Hz), 1.73-1.43 (10H, m), 1.38-1.20 (16H, m), 1.01 (6H, t, J = 7.1 Hz), 0.93-0.83 (6H, m).

MS m/z: 515 (M+H)⁺

(6)
2-(Benzyloxy)acetaldehyde (0.58 g) and sodium triacetoxyborohydride (1.04 g) were added to a solution of heptyl 3-ethyl-12-hexyl-11-oxo-10-oxa-3,7,12-triazaoctadecane-18-oate (C6) (1.26 g) in ethyl acetate (13 mL) at room temperature, and the mixture was stirred at room temperature for 4 hours. Ethyl acetate and a saturated aqueous solution of sodium hydrogen carbonate were added to the reaction mixture, and then the organic layer was separated and washed with saturated saline. Anhydrous sodium sulfate was added to the organic layer for drying, and the solvent was distilled off under reduced pressure. The obtained residue was purified by NH silica gel column chromatography (ethyl acetate-hexane), thereby obtaining heptyl 7-(2-(benzyloxy)ethyl)-3-ethyl-12-hexyl-11-oxo-10-oxa-3,7,12-triazaoctadecane-18-oate (C7) (1.44 g) as a colorless oily substance.

¹H-NMR (CDCl₃) δ: 7.36-7.22 (5H, m), 4.51 (2H, s), 4.11 (2H, t, J = 6.0 Hz), 4.05 (2H, t, J = 6.8 Hz), 3.54 (2H, t, J = 6.2 Hz), 3.25-3.08 (4H, m), 2.81-2.72 (4H, m), 2.59-2.45 (6H, m), 2.43-2.36 (2H, m), 2.33-2.24 (2H, m), 1.70-1.41 (10H, m), 1.39-1.18 (16H, m), 1.00 (6H, t, J = 7.2 Hz), 0.92-0.84 (6H, m).

MS m/z: 515 (M+H)⁺

(7)
Heptyl 7-(2-(benzyloxy)ethyl)-3-ethyl-12-hexyl-11-oxo-10-oxa-3,7,12-triazaoctadecane-18-oate (C7) (1.44 g), methanol (44 mL), 10% palladium/carbon (about 55% water-wetted product) (0.43 g), and ammonium formate (1.4 g) were mixed and stirred under heating and reflux for 2 hours. 10% palladium/carbon (0.43 g) and ammonium formate (1.4 g) were added thereto again, and the mixture was stirred under heating and reflux for 2 hours. The mixture was cooled to room temperature, insoluble matter was filtered off by celite filtration, and then the solvent was distilled off under reduced pressure. The obtained residue was purified by NH silica gel column chromatography (methanol-ethyl acetate-hexane), thereby obtaining heptyl 3-ethyl-12-hexyl-7-(2-hydroxyethyl)-11-oxo-10-oxa-3,7,12-triazaoctadecane-18-oate (C8) (1.07 g) as a colorless oily substance.

¹H-NMR (CDCl₃) δ: 4.13 (2H, t, J = 6.0 Hz), 4.05 (2H, t, J = 6.8 Hz), 3.62-3.49 (2H, m), 3.27-3.08 (4H, m), 2.73 (2H, t, J = 6.0 Hz), 2.66-2.61 (2H, m), 2.59 (2H, t, J = 6.8 Hz), 2.55-2.42 (6H, m), 2.30 (2H, m), 1.72-1.41 (10H, m), 1.39-1.19 (16H, m), 1.01 (6H, t, J = 7.2 Hz), 0.92-0.84 (6H, m).

MS m/z: 559 (M+H)⁺

(8)
2-Hexyloctanoic acid (AA1) (93 mg), N,N'-diisopropylcarbodiimide (84 µL), 9-azajulolidine (141 mg), and triethylamine (0.23 mL) were added to a dichloromethane (1.5 mL) solution of heptyl 3-ethyl-12-hexyl-7-(2-hydroxyethyl)-11-oxo-10-oxa-3,7,12-triazaoctadecane-18-oate (C8) (150 mg), and the mixture was stirred at room temperature overnight. The solvent was distilled off from the reaction mixture under reduced pressure, ethyl acetate and water were added thereto, and the organic layer was isolated. The organic layer was washed with saturated saline, and anhydrous sodium sulfate was then added thereto to dry the organic layer, and the solvent was distilled off under reduced pressure. The obtained residue was purified by silica gel column chromatography (methanol-ethyl acetate), and then purified by NH silica gel column chromatography (ethyl acetate-hexane), thereby obtaining heptyl 3-ethyl-12-hexyl-7-(2-((2-hexyloctanoyl)oxy)ethyl)-11-oxo-10-oxa-3,7,12-triazaoctadecane-1 8-oate (compound 18) (140 mg) as a colorless oily substance.

¹H-NMR (CDCl₃) δ: 4.15-4.01 (6H, m), 3.26-3.09 (4H, m), 2.76 (4H, t, J = 6.4 Hz), 2.59-2.46 (6H, m), 2.45-2.37 (2H, m), 2.35-2.25 (3H, m), 1.70-1.38 (16H, m), 1.37-1.18 (30H, m), 1.01 (6H, t, J = 7.1 Hz), 0.92-0.82 (12H, m).

MS m/z: 769 (M+H)⁺

### [Synthesis Example 19]

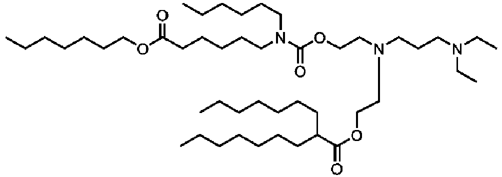

Heptyl 3-ethyl-7-(2-((2-heptylnonanoyl)oxy)ethyl)-12-hexyl-11-oxo-10-oxa-3,7,12-triazaoctadecane-18-oate was synthesized by the same method as in (8) of Synthesis Example 18 using 3-ethyl-12-hexyl-7-(2-hydroxyethyl)-11-oxo-10-oxa-3,7,12-triazaoctadecane-18-oate heptyl and 2-heptylnonanoic acid.

¹H-NMR (CDCl₃) δ: 4.15-4.01 (6H, m), 3.26-3.09 (4H, m), 2.76 (4H, t, J = 6.4 Hz), 2.59-2.46 (6H, m), 2.45-2.37 (2H, m), 2.35-2.25 (3H, m), 1.70-1.38 (16H, m), 1.37-1.18 (34H, m), 1.00 (6H, t, J = 7.1 Hz), 0.92-0.83 (12H, m).

MS (MSI, m/z): 796.9 [M+H]⁺

### [Synthesis Example 20]

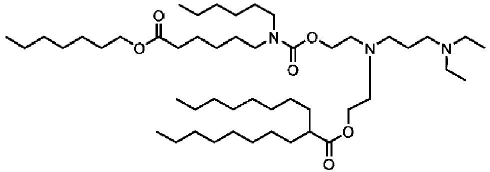

Heptyl 3-ethyl-12-hexyl-7-(2-((2-octyldodecanoyl)oxy)ethyl)-11-oxo-10-oxa-3,7,12-triazaoctadecane -18-oate was synthesized by the same method as in (8) of Synthesis Example 18 using 3-ethyl-12-hexyl-7-(2-hydroxyethyl)-11-oxo-10-oxa-3,7,12-triazaoctadecane-18-oate heptyl and 2-octyldodecanoic acid.

¹H-NMR (CDCl₃) δ: 4.16-4.01 (6H, m), 3.26-3.09 (4H, m), 2.76 (4H, t, J = 6.4 Hz), 2.62-2.36 (8H, m), 2.35-2.25 (3H, m), 1.70-1.38 (16H, m), 1.37-1.18 (38H, m), 1.07-0.96 (6H, m), 0.94-0.83 (12H, m).

MS (MSI, m/z): 825.1 [M+H]⁺

### [Synthesis Example 21]

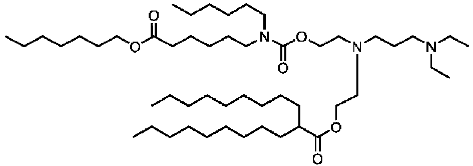

Heptyl 3-ethyl-12-hexyl-7-(2-((2-nonylundecanoyl)oxy)ethyl)-11-oxo-10-oxa-3,7,12-triazaoctadecane -18-oate was synthesized by the same method as in (8) of Synthesis Example 18 using 3-ethyl-12-hexyl-7-(2-hydroxyethyl)-11-oxo-10-oxa-3,7,12-triazaoctadecane-18-oate heptyl and 2-nonylundecanoic acid.

¹H-NMR (CDCl₃) δ: 4.15-4.02 (6H, m), 3.27-3.09 (4H, m), 2.76 (4H, t, J = 6.4 Hz), 2.62-2.36 (8H, m), 2.35-2.25 (3H, m), 1.70-1.38 (16H, m), 1.37-1.18 (42H, m), 1.07-0.96 (6H, m), 0.94-0.82 (12H, m).

MS (MSI, m/z): 854.1 [M+H]⁺

### [Synthesis Example 22]

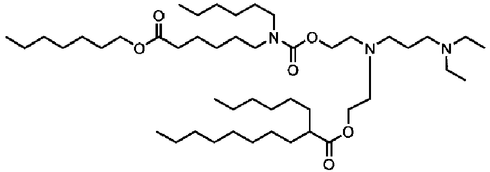

Heptyl 3-ethyl-12-hexyl-7-(2-((2-hexyldecanoyl)oxy)ethyl)-11-oxo-10-oxa-3,7,12-triazaoctadecane-1 8-oate was synthesized by the same method as in (8) of Synthesis Example 18 using 3-ethyl-12-hexyl-7-(2-hydroxyethyl)-11-oxo-10-oxa-3,7,12-triazaoctadecane-18-oate heptyl and 2-hexyldecanoic acid.

¹H-NMR (CDCl₃) δ: 4.15-4.08 (4H, m), 4.05 (2H, t, J = 6.8 Hz), 3.25-3.10 (4H, m), 2.76 (4H, t, J = 6.4 Hz), 2.59-2.46 (6H, m), 2.46-2.38 (2H, m), 2.35-2.25 (3H, m), 1.70-1.38 (16H, m), 1.37-1.18 (34H, m), 1.01 (6H, t, J = 7.1 Hz), 0.92-0.83 (12H, m).

MS (MSI, m/z): 797 [M+H]⁺

### [Synthesis Example 23]

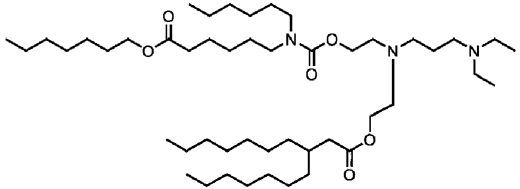

Heptyl 3-ethyl-7-(2-((3-heptyldecanoyl)oxy)ethyl)-12-hexyl-11-oxo-10-oxa-3,7,12-triazaoctadecane-18-oate was synthesized by the same method as in (8) of Synthesis Example 18 using 3-ethyl-12-hexyl-7-(2-hydroxyethyl)-11-oxo-10-oxa-3,7,12-triazaoctadecane-18-oate heptyl and 3-heptyldecanoic acid.

¹H-NMR (CDCl₃) δ: 4.15-4.02 (6H, m), 3.25-3.10 (4H, m), 2.76 (4H, t, J = 6.3 Hz), 2.59-2.46 (6H, m), 2.46-2.37 (2H, m), 2.30 (2H, t, J = 7.5 Hz), 2.22 (2H, d, J = 6.9 Hz), 1.87-1.79 (1H, m), 1.72-1.42 (12H, m), 1.38-1.18 (38H, m), 1.01 (6H, t, J = 7.1 Hz), 0.92-0.83 (12H, m).

MS (MSI, m/z): 811.1 [M+H]⁺

### [Synthesis Example 24]

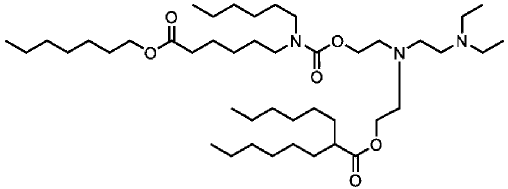

Heptyl 3-ethyl-11-hexyl-6-(2-((2-hexyloctanoyl)oxy)ethyl)-10-oxo-9-oxa-3,6,11-triazahexadecane-17 -oate was synthesized by the same method as in (8) of Synthesis Example 18 using 3-ethyl-11-hexyl-6-(2-hydroxyethyl)-10-oxo-9-oxa-3,6,11-triazahexadecane-17-oate heptyl and 2-hexyloctanoic acid.

¹H-NMR (CDCl₃) δ: 4.14-4.02 (6H, m), 3.26-3.09 (4H, m), 2.80 (4H, t, J = 6.3 Hz), 2.71-2.62 (2H, m), 2.56-2.47 (6H, m), 2.35-2.25 (3H, m), 1.71-1.37 (16H, m), 1.37-1.18 (32H, m), 1.02 (6H, t, J = 7.1 Hz), 0.92-0.83 (12H, m).

MS (MSI, m/z): 755.1 [M+H]⁺

### [Synthesis Example 25]

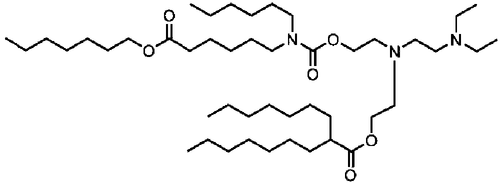

Heptyl 3-ethyl-6-(2-((2-heptylnonanoyl)oxy)ethyl)-11-hexyl-10-oxo-9-oxa-3,6,11-triazahexadecane-1 7-oate was synthesized by the same method as in (8) of Synthesis Example 18 using 3-ethyl-11-hexyl-6-(2-hydroxyethyl)-10-oxo-9-oxa-3,6,11-triazahexadecane-17-oate heptyl and 2-heptylnonanoic acid.

¹H-NMR (CDCl₃) δ: 4.14-4.02 (6H, m), 3.26-3.09 (4H, m), 2.80 (4H, t, J = 6.3 Hz), 2.71-2.62 (2H, m), 2.56-2.47 (6H, m), 2.35-2.25 (3H, m), 1.71-1.37 (16H, m), 1.37-1.18 (36H, m), 1.01 (6H, t, J = 7.1 Hz), 0.93-0.82 (12H, m).

MS (MSI, m/z): 783.1 [M+H]⁺

### [Synthesis Example 26]

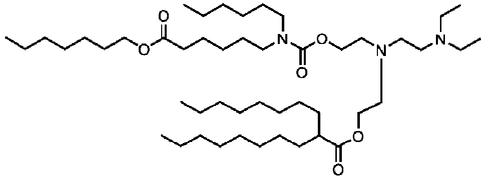

Heptyl 3-ethyl-11-hexyl-6-(2-((2-octyldecanoyl)oxy)ethyl)-10-oxo-9-oxa-3,6,11-triazahexadecane-17 -oate was synthesized by the same method as in (8) of Synthesis Example 18 using 3-ethyl-11-hexyl-6-(2-hydroxyethyl)-10-oxo-9-oxa-3,6,11-triazahexadecane-17-oate heptyl and 2-octyldecanoic acid.

¹H-NMR (CDCl₃) δ: 4.16-4.01 (6H, m), 3.26-3.08 (4H, m), 2.80 (4H, t, J = 6.3 Hz), 2.71-2.62 (2H, m), 2.56-2.47 (6H, m), 2.35-2.25 (3H, m), 1.71-1.37 (16H, m), 1.37-1.18 (40H, m), 1.01 (6H, t, J = 7.1 Hz), 0.93-0.81 (12H, m).

MS (MSI, m/z): 811.2 [M+H]⁺

### [Synthesis Example 27]

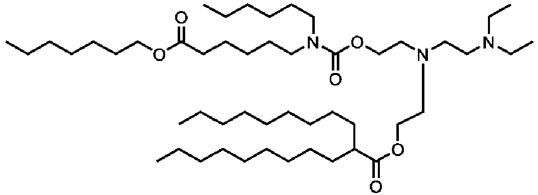

Heptyl 3-ethyl-11-hexyl-6-(2-((2-nonylundecanoyl)oxy)ethyl)-10-oxo-9-oxa-3,6,11-triazahexadecane-17-oate was synthesized by the same method as in (8) of Synthesis Example 18 using 3-ethyl-11-hexyl-6-(2-hydroxyethyl)-10-oxo-9-oxa-3,6,11-triazahexadecane-17-oate heptyl and 2-nonylundecanoic acid.

¹H-NMR (CDCl₃) δ: 4.17-4.08 (4H, m), 4.05 (2H, t, J = 6.8 Hz), 3.27-3.08 (4H, m), 2.80 (4H, t, J = 6.3 Hz), 2.72-2.62 (2H, m), 2.57-2.46 (6H, m), 2.35-2.25 (3H, m), 1.71-1.37 (16H, m), 1.37-1.18 (44H, m), 1.01 (6H, t, J = 7.1 Hz), 0.93-0.81 (12H, m).

MS (MSI, m/z): 839.2 [M+H]⁺

### [Synthesis Example 28]

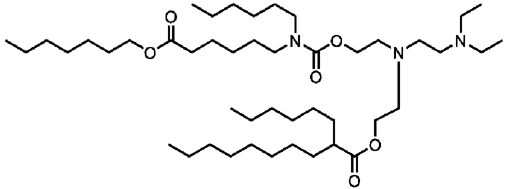

Heptyl 3-ethyl-11-hexyl-6-(2-((2-hexyldecanoyl)oxy)ethyl)-10-oxo-9-oxa-3,6,11-triazahexadecane-17 -oate was synthesized by the same method as in (8) of Synthesis Example 18 using 3-ethyl-11-hexyl-6-(2-hydroxyethyl)-10-oxo-9-oxa-3,6,11-triazahexadecane-17-oate heptyl and 2-hexyldecanoic acid.

¹H-NMR (CDCl₃) δ: 4.17-4.08 (4H, m), 4.05 (2H, t, J = 6.8 Hz), 3.27-3.08 (4H, m), 2.80 (4H, t, J = 6.3 Hz), 2.70-2.63 (2H, m), 2.57-2.46 (6H, m), 2.34-2.25 (3H, m), 1.71-1.37 (16H, m), 1.37-1.18 (36H, m), 1.02 (6H, t, J = 7.1 Hz), 0.94-0.83 (12H, m).

MS (MSI, m/z): 783.1 [M+H]⁺

### [Synthesis Example 29]

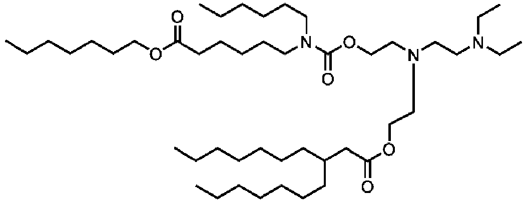

Heptyl 3-ethyl-6-(2-((3-heptyldecanoyl)oxy)ethyl)-11-hexyl-10-oxo-9-oxa-3,6,11-triazaheptadecane-1 7-oate was synthesized by the same method as in (8) of Synthesis Example 18 using 3-ethyl-11-hexyl-6-(2-hydroxyethyl)-10-oxo-9-oxa-3,6,11-triazahexadecane-17-oate heptyl and 3-heptyldecanoic acid.

¹H-NMR (CDCl₃) δ: 4.16-4.01 (6H, m), 3.26-3.08 (4H, m), 2.79 (4H, t, J = 6.3 Hz), 2.71-2.62 (2H, m), 2.56-2.47 (6H, m), 2.30 (2H, t, J = Hz), 2.22 (2H, d, J = Hz), 1.90-1.76 (1H, m), 1.71-1.37 (10H, m), 1.37-1.18 (38H, m), 1.01 (6H, t, J = 7.1 Hz), 0.93-0.81 (12H, m).

MS (MSI, m/z): 797.2 [M+H]⁺

### [Synthesis Example 30]

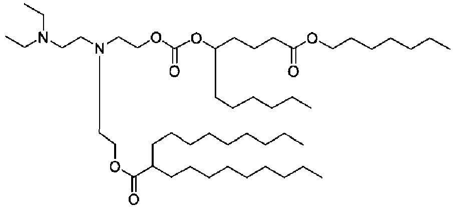

Heptyl 3-ethyl-12-hexyl-6-(2-((2-nonylundecanoyl)oxy)ethyl)-10-oxo-9,11-dioxa-3,6-diazahexadecan e-16-oate (compound 30) was synthesized according to Examples described in WO2024/190817A.

### [Synthesis Example 31]

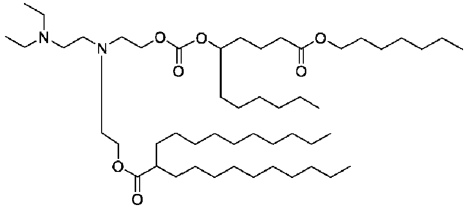

Heptyl 6-(2-((2-decyldodecanoyl)oxy)ethyl)-3-ethyl-12-hexyl-10-oxo-9,11-dioxa-3,6-diazahexadecan e-16-oate (compound 31) was synthesized according to Examples described in WO2024/190817A.

### [Synthesis Example 32]

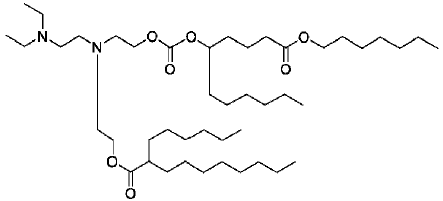

Heptyl 3-ethyl-12-hexyl-6-(2-((2-hexyldecanoyl)oxy)ethyl)-10-oxo-9,11-dioxa-3,6-diazahexadecane-16-oate (compound 32) was synthesized according to Examples described in WO2024/190817A.

### [Synthesis Example 33]

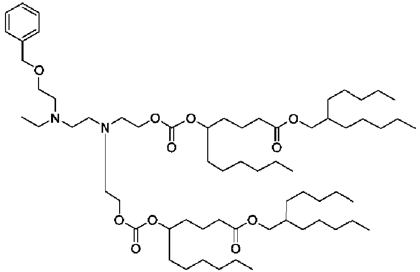

Bis(2-pentylheptyl) 11-(2-((2-(benzyloxy)ethyl)(ethyl)amino)ethyl)-5,17-dihexyl-7,15-dioxo-6,8,14,16-tetraoxa-11 -azahenicosanedioate was synthesized as a colorless oily substance by the same method as in Synthesis Example 9 (4) using 2,2'-((2-((2-(benzyloxy)ethyl)(ethyl)amino)ethyl)azanediyl)bis(ethan-1-ol) and 1-oxo-1-((2-pentylheptyl)oxy)undecan-5-yl 1H-1,2,4-triazole-1-carboxylate.
LC/MS
rt (min): 1.57
MS (MSI, m/z): 1104.4 [M+H]⁺

### [Synthesis Example 34]

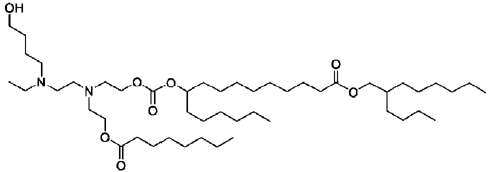

2-Butyloctyl 5-ethyl-14-hexyl-1-hydroxy-8-(2-(octanoyloxy)ethyl)-12-oxo-11,13-dioxa-5,8-diazatricosan-2 3-oate was synthesized as a colorless oily substance by the same method as in Synthesis Example 9 (5) using 2-butyloctyl 7-ethyl-16-hexyl-10-(2-(octanoyloxy)ethyl)-14-oxo-1-phenyl-2,13,15-trioxa-7,10-diazapentac osan-25-oate.
LC/MS
rt (min): 0.83
MS (MSI, m/z): 842.1 [M+H]⁺

### [Synthesis Example 35]

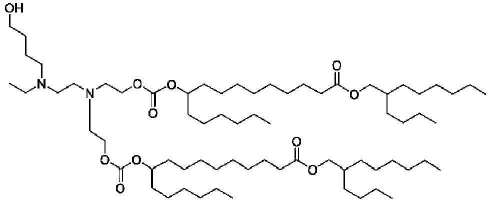

Bis(2-butyloctyl) 16-(2-(ethyl(4-hydroxybutyl)amino)ethyl)-10,22-dihexyl-12,20-dioxo-11,13,19,21-tetraoxa-16 -azahentriacontanedioate was synthesized as a colorless oily substance by the same method as in Synthesis Example 9 (5) using bis(2-butyloctyl) 16-(2-((4-(benzyloxy)butyl)(ethyl)amino)ethyl)-10,22-dihexyl-12,20-dioxo-11,13,19,21-tetrao xa-16-azahentriacontanedioate.
LC/MS
rt (min): 1.97
MS (MSI, m/z): 1182.5 [M+H]⁺

### [Synthesis Example 36]

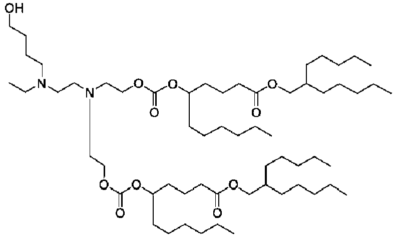

Bis(2-pentylheptyl) 11-(2-(ethyl(4-hydroxybutyl)amino)ethyl)-5,17-dihexyl-7,15-dioxo-6,8,14,16-tetraoxa-11-aza henicosanedioate was synthesized as a colorless oily substance by the same method as in Synthesis Example 9 (5) using bis(2-pentylheptyl) 11-(2-((4-(benzyloxy)butyl)(ethyl)amino)ethyl)-5,17-dihexyl-7,15-dioxo-6,8,14,16-tetraoxa-1 1-azahenicosanedioate.
LC/MS
rt (min): 1.48
MS (MSI, m/z): 1042.3 [M+H]⁺

### [Synthesis Example 37]

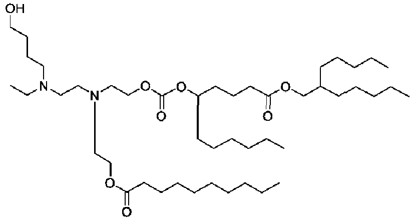

2-Pentylheptyl 8-(2-(decanoyloxy)ethyl)-5-ethyl-14-hexyl-1-hydroxy-12-oxo-11,13-dioxa-5,8-diazaoctadecan -18-oate was synthesized as a colorless oily substance by the same method as in Synthesis Example 9 (5) using 2-pentylheptyl 10-(2-(decanoyloxy)ethyl)-7-ethyl-16-hexyl-14-oxo-1-phenyl-2,13,15-trioxa-7,10-diazaeicos-20-oate.
LC/MS
rt (min): 0.62
MS (MSI, m/z): 800.0 [M+H]⁺

### [Synthesis Example 38]

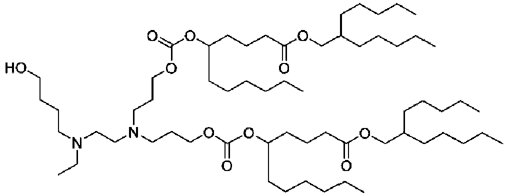

Bis(2-pentylheptyl) 12-(2-(ethyl(4-hydroxybutyl)amino)ethyl)-5,19-dihexyl-7,17-dioxo-6,8,16,18-tetraoxa-12-azat ricosandioate was synthesized as a colorless oily substance by the same method as in Synthesis Example 9 (5) using bis(2-pentylheptyl) 12-(2-((4-(benzyloxy)butyl)(ethyl)amino)ethyl)-5,19-dihexyl-7,17-dioxo-6,8,16,18-tetraoxa-1 2-azatricosandioate.
LC/MS
rt (min): 1.61
MS (MSI, m/z): 1070.4 [M+H]⁺

### [Synthesis Example 39]

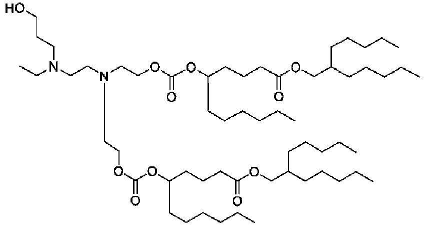

Bis(2-pentylheptyl) 11-(2-(ethyl(3-hydroxypropyl)amino)ethyl)-5,17-dihexyl-7,15-dioxo-6,8,14,16-tetraoxa-11-az ahenicosandioate was synthesized as a colorless oily substance by the same method as in Synthesis Example 9 (5) using bis(2-pentylheptyl) 11-(2-((3-(benzyloxy)propyl)(ethyl)amino)ethyl)-5,17-dihexyl-7,15-dioxo-6,8,14,16-tetraoxa-11-azahenicosandioate.
LC/MS
rt (min): 1.40
MS (MSI, m/z): 1028.3 [M+H]⁺

### [Synthesis Example 40]

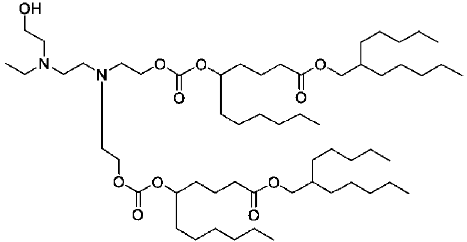

Bis(2-pentylheptyl) 11-(2-(ethyl(2-hydroxyethyl)amino)ethyl)-5,17-dihexyl-7,15-dioxo-6,8,14,16-tetraoxa-11-azah enicosanedioic acid was synthesized as a colorless oily substance by the same method as in Synthesis Example 9 (5) using bis(2-pentylheptyl) 11-(2-((2-(benzyloxy)ethyl)(ethyl)amino)ethyl)-5,17-dihexyl-7,15-dioxo-6,8,14,16-tetraoxa-11 -azahenicosanedioic acid.
LC/MS
rt (min): 1.44
MS (MSI, m/z): 1014.3 [M+H]⁺

### [Synthesis Example 41]

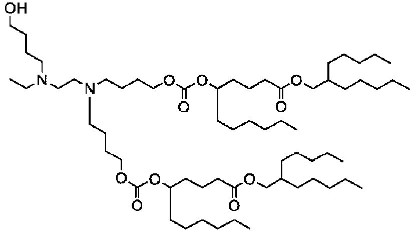

Bis(2-pentylheptyl) 13-(2-(ethyl(4-hydroxybutyl)amino)ethyl)-5,21-dihexyl-7,19-dioxo-6,8,18,20-tetraoxa-13-aza pentacosanedioic acid was synthesized as a colorless oily substance by the same method as in Synthesis Example 9 (5) using bis(2-pentylheptyl) 13-(2-((4-(benzyloxy)butyl)(ethyl)amino)ethyl)-5,21-dihexyl-7,19-dioxo-6,8,18,20-tetraoxa-1 3-azapentacosanedioic acid.
LC/MS
rt (min): 1.44
MS (MSI, m/z): 1098.5 [M+H]⁺

### [Synthesis Example 42]

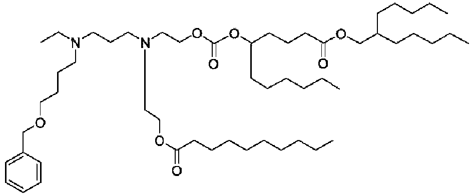

2-Pentylheptyl 11-(2-(decanoyloxy)ethyl)-7-ethyl-17-hexyl-15-oxo-1-phenyl-2,14,16-trioxa-7,11-diazahenico san-21-oate was synthesized as a colorless oily substance by the same method as in Synthesis Example 18 (8) using 2-pentylheptyl 7-ethyl-17-hexyl-11-(2-hydroxyethyl)-15-oxo-1-phenyl-2,14,16-trioxa-7,11-diazahenicosan-2 1-oate and decanoic acid.
LC/MS
rt (min): 0.87
MS (MSI, m/z): 904.2 [M+H]⁺

### [Synthesis Example 43]

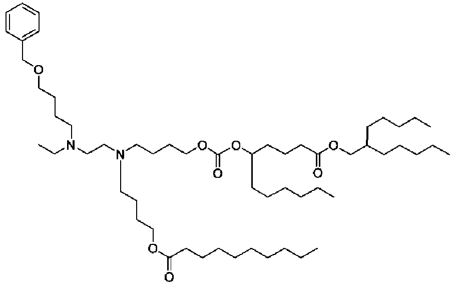

2-Pentylheptyl 10-(4-(decanoyloxy)butyl)-7-ethyl-18-hexyl-16-oxo-1-phenyl-2,15,17-trioxa-7,10-diazadocos an-22-oate was synthesized as a colorless oily substance by the same method as in Synthesis Example 18 (8) using 2-pentylheptyl 7-ethyl-18-hexyl-10-(4-hydroxybutyl)-16-oxo-1-phenyl-2,15,17-trioxa-7,10-diazadocosan-22-oate and decanoic acid.
LC/MS
rt (min): 0.99
MS (MSI, m/z): 946.2 [M+H]⁺

### [Synthesis Example 44]

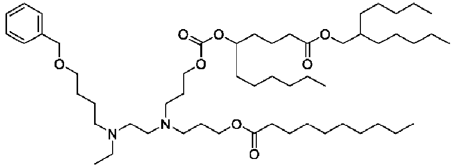

2-Pentylheptyl 10-(3-(decanoyloxy)propyl)-7-ethyl-17-hexyl-15-oxo-1-phenyl-2,14,16-trioxa-7,10-diazahenic osan-21-oate was synthesized as a colorless oily substance by the same method as in Synthesis Example 18 (8) using 2-pentylheptyl 7-ethyl-17-hexyl-10-(3-hydroxypropyl)-15-oxo-1-phenyl-2,14,16-trioxa-7,10-diazahenicosan-21-oate and decanoic acid.
LC/MS
rt (min): 1.07
MS (MSI, m/z): 918.2 [M+H]⁺

### [Synthesis Example 45]

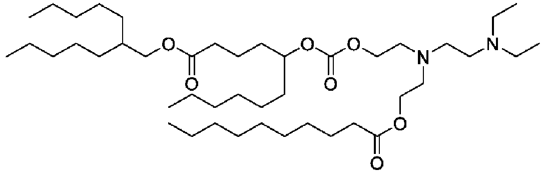

2-Pentylheptyl 6-(2-(decanoyloxy)ethyl)-3-ethyl-12-hexyl-10-oxo-9,11-dioxa-3,6-diazahexadecan-16-oate (compound 45) was synthesized according to Examples described in WO2021/095876A.

### [Synthesis Example 46]

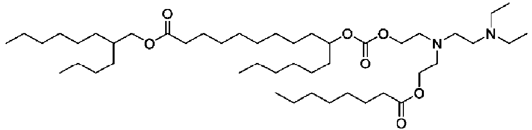

2-Butyloctyl 3-ethyl-12-hexyl-6-(2-(octanoyloxy)ethyl)-10-oxo-9,11-dioxa-3,6-diazahenicosan-21-oate (compound 46) was synthesized according to Examples described in WO2019/235635A.

### [Synthesis Example 47]

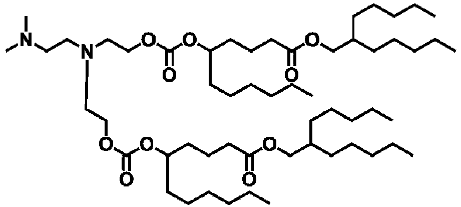

Bis(2-pentylheptyl) 11-(2-(dimethylamino)ethyl)-5,17-dihexyl-7,15-dioxo-6,8,14,16-tetraoxa-11-azahenicosanedio ate (compound 47) was synthesized according to Examples described in WO2022/230964A.

### [Synthesis Example 48]

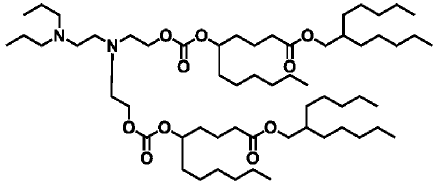

Bis(2-pentylheptyl) 11-(2-(dipropylamino)ethyl)-5,17-dihexyl-7,15-dioxo-6,8,14,16-tetraoxa-11-azahenicosanedio ate (compound 48) was synthesized according to Examples described in WO2022/230964A.

### [Synthesis Example 49]

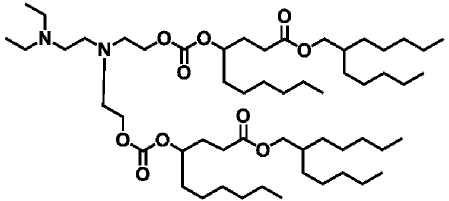

Bis(2-pentylheptyl) 10-(2-(diethylamino)ethyl)-4,16-dihexyl-6,14-dioxo-5,7,13,15-tetraoxa-10-azanonanedioate (compound 49) was synthesized according to Examples described in WO2022/230964A.

### [Synthesis Example 50]

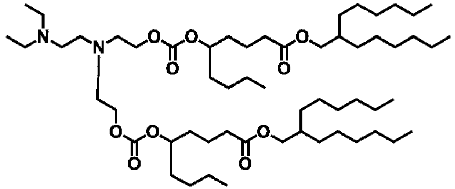

Bis(2-hexyloctyl) 5,17-dibutyl-11-(2-(diethylamino)ethyl)-7,15-dioxo-6,8,14,16-tetraoxa-11-azahenicosanedioat e (compound 50) was synthesized according to Examples described in WO2022/230964A.

### [Synthesis Example 51]

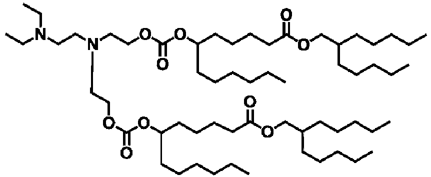

Bis(2-pentylheptyl) 12-(2-(diethylamino)ethyl)-6,18-dihexyl-8,16-dioxo-7,9,15,17-tetraoxa-12-azatricosanedioate (compound 51) was synthesized according to Examples described in WO2022/230964A.

### [Synthesis Example 52]

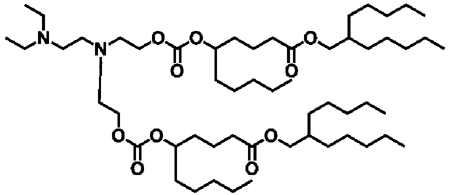

Bis(2-pentylheptyl) 11-(2-(diethylamino)ethyl)-7,15-dioxo-5,17-dipentyl-6,8,14,16-tetraoxa-11-azahenicosanedioa te (compound 52) was synthesized according to Examples described in WO2022/230964A.

### [Synthesis Example 53]

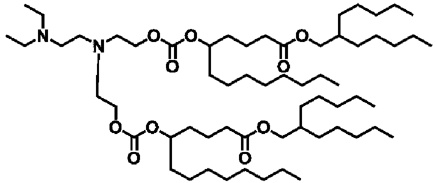

Bis(2-pentylheptyl) 11-(2-(diethylamino)ethyl)-5,17-dioctyl-7,15-dioxo-6,8,14,16-tetraoxa-11-azahenicosanedioat e (compound 53) was synthesized according to Examples described in WO2022/230964A.

### [Synthesis Example 54]

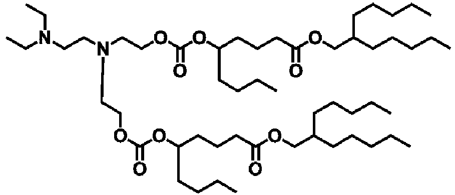

Bis(2-pentylheptyl) 5,17-dibutyl-11-(2-(diethylamino)ethyl)-7,15-dioxo-6,8,14,16-tetraoxa-11-azahenicosanedioat e (compound 54) was synthesized according to Examples described in WO2022/230964A.

### [Synthesis Example 55]

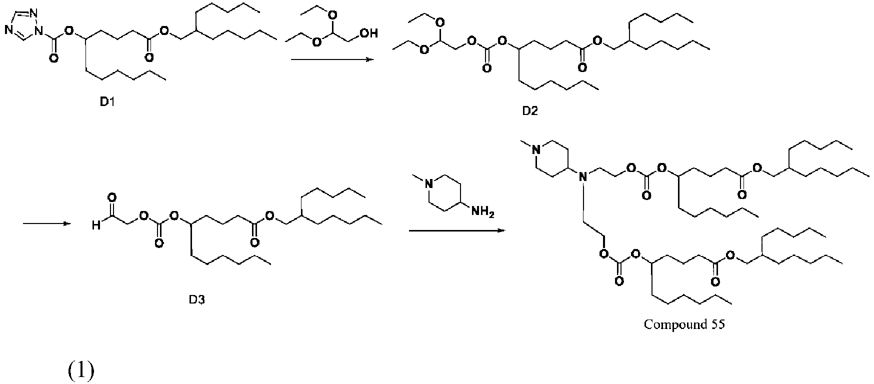

1-oxo-1-((2-pentylheptyl)oxy)undecan-5-yl 1H-1,2,4-triazole-1-carboxylate (D1) (5 g) and a 10 mL acetonitrile solution of 2,2-diethoxyethan-1-ol (1.9 g), which were synthesized according to Examples described in WO2024/014430A, were added to diazabicycloundecene (2.4 mL), and the mixture was stirred at 50°C for 20 minutes. The reaction product was separated into an ethyl acetate and water, the obtained organic phase was dried over anhydrous sodium sulfate, and the solvent was distilled off to obtain 2-pentylheptyl 5-(((2,2-diethoxyethoxy)carbonyl)oxy)undecanoate (D2) (5.77 g).

¹H-NMR (CDCl₃) δ: 4.71 (2H, t, J = 5.4 Hz), 4.13 (2H, t, J = 5.3 Hz), 3.97 (2H, d, J = 5.8 Hz), 3.76-3.54 (4H, m), 2.32 (2H, t, J = 7.0 Hz), 1.78-1.58 (7H, m), 1.33-1.20 (30H, m), 0.90-0.86 (9H, m).

(2)
p-Toluenesulfonic acid (0.32 g) was added to a solution of 5-(((2,2-diethoxyethoxy)carbonyl)oxy)undecanoate (D2) (1.00 g), acetic acid (4 mL), and water (1 mL), and the mixture was stirred at 60°C for 1 hour. The reaction product was separated into ethyl acetate and saturated saline, and the obtained organic phase was washed with a 10% potassium carbonate aqueous solution and saturated saline. After drying over anhydrous sodium sulfate, the solvent was distilled off to obtain 2-pentylheptyl 5-(((2-oxoethoxy)carbonyl)oxy)undecanoate (D3) (0.86 g).

MS m/z: 458 (M+H)⁺

(3)
A solution of 2-pentylheptyl 5-(((2-oxoethoxy)carbonyl)oxy)undecanoate (D3) (0.86 g) and ethyl acetate (8.6 mL) was cooled to 0°C, 1-methylpiperidin-4-amine (99 mg) was added thereto, and then sodium triacetoxyborohydride (0.60 g) was added thereto, and the mixture was stirred, and then the cooling layer was removed, and the mixture was stirred at room temperature for 2 hours. The obtained reaction solution was diluted with ethyl acetate, a 10% sodium hydrogen carbonate aqueous solution was carefully added thereto to stop the reaction, and the organic phase of the obtained mixture was washed with a 10% sodium hydrogen carbonate aqueous solution and then saturated saline. After drying over anhydrous sodium sulfate, the solvent was distilled off, the obtained residue was purified by silica gel column chromatography (hexane/ethyl acetate, and then methanol/ethyl acetate), and further purified by NH silica gel column chromatography (hexane/ethyl acetate) to obtain bis(2-pentylheptyl) 5,17-dihexyl-11-(1-methylpiperidin-4-yl)-7,15-dioxo-6,8,14,16-tetraoxa-11-azahenicosanedioa te (compound 55) (0.49 g) as a colorless oily substance.

¹H-NMR (CDCl₃) δ: 4.71-4.65 (2H, m), 4.15-4.03 (4H, m), 3.96 (4H, d, J = 5.8 Hz), 2.88 (2H, d, J = 11.3 Hz), 2.80 (4H, t, J = 6.7 Hz), 2.50-2.42 (1H, m), 2.32 (4H, t, J = 7.0 Hz), 2.24 (3H, s), 1.91 (2H, t, J = 10.9 Hz), 1.73-1.49 (18H, m), 1.34-1.21 (48H, m), 0.90-0.86 (18H, m).

MS m/z: 997 (M+H)⁺

A commercially available product of a compound 56 (product name: DLin-MC3-DMG, FUJIFILM Wako Pure Chemical Corporation) and a compound 57 (product name: COSMETOC (R) SS-OP, manufactured by NOF Corporation) was purchased.

### [Production of lipid particles]

### <Preparation of lipid particles not containing nucleic acid (empty LNP)>

The ionizable lipid shown in Table 1, one phospholipid (helper lipid) selected from DOPE (L-α-dioleoyl phosphatidylethanolamine, product name: COATSOME (R) ME-8181, manufactured by NOF Corporation), DSPC (1,2-distearoyl-sn-glycero-3-phosphocholine, product name: COATSOME (R) MC-8080, manufactured by NOF Corporation), DOPC (1,2-dioleoyl-sn-glycero-3-phosphocholine, product name: COATSOME (R) MC-8181, manufactured by NOF Corporation), DPPC (1,2-dipalmitoyl-sn-glycero-3-phosphocholine, product name: COATSOME (R) MC-6060, manufactured by NOF Corporation), and DMPC (1,2-dimyristoyl-sn-glycero-3-phosphocholine, product name: COATSOME (R) MC-4040, manufactured by NOF Corporation), cholesterol, and DMG-PEG2000 (1,2-dimyristoyl-rac-glycero-3-methoxypolyethylene glycol-2000, product name: SUNBRIGHT (R) GM-020, manufactured by NOF Corporation) were dissolved in ethanol at the molar ratios shown in Table 1 so that the total lipid concentration was 12.5 mmol/L or 62.5 mmol/L, thereby obtaining an oil phase.

A 50 mmol/L citrate buffer at a pH of 4 was mixed with the above-described oil phase using NanoAssemblr (Precision NanoSystems) so that the volume ratio of the citrate buffer to the oil phase was citrate buffer:oil phase = 3:1, and the mixed solution was diluted 2-fold with water to obtain a dispersion liquid of lipid particles. The dispersion liquid was dialyzed against a 20 mmol/L MES buffer solution at pH 6.0 containing 8% sucrose using a dialysis cassette (Slide-A-Lyzer G2, MWCO: 10 kD, Thermo Fisher Scientific, Inc.) to remove ethanol, and a concentration step was performed using an ultrafiltration filter (Amicon ultra 100 kDa, Merck KGaA) as necessary, thereby obtaining lipid particles (empty LNP) not containing a nucleic acid. The empty LNP was stored at -70°C until use.

### <Encapsulation of GFP mRNA in empty LNP (post-addition method)>

RNA solution was prepared by diluting CleanCap (registered trademark) EGFP mRNA (5 moU) (TriLink, L-7201) with water for injection. The empty LNP stored at -70°C was thawed at 4°C. The RNA solution was added to the present LNP liquid in an equal liquid amount and mixed by pipetting (LNP-RNA mixed solution). After being allowed to stand at room temperature for 5 minutes, 20 mmol/L Tris buffer (pH 8.4) containing 8% sucrose was added to the present LNP-RNA mixed solution in an equal liquid amount, and the mixture was mixed by pipetting, thereby preparing GFP mRNA-encapsulating LNP by a post-addition method.

### <Encapsulation of gRNA and Cas9 mRNA in empty LNP (post-addition method)>

CleanCap (registered trademark) Cas9 mRNA (5 moU) (TriLink, L-7206) and sgRNA (sequence; A*G*A*GUCUCUCAGCUGGUACA + modified Scaffold, Thermo Fisher A35514, custom synthesis) targeting a human T cell receptor alpha constant (TRAC) gene were mixed at a weight ratio shown in Table 1, and the mixture was diluted with water for injection to prepare an RNA solution. The empty LNP stored at -70°C was thawed at 4°C. The RNA solution was added to the present LNP liquid in an equal liquid amount and mixed by pipetting (LNP-RNA mixed solution). After allowing the mixture to stand at room temperature for 5 minutes, a 20 mmol/L Tris buffer containing 8% sucrose was added to the present LNP-RNA mixed solution in an equal liquid amount, and the mixture was mixed by pipetting, thereby preparing Cas9 mRNA/gRNA-encapsulating LNP by a post-addition method.

**[Table 1]**

| Cas9 mRNA/sgRNA-encapsulating lipid particles prepared by post-addition method | | | | | | |
|---|---|---|---|---|---|---|
| | Compound No. | Helper lipid | Formulation of ionizable lipid/helper lipid/cholesterol/DMG-PEG2000 (mol%) | Lipid/RNA (wt/wt) | Cas9 mRNA: sgRNA (wt/wt) | Oil-phase lipid concentration (mM) |
| Example 1 | Compound 5 | DOPE | 40/10/48.5/1.5 | 20 | 4:1 | 12.5 |
| Example 2 | Compound 5 | DOPE | 35/15/48.5/1.5 | 20 | 4:1 | 12.5 |
| Example 3 | Compound 5 | DOPE | 40/10/48.5/1.5 | 20 | 4:1 | 12.5 |
| Example 4 | Compound 5 | DOPE | 35/15/48.5/1.5 | 20 | 4:1 | 12.5 |
| Example 5 | Compound 5 | DOPE | 30/20/48.5/1.5 | 20 | 4:1 | 12.5 |
| Example 6 | Compound 5 | DOPE | 40/10/48.5/1.5 | 6.7 | 4:1 | 12.5 |
| Example 7 | Compound 5 | DOPE | 35/15/48.5/1.5 | 6.7 | 4:1 | 12.5 |
| Example 8 | Compound 5 | DOPE | 30/20/48.5/1.5 | 6.7 | 4:1 | 12.5 |
| Example 36 | Compound 13 | DSPC | 50/10/38.5/1.5 | 20 | 4:1 | 12.5 |
| Example 37 | Compound 13 | DSPC | 35/15/47.5/2.5 | 20 | 4:1 | 12.5 |
| Example 38 | Compound 10 | DSPC | 40/10/48.5/1.5 | 20 | 4:1 | 12.5 |
| Example 39 | Compound 8 | DSPC | 40/10/48.5/1.5 | 20 | 4:1 | 12.5 |
| Example 40 | Compound 14 | DSPC | 40/10/48.5/1.5 | 20 | 4:1 | 12.5 |
| Example 41 | Compound 15 | DSPC | 40/10/48.5/1.5 | 20 | 4:1 | 12.5 |
| Example 42 | Compound 16 | DSPC | 40/10/48.5/1.5 | 20 | 4:1 | 12.5 |
| Example 43 | Compound 17 | DSPC | 40/10/48.5/1.5 | 20 | 4:1 | 12.5 |
| Example 44 | Compound 9 | DOPE | 35/15/48.5/1.5 | 20 | 4:1 | 12.5 |
| Example 45 | Compound 10 | DOPE | 35/15/48.5/1.5 | 20 | 4:1 | 12.5 |
| Example 46 | Compound 8 | DOPE | 35/15/48.5/1.5 | 20 | 4:1 | 12.5 |
| Example 47 | Compound 14 | DOPE | 35/15/48.5/1.5 | 20 | 4:1 | 12.5 |
| Example 48 | Compound 15 | DOPE | 35/15/48.5/1.5 | 20 | 4:1 | 12.5 |
| Example 49 | Compound 16 | DOPE | 35/15/48.5/1.5 | 20 | 4:1 | 12.5 |
| Example 50 | Compound 17 | DOPE | 35/15/48.5/1.5 | 20 | 4:1 | 12.5 |
| Example 51 | Compound 18 | DSPC | 40/10/48.5/1.5 | 20 | 4:1 | 12.5 |
| Example 52 | Compound 19 | DSPC | 40/10/48.5/1.5 | 20 | 4:1 | 12.5 |
| Example 53 | Compound 20 | DSPC | 40/10/48.5/1.5 | 20 | 4:1 | 12.5 |
| Example 54 | Compound 21 | DSPC | 40/10/48.5/1.5 | 20 | 4:1 | 12.5 |
| Example 55 | Compound 22 | DSPC | 40/10/48.5/1.5 | 20 | 4:1 | 12.5 |
| Example 56 | Compound 23 | DSPC | 40/10/48.5/1.5 | 20 | 4:1 | 12.5 |
| Example 57 | Compound 24 | DSPC | 40/10/48.5/1.5 | 20 | 4:1 | 12.5 |
| Example 58 | Compound 25 | DSPC | 40/10/48.5/1.5 | 20 | 4:1 | 12.5 |
| Example 59 | Compound 26 | DSPC | 40/10/48.5/1.5 | 20 | 4:1 | 12.5 |
| Example 60 | Compound 27 | DSPC | 40/10/48.5/1.5 | 20 | 4:1 | 12.5 |
| Example 61 | Compound 28 | DSPC | 40/10/48.5/1.5 | 20 | 4:1 | 12.5 |
| Example 62 | Compound 29 | DSPC | 40/10/48.5/1.5 | 20 | 4:1 | 12.5 |
| Example 63 | Compound 30 | DSPC | 40/10/48.5/1.5 | 20 | 4:1 | 12.5 |
| Example 64 | Compound 31 | DSPC | 40/10/48.5/1.5 | 20 | 4:1 | 12.5 |
| Example 65 | Compound 32 | DSPC | 40/10/48.5/1.5 | 20 | 4:1 | 12.5 |
| Example 66 | Compound 18 | DOPE | 40/10/48.5/1.5 | 20 | 4:1 | 12.5 |
| Example 67 | Compound 19 | DOPE | 40/10/48.5/1.5 | 20 | 4:1 | 12.5 |
| Example 68 | Compound 20 | DOPE | 40/10/48.5/1.5 | 20 | 4:1 | 12.5 |
| Example 69 | Compound 21 | DOPE | 40/10/48.5/1.5 | 20 | 4:1 | 12.5 |
| Example 70 | Compound 22 | DOPE | 40/10/48.5/1.5 | 20 | 4:1 | 12.5 |
| Example 71 | Compound 23 | DOPE | 40/10/48.5/1.5 | 20 | 4:1 | 12.5 |
| Example 72 | Compound 24 | DOPE | 40/10/48.5/1.5 | 20 | 4:1 | 12.5 |
| Example 73 | Compound 25 | DOPE | 40/10/48.5/1.5 | 20 | 4:1 | 12.5 |
| Example 74 | Compound 26 | DOPE | 40/10/48.5/1.5 | 20 | 4:1 | 12.5 |
| Example 75 | Compound 27 | DOPE | 40/10/48.5/1.5 | 20 | 4:1 | 12.5 |
| Example 76 | Compound 28 | DOPE | 40/10/48.5/1.5 | 20 | 4:1 | 12.5 |
| Example 77 | Compound 29 | DOPE | 40/10/48.5/1.5 | 20 | 4:1 | 12.5 |
| Example 78 | Compound 30 | DOPE | 40/10/48.5/1.5 | 20 | 4:1 | 12.5 |
| Example 79 | Compound 31 | DOPE | 40/10/48.5/1.5 | 20 | 4:1 | 12.5 |
| Example 80 | Compound 32 | DOPE | 40/10/48.5/1.5 | 20 | 4:1 | 12.5 |
| Example 81 | Compound 15 | DOPE | 35/15/48.5/1.5 | 20 | 4:1 | 12.5 |
| Example 82 | Compound 15 | DOPE | 40/10/48.5/1.5 | 20 | 4:1 | 12.5 |
| Example 83 | Compound 15 | DSPC | 40/10/48.5/1.5 | 20 | 4:1 | 12.5 |
| Example 84 | Compound 5 | DOPE | 35/15/48.5/1.5 | 20 | 4:1 | 12.5 |
| Example 85 | Compound 33 | DSPC | 40/10/48.5/1.5 | 20 | 4:1 | 12.5 |
| Example 86 | Compound 34 | DSPC | 40/10/48.5/1.5 | 20 | 4:1 | 12.5 |
| Example 87 | Compound 35 | DSPC | 40/10/48.5/1.5 | 20 | 4:1 | 12.5 |
| Example 88 | Compound 36 | DSPC | 40/10/48.5/1.5 | 20 | 4:1 | 12.5 |
| Example 89 | Compound 37 | DSPC | 40/10/48.5/1.5 | 20 | 4:1 | 12.5 |
| Example 90 | Compound 38 | DSPC | 40/10/48.5/1.5 | 20 | 4:1 | 12.5 |
| Example 91 | Compound 39 | DSPC | 40/10/48.5/1.5 | 20 | 4:1 | 12.5 |
| Example 92 | Compound 40 | DSPC | 40/10/48.5/1.5 | 20 | 4:1 | 12.5 |
| Example 93 | Compound 9 | DSPC | 33.7/10/48.5/1.5 | 20 | 4:1 | 12.5 |
| Example 94 | Compound 41 | DSPC | 40/10/48.5/1.5 | 20 | 4:1 | 12.5 |
| Example 95 | Compound 42 | DSPC | 40/10/48.5/1.5 | 20 | 4:1 | 12.5 |
| Example 96 | Compound 43 | DSPC | 40/10/48.5/1.5 | 20 | 4:1 | 12.5 |
| Example 97 | Compound 44 | DSPC | 40/10/48.5/1.5 | 20 | 4:1 | 12.5 |
| Example 98 | Compound 45 | DOPE | 40/5/55/1.5 | 20 | 4:1 | 12.5 |
| Example 99 | Compound 45 | DSPC | 40/5/55/1.5 | 20 | 4:1 | 12.5 |
| Example 100 | Compound 45 | DPPC | 40/5/55/1.5 | 20 | 4:1 | 12.5 |
| Example 101 | Compound 45 | DMPC | 40/5/55/1.5 | 20 | 4:1 | 12.5 |
| Example 102 | Compound 45 | DOPC | 40/5/55/1.5 | 20 | 4:1 | 12.5 |
| Example 103 | Compound 45 | DOPE | 40/10/50/1.5 | 20 | 4:1 | 12.5 |
| Example 104 | Compound 45 | DSPC | 40/10/50/1.5 | 20 | 4:1 | 12.5 |
| Example 105 | Compound 45 | DPPC | 40/10/50/1.5 | 20 | 4:1 | 12.5 |
| Example 106 | Compound 45 | DMPC | 40/10/50/1.5 | 20 | 4:1 | 12.5 |
| Example 107 | Compound 45 | DOPC | 40/10/50/1.5 | 20 | 4:1 | 12.5 |
| Example 108 | Compound 45 | DOPE | 40/30/30/1.5 | 20 | 4:1 | 12.5 |
| Example 109 | Compound 45 | DSPC | 40/30/30/1.5 | 20 | 4:1 | 12.5 |
| Example 110 | Compound 45 | DPPC | 40/30/30/1.5 | 20 | 4:1 | 12.5 |
| Example 111 | Compound 45 | DMPC | 40/30/30/1.5 | 20 | 4:1 | 12.5 |
| Example 112 | Compound 45 | DOPC | 40/30/30/1.5 | 20 | 4:1 | 12.5 |
| Example 113 | Compound 46 | DOPE | 40/5/55/1.5 | 20 | 4:1 | 12.5 |
| Example 114 | Compound 46 | DSPC | 40/5/55/1.5 | 20 | 4:1 | 12.5 |
| Example 115 | Compound 46 | DPPC | 40/5/55/1.5 | 20 | 4:1 | 12.5 |
| Example 116 | Compound 46 | DMPC | 40/5/55/1.5 | 20 | 4:1 | 12.5 |
| Example 117 | Compound 46 | DOPC | 40/5/55/1.5 | 20 | 4:1 | 12.5 |
| Example 118 | Compound 46 | DOPE | 40/10/50/1.5 | 20 | 4:1 | 12.5 |
| Example 119 | Compound 46 | DSPC | 40/10/50/1.5 | 20 | 4:1 | 12.5 |
| Example 120 | Compound 46 | DPPC | 40/10/50/1.5 | 20 | 4:1 | 12.5 |
| Example 121 | Compound 46 | DMPC | 40/10/50/1.5 | 20 | 4:1 | 12.5 |
| Example 122 | Compound 46 | DOPC | 40/10/50/1.5 | 20 | 4:1 | 12.5 |
| Example 123 | Compound 46 | DOPE | 40/30/30/1.5 | 20 | 4:1 | 12.5 |
| Example 124 | Compound 46 | DSPC | 40/30/30/1.5 | 20 | 4:1 | 12.5 |
| Example 125 | Compound 46 | DPPC | 40/30/30/1.5 | 20 | 4:1 | 12.5 |
| Example 126 | Compound 46 | DMPC | 40/30/30/1.5 | 20 | 4:1 | 12.5 |
| Example 127 | Compound 46 | DOPC | 40/30/30/1.5 | 20 | 4:1 | 12.5 |
| Example 128 | Compound 17 | DSPC | 40/10/48.5/1.5 | 20 | 4:1 | 12.5 |
| Example 129 | Compound 5 | DOPE | 35/15/48.5/1.5 | 20 | 4:1 | 62.5 |
| Example 130 | Compound 10 | DOPE | 35/15/48.5/1.5 | 20 | 4:1 | 12.5 |
| Example 131 | Compound 8 | DOPE | 35/15/48.5/1.5 | 20 | 4:1 | 12.5 |
| Example 132 | Compound 14 | DOPE | 35/15/48.5/1.5 | 20 | 4:1 | 12.5 |
| Example 133 | Compound 15 | DOPE | 35/15/48.5/1.5 | 20 | 4:1 | 12.5 |
| Example 134 | Compound 16 | DOPE | 35/15/48.5/1.5 | 20 | 4:1 | 12.5 |
| Example 135 | Compound 17 | DOPE | 35/15/48.5/1.5 | 20 | 4:1 | 12.5 |
| Example 136 | Compound 5 | DOPE | 35/15/48.5/1.5 | 20 | 4:1 | 62.5 |
| Example 137 | Compound 5 | DOPE | 35/15/48.5/1.5 | 20 | 4:1 | 62.5 |
| Example 138 | Compound 5 | DOPE | 35/15/48.5/1.5 | 20 | 4:1 | 62.5 |
| Example 139 | Compound 36 | DSPC | 40/10/48.5/1.5 | 20 | 4:1 | 62.5 |
| Example 140 | Compound 39 | DSPC | 40/10/48.5/1.5 | 20 | 4:1 | 62.5 |
| Example 141 | Compound 38 | DSPC | 40/10/48.5/1.5 | 20 | 4:1 | 62.5 |
| Example 142 | Compound 15 | DSPC | 40/10/48.5/1.5 | 20 | 4:1 | 62.5 |
| Example 143 | Compound 5 | DOPE | 35/15/48.5/1.5 | 20 | 4:1 | 62.5 |
| Example 144 | Compound 32 | DOPE | 40/10/48.5/1.5 | 20 | 4:1 | 12.5 |
| Example 145 | Compound 36 | DSPC | 40/10/48.5/1.5 | 20 | 4:1 | 12.5 |
| Example 146 | Compound 5 | DOPE | 35/15/48.5/1.5 | 20 | 4:1 | 12.5 |
| Example 147 | Compound 4 | DOPE | 35/15/48.5/1.5 | 20 | 4:1 | 12.5 |
| Example 148 | Compound 47 | DOPE | 35/15/48.5/1.5 | 20 | 4:1 | 12.5 |
| Example 149 | Compound 48 | DOPE | 35/15/48.5/1.5 | 20 | 4:1 | 12.5 |
| Example 150 | Compound 49 | DOPE | 35/15/48.5/1.5 | 20 | 4:1 | 12.5 |
| Example 151 | Compound 50 | DOPE | 35/15/48.5/1.5 | 20 | 4:1 | 12.5 |
| Example 152 | Compound 51 | DOPE | 35/15/48.5/1.5 | 20 | 4:1 | 12.5 |
| Example 153 | Compound 52 | DOPE | 35/15/48.5/1.5 | 20 | 4:1 | 12.5 |
| Example 154 | Compound 53 | DOPE | 35/15/48.5/1.5 | 20 | 4:1 | 12.5 |
| Example 155 | Compound 54 | DOPE | 35/15/48.5/1.5 | 20 | 4:1 | 12.5 |
| Example 156 | Compound 6 | DOPE | 35/15/48.5/1.5 | 20 | 4:1 | 12.5 |
| Example 157 | Compound 55 | DSPC | 40/10/48.5/1.5 | 20 | 4:1 | 12.5 |
| Example 158 | Compound 55 | DOPE | 35/15/48.5/1.5 | 20 | 4:1 | 12.5 |

### <Encapsulation of GFP pDNA in empty LNP (post-addition method)>

GFP pDNA (GenScript, custom synthesized plasmid DNA) was diluted with water for injection to prepare a DNA solution. The empty LNP stored at -70°C was thawed at 4°C. The DNA solution was added to the present LNP solution in an equal liquid amount and mixed by pipetting (LNP-DNA mixed solution). After allowing the mixture to stand at room temperature for 5 minutes, a 20 mmol/L Tris buffer (pH 8.4) containing 8% sucrose was added to the present LNP-DNA mixed solution in an equal liquid amount, and the mixture was mixed by pipetting, thereby preparing GFP pDNA-encapsulating LNP by a post-addition method.

**[Table 2]**

| GFP pDNA-encapsulating lipid particles prepared by post-addition method | | | | | |
|---|---|---|---|---|---|
| | Compound No. | Helper lipid | Formulation of ionizable lipid/helper lipid/cholesterol/DMG-PEG2000 (mol%) | Lipid/DNA (wt/wt) | Oil-phase lipid concentration (mM) |
| Example 203 | Compound 32 | DOPE | 40/5/53.5/1.5 | 20 | 12.5 |
| Example 204 | Compound 32 | DOPE | 40/10/48.5/1.5 | 20 | 12.5 |
| Example 205 | Compound 32 | DOPE | 40/20/38.5/1.5 | 20 | 12.5 |
| Example 206 | Compound 32 | DOPE | 40/30/28.5/1.5 | 20 | 12.5 |
| Example 207 | Compound 32 | DOPE | 40/40/18.5/1.5 | 20 | 12.5 |
| Example 208 | Compound 5 | DOPE | 40/5/53.5/1.5 | 20 | 12.5 |
| Example 209 | Compound 5 | DOPE | 40/10/48.5/1.5 | 20 | 12.5 |
| Example 210 | Compound 5 | DOPE | 40/20/38.5/1.5 | 20 | 12.5 |
| Example 211 | Compound 5 | DOPE | 40/30/28.5/1.5 | 20 | 12.5 |
| Example 212 | Compound 5 | DOPE | 40/40/18.5/1.5 | 20 | 12.5 |
| Example 213 | Compound 31 | DOPE | 40/5/53.5/1.5 | 20 | 12.5 |
| Example 214 | Compound 31 | DOPE | 40/10/48.5/1.5 | 20 | 12.5 |
| Example 215 | Compound 31 | DOPE | 40/20/38.5/1.5 | 20 | 12.5 |
| Example 216 | Compound 31 | DOPE | 40/30/28.5/1.5 | 20 | 12.5 |
| Example 217 | Compound 31 | DOPE | 40/40/18.5/1.5 | 20 | 12.5 |
| Example 218 | Compound 5 | DOPE | 40/0/58.5/1.5 | 20 | 12.5 |
| Example 219 | Compound 5 | DOPE | 40/2.5/56/1.5 | 20 | 12.5 |
| Example 220 | Compound 31 | DOPE | 40/2.5/56/1.5 | 20 | 12.5 |
| Example 221 | Compound 32 | DOPE | 40/2.5/56/1.5 | 20 | 12.5 |
| Example 222 | Compound 36 | DSPC | 40/0/58.5/1.5 | 20 | 12.5 |
| Example 223 | Compound 36 | DSPC | 40/2.5/56/1.5 | 20 | 12.5 |
| Example 224 | Compound 38 | DSPC | 40/5/53.5/1.5 | 20 | 12.5 |
| Example 225 | Compound 36 | DSPC | 40/10/48.5/1.5 | 20 | 12.5 |
| Example 226 | Compound 36 | DSPC | 40/20/38.5/1.5 | 20 | 12.5 |
| Example 227 | Compound 36 | DSPC | 40/30/28.5/1.5 | 20 | 12.5 |
| Example 228 | Compound 36 | DSPC | 40/40/18.5/1.5 | 20 | 12.5 |
| Example 229 | Compound 36 | DSPC | 40/50/8.5/1.5 | 20 | 12.5 |
| Example 230 | Compound 36 | DSPC | 40/58.5/0/1.5 | 20 | 12.5 |
| Example 231 | Compound 9 | DSPC | 40/0/58.5/1.5 | 20 | 12.5 |
| Example 232 | Compound 9 | DSPC | 40/2.5/56/1.5 | 20 | 12.5 |
| Example 233 | Compound 9 | DSPC | 40/5/53.5/1.5 | 20 | 12.5 |
| Example 234 | Compound 9 | DSPC | 40/10/48.5/1.5 | 20 | 12.5 |
| Example 235 | Compound 9 | DSPC | 40/20/38.5/1.5 | 20 | 12.5 |
| Example 236 | Compound 9 | DSPC | 40/30/28.5/1.5 | 20 | 12.5 |
| Example 237 | Compound 9 | DSPC | 40/40/18.5/1.5 | 20 | 12.5 |
| Example 238 | Compound 9 | DSPC | 40/50/8.5/1.5 | 20 | 12.5 |
| Example 239 | Compound 9 | DSPC | 40/58.5/0/1.5 | 20 | 12.5 |
| Example 240 | Compound 5 | DOPE | 40/5/53.5/1.5 | 20 | 12.5 |
| Example 241 | Compound 5 | DOPE | 40/10/48.5/1.5 | 20 | 12.5 |
| Example 242 | Compound 5 | DOPE | 40/20/38.5/1.5 | 20 | 12.5 |
| Example 243 | Compound 5 | DOPE | 40/30/28.5/1.5 | 20 | 12.5 |
| Example 244 | Compound 5 | DOPE | 40/40/18.5/1.5 | 20 | 12.5 |
| Example 245 | Compound 5 | DOPE | 40/5/53.5/1.5 | 20 | 12.5 |
| Example 246 | Compound 5 | DOPE | 40/10/48.5/1.5 | 20 | 12.5 |
| Example 247 | Compound 5 | DOPE | 35/15/48.5/1.5 | 20 | 62.5 |
| Example 248 | Compound 5 | DOPE | 35/15/48.5/1.5 | 20 | 62.5 |
| Example 249 | Compound 5 | DOPE | 40/5/53.5/1.5 | 20 | 62.5 |
| Example 250 | Compound 5 | DOPE | 40/10/48.5/1.5 | 20 | 62.5 |
| Example 251 | Compound 5 | DOPE | 40/20/38.5/1.5 | 20 | 62.5 |
| Example 252 | Compound 5 | DOPE | 36.5/10/48.5/1.5 | 20 | 62.5 |
| Example 253 | Compound 5 | DOPE | 35/15/48.5/1.5 | 20 | 62.5 |
| Example 254 | Compound 5 | DOPE | 35/15/48.5/1.5 | 20 | 62.5 |
| Example 255 | Compound 5 | DOPE | 40/5/53.5/1.5 | 20 | 62.5 |
| Example 256 | Compound 5 | DOPE | 40/10/48.5/1.5 | 20 | 62.5 |
| Example 257 | Compound 5 | DOPE | 40/20/38.5/1.5 | 20 | 62.5 |
| Example 258 | Compound 5 | DOPE | 36.5/10/48.5/1.5 | 20 | 62.5 |
| Example 259 | Compound 5 | DOPE | 35/15/48.5/1.5 | 20 | 62.5 |
| Example 260 | Compound 5 | DOPE | 35/15/48.5/1.5 | 20 | 62.5 |
| Example 261 | Compound 5 | DOPE | 40/5/53.5/1.5 | 20 | 62.5 |
| Example 262 | Compound 5 | DOPE | 40/10/48.5/1.5 | 20 | 62.5 |
| Example 263 | Compound 5 | DOPE | 40/20/38.5/1.5 | 20 | 62.5 |
| Example 264 | Compound 5 | DOPE | 36.5/10/48.5/1.5 | 20 | 62.5 |
| Example 265 | Compound 5 | DOPE | 35/15/48.5/1.5 | 20 | 62.5 |
| Example 266 | Compound 5 | DOPE | 35/15/48.5/1.5 | 20 | 62.5 |
| Example 267 | Compound 5 | DOPE | 40/5/53.5/1.5 | 20 | 62.5 |
| Example 268 | Compound 5 | DOPE | 40/10/48.5/1.5 | 20 | 62.5 |
| Example 269 | Compound 5 | DOPE | 40/20/38.5/1.5 | 20 | 62.5 |
| Example 270 | Compound 5 | DOPE | 36.5/10/48.5/1.5 | 20 | 62.5 |
| Example 271 | Compound 5 | DOPE | 35/15/48.5/1.5 | 20 | 62.5 |
| Example 272 | Compound 5 | DOPE | 35/15/48.5/1.5 | 20 | 62.5 |
| Example 273 | Compound 5 | DOPE | 40/5/53.5/1.5 | 20 | 62.5 |
| Example 274 | Compound 5 | DOPE | 40/10/48.5/1.5 | 20 | 62.5 |
| Example 275 | Compound 5 | DOPE | 40/20/38.5/1.5 | 20 | 62.5 |
| Example 276 | Compound 5 | DOPE | 36.5/10/48.5/1.5 | 20 | 62.5 |
| Example 277 | Compound 5 | DOPE | 35/15/48.5/1.5 | 20 | 62.5 |
| Example 278 | Compound 5 | DOPE | 35/15/48.5/1.5 | 20 | 62.5 |
| Example 279 | Compound 5 | DOPE | 40/5/53.5/1.5 | 20 | 62.5 |
| Example 280 | Compound 5 | DOPE | 40/10/48.5/1.5 | 20 | 62.5 |
| Example 281 | Compound 5 | DOPE | 40/20/38.5/1.5 | 20 | 62.5 |
| Example 282 | Compound 5 | DOPE | 36.5/10/48.5/1.5 | 20 | 62.5 |
| Example 283 | Compound 5 | DOPE | 35/15/48.5/1.5 | 20 | 62.5 |
| Example 284 | Compound 5 | DOPE | 35/15/48.5/1.5 | 20 | 62.5 |
| Example 285 | Compound 5 | DOPE | 40/5/53.5/1.5 | 20 | 62.5 |
| Example 286 | Compound 5 | DOPE | 40/10/48.5/1.5 | 20 | 62.5 |
| Example 287 | Compound 5 | DOPE | 40/20/38.5/1.5 | 20 | 62.5 |
| Example 288 | Compound 5 | DOPE | 36.5/10/48.5/1.5 | 20 | 62.5 |
| Example 289 | Compound 5 | DOPE | 35/15/48.5/1.5 | 20 | 62.5 |
| Example 290 | Compound 5 | DOPE | 35/15/48.5/1.5 | 20 | 62.5 |
| Example 291 | Compound 5 | DOPE | 35/15/48.5/1.5 | 20 | 62.5 |
| Example 292 | Compound 13 | DSPC | 50/10/38.5/1.5 | 20 | 12.5 |
| Example 293 | Compound 56 | DSPC | 50/10/38.5/1.5 | 20 | 12.5 |
| Example 294 | Compound 5 | DOPE | 35/15/48.5/1.5 | 20 | 62.5 |
| Example 295 | Compound 5 | DOPE | 35/15/48.5/1.5 | 20 | 62.5 |
| Example 296 | Compound 5 | DOPE | 35/15/48.5/1.5 | 20 | 62.5 |
| Example 297 | Compound 13 | DSPC | 50/10/38.5/1.5 | 20 | 12.5 |
| Example 298 | Compound 57 | DOPC | 52.5/7.5/40/1.5 | 20 | 12.5 |
| Example 299 | Compound 5 | DOPE | 35/15/48.5/1.5 | 20 | 62.5 |
| Example 300 | Compound 5 | DOPE | 35/15/48.5/1.5 | 20 | 62.5 |
| Example 301 | Compound 5 | DOPE | 35/15/48.5/1.5 | 20 | 62.5 |
| Example 302 | Compound 13 | DSPC | 50/10/38.5/1.5 | 20 | 12.5 |
| Example 303 | Compound 56 | DSPC | 50/10/38.5/1.5 | 20 | 12.5 |
| Example 304 | Compound 57 | DOPC | 52.5/7.5/40/1.5 | 20 | 12.5 |
| Example 305 | Compound 36 | DSPC | 40/0/58.5/1.5 | 20 | 12.5 |
| Example 306 | Compound 36 | DSPC | 40/2.5/56/1.5 | 20 | 12.5 |
| Example 307 | Compound 36 | DSPC | 40/5/53.5/1.5 | 20 | 12.5 |
| Example 308 | Compound 36 | DSPC | 40/10/48.5/1.5 | 20 | 12.5 |
| Example 309 | Compound 36 | DSPC | 40/20/38.5/1.5 | 20 | 12.5 |
| Example 310 | Compound 9 | DSPC | 40/0/58.5/1.5 | 20 | 12.5 |
| Example 311 | Compound 9 | DSPC | 40/2.5/56/1.5 | 20 | 12.5 |
| Example 312 | Compound 9 | DSPC | 40/5/53.5/1.5 | 20 | 12.5 |
| Example 313 | Compound 9 | DSPC | 40/10/48.5/1.5 | 20 | 12.5 |
| Example 314 | Compound 9 | DSPC | 40/20/38.5/1.5 | 20 | 12.5 |
| Example 315 | Compound 5 | DOPE | 35/15/48.5/1.5 | 20 | 62.5 |
| Example 316 | Compound 5 | DOPE | 35/15/48.5/1.5 | 20 | 62.5 |
| Example 317 | Compound 5 | DOPE | 35/15/48.5/1.5 | 20 | 62.5 |
| Example 318 | Compound 21 | DSPC | 40/10/48.5/1.5 | 20 | 12.5 |
| Example 319 | Compound 27 | DSPC | 40/10/48.5/1.5 | 20 | 12.5 |
| Example 320 | Compound 30 | DSPC | 40/10/48.5/1.5 | 20 | 12.5 |
| Example 321 | Compound 31 | DSPC | 40/10/48.5/1.5 | 20 | 12.5 |
| Example 322 | Compound 32 | DSPC | 40/10/48.5/1.5 | 20 | 12.5 |
| Example 323 | Compound 18 | DOPE | 40/10/48.5/1.5 | 20 | 12.5 |
| Example 324 | Compound 19 | DOPE | 40/10/48.5/1.5 | 20 | 12.5 |
| Example 325 | Compound 20 | DOPE | 40/10/48.5/1.5 | 20 | 12.5 |
| Example 326 | Compound 21 | DOPE | 40/10/48.5/1.5 | 20 | 12.5 |
| Example 327 | Compound 22 | DOPE | 40/10/48.5/1.5 | 20 | 12.5 |
| Example 328 | Compound 24 | DOPE | 40/10/48.5/1.5 | 20 | 12.5 |
| Example 329 | Compound 25 | DOPE | 40/10/48.5/1.5 | 20 | 12.5 |
| Example 330 | Compound 26 | DOPE | 40/10/48.5/1.5 | 20 | 12.5 |
| Example 331 | Compound 27 | DOPE | 40/10/48.5/1.5 | 20 | 12.5 |
| Example 332 | Compound 28 | DOPE | 40/10/48.5/1.5 | 20 | 12.5 |
| Example 333 | Compound 29 | DOPE | 40/10/48.5/1.5 | 20 | 12.5 |
| Example 334 | Compound 30 | DOPE | 40/10/48.5/1.5 | 20 | 12.5 |
| Example 335 | Compound 31 | DOPE | 40/10/48.5/1.5 | 20 | 12.5 |
| Example 336 | Compound 32 | DOPE | 40/10/48.5/1.5 | 20 | 12.5 |
| Example 337 | Compound 15 | DOPE | 35/15/48.5/1.5 | 20 | 12.5 |
| Example 338 | Compound 15 | DOPE | 40/10/48.5/1.5 | 20 | 12.5 |
| Example 339 | Compound 15 | DSPC | 40/10/48.5/1.5 | 20 | 12.5 |
| Example 340 | Compound 5 | DOPE | 35/15/48.5/1.5 | 20 | 12.5 |
| Example 341 | Compound 33 | DSPC | 40/10/48.5/1.5 | 20 | 12.5 |
| Example 342 | Compound 35 | DSPC | 40/10/48.5/1.5 | 20 | 12.5 |
| Example 343 | Compound 36 | DSPC | 40/10/48.5/1.5 | 20 | 12.5 |
| Example 344 | Compound 38 | DSPC | 40/10/48.5/1.5 | 20 | 12.5 |
| Example 345 | Compound 39 | DSPC | 40/10/48.5/1.5 | 20 | 12.5 |
| Example 346 | Compound 40 | DSPC | 40/10/48.5/1.5 | 20 | 12.5 |
| Example 347 | Compound 9 | DSPC | 33.7/10/48.5/1.5 | 20 | 11.7 |
| Example 348 | Compound 41 | DSPC | 40/10/48.5/1.5 | 20 | 12.5 |
| Example 349 | Compound 42 | DSPC | 40/10/48.5/1.5 | 20 | 12.5 |
| Example 350 | Compound 43 | DSPC | 40/10/48.5/1.5 | 20 | 12.5 |
| Example 351 | Compound 44 | DSPC | 40/10/48.5/1.5 | 20 | 12.5 |
| Example 352 | Compound 45 | DOPE | 40/5/55/1.5 | 20 | 12.5 |
| Example 353 | Compound 45 | DSPC | 40/5/55/1.5 | 20 | 12.5 |
| Example 354 | Compound 45 | DPPC | 40/5/55/1.5 | 20 | 12.5 |
| Example 355 | Compound 45 | DMPC | 40/5/55/1.5 | 20 | 12.5 |
| Example 356 | Compound 45 | DOPC | 40/5/55/1.5 | 20 | 12.5 |
| Example 357 | Compound 45 | DOPE | 40/10/50/1.5 | 20 | 12.5 |
| Example 358 | Compound 45 | DSPC | 40/10/50/1.5 | 20 | 12.5 |
| Example 359 | Compound 45 | DOPC | 40/10/50/1.5 | 20 | 12.5 |
| Example 360 | Compound 46 | DOPE | 40/5/55/1.5 | 20 | 12.5 |
| Example 361 | Compound 46 | DOPC | 40/5/55/1.5 | 20 | 12.5 |
| Example 362 | Compound 46 | DOPE | 40/10/50/1.5 | 20 | 12.5 |
| Example 363 | Compound 46 | DSPC | 40/10/50/1.5 | 20 | 12.5 |
| Example 364 | Compound 46 | DPPC | 40/10/50/1.5 | 20 | 12.5 |
| Example 365 | Compound 46 | DOPC | 40/10/50/1.5 | 20 | 12.5 |
| Example 366 | Compound 46 | DOPE | 40/30/30/1.5 | 20 | 12.5 |
| Example 367 | Compound 46 | DPPC | 40/30/30/1.5 | 20 | 12.5 |
| Example 368 | Compound 17 | DSPC | 40/10/48.5/1.5 | 20 | 12.5 |
| Example 369 | Compound 5 | DOPE | 35/15/48.5/1.5 | 20 | 62.5 |
| Example 370 | Compound 10 | DOPE | 35/15/48.5/1.5 | 20 | 12.5 |
| Example 371 | Compound 8 | DOPE | 35/15/48.5/1.5 | 20 | 12.5 |
| Example 372 | Compound 14 | DOPE | 35/15/48.5/1.5 | 20 | 12.5 |
| Example 373 | Compound 15 | DOPE | 35/15/48.5/1.5 | 20 | 12.5 |
| Example 374 | Compound 16 | DOPE | 35/15/48.5/1.5 | 20 | 12.5 |
| Example 375 | Compound 17 | DOPE | 35/15/48.5/1.5 | 20 | 12.5 |
| Example 376 | Compound 5 | DOPE | 35/15/48.5/1.5 | 20 | 62.5 |
| Example 377 | Compound 32 | DOPE | 40/10/48.5/1.5 | 20 | 12.5 |
| Example 378 | Compound 36 | DSPC | 40/10/48.5/1.5 | 20 | 12.5 |
| Example 379 | Compound 5 | DOPE | 35/15/48.5/1.5 | 20 | 12.5 |
| Example 380 | Compound 4 | DOPE | 35/15/48.5/1.5 | 20 | 12.5 |
| Example 381 | Compound 47 | DOPE | 35/15/48.5/1.5 | 20 | 12.5 |
| Example 382 | Compound 48 | DOPE | 35/15/48.5/1.5 | 20 | 12.5 |
| Example 383 | Compound 49 | DOPE | 35/15/48.5/1.5 | 20 | 12.5 |
| Example 384 | Compound 50 | DOPE | 35/15/48.5/1.5 | 20 | 12.5 |
| Example 385 | Compound 51 | DOPE | 35/15/48.5/1.5 | 20 | 12.5 |
| Example 386 | Compound 52 | DOPE | 35/15/48.5/1.5 | 20 | 12.5 |
| Example 387 | Compound 53 | DOPE | 35/15/48.5/1.5 | 20 | 12.5 |
| Example 388 | Compound 54 | DOPE | 35/15/48.5/1.5 | 20 | 12.5 |
| Example 389 | Compound 6 | DOPE | 35/15/48.5/1.5 | 20 | 12.5 |
| Example 390 | Compound 55 | DSPC | 40/10/48.5/1.5 | 20 | 12.5 |
| Example 391 | Compound 55 | DOPE | 35/15/48.5/1.5 | 20 | 12.5 |

### <Preparation of Cas9 mRNA or GFP mRNA-encapsulating lipid particles (conventional method)>

The ionizable lipid, DOPE (L-α-dioleoyl phosphatidylethanolamine, product name: COATSOME (R) ME-8181, manufactured by NOF Corporation) or DSPC (1,2-distearoyl-sn-glycero-3-phosphocholine, product name: COATSOME (R) MC-8080, manufactured by NOF Corporation), cholesterol, and DMG-PEG2000 (1,2-dimyristoyl-rac-glycero-3-methoxypolyethylene glycol-2000, product name: SUNBRIGHT (R) GM-020, manufactured by NOF Corporation) described in Table 3 and Table 4 were dissolved in ethanol at a molar ratio described in Table 2 and Table 3 so that the total lipid concentration was 12.5 mmol/L, thereby obtaining an oil phase.

A nucleic acid mixed solution (Cas9/sgRNA) obtained by mixing CleanCap (registered trademark) Cas9 mRNA (5 moU) (TriLink, L-7206) and a sgRNA (sequence; A*G*A*GUCUCUCAGCUGGUACA + modified Scaffold, Thermo Fisher A35514, custom synthesized) targeting a human T cell receptor alpha constant (TRAC) gene at a weight ratio described in Table 3 and Table 4, or GFP mRNA (product name: CleanCap GFP mRNA; manufactured by TriLink) was diluted with a 50 mmol/L citrate buffer at pH 4 so that the weight ratio of the total lipid concentration after mixing the oil phase and the water phase to the mRNA concentration was as described in Table 2 and Table 3, thereby obtaining a water phase. Subsequently, the water phase and the oil phase were mixed using NanoAssemblr (Precision NanoSystems) so that the volume ratio of the water phase to the oil phase was water phase:oil phase = 3:1, and the mixed solution was diluted 2-fold with water to obtain a dispersion liquid of mRNA lipid particles. The dispersion liquid was dialyzed against a 20 mmol/L Tris buffer solution at pH 7.4 containing 8% sucrose using a dialysis cassette (Slide-A-Lyzer G2, MWCO: 10 kD, Thermo Fisher Scientific) to remove ethanol, thereby obtaining nucleic acid-encapsulating lipid particles by the conventional method.

**[Table 3]**

| Cas9 mRNA/sgRNA-encapsulating lipid particles prepared by conventional method | | | | | | |
|---|---|---|---|---|---|---|
| | Compound No. | Helper lipid | Formulation of ionizable lipid/helper lipid/cholesterol/DMG-PEG2000 (mol%) | Lipid/RNA (wt/wt) | Cas9 mRNA: sgRNA (wt/wt) | Oil-phase lipid concentration (mM) |
| Comparative Example 2 | Compound 13 | DSPC | 50/10/38.5/1.5 | 20 | 4:1 | 12.5 |
| Comparative Example 2 | Compound 13 | DSPC | 50/10/38.5/1.5 | 20 | 4:1 | 12.5 |
| Comparative Example 3 | Compound 13 | DSPC | 35/1.5/47.5/2.5 | 20 | 4:1 | 12.5 |
| Example 9 | Compound 5 | DOPE | 40/10/38.5/1.5 | 20 | 4:1 | 12.5 |
| Example 9 | Compound 5 | DOPE | 40/10/38.5/1.5 | 20 | 4:1 | 12.5 |
| Example 10 | Compound 5 | DOPE | 35/15/48.5/1.5 | 20 | 4:1 | 12.5 |
| Example 11 | Compound 5 | DOPE | 30/20/48.5/1.5 | 20 | 4:1 | 12.5 |
| Example 12 | Compound 5 | DSPC | 50/10/38.5/1.5 | 20 | 4:1 | 12.5 |
| Example 13 | Compound 5 | DOPE | 50/10/38.5/1.5 | 20 | 4:1 | 12.5 |
| Example 13 | Compound 5 | DOPE | 50/10/38.5/1.5 | 20 | 4:1 | 12.5 |
| Example 14 | Compound 5 | DOPE | 40/20/38.5/1.5 | 20 | 4:1 | 12.5 |
| Example 15 | Compound 5 | DOPE | 35/10/53.5/1.5 | 20 | 4:1 | 12.5 |
| Example 16 | Compound 5 | DOPE | 30/10/58.5/1.5 | 20 | 4:1 | 12.5 |
| Example 17 | Compound 5 | DOPE | 40/10/48.5/1.5 | 30 | 4:1 | 12.5 |
| Example 18 | Compound 5 | DOPE | 40/10/48.5/1.5 | 40 | 4:1 | 12.5 |
| Example 19 | Compound 5 | DOPE | 40/10/47/3 | 20 | 4:1 | 12.5 |

**[Table 4]**

| GFP mRNA-encapsulating lipid particles prepared by conventional method | | | | | |
|---|---|---|---|---|---|
| | Compound No. | Helper lipid | Formulation of ionizable lipid/helper lipid/cholesterol/DMG-PEG2000 (mol%) | Lipid/RNA (wt/wt) | Oil-phase lipid concentration (mM) |
| Example 28 | Compound 9 | DSPC | 50/10/38.5/1.5 | 20 | 12.5 |
| Example 29 | Compound 10 | DSPC | 50/10/38.5/1.5 | 20 | 12.5 |
| Example 30 | Compound 11 | DSPC | 50/10/38.5/1.5 | 20 | 12.5 |
| Example 31 | Compound 12 | DSPC | 50/10/38.5/1.5 | 20 | 12.5 |
| Example 32 | Compound 5 | DOPE | 50/10/38.5/1.5 | 20 | 12.5 |
| Example 33 | Compound 6 | DOPE | 50/10/38.5/1.5 | 20 | 12.5 |
| Example 34 | Compound 7 | DOPE | 50/10/38.5/1.5 | 20 | 12.5 |
| Example 35 | Compound 11 | DOPE | 50/10/38.5/1.5 | 20 | 12.5 |

### <Preparation of GFP pDNA-encapsulating lipid particles (conventional method)>

The ionizable lipid, DOPE (L-α-dioleoyl phosphatidylethanolamine, product name: COATSOME (R) ME-8181, manufactured by NOF Corporation) or DSPC (1,2-distearoyl-sn-glycero-3-phosphocholine, product name: COATSOME (R) MC-8080, manufactured by NOF Corporation), cholesterol, and DMG-PEG2000 (1,2-dimyristoyl-rac-glycero-3-methoxypolyethylene glycol-2000, product name: SUNBRIGHT (R) GM-020, manufactured by NOF Corporation) described in Table 5 were dissolved in ethanol at a molar ratio described in Table 5 so that the total lipid concentration was 12.5 mmol/L, thereby obtaining an oil phase.

GFP pDNA (GenScript, custom synthesized plasmid DNA) was diluted with a 50 mmol/L citrate buffer at pH 4 so that the weight ratio of the total lipid concentration after mixing the oil phase and the water phase to the DNA concentration was as described in Table 5, thereby obtaining a water phase. Subsequently, the water phase and the oil phase were mixed using NanoAssemblr (Precision NanoSystems) so that the volume ratio of the water phase to the oil phase was water phase:oil phase = 3:1, and the mixed solution was diluted 2-fold with water to obtain a dispersion liquid of pDNA lipid particles. The dispersion liquid was dialyzed against a 20 mmol/L Tris buffer solution at pH 7.4 containing 8% sucrose using a dialysis cassette (Slide-A-Lyzer G2, MWCO: 10 kD, Thermo Fisher Scientific) to remove ethanol, thereby obtaining nucleic acid-encapsulating lipid particles by the conventional method.

**[Table 5]**

| GFP pDNA-encapsulating lipid particles prepared by conventional method | | | | | |
|---|---|---|---|---|---|
| | Compound No. | Helper lipid | Formulation of ionizable lipid/helper lipid/cholesterol/DMG-PEG2000 (mol%) | Lipid/DNA (wt/wt) | Oil-phase lipid concentration (mM) |
| Example 159 | Compound 32 | DOPE | 40/5/53.5/1.5 | 20 | 12.5 |
| Example 160 | Compound 32 | DOPE | 40/10/48.5/1.5 | 20 | 12.5 |
| Example 161 | Compound 32 | DOPE | 40/20/38.5/1.5 | 20 | 12.5 |
| Example 162 | Compound 32 | DOPE | 40/30/28.5/1.5 | 20 | 12.5 |
| Example 163 | Compound 32 | DOPE | 40/40/18.5/1.5 | 20 | 12.5 |
| Example 164 | Compound 5 | DOPE | 40/5/53.5/1.5 | 20 | 12.5 |
| Example 165 | Compound 5 | DOPE | 40/10/48.5/1.5 | 20 | 12.5 |
| Example 166 | Compound 5 | DOPE | 40/20/38.5/1.5 | 20 | 12.5 |
| Example 167 | Compound 5 | DOPE | 40/30/28.5/1.5 | 20 | 12.5 |
| Example 168 | Compound 5 | DOPE | 40/40/18.5/1.5 | 20 | 12.5 |
| Example 169 | Compound 31 | DOPE | 40/5/53.5/1.5 | 20 | 12.5 |
| Example 170 | Compound 31 | DOPE | 40/10/48.5/1.5 | 20 | 12.5 |
| Example 171 | Compound 31 | DOPE | 40/20/38.5/1.5 | 20 | 12.5 |
| Example 172 | Compound 31 | DOPE | 40/30/28.5/1.5 | 20 | 12.5 |
| Example 173 | Compound 31 | DOPE | 40/40/18.5/1.5 | 20 | 12.5 |
| Example 174 | Compound 5 | DOPE | 40/0/58.5/1.5 | 20 | 12.5 |
| Example 175 | Compound 5 | DOPE | 40/2.5/56/1.5 | 20 | 12.5 |
| Example 176 | Compound 5 | DOPE | 40/50/8.5/1.5 | 20 | 12.5 |
| Example 177 | Compound 5 | DOPE | 40/58.5/0/1.5 | 20 | 12.5 |
| Example 178 | Compound 31 | DOPE | 40/0/58.5/1.5 | 20 | 12.5 |
| Example 179 | Compound 31 | DOPE | 40/2.5/56/1.5 | 20 | 12.5 |
| Example 180 | Compound 31 | DOPE | 40/50/8.5/1.5 | 20 | 12.5 |
| Example 181 | Compound 31 | DOPE | 40/58.5/0/1.5 | 20 | 12.5 |
| Example 182 | Compound 32 | DOPE | 40/0/58.5/1.5 | 20 | 12.5 |
| Example 183 | Compound 32 | DOPE | 40/2.5/56/1.5 | 20 | 12.5 |
| Example 184 | Compound 32 | DOPE | 40/50/8.5/1.5 | 20 | 12.5 |
| Example 185 | Compound 32 | DOPE | 40/58.5/0/1.5 | 20 | 12.5 |
| Example 185 | Compound 32 | DOPE | 40/58.5/0/1.5 | 20 | 12.5 |
| Example 186 | Compound 36 | DSPC | 40/2.5/56/1.5 | 20 | 12.5 |
| Example 187 | Compound 36 | DSPC | 40/5/53.5/1.5 | 20 | 12.5 |
| Example 188 | Compound 36 | DSPC | 40/10/48.5/1.5 | 20 | 12.5 |
| Example 189 | Compound 36 | DSPC | 40/20/38.5/1.5 | 20 | 12.5 |
| Example 190 | Compound 36 | DSPC | 40/30/28.5/1.5 | 20 | 12.5 |
| Example 191 | Compound 36 | DSPC | 40/40/18.5/1.5 | 20 | 12.5 |
| Example 192 | Compound 36 | DSPC | 40/50/8.5/1.5 | 20 | 12.5 |
| Example 193 | Compound 36 | DSPC | 40/58.5/0/1.5 | 20 | 12.5 |
| Example 194 | Compound 9 | DSPC | 40/0/58.5/1.5 | 20 | 12.5 |
| Example 195 | Compound 9 | DSPC | 40/2.5/56/1.5 | 20 | 12.5 |
| Example 196 | Compound 9 | DSPC | 40/5/53.5/1.5 | 20 | 12.5 |
| Example 197 | Compound 9 | DSPC | 40/10/48.5/1.5 | 20 | 12.5 |
| Example 198 | Compound 9 | DSPC | 40/20/38.5/1.5 | 20 | 12.5 |
| Example 199 | Compound 9 | DSPC | 40/30/28.5/1.5 | 20 | 12.5 |
| Example 200 | Compound 9 | DSPC | 40/40/18.5/1.5 | 20 | 12.5 |
| Example 201 | Compound 9 | DSPC | 40/50/8.5/1.5 | 20 | 12.5 |
| Example 202 | Compound 9 | DSPC | 40/58.5/0/1.5 | 20 | 12.5 |

### <Preparation of GenVoy T cell kit>

As a comparative example, a GenVoy T cell kit (Precision nanosystems) was prepared according to a recommended protocol.

Specifically, after all the kit contents were returned to room temperature, the Lipid mix was heated in a hot water bath at 52°C for 10 minutes to be sufficiently melted. A nucleic acid mixed solution (Cas9/sgRNA) obtained by mixing CleanCap (registered trademark) Cas9 mRNA (5 moU) (TriLink, L-7206) and sgRNA (sequence; A*G*A*GUCUCUCAGCUGGUACA + modified Scaffold, Thermo Fisher A35514, custom synthesis) targeting a human T cell receptor alpha constant (TRAC) gene at a weight ratio of 1:1 was diluted with water and 10x formulation buffer to prepare an RNA solution such that the final concentration of RNA was 312 µg/mL.

A 50 mmol/L citrate buffer at pH 4 was mixed using NanoAssemblr Ignite (Precision NanoSystems) so that the volume ratio of the water phase:oil phase was water phase:oil phase = 2:1. The mixed solution was diluted 30 times using a 1x dilution buffer to obtain a dispersion liquid of lipid particles. The dispersion liquid was concentrated using an ultrafiltration filter (Amicon ultra 100 kDa, Merck), and an equal volume of Cryoprotect buffer was added to the concentrated solution to obtain GenVoy (Comparative Example 1). The prepared GenVoy was stored frozen at -70°C until use.

### <Measurement of particle size>

The particle diameter of the nucleic acid-encapsulating lipid particles was measured using a particle diameter measurement system NanoSAQLA (Otsuka Electronics Co., Ltd.) after optionally diluting the lipid particles with phosphate buffered saline (PBS). The measurement results of the particle diameter and the polydispersity index (PDI) are shown in Table 4.

### <Evaluation of encapsulation rate of nucleic acid>

### (Quantification of total nucleic acid concentration)

The nucleic acid was diluted with MilliQ water to prepare a diluted sample in 2-fold dilution series from 100 µg/mL to 3.1 µg/mL, and a standard solution for calibration was prepared. 50 µL of the calibration curve solution or the lipid particles was mixed with 450 µL of methanol to prepare a measurement solution.

The absorbance of each measurement solution at 260 nm and 330 nm was measured using a UV plate reader (Multiskan Go, Thermo Fisher Scientific), the absorbance at 330 nm was subtracted from the absorbance at 260 nm, and the result was defined as the absorbance of each measurement solution. The total RNA concentration was calculated from the calibration curve using the absorbance of each sample measurement solution.

### (Quantification of nucleic acid concentration in outer water phase)

The concentration of the outer water phase nucleic acid was quantified by a standard addition method using a QuanT-iT RiboGreen RNA Assay Kit (Thermo Fisher Scientific). First, a 20× TE buffer included in the above kit was diluted with water, thereby obtaining a 1× TE buffer. TE represents Tris/EDTA (ethylenediaminetetraacetic acid). The nucleic acid was diluted with a TE buffer so that the final concentration was 0 to 400 ng/mL, and a nucleic acid dilution series was prepared. 10 µL of the lipid particles diluted with the TE buffer and 90 µL of the nucleic acid dilution series were mixed in a 96-well plate, 100 µL of a RiboGreen reagent diluted 200 times with the TE buffer was added to each well, and fluorescence (excitation wavelength: 485 nm, fluorescence wavelength: 535 nm) was measured using a fluorescence plate reader (Infinite 200 Pro M nano+, TECAN). From the obtained results, the outer water phase RNA concentration of each measurement solution was calculated according to the standard addition method.

### (Calculation of encapsulation rate)

The RNA encapsulation rate of the nucleic acid lipid nanoparticles was calculated according to the following expression using the quantification results of the total nucleic acid concentration and the nucleic acid concentration in the outer water phase, which were obtained in the above-described steps. 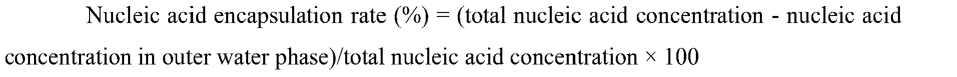 Nucleic acid encapsulation rate (%) = (total nucleic acid concentration - nucleic acid concentration in outer water phase)/total nucleic acid concentration × 100

The results are shown in Table 6.

**[Table 6]**

| | Particle size (nm) | PdI | Encapsulation rate % |
|---|---|---|---|
| Comparative Example 1 | 147 | 0.10 | 92% |
| Comparative Example 2 | 113 | 0.12 | 88% |
| Comparative Example 3 | 86 | 0.17 | 95% |
| Example 1 | 146 | 0.06 | 95% |
| Example 2 | 151 | 0.09 | 96% |
| Example 3 | 110 | 0.03 | 97% |
| Example 4 | 110 | 0.05 | 97% |
| Example 5 | 117 | 0.05 | 94% |
| Example 6 | 129 | 0.16 | 75% |
| Example 7 | 122 | 0.08 | 64% |
| Example 9 | 78 | 0.11 | 97% |
| Example 10 | 67 | 0.07 | 96% |
| Example 11 | 67 | 0.10 | 96% |
| Example 12 | 88 | 0.17 | 84% |
| Example 13 | 86 | 0.12 | 97% |
| Example 14 | 84 | 0.15 | 96% |
| Example 15 | 69 | 0.05 | 96% |
| Example 16 | 66 | 0.07 | 95% |
| Example 17 | 73 | 0.07 | 96% |
| Example 18 | 71 | 0.06 | 96% |
| Example 19 | 56 | 0.11 | 96% |
| Example 20 | 105 | 0.12 | 85% |
| Example 21 | 148 | 0.10 | 65% |
| Example 22 | 125 | 0.10 | 83% |
| Example 23 | 84 | 0.14 | 96% |
| Example 24 | 82 | 0.12 | 95% |
| Example 25 | 86 | 0.13 | 96% |
| Example 26 | 145 | 0.14 | 90% |
| Example 27 | 101 | 0.10 | 88% |
| Example 28 | 83 | 0.14 | 96% |
| Example 29 | 104 | 0.13 | 82% |
| Example 30 | 80 | 0.14 | 97% |
| Example 31 | 85 | 0.13 | 96% |
| Example 32 | 87 | 0.10 | 96% |
| Example 33 | 87 | 0.09 | 97% |
| Example 34 | 108 | 0.13 | 98% |
| Example 35 | 86 | 0.07 | 97% |
| Example 38 | 148 | 0.02 | 86% |
| Example 39 | 155 | 0.09 | 75% |
| Example 40 | 145 | 0.07 | 95% |
| Example 41 | 102 | 0.09 | 96% |
| Example 42 | 133 | 0.08 | 89% |
| Example 43 | 187 | 0.12 | 63% |
| Example 44 | 130 | 0.05 | 64% |
| Example 46 | 138 | 0.04 | 74% |
| Example 48 | 212 | 0.11 | 50% |
| Example 49 | 171 | 0.09 | 54% |
| Example 50 | 148 | 0.13 | 53% |
| Example 52 | 192 | 0.06 | 54% |
| Example 53 | 171 | 0.09 | 92% |
| Example 54 | 132 | 0.10 | 97% |
| Example 55 | 208 | 0.07 | 56% |
| Example 59 | 130 | 0.09 | 83% |
| Example 60 | 106 | 0.05 | 95% |
| Example 63 | 90 | 0.09 | 95% |
| Example 64 | 95 | 0.11 | 92% |
| Example 65 | 114 | 0.07 | 94% |
| Example 66 | 147 | 0.12 | 89% |
| Example 67 | 207 | 0.07 | 55% |
| Example 69 | 147 | 0.16 | 58% |
| Example 70 | 156 | 0.12 | 85% |
| Example 72 | 195 | 0.05 | 57% |
| Example 73 | 169 | 0.03 | 63% |
| Example 76 | 188 | 0.06 | 87% |
| Example 77 | 178 | 0.11 | 66% |
| Example 78 | 130 | 0.08 | 65% |
| Example 79 | 126 | 0.03 | 58% |
| Example 80 | 130 | 0.05 | 78% |
| Example 81 | 168 | 0.08 | 86% |
| Example 83 | 102 | 0.09 | 96% |
| Example 84 | 110 | 0.05 | 97% |
| Example 87 | 106 | 0.09 | 96% |
| Example 88 | 91 | 0.17 | 95% |
| Example 90 | 120 | 0.22 | 91% |
| Example 91 | 90 | 0.21 | 95% |
| Example 92 | 87 | 0.22 | 94% |
| Example 93 | 111 | 0.23 | 95% |
| Example 94 | 161 | 0.16 | 86% |
| Example 95 | 122 | 0.06 | 94% |
| Example 100 | 178 | 0.19 | 50% |
| Example 101 | 163 | 0.16 | 62% |
| Example 102 | 196 | 0.11 | 50% |
| Example 104 | 195 | 0.13 | 53% |
| Example 107 | 179 | 0.16 | 53% |
| Example 113 | 152 | 0.12 | 70% |
| Example 117 | 152 | 0.09 | 74% |
| Example 118 | 179 | 0.09 | 61% |
| Example 128 | 105 | 0.08 | 96% |
| Example 129 | 137 | 0.03 | 98% |
| Example 131 | 138 | 0.04 | 74% |
| Example 133 | 212 | 0.11 | 50% |
| Example 134 | 171 | 0.09 | 54% |
| Example 135 | 148 | 0.13 | 53% |
| Example 136 | 155 | 0.07 | 91% |
| Example 137 | 152 | 0.07 | 96% |
| Example 138 | 155 | 0.07 | 91% |
| Example 139 | 116 | 0.16 | 84% |
| Example 140 | 118 | 0.10 | 89% |
| Example 141 | 154 | 0.26 | 75% |
| Example 143 | 155 | 0.07 | 91% |
| Example 144 | 130 | 0.05 | 78% |
| Example 145 | 91 | 0.17 | 95% |
| Example 157 | 71 | 0.14 | 95% |
| Example 158 | 109 | 0.14 | 74% |
| Example 159 | 102 | 0.14 | 95% |
| Example 160 | 114 | 0.13 | 94% |
| Example 161 | 109 | 0.18 | 96% |
| Example 162 | 113 | 0.13 | 94% |
| Example 163 | 116 | 0.09 | 89% |
| Example 164 | 85 | 0.06 | 96% |
| Example 165 | 80 | 0.06 | 97% |
| Example 166 | 87 | 0.12 | 97% |
| Example 167 | 85 | 0.17 | 94% |
| Example 168 | 96 | 0.14 | 91% |
| Example 169 | 71 | 0.12 | 96% |
| Example 170 | 70 | 0.15 | 97% |
| Example 171 | 80 | 0.06 | 96% |
| Example 172 | 82 | 0.14 | 94% |
| Example 173 | 82 | 0.16 | 94% |
| Example 174 | 85 | 0.04 | 95% |
| Example 175 | 84 | 0.05 | 95% |
| Example 176 | 97 | 0.16 | 92% |
| Example 177 | 94 | 0.17 | 92% |
| Example 178 | 95 | 0.12 | 93% |
| Example 179 | 87 | 0.10 | 96% |
| Example 180 | 92 | 0.13 | 93% |
| Example 181 | 94 | 0.15 | 91% |
| Example 182 | 96 | 0.06 | 76% |
| Example 183 | 88 | 0.10 | 91% |
| Example 184 | 79 | 0.18 | 95% |
| Example 185 | 85 | 0.15 | 92% |
| Example 186 | 71 | 0.11 | 92% |
| Example 187 | 68 | 0.06 | 93% |
| Example 188 | 70 | 0.10 | 93% |
| Example 189 | 65 | 0.15 | 94% |
| Example 190 | 71 | 0.13 | 94% |
| Example 191 | 78 | 0.16 | 92% |
| Example 192 | 104 | 0.10 | 83% |
| Example 193 | 119 | 0.18 | 80% |
| Example 194 | 88 | 0.00 | 95% |
| Example 195 | 78 | 0.09 | 94% |
| Example 196 | 74 | 0.07 | 95% |
| Example 197 | 76 | 0.08 | 94% |
| Example 198 | 69 | 0.15 | 95% |
| Example 199 | 73 | 0.19 | 96% |
| Example 200 | 85 | 0.22 | 96% |
| Example 201 | 112 | 0.16 | 93% |
| Example 202 | 124 | 0.19 | 88% |
| Example 208 | 105 | 0.03 | 96% |
| Example 209 | 113 | 0.01 | 95% |
| Example 213 | 131 | 0.05 | 85% |
| Example 214 | 116 | 0.07 | 72% |
| Example 215 | 99 | 0.14 | 63% |
| Example 216 | 111 | 0.12 | 65% |
| Example 217 | 180 | 0.20 | 68% |
| Example 218 | 109 | 0.03 | 86% |
| Example 219 | 107 | 0.06 | 91% |
| Example 222 | 125 | 0.11 | 91% |
| Example 223 | 109 | 0.11 | 95% |
| Example 224 | 103 | 0.06 | 96% |
| Example 225 | 98 | 0.08 | 97% |
| Example 226 | 84 | 0.12 | 96% |
| Example 227 | 83 | 0.16 | 96% |
| Example 228 | 91 | 0.16 | 91% |
| Example 229 | 105 | 0.13 | 64% |
| Example 231 | 168 | 0.23 | 82% |
| Example 232 | 132 | 0.12 | 96% |
| Example 233 | 122 | 0.09 | 94% |
| Example 234 | 122 | 0.05 | 97% |
| Example 235 | 93 | 0.13 | 96% |
| Example 236 | 90 | 0.14 | 95% |
| Example 237 | 116 | 0.25 | 57% |
| Example 238 | 129 | 0.14 | 73% |
| Example 239 | 225 | 0.25 | 58% |
| Example 240 | 116 | 0.08 | 94% |
| Example 241 | 118 | 0.07 | 95% |
| Example 245 | 116 | 0.04 | 95% |
| Example 246 | 131 | 0.04 | 95% |
| Example 247 | 120 | 0.05 | 95% |
| Example 248 | 124 | 0.05 | 95% |
| Example 249 | 107 | 0.03 | 88% |
| Example 250 | 119 | 0.07 | 93% |
| Example 251 | 143 | 0.03 | 95% |
| Example 252 | 115 | 0.06 | 92% |
| Example 253 | 120 | 0.05 | 95% |
| Example 254 | 124 | 0.05 | 95% |
| Example 255 | 107 | 0.03 | 88% |
| Example 256 | 119 | 0.07 | 93% |
| Example 257 | 143 | 0.03 | 95% |
| Example 258 | 115 | 0.06 | 92% |
| Example 259 | 120 | 0.05 | 95% |
| Example 260 | 124 | 0.05 | 95% |
| Example 261 | 107 | 0.03 | 88% |
| Example 262 | 119 | 0.07 | 93% |
| Example 263 | 143 | 0.03 | 95% |
| Example 264 | 115 | 0.06 | 92% |
| Example 265 | 120 | 0.05 | 95% |
| Example 266 | 124 | 0.05 | 95% |
| Example 267 | 107 | 0.03 | 88% |
| Example 268 | 119 | 0.07 | 93% |
| Example 269 | 143 | 0.03 | 95% |
| Example 270 | 115 | 0.06 | 92% |
| Example 271 | 120 | 0.05 | 95% |
| Example 272 | 124 | 0.05 | 95% |
| Example 273 | 107 | 0.03 | 88% |
| Example 274 | 119 | 0.07 | 93% |
| Example 275 | 143 | 0.03 | 95% |
| Example 276 | 115 | 0.06 | 92% |
| Example 277 | 120 | 0.05 | 95% |
| Example 278 | 124 | 0.05 | 95% |
| Example 279 | 107 | 0.03 | 88% |
| Example 280 | 119 | 0.07 | 93% |
| Example 281 | 143 | 0.03 | 95% |
| Example 282 | 115 | 0.06 | 92% |
| Example 283 | 120 | 0.05 | 95% |
| Example 284 | 124 | 0.05 | 95% |
| Example 285 | 107 | 0.03 | 88% |
| Example 286 | 119 | 0.07 | 93% |
| Example 287 | 143 | 0.03 | 95% |
| Example 288 | 115 | 0.06 | 92% |
| Example 289 | 126 | 0.00 | 93% |
| Example 290 | 129 | 0.01 | 92% |
| Example 291 | 132 | 0.00 | 94% |
| Example 294 | 126 | 0.00 | 93% |
| Example 295 | 129 | 0.01 | 92% |
| Example 296 | 132 | 0.00 | 94% |
| Example 299 | 126 | 0.00 | 93% |
| Example 300 | 129 | 0.01 | 92% |
| Example 301 | 132 | 0.00 | 94% |
| Example 305 | 137 | 0.14 | 91% |
| Example 306 | 114 | 0.12 | 92% |
| Example 307 | 92 | 0.07 | 77% |
| Example 308 | 100 | 0.11 | 96% |
| Example 309 | 86 | 0.17 | 94% |
| Example 310 | 172 | 0.26 | 67% |
| Example 311 | 141 | 0.15 | 92% |
| Example 312 | 126 | 0.18 | 93% |
| Example 313 | 110 | 0.12 | 94% |
| Example 314 | 90 | 0.10 | 96% |
| Example 315 | 126 | 0.00 | 93% |
| Example 316 | 126 | 0.00 | 93% |
| Example 317 | 126 | 0.00 | 93% |
| Example 318 | 132 | 0.10 | 97% |
| Example 319 | 106 | 0.05 | 95% |
| Example 320 | 90 | 0.09 | 95% |
| Example 321 | 95 | 0.11 | 92% |
| Example 322 | 114 | 0.07 | 94% |
| Example 323 | 147 | 0.12 | 89% |
| Example 324 | 207 | 0.07 | 55% |
| Example 326 | 147 | 0.16 | 58% |
| Example 327 | 156 | 0.12 | 85% |
| Example 328 | 195 | 0.05 | 57% |
| Example 329 | 169 | 0.03 | 63% |
| Example 332 | 188 | 0.06 | 87% |
| Example 333 | 178 | 0.11 | 66% |
| Example 334 | 130 | 0.08 | 65% |
| Example 335 | 126 | 0.03 | 58% |
| Example 336 | 130 | 0.05 | 78% |
| Example 337 | 168 | 0.08 | 86% |
| Example 339 | 102 | 0.09 | 96% |
| Example 340 | 110 | 0.05 | 97% |
| Example 342 | 106 | 0.09 | 96% |
| Example 343 | 91 | 0.17 | 95% |
| Example 344 | 120 | 0.22 | 91% |
| Example 345 | 90 | 0.21 | 95% |
| Example 346 | 87 | 0.22 | 94% |
| Example 347 | 111 | 0.23 | 95% |
| Example 348 | 161 | 0.16 | 86% |
| Example 349 | 122 | 0.06 | 94% |
| Example 354 | 178 | 0.19 | 50% |
| Example 355 | 163 | 0.16 | 62% |
| Example 356 | 196 | 0.11 | 50% |
| Example 358 | 195 | 0.13 | 53% |
| Example 359 | 179 | 0.16 | 53% |
| Example 360 | 152 | 0.12 | 70% |
| Example 361 | 152 | 0.09 | 74% |
| Example 362 | 179 | 0.09 | 61% |
| Example 368 | 105 | 0.08 | 96% |
| Example 369 | 137 | 0.03 | 98% |
| Example 371 | 138 | 0.04 | 74% |
| Example 373 | 212 | 0.11 | 50% |
| Example 374 | 171 | 0.09 | 54% |
| Example 375 | 148 | 0.13 | 53% |
| Example 376 | 155 | 0.07 | 91% |
| Example 377 | 130 | 0.05 | 78% |
| Example 378 | 91 | 0.17 | 95% |
| Example 390 | 71 | 0.14 | 95% |
| Example 391 | 109 | 0.14 | 74% |

### [T cell culture and evaluation method]

### [Materials and methods]

### <Preparation of culture medium for activation>

The culture medium used for culturing T cells under the activation culture conditions consists of TexMACStm Medium (Miltenyi Biotec, 130-097-196) and 5 ng/ml human interleukin-2 (IL-2, Roche, 11147528001) (hereinafter, referred to as a culture medium for activation), or consists of PRIME-XV T Cell CDM (FUJIFILM Irvine Scientific, 91154) and 5 ng/ml human interleukin-2 (IL-2, Roche, 11147528001) (hereinafter, referred to as a CDM culture medium for activation).

### <Preparation and activation culture of T cells>

Frozen T cells (Human PB Pan-T, Cryo, STEMCELL Technologies, ST-70024) derived from peripheral blood of a healthy human donor were thawed by being immersed in a water bath at 37°C for several minutes. The thawed T cells were resuspended in TexMACS Medium containing 1% BSA (SIGMA, A9576) and 20 U/ml DNase I (Worthington Biochemical, LS002139), further washed by centrifugation, and resuspended in a culture medium for activation. The T cells were prepared at a cell concentration of 1.0 × 10⁶ cells/ml using the present culture medium, and Dynabeads Human T-Activator CD3/CD28 (Thermo Fisher DB11131) was added thereto such that the concentration thereof was 1.0 × 10⁶ beads/ml, or Immunocult^{™} Human CD3/CD28 T Cell Activator (STEMCELL Technologies, 10971) was added thereto such that the concentration thereof was 25 µL/mL. The cells were seeded in a 24-well plate for cell culture and activated by being cultured in a 37°C, 5% CO₂ incubator for 3 days. On the third day of activation, the Dynabeads were removed from the T cell culture solution. The T cells pretreated as described above were used as activated T cells.

### <Preparation of culture medium for inactivation>

The culture medium used in a case of culturing T cells under inactivation culture conditions consists of TexMACStm Medium (Miltenyi Biotec, 130-097-196), 5 ng/ml human interleukin-2 (IL-2, Roche, 11147528001), 5 ng/ml Human IL-7 (Miltenyi Biotec 130-095-367), and 5 ng/ml Human IL-15 (Miltenyi Biotec, 130-095-760) (hereinafter, culture medium for inactivation).

### <Preparation and inactivation culture of T cells>

Frozen T cells (Human PB Pan-T, Cryo, STEMCELL Technologies, ST-70024) derived from peripheral blood of a healthy human donor were thawed by being placed in a water bath at 37°C for several minutes. The thawed T cells were resuspended in TexMACS Medium containing 1% BSA and 20 U/ml DNase I, further washed by centrifugation, and resuspended in a culture medium for inactivation. The T cells were adjusted to a cell concentration of 1.0 × 10⁶ cells/ml using the present culture medium, seeded in a 24-well plate for cell culture, and cultured in a 37°C, 5% CO₂ incubator for 3 days. The T cells pretreated as described above were used as inactivated T cells.

### <Evaluation of cell viability and cell proliferation rate>

Viable cells and dead cells of the T cells were stained using an Acridine Orange/Propidium Iodide (AO/PI) Cell Viability Kit (Logos Biosystems, F23001). After the staining, the viable cell concentration, the dead cell concentration, and the viability were immediately measured with an automatic cell counter Luna-FL device (Logos Biosystems). The cell viability was calculated as a numerical value in a case where the viability measurement value of the culture medium treatment condition was set to 100%, as shown in the following Expression 1. In addition, the cell proliferation rate on the 7th day after the start of the culture was calculated according to the following Expression 2. 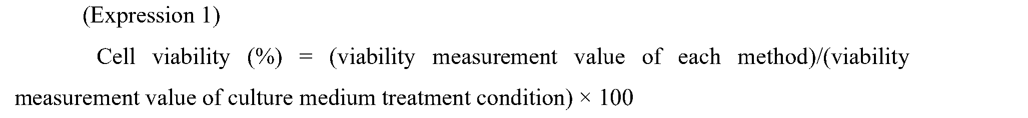 Cell viability (%) = (viability measurement value of each method)/(viability measurement value of culture medium treatment condition) × 100 (Equation 1) 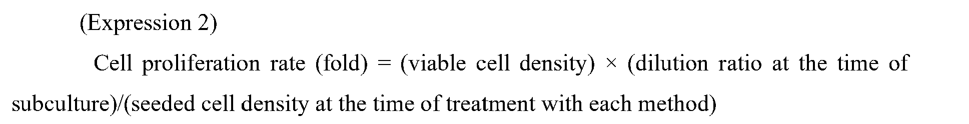 Cell proliferation rate (fold) = (viable cell density) × (dilution ratio at the time of subculture)/(seeded cell density at the time of treatment with each method) (Equation 1)

### <Flow cytometry>

On the 4th day, the 7th day, or the 14th day after the start of the culture, the efficiency of endogenous TCR knockout (KO) or the GFP-positive rate was evaluated by flow cytometry for the T cells edited by each method.

For the evaluation of the TCRKO efficiency, the dead cells of the T cells were stained using BD Horizon^{TM} Fixable Viability Stain (FVS) Reagents (BD, 565388), and then incubated with an antibody targeting TCR. For the evaluation of the GFP-positive rate, the T cells were stained with dead cells using FVS Reagents. After each staining operation, the cells were fixed and washed, and the cell state was analyzed with an Attune device (Thermo Fisher). The analysis data was analyzed using Flowjo software. The T cells were gated by size, single cell, and viable cell, and then the ratio of TCR-negative cells or the ratio of GFP-positive cells and the median fluorescence intensity (MFI) were analyzed.

### <Test Example 1> Delivery of nucleic acid to activated T cells using post-addition LNP

### <1-1> LNP treatment of activated T cells

The activated T cells on the 3rd day of culture were collected in the required number of cells, centrifuged, and the supernatant was removed. The activated T cells were adjusted to a concentration of 1.0 × 10⁶ cells/ml in an activation culture medium containing recombinant human apolipoprotein E3 (ApoE3) (FUJIFILM Wako Pure Chemical Corporation, 010-20261) at a final concentration of 1 µg/ml, which was prepared in advance, and seeded in a 96-well plate.

Using the RNA-encapsulating LNPs prepared by the post-addition method of Examples 1, 2, 36, and 37 or the RNA-encapsulating LNP prepared by the conventional method of Comparative Example 3, the LNPs were added such that the amount of RNA was 1.8 to 6.0 µg (total RNA amount) per 1.0 × 10⁶ cells, and the cells were cultured in a 37°C, 5% CO₂ incubator.

24 hours after the addition of the LNP, the activation culture medium was added to the T cell suspension at a volume ratio of 1:3, and the cells were expanded and cultured, and further cultured for 3 days.

### <1-2> TCR KO efficiency in activated T cells

On the 7th day of the start of the culture, the TCR-negative cell ratio of the T cells treated with each method was measured by flow cytometry, and the TCR KO efficiency was evaluated.

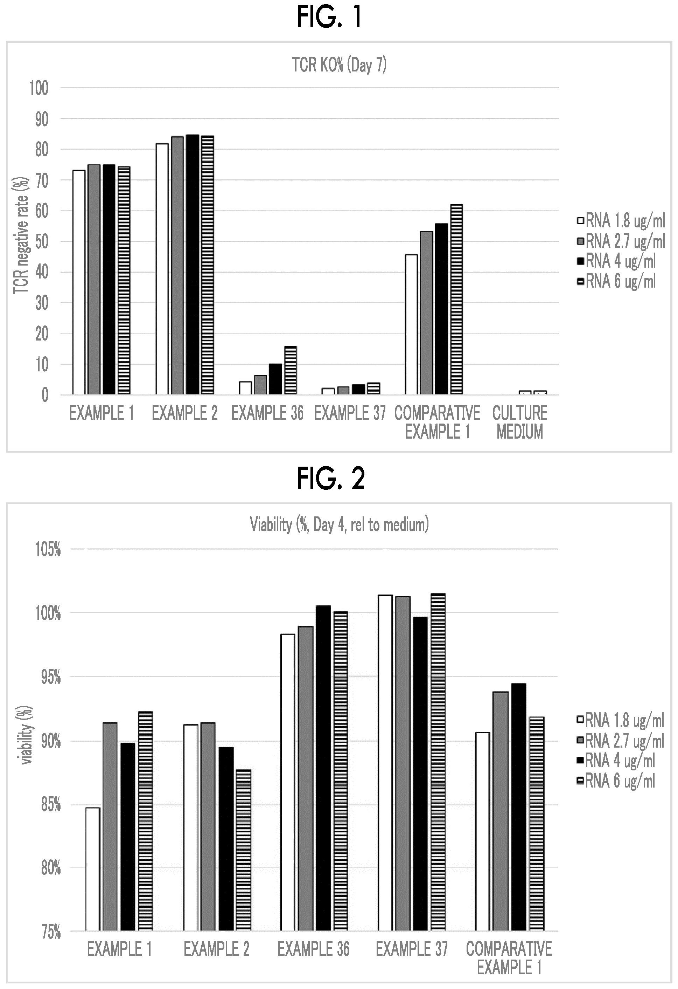

FIG. 1 shows the frequency (%) of TCR-negative cells among the T cells treated in Examples 1, 2, 36, and 37, Comparative Example 1, or with culture medium (control condition). From the results in FIG. 1, it was shown that Examples 1, 2, 36, and 37 were all capable of introducing mRNA into T cells and performing TCR KO, and particularly, Examples 1 and 2 had a high TCR KO efficiency. Furthermore, it was shown that Examples 1 and 2 had a higher TCR KO efficiency than the treatment in Comparative Example 1.

### <1-3> Cell survival rate and proliferation rate after treatment in activated T cells

On the 4th day after the start of the culture (1 day after the treatment with each method), the cell survival rate of the T cells treated with each LNP was evaluated. FIG. 2 shows the cell survival rate when the culture medium (control condition) is set to 100%. As a result, it was shown that the survival rate was maintained at a high level of 80% or more under any of the addition concentration conditions.

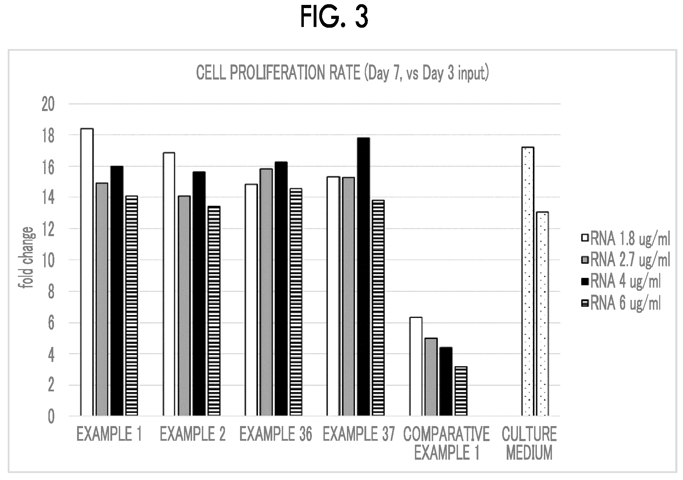

In addition, on the 7th day after the start of the culture, the proliferation rate of the T cells treated with each LNP was evaluated. FIG. 3 shows the cell proliferation rate on the 7th day. From the results in FIG. 3, it was shown that the proliferation rate was maintained at the same level as the control condition under any of the addition concentration conditions of Examples 1, 2, 36, and 37, and further, the proliferation rate was higher than that of Comparative Example 1.

### <2-1> Lipofectamine treatment of activated T cells

The activated T cells on the 3rd day of culture were collected in the required number of cells, centrifuged, and the supernatant was removed. The cells were adjusted to a concentration of 1.0 × 10⁶ cells/ml in an activation culture medium and seeded in a 96-well plate.

Lipofectamine MessengerMAX (hereinafter, Lipofectamine (M). A mixed solution of Lipofectamine and RNA was prepared according to the protocol of the manufacturer (Thermo Fisher, LMRNA001). Briefly, Opti-MEM I Medium (hereinafter, Opti-MEM, Thermo Fisher, 31985-062) and Lipofectamine (M) were mixed in the liquid amount shown in Table 7, mixed with a vortex for 2 to 3 seconds, and incubated at room temperature for 10 minutes (liquid 1). In parallel, an RNA solution (1 µg/ml) obtained by mixing Cas9 mRNA and TRAC sgRNA at a weight ratio of 4:1 and Opti-MEM were mixed in the liquid amount shown in Table 7 (liquid 2). After 10 minutes from the mixing of the liquid 1, the liquid 1 and the liquid 2 were mixed in equal amounts and incubated at room temperature for 5 minutes (Lipofectamine-RNA mixed solution).

10 µl of (Lipofectamine-RNA mixed solution) was added per 1.0 × 10⁵ cells of the activated T cells seeded in the 96-well plate.

**[Table 7]**

| | | Lipofectamine (M) | Lipofectamine (M) |
|---|---|---|---|
| | | RNA 1.0 µg/ml | RNA 6.0 µg/ml |
| Liquid 1 | Opti-MEM | 5.0 µl | 3.8 µl |
| | Lipofectamine | 0.2 µl | 1.2 µl |
| Liquid 2 | Opti-MEM | 5.0 µl | 4.4 µl |
| | RNA (1 µg/ml) | 0.1 µl | 0.6 µl |
| RNA concentration (µg/ml) | | 1.0 | 6.0 |

A mixed solution of Lipofectamine (CR) and a Cas9 protein/sgRNA complex was prepared according to the protocol of the manufacturer of Lipofectamine CRISPRMAX (hereinafter, Lipofectamine (CR), Thermo Fisher, CMAX00001).

Lipofectamine (CR) is composed of Cas9 Plus Reagent and CRISPRMAX Reagent.

Briefly, 10 µl of Opti-MEM, 0.5 µl of TrueCut^{™} Cas9 Protein v2 (Thermo Fisher, A36498, 1 µg/µl), 0.1 µl of TRAC sgRNA (1 µg/µl), and 1 µl of Cas9 Plus Reagent were mixed and mixed with a vortex for 2 to 3 seconds (liquid 3). Subsequently, 10 µl of Opti-MEM and 0.6 µl of CRISPRMAX Reagent were added and mixed by pipetting (liquid 4). Immediately, the total amount of the liquid 3 was added to the liquid 4, mixed by pipetting, and incubated at room temperature for 10 minutes (Lipofectamine (CR) mixed solution).

10 µl of the Lipofectamine (CR) mixed solution was added per 1.0 × 10⁵ cells of the activated T cells seeded in the 96-well plate.

### <2-2> TCR KO efficiency in activated T cells

On the 7th day of the start of the culture, the TCR-negative cell ratio of the T cells treated with each method was measured by flow cytometry, and the TCR KO efficiency was evaluated.

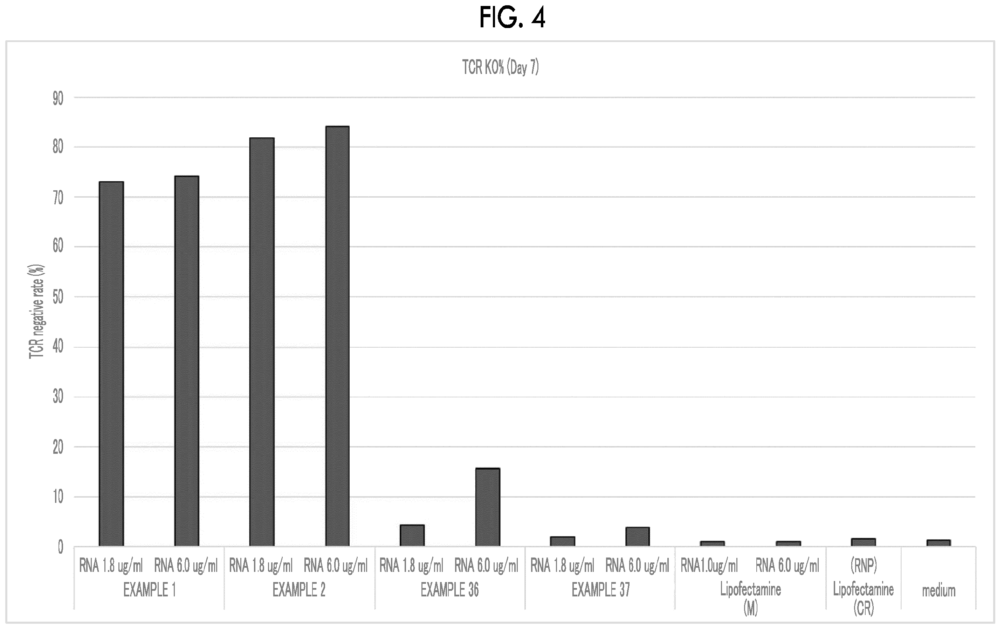

FIG. 4 shows the frequency (%) of TCR-negative cells among the T cells treated in Examples 1, 2, 36, and 37, or with Lipofectamine (M), Lipofectamine (CR), or the culture medium (control condition). From this result, it was shown that the TCR KO efficiency was significantly improved in Examples 1, 2, 36, and 37 as compared with the case where TCR KO was not performed in the treatment with Lipofectamine (M) and Lipofectamine (CR).

### <3-1> TCR KO by conventional method LNP and addition method LNP in activated T cells

The activated T cells on the 3rd day of culture were collected in the required number of cells, centrifuged, and the supernatant was removed. The activated T cells were adjusted to a concentration of 1.0 × 10⁶ cells/ml in an activation culture medium containing 1 µg/ml of ApoE3, which was prepared in advance, and seeded in a 96-well plate.

Using the RNA-encapsulating LNP prepared by the conventional method of Examples 9, 10, or 11, the RNA-encapsulating LNP prepared by the post-addition method (the ratio of LNP to RNA was 20) of Examples 3 to 5, and the RNA-encapsulating LNP prepared by the post-addition method (the ratio of LNP to RNA was 6.7) of Examples 6 to 8, the LNPs were added such that the amount of RNA was 1.0 to 5.4 µg (total RNA amount) per 1.0 × 10⁶ cells, and the cells were cultured in a 37°C, 5% CO₂ incubator.

24 hours after the addition of LNP, the activation culture medium was added to the T cell suspension at a volume ratio of 1:3, and the cells were expanded and cultured for another 3 days.

### <3-2> TCR KO efficiency by conventional method and post-addition method LNP treatment

On the 7th day of the start of the culture, the TCR-negative cell ratio of the T cells treated with each method was measured by flow cytometry, and the TCR KO efficiency was evaluated.

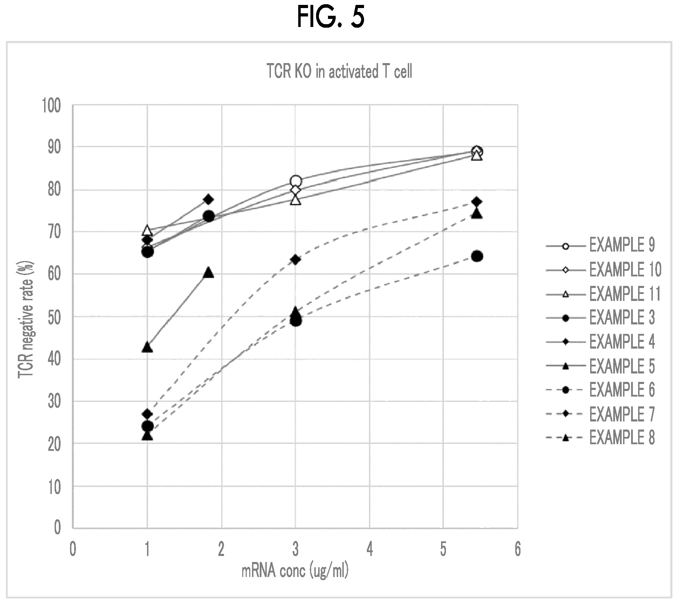

FIG. 5 shows the frequency (%) of TCR-negative cells among T cells treated by each method. From this result, it was shown that the post-addition method (the ratio of LNP to RNA was 20) of Examples 3 and 4 had the same TCR KO efficiency as Examples 9 and 10.

In addition, the post-addition method (the ratio of LNP to RNA was 6.7) was shown to provide KO efficiency equivalent to that of the conventional method under an RNA addition condition of 5.4 µg/ml. On the other hand, it was shown that the post-addition method (the ratio of LNP to RNA was 6.7) had a lower KO efficiency than the post-addition method (the ratio of LNP to RNA was 20) and the conventional method under the RNA 1.0 or 1.8 µg/ml addition condition. From the above results, it was shown that the post-addition method (the ratio of LNP to RNA was 20) had the same mRNA delivery efficiency as the conventional method, and the delivery efficiency was higher in the post-addition method with the ratio of LNP to RNA of 20 than 6.7.

### <3-3> Cell viability after treatment in activated T cells

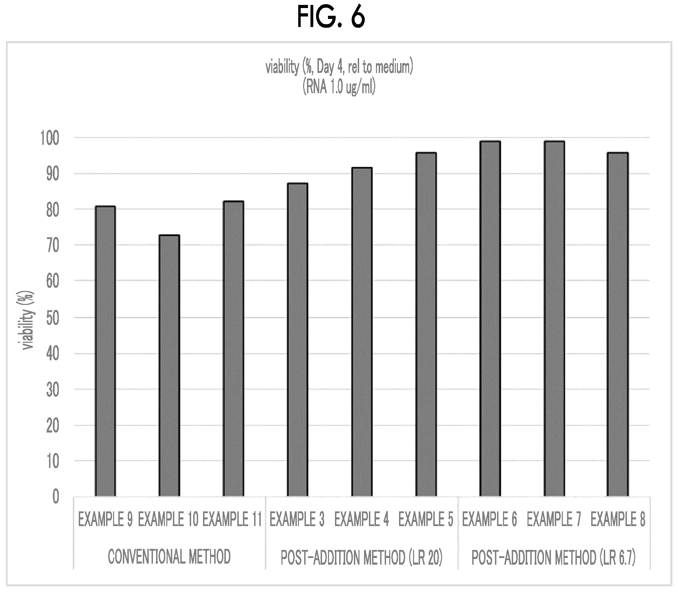

On the 4th day after the start of the culture (1 day after the treatment by each method), the cell viability of the T cells treated under the RNA 1.0 µg/ml addition condition with each LNP was evaluated. FIG. 6 shows the cell viability when the culture medium (control condition) was set to 100%.

From these results, it was shown that the same cell viability was obtained in a case of being treated with LNP prepared by the conventional method and the post-addition method.

### <Test Example 2> Nucleic acid delivery to inactivated T cells using post-added LNP

### <1> LNP treatment of inactivated T cells

The inactivated T cells on the 3rd day of the culture were collected in the required number of cells, centrifuged, and the supernatant was removed. The inactivated T cells were adjusted to a concentration of 1.0 × 10⁶ cells/ml in a culture medium for inactivation containing recombinant human apolipoprotein E3 (ApoE3) (FUJIFILM Wako Pure Chemical Corporation, 010-20261) at a final concentration of 1 µg/ml, which had been prepared in advance, and seeded in a 96-well plate.

Using the RNA-encapsulating LNPs prepared by the post-addition method of Examples 3 to 5, the LNPs were added such that the amount of RNA was 1.0 to 1.8 µg (total RNA amount) per 1.0 × 10⁶ cells, and cultured in a 37°C, 5% CO₂ incubator.

24 hours after the addition of the LNP, the culture medium for inactivation was added to the T cell suspension at a volume ratio of 1:3, and the cells were expanded and cultured for another 11 days. During this period, the culture medium was replaced or the cells were subcultured every 2 to 4 days so that the cell concentration was maintained at 0.1 to 6.0 × 10⁶ cells/ml.

### <2> TCR KO efficiency in inactivated T cells

On the 14th day of the culture, the TCR-negative cell ratio of the T cells treated with each method was measured by flow cytometry, and the TCR KO efficiency was evaluated.

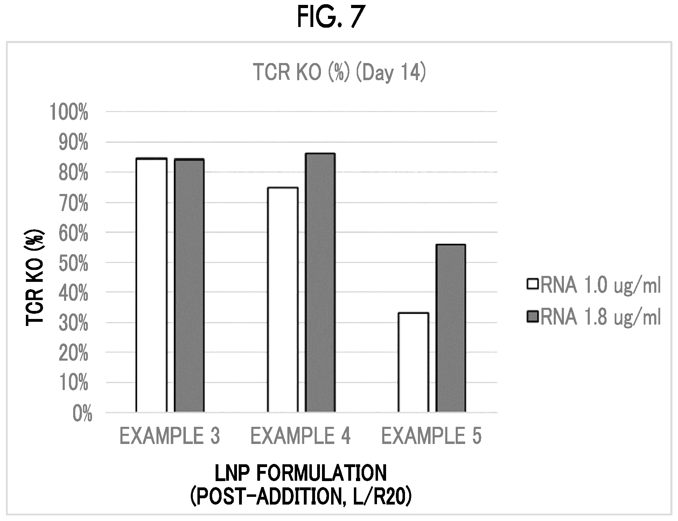

FIG. 7 shows the frequency (%) of TCR-negative cells among the T cells treated with the RNA-encapsulating LNP prepared by the post-addition method of Examples 3 to 5. From these results, it was shown that the treatments according to Examples 3 and 4 had a high mRNA introduction efficiency and a high TCR KO efficiency in the inactivated T cells.

### <3> Cell viability after treatment in inactivated T cells

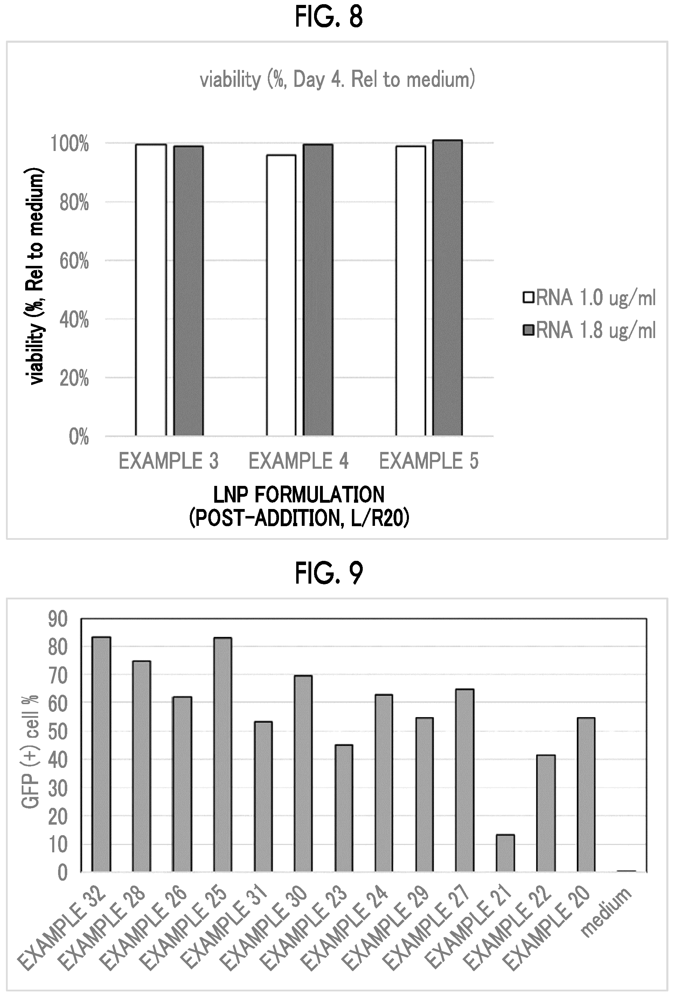

On the 4th day after the start of the culture (1 day after the treatment with each method), the cell survival rate of the T cells treated with each LNP was evaluated. FIG. 8 shows the cell viability when the culture medium (control condition) was set to 100%. As a result, it was shown that the viability was maintained at a high level of 95% or more under any of the addition concentration conditions.

### <Test Example 3> Nucleic acid delivery to activated T cells using LNP of the related art

### <1> Lipid selection in mRNA delivery to activated T cells

The activated T cells on the 3rd day of culture were collected in the required number of cells, centrifuged, and the supernatant was removed. The activated T cells were adjusted to a concentration of 1.0 × 10⁶ cells/ml in an activation culture medium containing 1 µg/ml of ApoE3, which was prepared in advance, and seeded in a 96-well plate.

Using the GFP mRNA-encapsulating LNPs prepared by the conventional method of Examples 20 to 32, the LNPs were added such that the amount of RNA was 1.0 µg (total RNA amount) per 1.0 × 10⁶ cells, and the cells were cultured in a 37°C, 5% CO₂ incubator.

The cells were collected 24 hours after the addition of the LNP, the GFP-positive cell ratio of the T cells treated with each LNP was measured by flow cytometry, and the GFP mRNA introduction efficiency was evaluated. The present results are shown in FIG. 9. It was shown that all the lipids of Examples 20 to 32 could efficiently deliver mRNA to activated T cells, and particularly, Examples 24 to 28, 30, and 32 were highly efficient.

### <2> Comparison of helper lipids in GFP mRNA delivery to activated T cells

The activated T cells on the 3rd day of culture were collected in the required number of cells, centrifuged, and the supernatant was removed. The activated T cells were adjusted to a concentration of 1.0 × 10⁶ cells/ml in an activation culture medium containing 1 µg/ml of ApoE3, which was prepared in advance, and seeded in a 96-well plate.

Using the RNA-encapsulating LNPs prepared by the conventional method of Examples 24 and 32, the LNPs were added such that the amount of RNA was 1.0 µg (total RNA amount) per 1.0 × 10⁶ cells, and the cells were cultured in a 37°C, 5% CO₂ incubator.

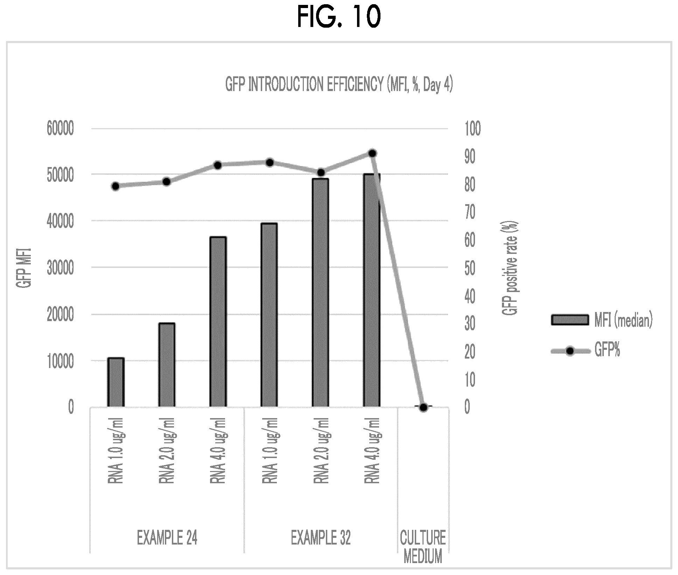

The cells were collected 24 hours after the addition of the LNP, the GFP-positive cell ratio and the GFP MFI of the T cells treated with each LNP were measured by flow cytometry, and the GFP mRNA introduction efficiency was evaluated. The present results are shown in FIG. 10.

### <3> Comparison of helper lipids in mRNA delivery to activated T cells

The activated T cells on the 3rd day of culture were collected in the required number of cells, centrifuged, and the supernatant was removed. The activated T cells were adjusted to a concentration of 1.0 × 10⁶ cells/ml in an activation culture medium containing 1 µg/ml of ApoE3, which was prepared in advance, and seeded in a 96-well plate.

Using the RNA-encapsulating LNPs prepared by the conventional method of Examples 12 and 13 and Comparative Example 2, the LNPs were added such that the amount of RNA was 1.0 to 3.6 µg (total RNA amount) per 1.0 × 10⁶ cells, and the cells were cultured in a 37°C, 5% CO₂ incubator.

24 hours after the addition of LNP, the activation culture medium was added to the T cell suspension at a volume ratio of 1:3, and the cells were expanded and cultured for another 3 days.

On the 7th day of the start of the culture, the TCR-negative cell ratio of the T cells treated with each method was measured by flow cytometry, and the TCR KO efficiency was evaluated.

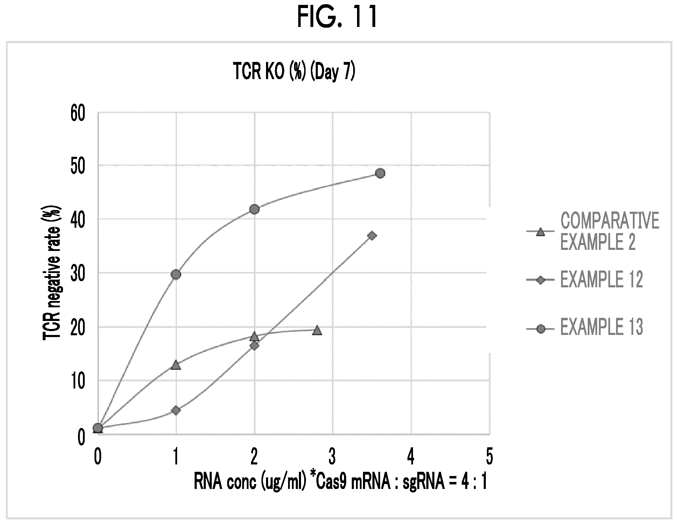

FIG. 11 shows the frequency (%) of TCR-negative cells among T cells. From this result, it was shown that the treatment of Example 13 had a higher TCR KO efficiency in the activated T cells as compared with Example 12.

From the above results, it was shown that in a case where the present lipid and the T cells were combined, the helper lipid using DOPE had a higher mRNA delivery efficiency than that using DSPC.

### <4> Ratio of each composition in mRNA delivery to activated T cells

The activated T cells on the 3rd day of culture were collected in the required number of cells, centrifuged, and the supernatant was removed. The activated T cells were adjusted to a concentration of 1.0 × 10⁶ cells/ml in an activation culture medium containing 1 µg/ml of ApoE3, which was prepared in advance, and seeded in a 96-well plate.

Using the RNA-encapsulating LNPs prepared by the conventional method of Examples 13 and 9, Examples 9, 10, 11, and 14 to 19, and Comparative Examples 2 and 3, the LNPs were added such that the amount of RNA was 1.0 to 5.45 µg (total RNA amount) per 1.0 × 10⁶ cells, and the cells were cultured in a 37°C, 5% CO₂ incubator.

24 hours after the addition of LNP, the activation culture medium was added to the T cell suspension at a volume ratio of 1:3, and the cells were expanded and cultured for another 3 days.

On the 7th day of the start of the culture, the TCR-negative cell ratio of the T cells treated with each method was measured by flow cytometry, and the TCR KO efficiency was evaluated.

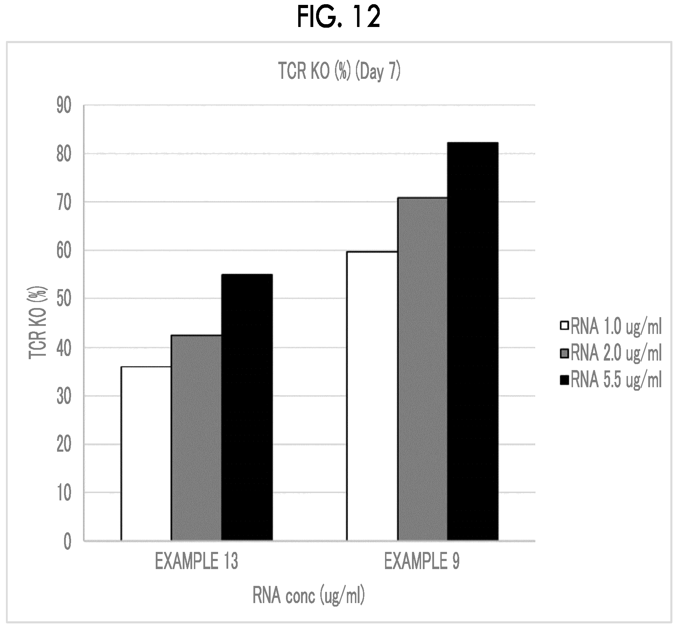

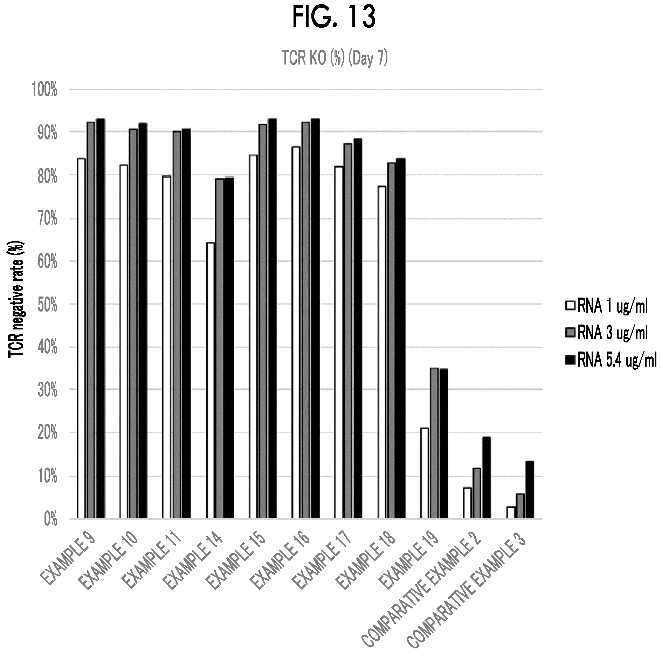

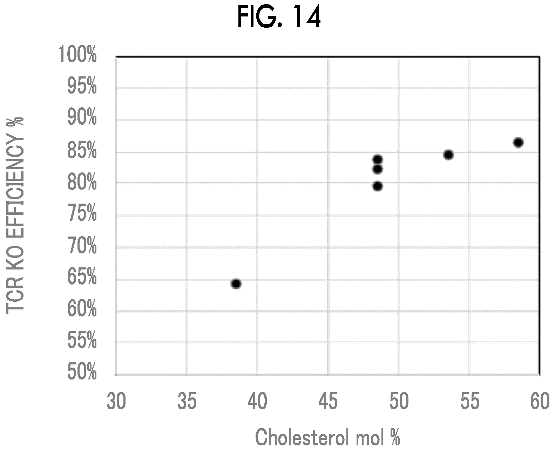

FIGS. 12 and 13 show the frequency (percentage) of TCR-negative cells among the T cells treated with each LNP. In addition, FIG. 14 shows the relationship between the cholesterol ratio and the TCR KO efficiency.

From the results of FIGS. 12 and 13, in Examples 9 to 11 and 13 to 19, the TCR KO efficiency in the activated T cells was significantly improved as compared with Comparative Examples 2 and 3. In addition, from the results of FIG. 14, it was shown that the cholesterol ratio of 48.5% or more had a higher mRNA delivery efficiency.

### <Test Example 4> Delivery of Nucleic Acid to Inactivated T Cells Using LNP of Related Art

### <1> Selection of Lipid in mRNA Delivery to Inactivated T Cells

The inactivated T cells on the 3rd day of the culture were collected in the required number of cells, centrifuged, and the supernatant was removed. The inactivated T cells were adjusted to a concentration of 1.0 × 10⁶ cells/ml in an activation culture medium containing ApoE3 at a final concentration of 1 µg/ml, which had been prepared in advance, and seeded in a 96-well plate.

In Examples 23 to 35, using the GFP mRNA-encapsulating LNPs prepared by the conventional method, the LNPs were added such that the amount of the RNA was 1.0 µg (total RNA amount) per 1.0 × 10⁶ cells, and the cells were cultured in a 37°C, 5% CO₂ incubator.

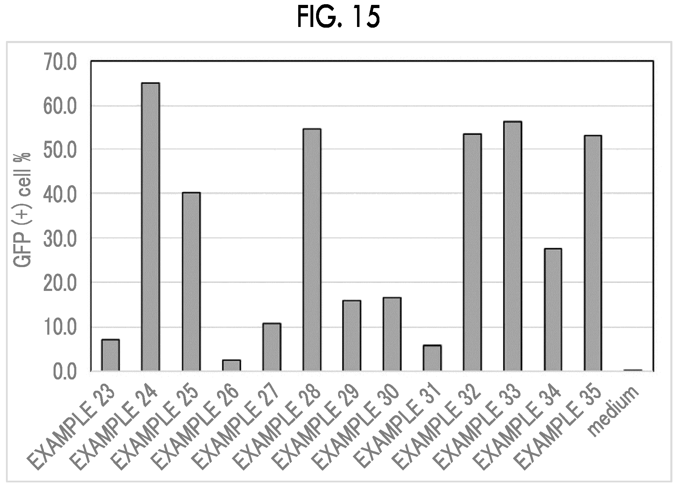

The cells were collected 24 hours after the addition of the LNP, the GFP-positive cell ratio of the T cells treated with each LNP was measured by flow cytometry, and the GFP mRNA introduction efficiency was evaluated. The present results are shown in FIG. 15. All of the LNPs of Examples 23 to 35 were capable of efficiently delivering mRNA to activated T cells, and in particular, it was shown that Example 24 had a high efficiency.

### <2> TCR KO efficiency in inactivated T cells

The inactivated T cells on the 3rd day of the culture were collected in the required number of cells, centrifuged, and the supernatant was removed. The inactivated T cells were adjusted to a concentration of 1.0 × 10⁶ cells/ml in a culture medium for inactivation containing recombinant human apolipoprotein E3 (ApoE3) (FUJIFILM Wako Pure Chemical Corporation, 010-20261) at a final concentration of 1 µg/ml, which had been prepared in advance, and seeded in a 96-well plate.

In Examples 13 and 9, using the RNA-encapsulating LNP prepared by the conventional method, the LNP was added such that the amount of the RNA was 1.0 to 5.5 µg (total RNA amount) per 1.0 × 10⁶ cells, and the cells were cultured in a 37°C, 5% CO₂ incubator.

24 hours after the addition of the LNP, the culture medium for inactivation was added to the T cell suspension at a volume ratio of 1:3, and the cells were expanded and cultured for another 11 days. During this period, the culture medium was replaced or the cells were subcultured every 2 to 4 days so that the cell concentration was maintained at 0.1 to 6.0 × 10⁶ cells/ml.

On the 14th day of the culture, the TCR-negative cell ratio of the T cells treated with each method was measured by flow cytometry, and the TCR KO efficiency was evaluated.

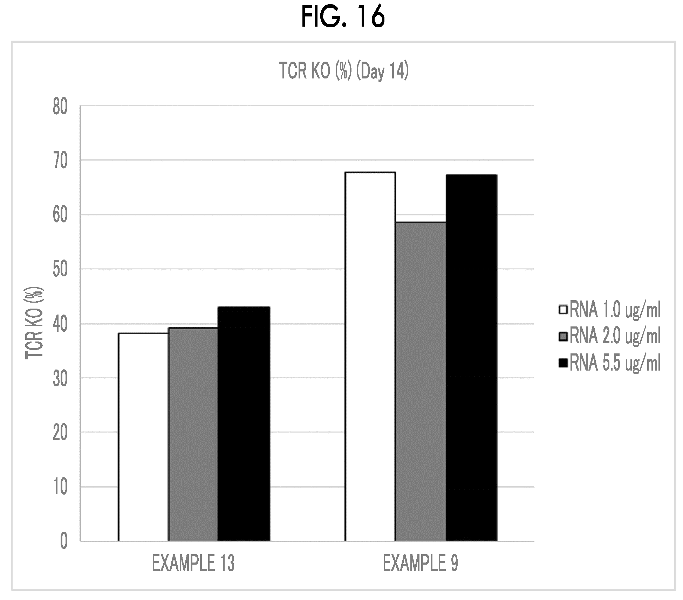

FIG. 16 shows the frequency (%) of TCR-negative cells among T cells. From these results, it was shown that the treatment according to Examples 13 and 9 achieves highly efficient mRNA introduction into inactivated T cells. In addition, since the treatment of Example 9 has a higher TCR KO efficiency in activated T cells than that of Example 13, the cholesterol ratio of 48.5% or more has a higher mRNA delivery efficiency.

As a method of increasing the concentration of the empty LNP by 10 times, a method of concentrating the empty LNP prepared as described in Example 2 with a total lipid concentration of 12.5 mM by 10 times by ultrafiltration (empty LNP concentration method 1) and a method of concentrating the empty LNP prepared with a total lipid concentration of 62.5 mM by 2 times by ultrafiltration (empty LNP concentration method 2) were compared. The empty LNP was prepared according to the above-described examples, and a nucleic acid mixture solution (Cas9/sgRNA) obtained by mixing CleanCap (registered trademark) Cas9 mRNA (5 moU) (TriLink, L-7206) and sgRNA targeting a human T cell receptor alpha constant (TRAC) gene (sequence; A*G*A*GUCUCUCAGCUGGUACA + modified Scaffold, Thermo Fisher A35514, custom synthesis) at a weight ratio of 4:1 was encapsulated by a post-addition method.

The results of the TCR KO efficiency test in T cells according to the above-described test examples are shown in Table 8.

From the results in Table 8, it was shown that the empty LNP concentration methods 1 and 2 had the same survival rate, TCR KO efficiency, and proliferation rate, and both were very high efficiency.

**[Table 8]**

| LNP addition condition | | Day 4 | Day 7 | |
|---|---|---|---|---|
| | | Viability (%) (rel to medium) | TCR KO efficiency (%) | Cell proliferative property (fold change, vs Day 3 input) |
| Example 2 (empty LNP concentration method 1) | RNA 6 ug/ml | 87.7 | 84.2 | 13.4 |
| | RNA 4 ug/ml | 89.5 | 84.6 | 15.6 |
| | RNA 2.7 ug/ml | 91.3 | 84 | 14.1 |
| | RNA 1.8 ug/ml | 91.2 | 81.8 | 16.8 |
| (Empty LNP concentration method 1) | RNA 6 ug/ml | 90.2 | 83.9 | 12.8 |
| | RNA 4 ug/ml | 92.0 | 84.8 | 16.5 |
| | RNA 2.7 ug/ml | 92.1 | 84.3 | 15.9 |
| | RNA 1.8 ug/ml | 88.6 | 83.9 | 15.8 |

### <Encapsulation of various RNAs in empty LNP (post-addition method)>

gp46 siRNA (Gene design), CleanCap (registered trademark) EPO mRNA (5 moU) (TriLink, L-7209), CleanCap FLuc mRNA (TriLink, L-7602), CleanCap Cas9 mRNA (5 moU) (TriLink, L-7206), and GFP pDNA (GenScript, custom synthesized plasmid DNA) were diluted with water for injection to prepare RNA solutions having different concentrations. The empty LNP of Example 2 stored at -70°C was thawed at 4°C. The RNA solution was added to the present LNP liquid in an equal liquid amount and mixed by pipetting (LNP-RNA mixed solution). After being allowed to stand at room temperature for 5 minutes, 20 mmol/L Tris buffer containing 8% sucrose was added to the present LNP-RNA mixture solution in an equal liquid amount, and the mixture was mixed by pipetting to prepare RNA-encapsulating LNP by a post-addition method.

### <Measurement of encapsulation rate>

The encapsulation rate was measured using Quant-iT RiboGreen RNA Assay Kit (Thermo Fisher Scientific). First, the RNA-encapsulating LNP was diluted with TE buffer or TE buffer containing 0.1% Triton X-100. Thereafter, Ribogreen diluted 200 times with TE buffer was added in an equal liquid amount, and fluorescence (excitation wavelength: 485 nm, fluorescence wavelength: 535 nm) was measured using a fluorescence plate reader (Infinite 200 Pro M nano+, TECAN). The encapsulation rate was calculated from a value obtained by multiplying the obtained fluorescence intensity by a dilution ratio, according to the following expression. 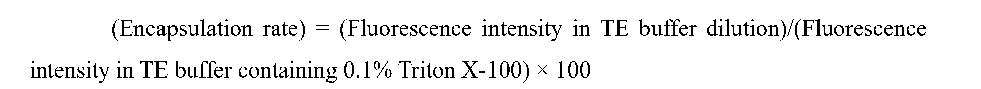 (Encapsulation rate) = (Fluorescence intensity in TE buffer dilution)/(Fluorescence intensity in TE buffer containing 0.1% Triton X-100) × 100

The results are shown in Table 9 below.

**[Table 9]**

| Lipid/nucleic acid (wt/wt) | Encapsulated nucleic acid | | | | |
|---|---|---|---|---|---|
| | gp46 siRNA | EPO mRNA | Fluc mRNA | Cas9 mRNA | GFP-pDNA |
| 1.25 | 31% | 27% | 35% | 14% | 32% |
| 2.5 | 29% | 35% | 29% | 26% | 18% |
| 5 | 63% | 76% | 68% | 67% | 45% |
| 10 | 75% | 94% | 94% | 95% | 93% |
| 20 | 81% | 95% | 95% | 95% | 93% |
| 40 | 84% | 93% | 92% | 93% | 93% |
| 80 | 87% | 89% | 88% | 90% | 90% |
| 160 | 86% | 84% | 83% | 86% | 87% |

### <Test Example 4> Delivery of nucleic acid to activated T cells using post-addition LNP

### <4-1> Encapsulation of gRNA and Cas9 mRNA in empty LNP (post-addition method)

CleanCap (registered trademark) Cas9 mRNA (5moU) (TriLink, L-7206) and sgRNA (sequence; A*G*A*GUCUCUCAGCUGGUACA + modified Scaffold, Thermo Fisher A35514, custom synthesis) targeting a human T cell receptor alpha constant (TRAC) gene were mixed at a weight ratio of 8:4, 8:2, or 8:1, and diluted with water for injection to prepare an RNA solution (total nucleic acid concentration: 480, 400, or 360 µg/mL).

The empty LNP of Example 2 stored at -70°C was thawed at 4°C. The RNA solution was added to the present LNP liquid in an equal liquid amount and mixed by pipetting (LNP-RNA mixed solution). After allowing the mixture to stand at room temperature for 5 minutes, a 20 mmol/L Tris buffer containing 8% sucrose was added to the present LNP-RNA mixed solution in an equal liquid amount, and the mixture was mixed by pipetting, thereby preparing Cas9 mRNA/gRNA-encapsulating LNP by a post-addition method.

### <4-2> LNP treatment of activated T cells

Activated T cells on the 1st to 4th days of activation were collected in the required number of cells, centrifuged, and the supernatant was removed. The activated T cells were adjusted to a concentration of 1.0 × 10⁶ cells/ml in an activation culture medium containing recombinant human apolipoprotein E3 (ApoE3) (FUJIFILM Wako Pure Chemical Corporation, 010-20261) at a final concentration of 1 µg/ml, which was prepared in advance, and seeded in a 96-well plate.

Using the RNA-encapsulating LNP prepared by the post-addition method of Example 2 was added such that the amount of RNA was 4.0 µg (total RNA amount) per 1.0 × 10⁶ cells, and the cells were cultured in a 37°C, 5% CO₂ incubator.

After 24 hours from the addition of LNP, the activation culture medium was added to the T cell suspension at a volume ratio of 1:3 for expansion culture, and the culture was further continued until the 7th day from the start of the culture.

### <4-3> TCR KO efficiency in activated T cells

On the 7th day of the start of the culture, the TCR-negative cell ratio of the T cells treated with each method was measured by flow cytometry, and the TCR KO efficiency was evaluated.

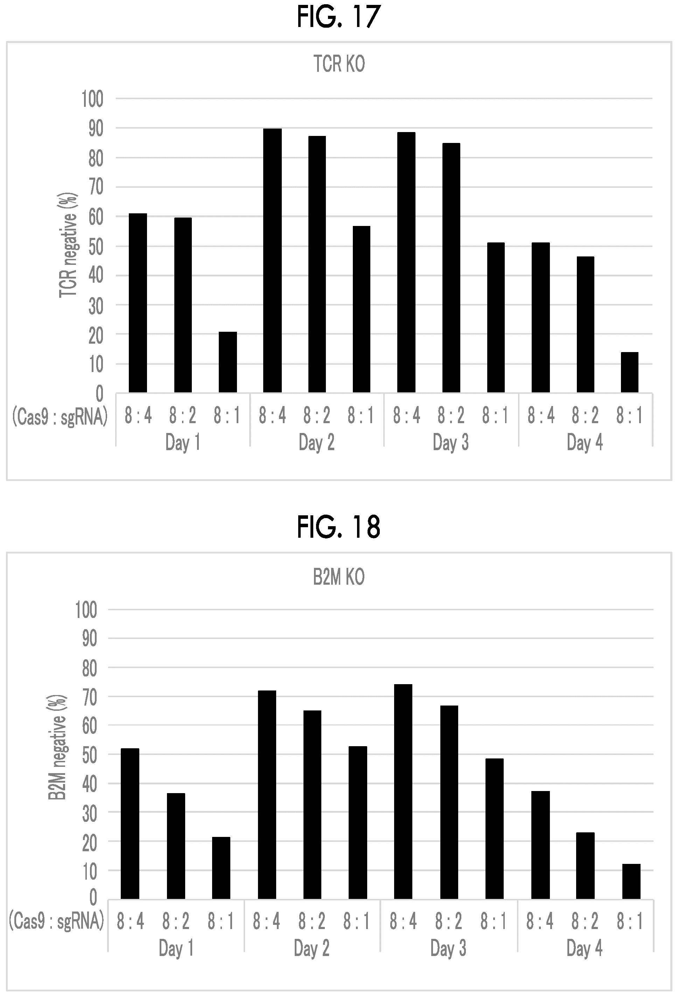

FIG. 17 shows the frequency (%) of TCR-negative cells among T cells treated with LNP in which Cas9 and sgRNA have been mixed and encapsulated in an empty LNP in Example 2 at each ratio at each LNP treatment timing. From the results of FIG. 17, it was shown that the TCR KO efficiency was high regardless of the ratio of Cas9 mRNA to sgRNA being 8:4 to 8:1. In addition, it was shown that the timing of performing the LNP treatment can be any of the 1st to 4th days of activation, and the TCR KO efficiency was particularly high on the 2nd and 3rd days of activation.

### <Test Example 5> Nucleic acid delivery to activated T cells using post-added LNP

### <5-1> Encapsulation of gRNA and Cas9 mRNA in empty LNP (post-addition method)

CleanCap (registered trademark) Cas9 mRNA (5 moU) (TriLink, L-7206) and sgRNA (sequence; GAGTAGCGCGAGCACAGCTA, Thermo Fisher A35514, Assay ID CRISPR983706_SGM) targeting a human β2-microglobulin (B2M) gene were mixed at a weight ratio of 8:4, 8:2, or 8:1, and diluted with water for injection to prepare an RNA solution (total nucleic acid concentration: 480, 400, or 360 µg/mL).

The empty LNP of Example 2 stored at -70°C was thawed at 4°C. The RNA solution was added to the present LNP liquid in an equal liquid amount and mixed by pipetting (LNP-RNA mixed solution). After allowing the mixture to stand at room temperature for 5 minutes, a 20 mmol/L Tris buffer containing 8% sucrose was added to the present LNP-RNA mixed solution in an equal liquid amount, and the mixture was mixed by pipetting, thereby preparing Cas9 mRNA/gRNA-encapsulating LNP by a post-addition method.

### <5-2> LNP treatment of activated T cells

Activated T cells on the 1st to 4th days of activation were collected in the required number of cells, centrifuged, and the supernatant was removed. The activated T cells were adjusted to a concentration of 1.0 × 10⁶ cells/ml in an activation culture medium containing recombinant human apolipoprotein E3 (ApoE3) (FUJIFILM Wako Pure Chemical Corporation, 010-20261) at a final concentration of 1 µg/ml, which was prepared in advance, and seeded in a 96-well plate.

Using the RNA-encapsulating LNP prepared by the post-addition method of Example 2 was added such that the amount of RNA was 4.0 µg (total RNA amount) per 1.0 × 10⁶ cells, and the cells were cultured in a 37°C, 5% CO₂ incubator.

After 24 hours from the addition of LNP, the activation culture medium was added to the T cell suspension at a volume ratio of 1:3 for expansion culture, and the culture was further continued until the 7th day from the start of the culture.

### <5-3> B2M KO efficiency in activated T cells

On the 7th day from the start of the culture, the B2M-negative cell ratio of the T cells treated with each method was measured by flow cytometry, and the B2M KO efficiency was evaluated.

FIG. 18 shows the frequency (%) of B2M-negative cells among T cells treated with LNP in which Cas9 and sgRNA have been mixed and encapsulated in an empty LNP in Example 2 at each ratio at each LNP treatment timing. From the results in FIG. 18, it was shown that the B2M KO efficiency was high regardless of the ratio of Cas9 mRNA to sgRNA being 8:4 to 8:1. In addition, it was shown that the timing of performing the LNP treatment can be any of the 1st to 4th days of activation, and the KO efficiency was particularly high on the 2nd and 3rd days of activation.

### <Test Example 6> Nucleic acid delivery to activated T cells using post-added LNP

### <6-1> Encapsulation of gRNA and Cas9 mRNA in empty LNP (post-addition method, co-encapsulation)

CleanCap (registered trademark) Cas9 mRNA (5 moU) (TriLink, L-7206), sgRNA targeting human TRAC gene (sequence; A*G*A*GUCUCUCAGCUGGUACA + modified Scaffold, Thermo Fisher A35514, custom synthesis), and sgRNA targeting human B2M gene (sequence; GAGTAGCGCGAGCACAGCTA, Thermo Fisher A35514, Assay ID CRISPR983706_SGM) were mixed at a weight ratio of 8:4:4, 8:2:2, or 8:1:1 and diluted with water for injection to prepare RNA solutions (total nucleic acid concentration: 640, 480, and 400 µg/mL).

The empty LNP of Example 2 stored at -70°C was thawed at 4°C. The RNA solution was added to the present LNP liquid in an equal liquid amount and mixed by pipetting (LNP-RNA mixed solution). After allowing the mixture to stand at room temperature for 5 minutes, a 20 mmol/L Tris buffer containing 8% sucrose was added to the present LNP-RNA mixed solution in an equal liquid amount, and the mixture was mixed by pipetting, thereby preparing Cas9 mRNA/gRNA-encapsulating LNP by a post-addition method.

### <6-2> Encapsulation of gRNA and Cas9 mRNA in empty LNP (post-addition method, separate encapsulation)

CleanCap (registered trademark) Cas9 mRNA (5 moU) (TriLink, L-7206) and sgRNA targeting human TRAC gene (sequence; A*G*A*GUCUCUCAGCUGGUACA + modified Scaffold, Thermo Fisher A35514, custom synthesis), or Cas9 mRNA (5 mU) and sgRNA targeting human B2M gene (sequence; GAGTAGCGCGAGCACAGCTA, Thermo Fisher A35514, Assay ID CRISPR983706_SGM) were mixed at a weight ratio of 8:2, respectively, and diluted with water for injection to prepare RNA solutions (total nucleic acid concentration: 400 µg/mL).

The empty LNP of Example 2 stored at -70°C was thawed at 4°C. One of the RNA solutions was added to the present LNP solution in an equal liquid amount and mixed by pipetting (LNP-RNA mixed solution). After being allowed to stand at room temperature for 5 minutes, 20 mmol/L Tris buffer containing 8% sucrose was added to the present LNP-RNA mixed solution in an equal liquid amount and mixed by pipetting to separately prepare Cas9 mRNA/gRNA (TRAC)-encapsulating LNP and Cas9 mRNA/gRNA (B2M)-encapsulating LNP by a post-addition method.

### <6-3> LNP treatment of activated T cells

Activated T cells on the 1st to 4th days of activation were collected in the required number of cells, centrifuged, and the supernatant was removed. The activated T cells were adjusted to a concentration of 1.0 × 10⁶ cells/ml in an activation culture medium containing recombinant human apolipoprotein E3 (ApoE3) (FUJIFILM Wako Pure Chemical Corporation, 010-20261) at a final concentration of 1 µg/ml, which was prepared in advance, and seeded in a 96-well plate.

Using the RNA-encapsulating LNP prepared by the post-addition method of Example 2, the RNA-encapsulating LNP was added thereto such that the amount of RNA was 4.0 or 8.0 µg (total RNA amount) per 1.0 × 10⁶ cells, and cultured in a 37°C, 5% CO₂ incubator.

After 24 hours from the addition of LNP, the activation culture medium was added to the T cell suspension at a volume ratio of 1:3 for expansion culture, and the culture was further continued until the 7th day from the start of the culture.

### <6-4> Double KO efficiency of TCR and B2M in activated T cells

On the 7th day from the start of the culture, the TCR-negative and B2M-negative cell ratio of the T cells treated by each method was measured by flow cytometry to evaluate the double KO efficiency.

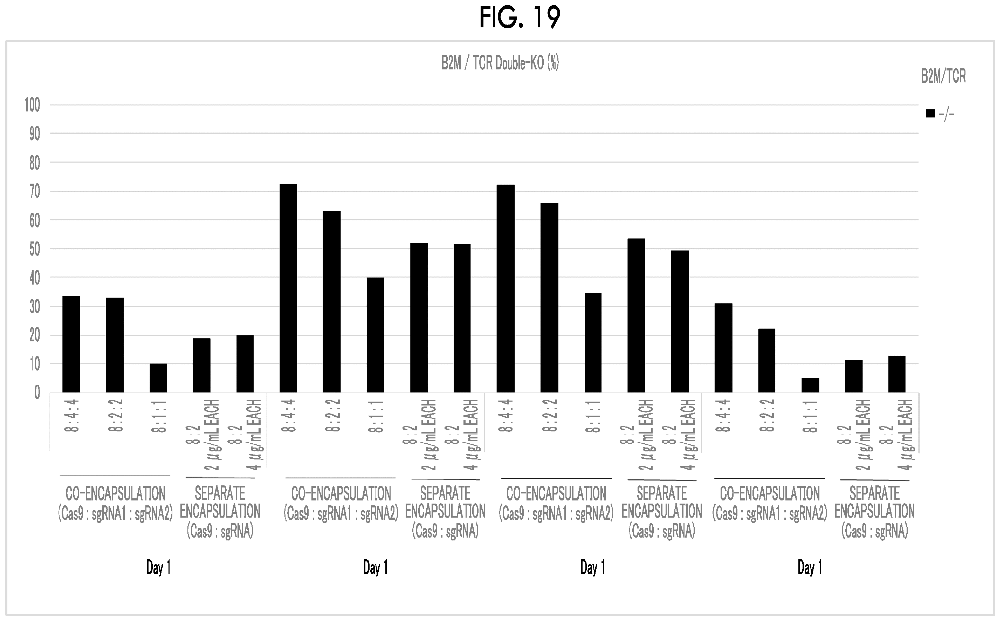

FIG. 19 shows the frequency (%) of TCR- and B2M-double-negative cells among T cells treated with LNP in which Cas9 and two types of sgRNAs have been mixed and encapsulated in an empty LNP in Example 2 at each ratio at each LNP treatment timing. From the results of FIG. 19, it was shown that a high double KO efficiency was obtained regardless of whether the Cas9 mRNA and the two types of sgRNA were encapsulated in one LNP or encapsulated in separate LNPs. In addition, it was shown that a higher double KO efficiency was obtained particularly in a case where the Cas9 mRNA and the two types of sgRNA were encapsulated in one LNP.

### <Test Example 7> Nucleic acid delivery to activated T cells using post-added LNP (cryopreservation)

### <7-1> Encapsulation of gRNA and Cas9 mRNA in empty LNP (post-addition method)

CleanCap (registered trademark) Cas9 mRNA (5 moU) (TriLink, L-7206) and sgRNA (sequence; A*G*A*GUCUCUCAGCUGGUACA + modified Scaffold, Thermo Fisher A35514, custom synthesis) targeting a human T cell receptor alpha constant (TRAC) gene were mixed at a weight ratio of 4:1 and diluted with water for injection to prepare an RNA solution (total nucleic acid concentration: 400 µg/mL).

The empty LNP of Example 2 stored at -70°C was thawed at 4°C. The RNA solution was added to the present LNP liquid in an equal liquid amount and mixed by pipetting (LNP-RNA mixed solution). After allowing the mixture to stand at room temperature for 5 minutes, a 20 mmol/L Tris buffer containing 8% sucrose was added to the present LNP-RNA mixed solution in an equal liquid amount, and the mixture was mixed by pipetting, thereby preparing Cas9 mRNA/gRNA-encapsulating LNP by a post-addition method. (Before freezing post-added LNP)

The present post-added LNP was re-frozen and stored at -70°C and thawed again at 4°C. (After freeze-thaw post-added LNP)

### <7-2> LNP treatment of activated T cells

The activated T cells on the 3rd day of activation were collected in the required number of cells, centrifuged, and the supernatant was removed. The activated T cells were adjusted to a concentration of 1.0 × 10⁶ cells/ml in an activation culture medium containing recombinant human apolipoprotein E3 (ApoE3) (FUJIFILM Wako Pure Chemical Corporation, 010-20261) at a final concentration of 1 µg/ml, which was prepared in advance, and seeded in a 96-well plate.

Using the RNA-encapsulating LNP prepared by the post-addition method of Example 2, the LNP was added such that the amount of RNA was 4.0 µg (total RNA amount) per 1.0 × 10⁶ cells, and the cells were cultured in a 37°C, 5% CO₂ incubator.

After 24 hours from the addition of LNP, the activation culture medium was added to the T cell suspension at a volume ratio of 1:3 for expansion culture, and the culture was further continued until the 7th day from the start of the culture.

### <7-3> TCR KO efficiency in activated T cells

On the 7th day of the start of the culture, the TCR-negative cell ratio of the T cells treated with each method was measured by flow cytometry, and the TCR KO efficiency was evaluated.

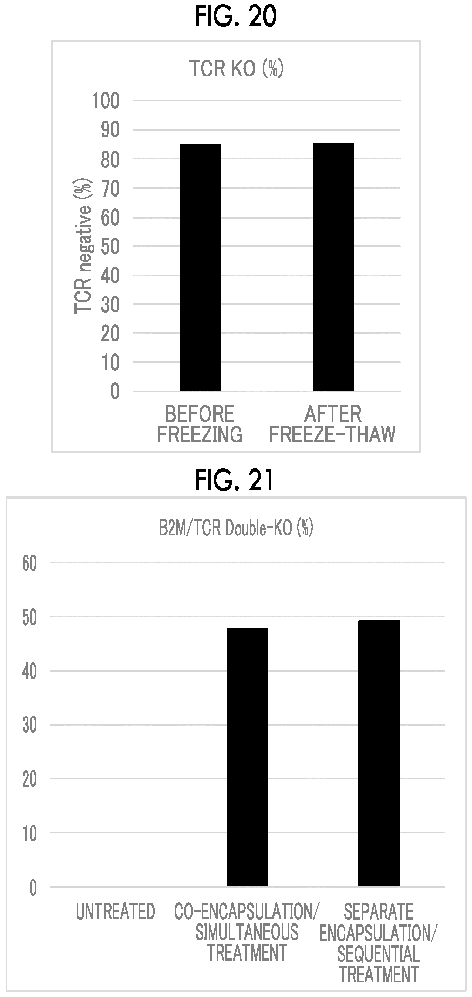

FIG. 20 shows the frequency (%) of TCR-negative cells among T cells treated with the post-addition LNP (before freezing) or the post-addition LNP (after freeze-thaw). From the results in FIG. 20, it was shown that the post-added LNP had the same high KO efficiency as before freezing even after being refrozen, stored, and thawed at -70°C.

### <Test Example 8> Nucleic acid delivery to activated T cells using post-added LNP (sequential treatment)

### <1> Encapsulation of gRNA and Cas9 mRNA in empty LNP (post-addition method, co-encapsulation)

CleanCap (registered trademark) Cas9 mRNA (5 moU) (TriLink, L-7206), sgRNA targeting a human TRAC gene (sequence; A*G*A*GUCUCUCAGCUGGUACA + modified Scaffold, Thermo Fisher A35514, custom synthesis), and sgRNA targeting a human B2M gene (sequence; GAGTAGCGCGAGCACAGCTA, Thermo Fisher A35514, Assay ID CRISPR983706_SGM) were mixed at a weight ratio of 8:4:4 and diluted with water for injection to prepare an RNA solution (total nucleic acid concentration: 640 µg/mL).

The empty LNP of Example 2 stored at -70°C was thawed at 4°C. The RNA solution was added to the present LNP liquid in an equal liquid amount and mixed by pipetting (LNP-RNA mixed solution). After being allowed to stand at room temperature for 5 minutes, 20 mmol/L Tris buffer containing 8% sucrose was added to the present LNP-RNA mixed solution in an equal liquid amount, and the mixture was mixed by pipetting to prepare Cas9 mRNA/gRNA-encapsulating LNP by a post-addition method (hereinafter, TRAC-B2M targeted LNP).

### <2> Encapsulation of gRNA and Cas9 mRNA in empty LNP (post-addition method, separate encapsulation)

CleanCap (registered trademark) Cas9 mRNA (5 moU) (TriLink, L-7206) and sgRNA targeting a human TRAC gene (sequence; A*G*A*GUCUCUCAGCUGGUACA + modified Scaffold, Thermo Fisher A35514, custom synthesis), or Cas9 mRNA (5 moU) and sgRNA targeting a human B2M gene (sequence; GAGTAGCGCGAGCACAGCTA, Thermo Fisher A35514, Assay ID CRISPR983706_SGM) were mixed at a weight ratio of 8:4, and the mixture was diluted with water for injection to prepare an RNA solution (total nucleic acid concentration: 400 µg/mL).

The empty LNP of Example 2 stored at -70°C was thawed at 4°C. One of the RNA solutions was added to the present LNP solution in an equal liquid amount and mixed by pipetting (LNP-RNA mixed solution). After being allowed to stand at room temperature for 5 minutes, 20 mmol/L Tris buffer containing 8% sucrose was added to the present LNP-RNA mixed solution in an equal liquid amount, and the mixture was mixed by pipetting to separately prepare Cas9 mRNA/gRNA (TRAC)-encapsulating LNP and Cas9 mRNA/gRNA (B2M)-encapsulating LNP by a post-addition method (hereinafter, each of which is referred to as TRAC targeted LNP and B2M targeted LNP).

### <3> LNP treatment of activated T cells

The activated T cells on the second day of activation were collected in the required number of cells, centrifuged, and the supernatant was removed. The activated T cells were adjusted to a concentration of 1.0 × 10⁶ cells/ml in an activation culture medium containing recombinant human apolipoprotein E3 (ApoE3) (FUJIFILM Wako Pure Chemical Corporation, 010-20261) at a final concentration of 1 µg/ml, which was prepared in advance, and seeded in a 96-well plate.

TRAC-B2M targeted LNP or B2M targeted LNP prepared by the post-addition method of Example 2 was added at 6.4 or 4.8 µg (total RNA amount) per 1.0 × 10⁶ cells, and the cells were cultured in a 37°C, 5% CO₂ incubator.

After 24 hours from the LNP addition, the culture supernatant was exchanged with half the amount in a newly adjusted activation culture medium for all culture conditions. Subsequently, B2M-targeted LNP and ApoE3 were added to the T cell suspension treated with the TCR-targeted LNP so that the amount of the B2M-targeted LNP was 4.8 µg in terms of total RNA and the final concentration of ApoE3 was 1 µg/mL, and the resulting suspension was cultured in a 37°C, 5% CO₂ incubator. Furthermore, 24 hours later, the activation culture medium was added to the T cell suspension at a volume ratio of 1:3, and the cells were expanded and cultured for 7 days from the start of the culture.

### <4> Double KO efficiency of TCR and B2M in activated T cells

On the 7th day from the start of the culture, the TCR-negative and B2M-negative cell ratio of the T cells treated by each method was measured by flow cytometry to evaluate the double KO efficiency.

FIG. 21 shows the frequency (%) of TCR- and B2M-double-negative cells among T cells treated with LNP in which Cas9 and two types of sgRNAs have been encapsulated in an empty LNP in Example 2 at each condition at each LNP treatment timing. From the results in FIG. 21, it was shown that a high double KO efficiency was obtained regardless of whether Cas9 mRNA and two types of sgRNA were encapsulated in one LNP or encapsulated in separate LNPs and sequentially treated.

### <5> Evaluation of translocation frequency at TRAC and B2M cleavage site

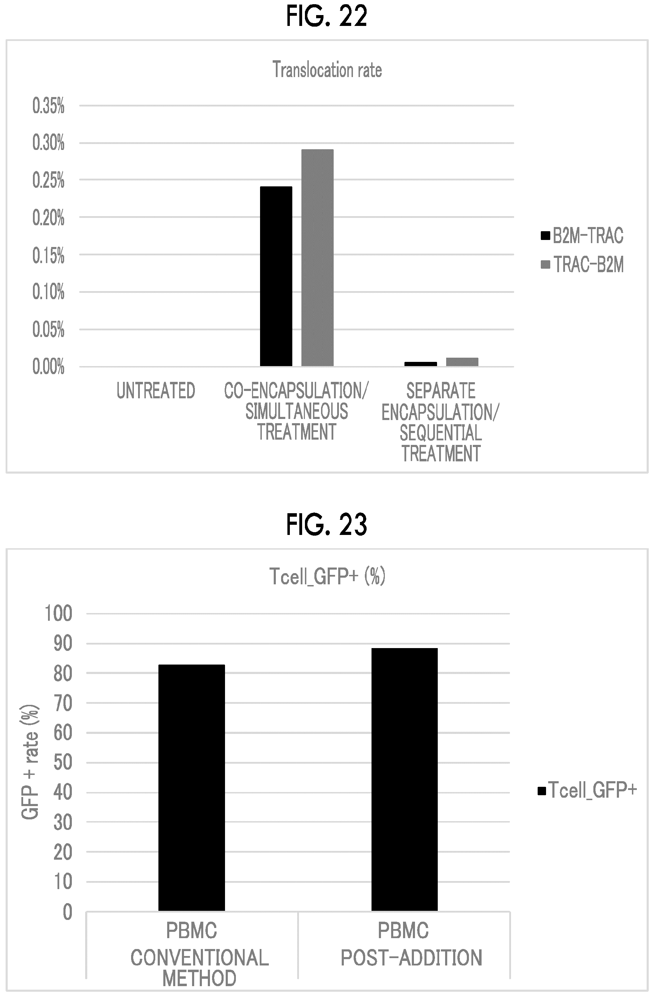

Genomic DNA was recovered from the T cells treated with each method on the 7th day of the culture. Genomic DNA was recovered using a QIAamp DNA Mini Kit (QIAGEN, 51306) according to the manufacturer's protocol. According to the protocol for ddPCR Supermix for Probes (No dUTP) (Bio-Rad, 1863024), ddPCR Supermix for Probes (No dUTP), the recovered genomic DNA, BamH1-HF (NEB, R3136S), HindIII-HF (NEB, R3104S), primers targeting the RPP30 gene locus, and a HEX-labeled probe (Fw primer: TCAGCATGGCGGTGTTT, Rv primer: GCTGTCTCCACAAGTC, probe: TTCTGACCTGAAGGCTCTGCGC) were mixed, and one set selected from primers and a FAM-labeled probe for detecting a normal B2M gene (Fw primer: tgggcacgcgtttaatataag, Rv primer: cttggagaagggaagtcacg, probe: cagagcgggagggtagga), primers and a FAM-labeled probe for detecting a normal TRAC gene (Fw primer: gtcccacagatatccagaaccc, Rv primer: tcagaatccttactttgtgacacat, probe: aatcggtgaataggcagacagact), primers and a FAM-labeled probe for detecting a B2M-TRAC gene translocation (Fw primer: tgggcacgcgtttaatataag, Rv primer: tcagaatccttactttgtgacacat, probe: aatcggtgaataggcagacagact), or primers and a FAM-labeled probe for detecting a TRAC-B2M gene translocation (Fw primer: gtcccacagatatccagaaccc, Rv primer: cttggagaagggaagtcacg, probe: cagagcgggagggtagga) was further added and mixed. Using these mixed solutions, the B2M-TRAC gene translocation frequency and the TRAC-B2M gene translocation frequency per cell were analyzed by the ddPCR method. FIG. 22 shows the analysis results of the translocation frequency in each treatment.

### <Test Example 9> Nucleic acid delivery to activated T cells using post-addition LNP (TCR KO)

### <1> LNP treatment of activated T cells

The activated T cells on the 3rd day of culture were collected in the required number of cells, centrifuged, and the supernatant was removed. The activated T cells were adjusted to a concentration of 1.0 × 10⁶ cells/ml in an activation culture medium containing recombinant human apolipoprotein E3 (ApoE3) (FUJIFILM Wako Pure Chemical Corporation, 010-20261) at a final concentration of 1 µg/ml, which was prepared in advance, and seeded in a 96-well plate.

Using the RNA-encapsulating LNP prepared by the post-addition method of Example 2, the LNP was added thereto such that the amount of RNA was 1.8 to 40.0 µg (total RNA amount) per 1.0 × 10⁶ cells, and cultured in a 37°C, 5% CO₂ incubator.

24 hours after the addition of the LNP, the activation culture medium was added to the T cell suspension at a volume ratio of 1:3, and the cells were expanded and cultured, and further cultured for 3 days.

### <2> TCR KO efficiency in activated T cells

On the 7th day of the start of the culture, the TCR-negative cell ratio of the T cells treated with each method was measured by flow cytometry, and the TCR KO efficiency was evaluated. The results are shown in Table 10.

**[Table 10]**

| | Addition concentration (µg/10⁶ cells) | TCR KO efficiency |
|---|---|---|
| Example 38 | 1.8 | 1.3% |
| Example 39 | 1.8 | 3.1% |
| Example 40 | 1.8 | 19.7% |
| Example 41 | 1.8 | 90.8% |
| Example 42 | 1.8 | 12.1% |
| Example 43 | 1.8 | 1.7% |
| Example 44 | 1.8 | 22.9% |
| Example 45 | 1.8 | 12.1% |
| Example 46 | 1.8 | 13.3% |
| Example 47 | 1.8 | 4.1% |
| Example 48 | 1.8 | 4.1% |
| Example 49 | 1.8 | 5.8% |
| Example 50 | 1.8 | 10.8% |
| Example 51 | 1.8 | 2.9% |
| Example 52 | 1.8 | 1.9% |
| Example 53 | 1.8 | 1.7% |
| Example 54 | 1.8 | 2.6% |
| Example 55 | 1.8 | 1.9% |
| Example 56 | 1.8 | 2.2% |
| Example 57 | 1.8 | 3.6% |
| Example 58 | 1.8 | 2.0% |
| Example 59 | 1.8 | 1.7% |
| Example 60 | 1.8 | 4.4% |
| Example 61 | 1.8 | 1.8% |
| Example 62 | 1.8 | 1.7% |
| Example 63 | 1.8 | 51.1% |
| Example 64 | 1.8 | 35.4% |
| Example 65 | 1.8 | 2.6% |
| Example 66 | 1.8 | 2.4% |
| Example 67 | 1.8 | 1.8% |
| Example 68 | 1.8 | 1.8% |
| Example 69 | 1.8 | 2.2% |
| Example 70 | 1.8 | 2.1% |
| Example 71 | 1.8 | 2.1% |
| Example 72 | 1.8 | 2.1% |
| Example 73 | 1.8 | 1.7% |
| Example 74 | 1.8 | 2.5% |
| Example 75 | 1.8 | 5.6% |
| Example 76 | 1.8 | 1.9% |
| Example 77 | 1.8 | 2.5% |
| Example 78 | 1.8 | 42.2% |
| Example 79 | 1.8 | 28.3% |
| Example 80 | 1.8 | 14.9% |
| Example 81 | 1.8 | 2.3% |
| Example 82 | 1.8 | 3.8% |
| Example 83 | 1.8 | 90.8% |
| Example 84 | 1.8 | 83.4% |
| Example 85 | 1.8 | 1.6% |
| Example 86 | 1.8 | 1.8% |
| Example 87 | 1.8 | 61.2% |
| Example 88 | 1.8 | 98.3% |
| Example 89 | 1.8 | 1.8% |
| Example 90 | 1.8 | 91.9% |
| Example 91 | 1.8 | 97.6% |
| Example 92 | 1.8 | 86.8% |
| Example 93 | 1.8 | 94.4% |
| Example 94 | 1.8 | 17.6% |
| Example 95 | 1.8 | 5.8% |
| Example 96 | 1.8 | 1.9% |
| Example 97 | 1.8 | 6.9% |
| Example 98 | 1.8 | 2.2% |
| Example 99 | 1.8 | 2.3% |
| Example 100 | 1.8 | 2.2% |
| Example 101 | 1.8 | 2.4% |
| Example 102 | 1.8 | 2.4% |
| Example 103 | 1.8 | 3.4% |
| Example 104 | 1.8 | 2.2% |
| Example 105 | 1.8 | 2.3% |
| Example 106 | 1.8 | 2.0% |
| Example 107 | 1.8 | 3.3% |
| Example 108 | 1.8 | 2.9% |
| Example 109 | 1.8 | 2.5% |
| Example 110 | 1.8 | 2.4% |
| Example 111 | 1.8 | 2.3% |
| Example 112 | 1.8 | 2.5% |
| Example 113 | 1.8 | 1.9% |
| Example 114 | 1.8 | 1.5% |
| Example 115 | 1.8 | 1.8% |
| Example 116 | 1.8 | 1.6% |
| Example 117 | 1.8 | 4.0% |
| Example 118 | 1.8 | 2.0% |
| Example 119 | 1.8 | 1.9% |
| Example 120 | 1.8 | 1.9% |
| Example 121 | 1.8 | 1.6% |
| Example 122 | 1.8 | 1.5% |
| Example 123 | 1.8 | 15.3% |
| Example 124 | 1.8 | 1.7% |
| Example 125 | 1.8 | 1.7% |
| Example 126 | 1.8 | 1.5% |
| Example 127 | 1.8 | 1.7% |
| Example 128 | 1.8 | 85.5% |
| Example 129 | 4.0 | 82.2% |
| Example 130 | 1.8 | 12.1% |
| Example 131 | 1.8 | 13.3% |
| Example 132 | 1.8 | 4.1% |
| Example 133 | 1.8 | 4.1% |
| Example 134 | 1.8 | 5.8% |
| Example 135 | 1.8 | 10.8% |
| Example 136 | 4.0 | 80.6% |
| Example 137 | 4.0 | 80.6% |
| Example 138 | 4.0 | 80.6% |
| Example 139 | 4.0 | 94.3% |
| Example 140 | 4.0 | 90.1% |
| Example 141 | 4.0 | 76.7% |
| Example 142 | 4.0 | 4.4% |
| Example 143 | 4.0 | 80.6% |
| Example 144 | 1.8 | 14.9% |
| Example 145 | 1.8 | 98.3% |
| Example 146 | 1.8 | 88.1% |
| Example 147 | 1.8 | 86.2% |
| Example 148 | 1.8 | 3.8% |
| Example 149 | 1.8 | 2.1% |
| Example 150 | 1.8 | 87.4% |
| Example 151 | 1.8 | 88.0% |
| Example 152 | 1.8 | 84.6% |
| Example 153 | 1.8 | 87.1% |
| Example 154 | 1.8 | 83.4% |
| Example 155 | 1.8 | 83.8% |
| Example 156 | 1.8 | 81.4% |
| Example 157 | 1.8 | 82.6% |
| Example 158 | 1.8 | 92.5% |

### <Test Example 10> Delivery of plasmid DNA to activated T cells using LNP of the related art

### <1> LNP treatment of activated T cells

The activated T cells on the 3rd day of culture were collected in the required number of cells, centrifuged, and the supernatant was removed. The activated T cells were adjusted to a concentration of 1.0 × 10⁶ cells/ml in a culture medium for activation or a CDM culture medium for activation, which contained recombinant human apolipoprotein E3 (ApoE3) (FUJIFILM Wako Pure Chemical Corporation, 010-20261) at a final concentration of 1 µg/ml, and seeded in a 96-well plate.

Using the GFP pDNA-encapsulating LNPs of Examples 159 to 202, the LNPs were added such that the amount of DNA was 0.4 to 10.0 µg (total DNA) per 1.0 × 10⁶ cells, and the cells were cultured in a 37°C, 5% CO₂ inclubator.

### <2> TCR KO efficiency in activated T cells

On the 4th day after the start of the culture, the GFP-positive cell ratio of the T cells treated with each method was measured by flow cytometry, and the plasmid DNA introduction efficiency was evaluated. The results are shown in Table 11.

**[Table 11]**

| | Addition concentration (µg/10⁶ cells) | GFP-positive cell ratio (%) |
|---|---|---|
| Example 159 | 1.8 | 9.5 |
| Example 160 | 1.8 | 9.4 |
| Example 161 | 1.8 | 5.0 |
| Example 162 | 1.8 | 1.5 |
| Example 163 | 1.8 | 0.7 |
| Example 164 | 1.8 | 3.7 |
| Example 165 | 1.8 | 5.7 |
| Example 166 | 1.8 | 6.4 |
| Example 167 | 1.8 | 10.0 |
| Example 168 | 1.8 | 7.8 |
| Example 169 | 1.8 | 5.2 |
| Example 170 | 1.8 | 7.0 |
| Example 171 | 1.8 | 5.8 |
| Example 172 | 1.8 | 1.9 |
| Example 173 | 1.8 | 2.9 |
| Example 174 | 1.8 | 2.9 |
| Example 175 | 1.8 | 1.0 |
| Example 176 | 1.8 | 1.0 |
| Example 177 | 1.8 | 1.4 |
| Example 178 | 1.8 | 1.1 |
| Example 179 | 1.8 | 1.0 |
| Example 180 | 1.8 | 0.6 |
| Example 181 | 1.8 | 0.3 |
| Example 182 | 1.8 | 1.3 |
| Example 183 | 1.8 | 0.6 |
| Example 184 | 1.8 | 0.4 |
| Example 185 | 1.8 | 0.9 |
| Example 185 | 1.8 | 0.9 |
| Example 186 | 1.8 | 9.6 |
| Example 187 | 1.8 | 5.5 |
| Example 188 | 1.8 | 4.0 |
| Example 189 | 1.8 | 2.1 |
| Example 190 | 1.8 | 1.2 |
| Example 191 | 1.8 | 5.7 |
| Example 192 | 1.8 | 1.4 |
| Example 193 | 1.8 | 4.0 |
| Example 194 | 1.8 | 8.4 |
| Example 195 | 1.8 | 7.8 |
| Example 196 | 1.8 | 5.3 |
| Example 197 | 1.8 | 5.5 |
| Example 198 | 1.8 | 4.2 |
| Example 199 | 1.8 | 1.6 |
| Example 200 | 1.8 | 3.1 |
| Example 201 | 1.8 | 3.4 |
| Example 202 | 1.8 | 3.7 |

### <Test Example 11> Delivery of plasmid DNA to activated T cells using post-addition LNP

### <1> LNP treatment of activated T cells

The activated T cells on the 3rd day of culture were collected in the required number of cells, centrifuged, and the supernatant was removed. The activated T cells were adjusted to a concentration of 1.0 × 10⁶ cells/ml in a culture medium for activation or a CDM culture medium for activation, which contained recombinant human apolipoprotein E3 (ApoE3) (FUJIFILM Wako Pure Chemical Corporation, 010-20261) at a final concentration of 1 µg/ml, and seeded in a 96-well plate.

Using the GFP pDNA-encapsulating LNPs of Examples 203 to 391, the LNPs were added such that the amount of DNA was in an amount corresponding to 0.4 to 10.0 µg of total DNA per 1.0 × 10⁶ cells, and the cells were cultured in a 37°C, 5% CO₂ inclubator.

### <2> TCR KO efficiency in activated T cells

On the 4th day after the start of the culture, the GFP-positive cell ratio of the T cells treated with each method was measured by flow cytometry, and the plasmid DNA introduction efficiency was evaluated. The results are shown in Table 12.

**[Table 12]**

| | Addition concentration (µg/10⁶ cells) | GFP-positive cell ratio (%) |
|---|---|---|
| Example 203 | 1.8 | 4.5 |
| Example 204 | 1.8 | 10.8 |
| Example 205 | 1.8 | 9.6 |
| Example 206 | 1.8 | 1.3 |
| Example 207 | 1.8 | 1.1 |
| Example 208 | 1.8 | 22.1 |
| Example 209 | 1.8 | 22.1 |
| Example 210 | 1.8 | 24.4 |
| Example 211 | 1.8 | 9.4 |
| Example 212 | 1.8 | 1.4 |
| Example 213 | 1.8 | 17.3 |
| Example 214 | 1.8 | 20.6 |
| Example 215 | 1.8 | 11.4 |
| Example 216 | 1.8 | 7.5 |
| Example 217 | 1.8 | 5.6 |
| Example 218 | 1.8 | 2.8 |
| Example 219 | 1.8 | 2.4 |
| Example 220 | 1.8 | 0.5 |
| Example 221 | 1.8 | 0.9 |
| Example 222 | 1.8 | 12.1 |
| Example 223 | 1.8 | 14.6 |
| Example 224 | 1.8 | 11.9 |
| Example 225 | 1.8 | 7.3 |
| Example 226 | 1.8 | 4.9 |
| Example 227 | 1.8 | 7.0 |
| Example 228 | 1.8 | 4.5 |
| Example 229 | 1.8 | 2.5 |
| Example 230 | 1.8 | 1.5 |
| Example 231 | 1.8 | 9.8 |
| Example 232 | 1.8 | 14.0 |
| Example 233 | 1.8 | 14.7 |
| Example 234 | 1.8 | 17.2 |
| Example 235 | 1.8 | 9.1 |
| Example 236 | 1.8 | 3.0 |
| Example 237 | 1.8 | 0.8 |
| Example 238 | 1.8 | 0.9 |
| Example 239 | 1.8 | 0.5 |
| Example 240 | 1.8 | 11.1 |
| Example 241 | 1.8 | 13.2 |
| Example 242 | 1.8 | 11.7 |
| Example 243 | 1.8 | 2.5 |
| Example 244 | 1.8 | 0.4 |
| Example 245 | 1.8 | 11.0 |
| Example 246 | 1.8 | 12.3 |
| Example 247 | 1.1 | 11.0 |
| Example 248 | 1.1 | 10.6 |
| Example 249 | 1.1 | 9.6 |
| Example 250 | 1.1 | 9.9 |
| Example 251 | 1.1 | 10.8 |
| Example 252 | 1.1 | 9.3 |
| Example 253 | 3.3 | 13.7 |
| Example 254 | 3.3 | 12.9 |
| Example 255 | 3.3 | 9.4 |
| Example 256 | 3.3 | 12.6 |
| Example 257 | 3.3 | 11.2 |
| Example 258 | 3.3 | 12.0 |
| Example 259 | 10.0 | 8.4 |
| Example 260 | 10.0 | 11.3 |
| Example 261 | 10.0 | 10.0 |
| Example 262 | 10.0 | 12.3 |
| Example 263 | 10.0 | 10.8 |
| Example 264 | 10.0 | 11.5 |
| Example 265 | 0.4 | 24.8 |
| Example 266 | 0.4 | 23.8 |
| Example 267 | 0.4 | 14.4 |
| Example 268 | 0.4 | 18.7 |
| Example 269 | 0.4 | 16.7 |
| Example 270 | 0.4 | 18.6 |
| Example 271 | 1.1 | 22.1 |
| Example 272 | 1.1 | 22.8 |
| Example 273 | 1.1 | 16.1 |
| Example 274 | 1.1 | 17.4 |
| Example 275 | 1.1 | 11.2 |
| Example 276 | 1.1 | 16.2 |
| Example 277 | 3.3 | 13.7 |
| Example 278 | 3.3 | 12.9 |
| Example 279 | 3.3 | 9.4 |
| Example 280 | 3.3 | 12.6 |
| Example 281 | 3.3 | 11.2 |
| Example 282 | 3.3 | 12.0 |
| Example 283 | 10.0 | 15.3 |
| Example 284 | 10.0 | 15.6 |
| Example 285 | 10.0 | 17.1 |
| Example 286 | 10.0 | 14.9 |
| Example 287 | 10.0 | 8.5 |
| Example 288 | 10.0 | 14.9 |
| Example 289 | 1.8 | 5.6 |
| Example 290 | 1.8 | 5.9 |
| Example 291 | 1.8 | 4.6 |
| Example 292 | 1.8 | 0.3 |
| Example 293 | 1.8 | 0.2 |
| Example 294 | 1.8 | 14.2 |
| Example 295 | 1.8 | 15.8 |
| Example 296 | 1.8 | 15.8 |
| Example 297 | 1.8 | 0.5 |
| Example 298 | 1.8 | 0.4 |
| Example 299 | 1.8 | 3.8 |
| Example 300 | 1.8 | 3.7 |
| Example 301 | 1.8 | 3.3 |
| Example 302 | 1.8 | 1.4 |
| Example 303 | 1.8 | 0.6 |
| Example 304 | 1.8 | 0.4 |
| Example 305 | 1.8 | 0.9 |
| Example 306 | 1.8 | 5.5 |
| Example 307 | 1.8 | 5.6 |
| Example 308 | 1.8 | 4.5 |
| Example 309 | 1.8 | 2.3 |
| Example 310 | 1.8 | 1.1 |
| Example 311 | 1.8 | 4.4 |
| Example 312 | 1.8 | 6.4 |
| Example 313 | 1.8 | 7.1 |
| Example 314 | 1.8 | 10.2 |
| Example 315 | 1.8 | 5.6 |
| Example 316 | 0.6 | 3.8 |
| Example 317 | 0.2 | 2.1 |
| Example 318 | 1.8 | 0.9 |
| Example 319 | 1.8 | 0.4 |
| Example 320 | 1.8 | 1.3 |
| Example 321 | 1.8 | 0.7 |
| Example 322 | 1.8 | 0.4 |
| Example 323 | 1.8 | 1.1 |
| Example 324 | 1.8 | 1.3 |
| Example 325 | 1.8 | 1.2 |
| Example 326 | 1.8 | 1.2 |
| Example 327 | 1.8 | 1.0 |
| Example 328 | 1.8 | 2.2 |
| Example 329 | 1.8 | 1.5 |
| Example 330 | 1.8 | 4.3 |
| Example 331 | 1.8 | 4.9 |
| Example 332 | 1.8 | 1.3 |
| Example 333 | 1.8 | 3.3 |
| Example 334 | 1.8 | 11.7 |
| Example 335 | 1.8 | 13.1 |
| Example 336 | 1.8 | 14.1 |
| Example 337 | 1.8 | 0.3 |
| Example 338 | 1.8 | 2.0 |
| Example 339 | 1.8 | 12.9 |
| Example 340 | 1.8 | 9.7 |
| Example 341 | 1.8 | 0.4 |
| Example 342 | 1.8 | 7.8 |
| Example 343 | 1.8 | 24.8 |
| Example 344 | 1.8 | 12.0 |
| Example 345 | 1.8 | 19.5 |
| Example 346 | 1.8 | 11.3 |
| Example 347 | 1.8 | 18.6 |
| Example 348 | 1.8 | 3.3 |
| Example 349 | 1.8 | 1.5 |
| Example 350 | 1.8 | 0.2 |
| Example 351 | 1.8 | 0.2 |
| Example 352 | 1.8 | 0.7 |
| Example 353 | 1.8 | 0.2 |
| Example 354 | 1.8 | 0.4 |
| Example 355 | 1.8 | 0.5 |
| Example 356 | 1.8 | 0.8 |
| Example 357 | 1.8 | 1.0 |
| Example 358 | 1.8 | 0.2 |
| Example 359 | 1.8 | 1.0 |
| Example 360 | 1.8 | 0.5 |
| Example 361 | 1.8 | 1.4 |
| Example 362 | 1.8 | 0.9 |
| Example 363 | 1.8 | 0.2 |
| Example 364 | 1.8 | 0.4 |
| Example 365 | 1.8 | 0.2 |
| Example 366 | 1.8 | 1.3 |
| Example 367 | 1.8 | 0.2 |
| Example 368 | 1.8 | 8.5 |
| Example 369 | 1.8 | 10.1 |
| Example 370 | 1.8 | 5.0 |
| Example 371 | 1.8 | 8.8 |
| Example 372 | 1.8 | 2.4 |
| Example 373 | 1.8 | 1.7 |
| Example 374 | 1.8 | 4.6 |
| Example 375 | 1.8 | 4.3 |
| Example 376 | 1.8 | 6.9 |
| Example 383 | 1.8 | 8.2 |
| Example 384 | 1.8 | 8.3 |
| Example 385 | 1.8 | 8.2 |
| Example 386 | 1.8 | 9.5 |
| Example 387 | 1.8 | 6.4 |
| Example 388 | 1.8 | 8.5 |
| Example 389 | 1.8 | 5.0 |
| Example 390 | 1.8 | 7.4 |
| Example 391 | 1.8 | 18.4 |

### <Test Example 12> Nucleic acid delivery to human peripheral blood mononuclear cells (hPBMC) using conventional method LNP or post-addition LNP

### <1> Preparation of culture medium

The culture medium used for culturing hPBMC consists of TexMACS^{™} Medium (Miltenyi Biotec, 130-097-196) and 5 ng/ml human interleukin-2 (IL-2, Roche, 11147528001) (hereinafter, referred to as an activation culture medium).

### <2> Preparation and culture of PBMC

Human peripheral blood mononuclear cells (hPBMC, Frozen, STEMCELL Technologies, 70025) derived from the peripheral blood of a healthy human donor were thawed by being placed in a water bath at 37°C for several minutes. The thawed hPBMC was resuspended in TexMACS Medium containing 1% BSA (SIGMA, A9576) and 20 U/ml DNase I (Worthington Biochemical, LS002139), further washed by centrifugation, and resuspended in a culture medium. The hPBMC was prepared at a cell concentration of 1.0 × 10⁶ cells/ml using the present culture medium, and Dynabeads Human T-Activator CD3/CD28 (Thermo Fisher DB11131) was added thereto such that the concentration of the beads was 1.0 × 10⁶ beads/ml. The cells were seeded in a 24-well plate for cell culture and cultured in a 37°C, 5% CO₂ incubator for 3 days. On the third day of culture, the Dynabeads were removed from the cell culture solution.

### <3> Encapsulation of GFP mRNA in empty LNP (post-addition method)

CleanCap (registered trademark) EGFP mRNA (5 moU) (TriLink, L-7201) was diluted with water for injection to prepare an RNA solution (total nucleic acid concentration: 400 µg/mL).

The empty LNP of Example 2 stored at -70°C was thawed at 4°C. The RNA solution was added to the present LNP liquid in an equal liquid amount and mixed by pipetting (LNP-RNA mixed solution). After being allowed to stand at room temperature for 5 minutes, 20 mmol/L Tris buffer containing 8% sucrose was added to the present LNP-RNA mixed solution in an equal liquid amount, and the mixture was mixed by pipetting to prepare GFP mRNA-encapsulating LNP by a post-addition method (post-addition method GFP mRNA-encapsulating LNP).

### <4> LNP treatment of PBMC

The cells on the third day of culture were collected in a required number of cells, centrifuged, and the supernatant was removed. The cells were adjusted to a concentration of 1.0 × 10⁶ cells/ml in a culture medium containing recombinant human apolipoprotein E3 (ApoE3) (FUJIFILM Wako Pure Chemical Corporation, 010-20261) at a final concentration of 1 µg/ml, which had been prepared in advance, and seeded in a 96-well plate.

Using the GFP mRNA-encapsulating LNP by conventional method of Example 32 and the GFP mRNA-encapsulating LNP prepared by the post-addition method in <3>, the LNPs were added such that the amount of the RNA was 4.0 µg (total RNA amount) per 1.0 × 10⁶ cells, and the cells were cultured in a 37°C, 5% CO₂ incubator.

### <5> mRNA expression efficiency in hPBMC

On the 4th day after the start of the culture, the GFP-positive rate of the hPBMC edited by each method was evaluated by flow cytometry.

For the evaluation of the GFP efficiency of the hPBMC, after staining dead cells with BD Horizon^{TM} Fixable Viability Stain (FVS) Reagents (BD, 565388), the cells were incubated with an antibody cocktail in which an anti-CD45 antibody (BD, 564105), an anti-CD3 antibody (BD, 563423), an anti-CD14 antibody (BD, 555399), an anti-CD19 antibody (R&D Systems, FAB4867V-100UG), an anti-CD16 antibody (BD, 562878), and an anti-CD56 antibody (BD, 740076) were mixed.

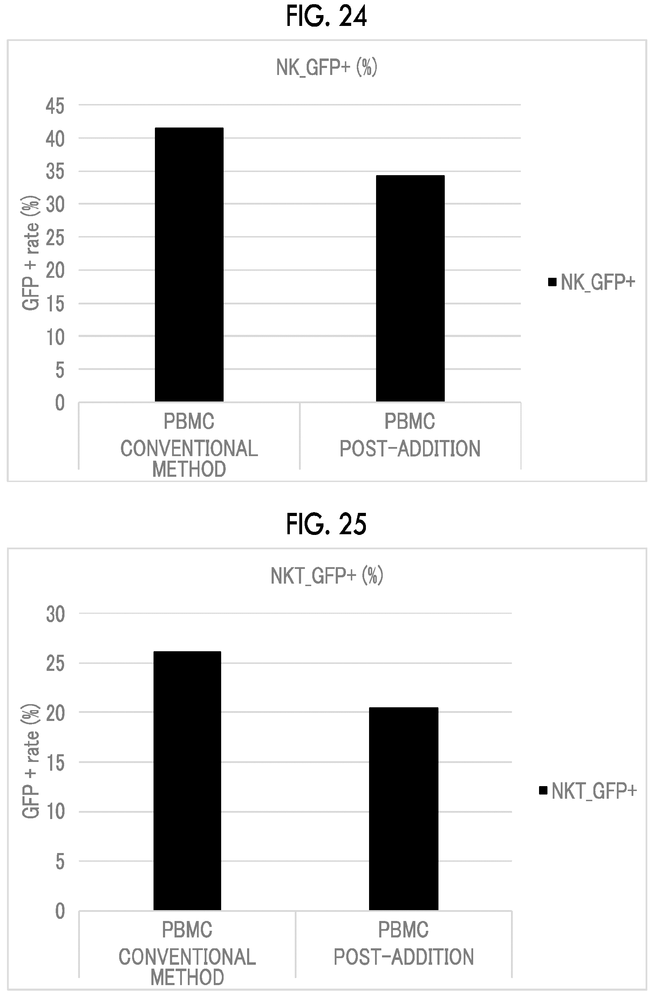

After each of these staining operations, the cells were fixed and washed, and the cell state was analyzed with an Attune device (Thermo Fisher Scientific, Inc.). The analysis data was analyzed using Flowjo software. The cell measurement data was gated for single cells, CD45-positive cells, and viable cells, and then CD3-positive CD16/56-negative cells were gated as T cells, CD3-negative CD16/56-positive cells were gated as NK cells, CD3-positive CD16/56-positive cells were gated as NKT cells, and CD3-negative CD19-positive cells were gated as B cells, and the ratio of GFP-positive cells was analyzed. From the results shown in FIGS. 23 to 26, it was shown that the mRNA can be introduced into T cells, NK cells, NKT cells, and B cells in the PBMC with high efficiency in both the method of the related art and the post-addition method.

### <Test Example 13> Nucleic acid delivery to human acute monocytic leukemia-derived cells (THP-1) using post-added LNP

### <1> Encapsulation of GFP mRNA in empty LNP (post-addition method)

CleanCap (registered trademark) EGFP mRNA (5 moU) (TriLink, L-7201) was diluted with water for injection to prepare an RNA solution (total nucleic acid concentration: 400 µg/mL).

The empty LNP of Example 2 stored at -70°C was thawed at 4°C. The RNA solution was added to the present LNP liquid in an equal liquid amount and mixed by pipetting (LNP-RNA mixed solution). After being allowed to stand at room temperature for 5 minutes, 20 mmol/L Tris buffer containing 8% sucrose was added to the present LNP-RNA mixed solution in an equal liquid amount, and the mixture was mixed by pipetting to prepare GFP mRNA-encapsulating LNP by the post-addition method (post-addition method GFP mRNA-encapsulating LNP).

### <2> Culture of THP-1 and LNP treatment

Human acute monocytic leukemia-derived cells (THP-1, JCRB Cell Bank, JCRB0112.1) were prepared at a cell concentration of 1.1 × 10⁶ cells/ml using TexMACS Medium containing a final concentration of 1 µg/ml ApoE3 or RPMI 1640 Medium (ATCC modification) (Thermo Fisher, A1049101) containing a final concentration of 1 µg/ml ApoE3 and 10% fetal bovine serum, and seeded in a 96-well plate for cell culture at 0.1 ml/well. Subsequently, 4 µL (final concentration: 4 µg/ml) or 8 µL (final concentration: 8 µg/ml) of the GFP mRNA-encapsulating LNP (total RNA concentration: 100 µg/mL) prepared by the post-addition method in <1> above was added to each well, and the cells were cultured in a 37°C, 5% CO₂ incubator.

### <3> mRNA expression efficiency in THP-1

The THP-1 cells treated under each condition were observed with a fluorescence microscope the day after the LNP treatment, and the GFP-positive rate was evaluated. In addition, the THP-1 cells treated under each condition were recovered from the 96-well plate, and the GFP-positive rate was evaluated by flow cytometry.

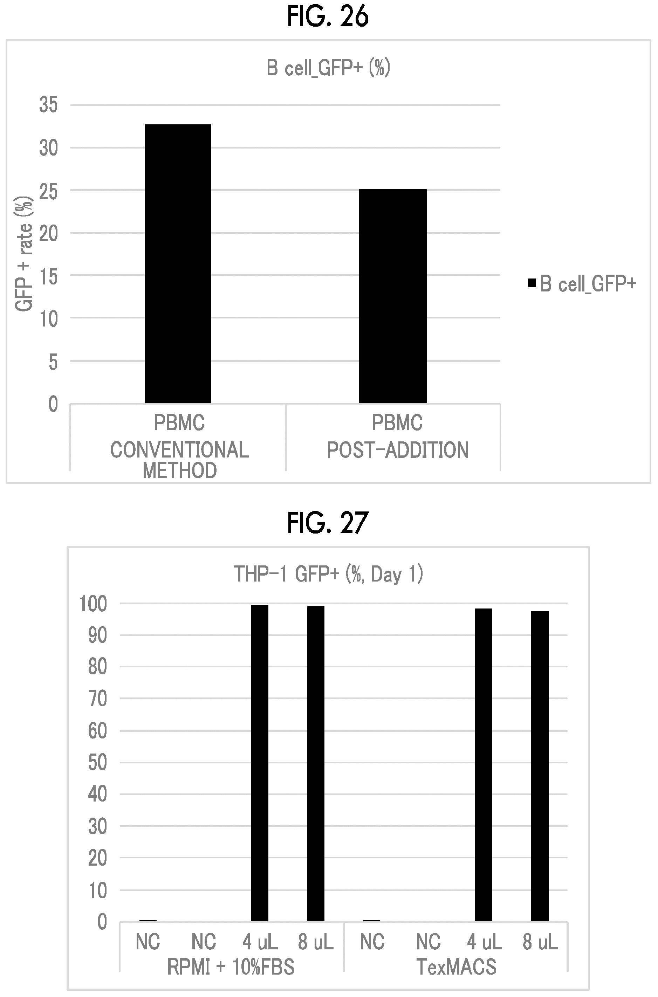

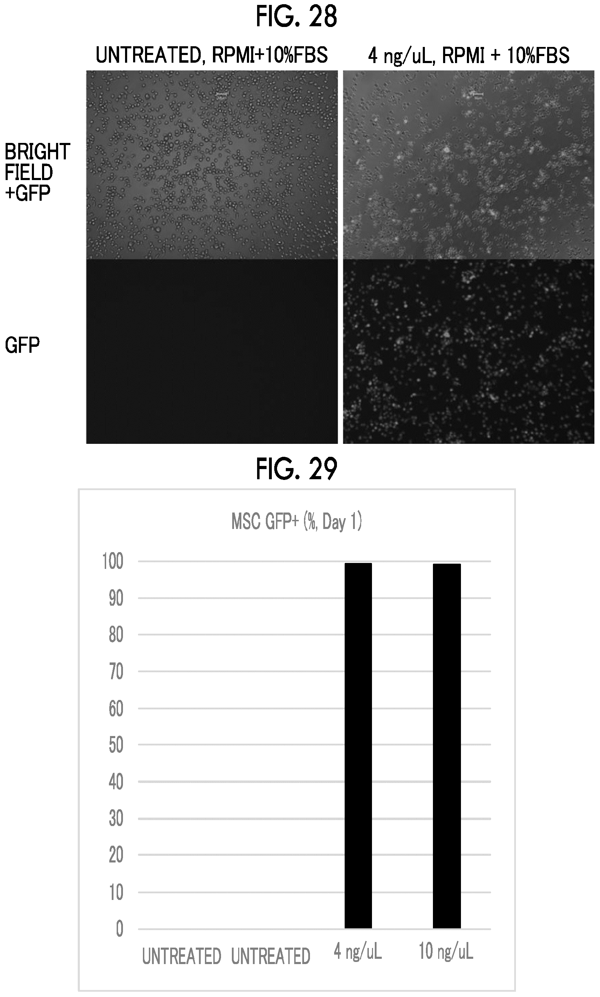

The GFP efficiency of THP-1 was evaluated by staining dead cells with BD Horizon^{TM} Fixable Viability Stain (FVS) Reagents (BD, 565388), fixing and washing the cells, and analyzing the cell state with an Attune device (Thermo Fisher). The analysis data was analyzed using Flowjo software. The cell measurement data was gated for size, single cells, and live cells, and then the ratio of GFP-positive cells was analyzed. From the results shown in FIGS. 27 and 28, it was shown that mRNA can be efficiently introduced into THP-1.

### <Test Example 14> Nucleic acid delivery to human bone marrow-derived mesenchymal stem cells (BM-MSCs) using post-added LNP

### <1> Preparation and culture of BM-MSCs

Mesenchymal stem cells (Xeno-Free RoosterVial (TM)-hBM, Roosterbio, MSC-031) derived from the bone marrow of a healthy human donor were suspended in PRIME-XV Expansion XSFM (FUJIFILM Irvine Scientific, 91149) (hereinafter, a culture medium for MSCs), prepared at a cell concentration of 0.015 × 10⁶ cells/ml, seeded in a 24-well plate for cell culture at 0.5 ml/well, and cultured in a 37°C, 5% CO₂ incubator for 4 days.

### <2> Encapsulation of GFP mRNA in empty LNP (post-addition method)

CleanCap (registered trademark) EGFP mRNA (5 moU) (TriLink, L-7201) was diluted with water for injection to prepare an RNA solution (total nucleic acid concentration: 400 µg/mL).

The empty LNP of Example 2 stored at -70°C was thawed at 4°C. The RNA solution was added to the present LNP liquid in an equal liquid amount and mixed by pipetting (LNP-RNA mixed solution). After being allowed to stand at room temperature for 5 minutes, 20 mmol/L Tris buffer containing 8% sucrose was added to the present LNP-RNA mixed solution in an equal liquid amount, and the mixture was mixed by pipetting to prepare GFP mRNA-encapsulating LNP by a post-addition method (post-addition method GFP mRNA-encapsulating LNP).

### <3> LNP treatment of BM-MSCs

On the 4th day of culture, the culture medium was removed by suction from the wells in which the cells had been seeded, and the culture medium for MSCs containing ApoE3 at a final concentration of 1 µg/ml, which had been prepared in advance, was added to each well in an amount of 500 µL/well. 20 µL (final concentration: 4 µg/ml) or 50 µL (final concentration: 10 µg/ml) of the GFP mRNA-encapsulating LNP (total RNA concentration: 100 µg/mL) prepared by the post-addition method in <3> above was added to each well, and the cells were cultured in a 37°C, 5% CO₂ incubator.

### <4> mRNA expression efficiency in BM-MSCs

The BM-MSCs treated under each condition were observed with a fluorescence microscope the day after the LNP treatment, and the GFP-positive rate was evaluated. In addition, the BM-MSCs treated under each condition were detached and collected from a 24-well plate, and the GFP-positive rate was evaluated by flow cytometry.

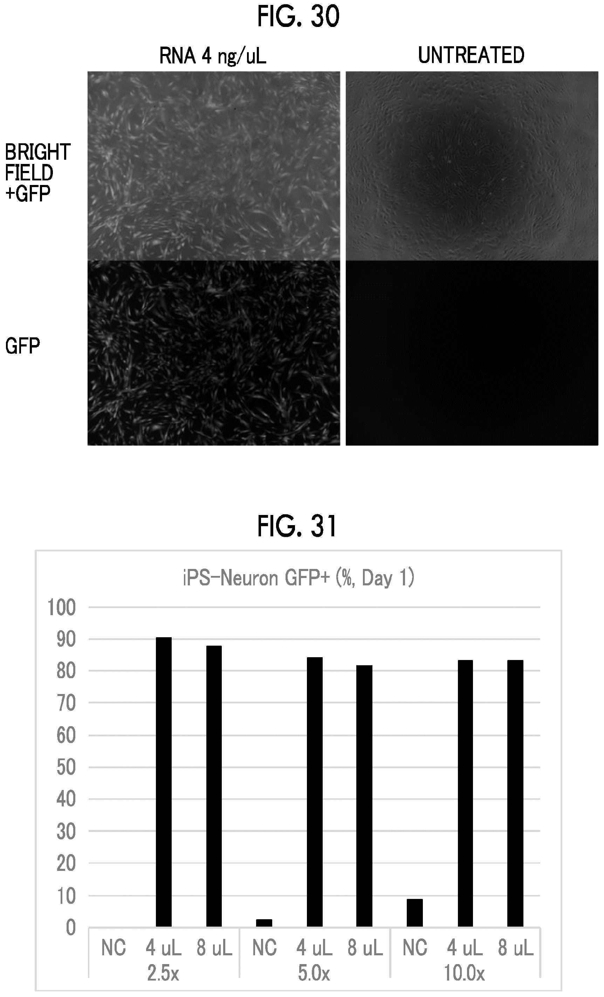

The GFP efficiency of the BM-MSCs was evaluated by staining dead cells with BD Horizon^{TM} Fixable Viability Stain (FVS) Reagents (BD, 565388), fixing and washing the cells, and analyzing the cell state with an Attune device (Thermo Fisher Scientific, Inc.). The analysis data was analyzed using Flowjo software. The cell measurement data was gated for size, single cells, and live cells, and then the ratio of GFP-positive cells was analyzed. From the results shown in FIGS. 29 and 30, it was shown that mRNA can be introduced into BM-MSCs with high efficiency.

### <Test Example 15> Nucleic acid delivery to iPS cell-derived nerve cells (iNeuron) using post-added LNP

### <1> Preparation of culture medium

The culture medium used for culturing iNeurons consists of Neurobasal Medium (Thermo Fisher Scientific, 21103-049), GlutaMAX Supplement (Thermo Fisher Scientific, 35050061) (final concentration: 1%), and B27 supplement (Thermo Fisher Scientific, 17504) (final concentration: 2%) (hereinafter, referred to as a culture medium for iNeurons).

### <2> Culture of iNeurons

The neurons were produced by forcibly expressing the Ngn2 genes from the iPS cells and the cells that had been frozen and stored were used. The frozen-stored cells were thawed in a warm bath at 37°C, and after melting, the cells were added to a culture medium for iNeurons and centrifuged at 600 × g at room temperature for 5 minutes. After centrifugation, the supernatant was removed, the cells were suspended in 1 mL of a culture medium for iNeurons, and the number of cells was counted. Nerve cells were seeded in a 96-well plate coated with iMatrix (Matrixome, 892021) at 2.5 to 10.0 × 10⁴ cells/well, and cultured in a 37°C, 5% CO₂ incubator for 1 day.

### <3> Encapsulation of GFP mRNA in empty LNP (post-addition method)

CleanCap (registered trademark) EGFP mRNA (5 moU) (TriLink, L-7201) was diluted with water for injection to prepare an RNA solution (total nucleic acid concentration: 400 µg/mL).

The empty LNP of Example 2 stored at -70°C was thawed at 4°C. The RNA solution was added to the present LNP liquid in an equal liquid amount and mixed by pipetting (LNP-RNA mixed solution). After being allowed to stand at room temperature for 5 minutes, 20 mmol/L Tris buffer containing 8% sucrose was added to the present LNP-RNA mixed solution in an equal liquid amount, and the mixture was mixed by pipetting to prepare GFP mRNA-encapsulating LNP by a post-addition method (post-addition method GFP mRNA-encapsulating LNP).

### <4> LNP treatment of iNeuron

On the first day of culture, the culture medium was removed by suction from the wells in which the cells were seeded, and a culture medium for iNeurons containing ApoE3 at a final concentration of 1 µg/ml, which had been prepared in advance, was added to each well in an amount of 100 µL/well. 4 µL (final concentration: 4 µg/ml) or 8 µL (final concentration: 10 µg/ml) of the GFP mRNA-encapsulating LNP (total RNA concentration: 100 µg/mL) prepared by the post-addition method in <3> above was added to each well, and the cells were cultured in a 37°C, 5% CO₂ incubator.

### <5> mRNA expression efficiency in iNeuron

The iNeurons treated under each condition were observed with a fluorescence microscope the day after the LNP treatment, and the GFP-positive rate was evaluated. In addition, the iNeurons treated under each condition were detached and recovered from a 96-well plate, and the GFP-positive rate was evaluated by flow cytometry.

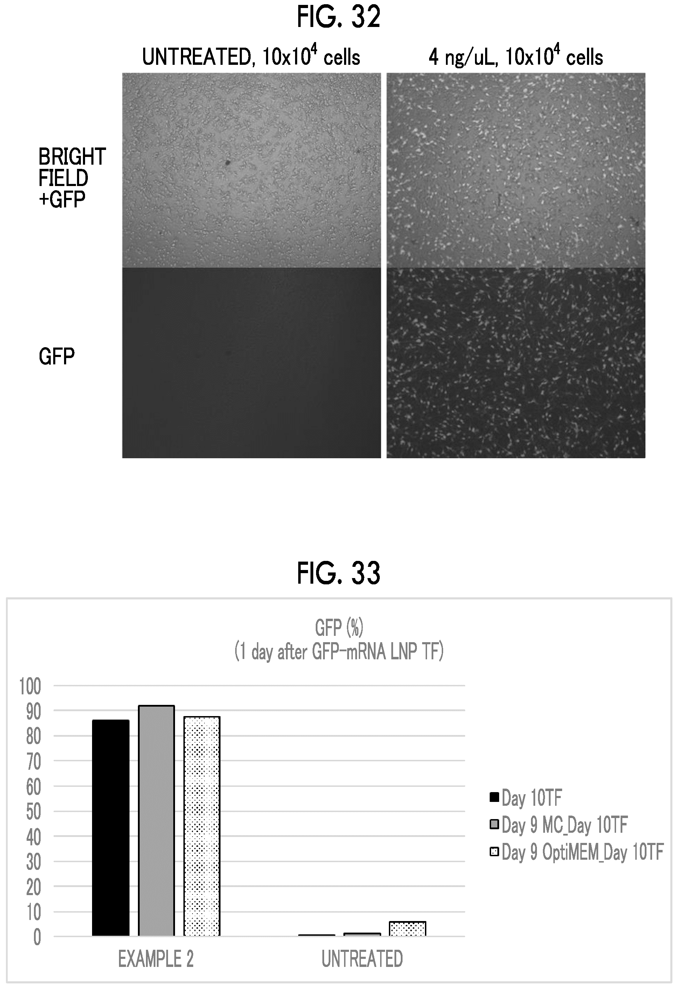

The GFP efficiency of iNeurons was evaluated by staining dead cells with BD Horizon^{TM} Fixable Viability Stain (FVS) Reagents (BD, 565388), fixing and washing the cells, and analyzing the cell state with an Attune device (Thermo Fisher Scientific, Inc.). The analysis data was analyzed using Flowjo software. The cell measurement data was gated for size, single cells, and live cells, and then the ratio of GFP-positive cells was analyzed. From the results shown in FIGS. 31 and 32, it was shown that mRNA can be efficiently introduced into iNeurons.

### <Test Example 16> Nucleic acid delivery to T cells after long-term culture

### <1> Adjustment of T cells after long-term culture

The T cells on the 10th day of the culture (7 days after activation) were collected in the required number of cells, centrifuged, and the supernatant was removed. The activated T cells were adjusted to a concentration of 1.0 × 10⁶ cells/ml in an activation culture medium containing recombinant human apolipoprotein E3 (ApoE3) (FUJIFILM Wako Pure Chemical Corporation, 010-20261) at a final concentration of 1 µg/ml, which was prepared in advance, and seeded in a 96-well plate.

### <2> Adjustment of T cells after long-term culture in which the culture medium was exchanged the day before the LNP treatment

The T cells on the 9th day of the culture (6 days after activation) in the activation culture medium were collected, resuspended in a newly adjusted activation culture medium or 5 ng/ml IL-2-containing Opti-MEM (TM) I Reduced Serum Medium (Thermo Fisher, 31985062), and cultured for 1 day. The T cells on the 10th day of the culture were collected in the required number of cells, centrifuged, and the supernatant was removed. The activated T cells were adjusted to a concentration of 1.0 × 10⁶ cells/ml in an activation culture medium containing recombinant human apolipoprotein E3 (ApoE3) (FUJIFILM Wako Pure Chemical Corporation, 010-20261) at a final concentration of 1 µg/ml, which was prepared in advance, and seeded in a 96-well plate.

### <3> Encapsulation of GFP mRNA in empty LNP (post-addition method)

CleanCap (registered trademark) EGFP mRNA (5 moU) (TriLink, L-7201) was diluted with water for injection to prepare an RNA solution (total nucleic acid concentration: 400 µg/mL).

The empty LNP of Example 2 stored at -70°C was thawed at 4°C. The RNA solution was added to the present LNP liquid in an equal liquid amount and mixed by pipetting (LNP-RNA mixed solution). After being allowed to stand at room temperature for 5 minutes, 20 mmol/L Tris buffer containing 8% sucrose was added to the present LNP-RNA mixed solution in an equal liquid amount, and the mixture was mixed by pipetting to prepare GFP mRNA-encapsulating LNP by a post-addition method (post-addition method GFP mRNA-encapsulating LNP).

The GFP mRNA-encapsulating LNP prepared by the post-addition method in <3> above was added thereto such that the amount of the GFP mRNA-encapsulating LNP was 4.0 µg (total RNA amount) per 1.0 × 10⁶ cells, and the mixture was cultured in a 37°C, 5% CO₂ incubator. The cells were collected 24 hours after the addition of the LNP, the GFP-positive cell ratio of the T cells treated with each LNP was measured by flow cytometry, and the GFP mRNA introduction efficiency was evaluated. The present results are shown in FIG. 33 (Day9 MC_Day10 TF condition, Day9 OptiMEM_Day10 TF condition).

[Sequence Table] International application 23F01287W1JP24046425_154.xml based on International Patent Cooperation Treaty

## Claims

1. A method for delivering nucleic acid to an immune cell (excluding an in vivo delivery method), the method comprising:
a step A of preparing lipid particles not containing nucleic acid using an ionizable lipid, a non-ionizable lipid, and a lipid having a nonionic polymer;
a step B of preparing nucleic acid-containing lipid particles by mixing the lipid particles not containing nucleic acid with nucleic acid; and
a step C of bringing the nucleic acid-containing lipid particles into contact with an immune cell.

2. The method according to claim 1, further comprising:
freezing and storing the lipid particles not containing nucleic acid and thawing the lipid particles not containing nucleic acid that have been frozen and stored before mixing with the nucleic acid.

3. The method according to claim 1,
wherein the step B includes a step of incubating the lipid particles not containing nucleic acid and an aqueous solution containing the nucleic acid at 0°C to 30°C for 0.1 to 120 minutes, and a step of adjusting a pH of the resulting mixture to 6.5 to 8.5.

4. The method according to claim 1,
wherein, in the step B, the mixing of the lipid particles not containing nucleic acid with the nucleic acid is performed by any one selected from mixing in which a liquid is moved back and forth in a reciprocating direction in a container, pipette mixing, stirrer mixing in a batch container, mixing in which a liquid content is agitated by rotating a container, or flask agitation.

5. The method according to claim 1, further comprising:
a step of adding an apolipoprotein to a culture solution containing the immune cell before bringing the nucleic acid-containing lipid particles into contact with the immune cell in the step C.

6. The method according to any one of claims 1 to 5,
wherein the ionizable lipid is a compound represented by Formula (1) or a salt thereof, 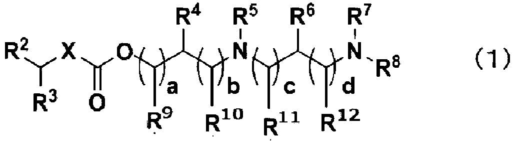
in the formula, X represents -NR¹- or -O-,
R¹ represents a hydrogen atom, a hydrocarbon group having 6 to 24 carbon atoms, or a group represented by R²¹-L¹-R²²-, where R²¹ represents a hydrocarbon group having 1 to 24 carbon atoms, L¹ represents -O(CO)O-, -O(CO)-, -(CO)O-, -O-, or 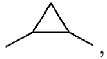 and R²² represents a divalent hydrocarbon linking group having 1 to 18 carbon atoms,
R² and R³ each independently represent a hydrogen atom, a hydrocarbon group having 3 to 24 carbon atoms, or a group represented by R³¹-L²-R³²-, where R³¹ represents a hydrocarbon group having 1 to 24 carbon atoms, L² represents -O(CO)O-, -O(CO)-, -(CO)O-, -O-, or 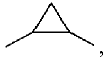 and R³² represents a divalent hydrocarbon linking group having 1 to 18 carbon atoms,
R⁴, R⁵, R⁶, R⁷, R⁸, R⁹, R¹⁰, R¹¹, and R¹² each independently represent a hydrogen atom or an alkyl group having 1 to 18 carbon atoms which may be substituted,
any one or more of the pairs of R⁴ and R⁵, R¹⁰ and R⁵, R⁵ and R¹², R⁴ and R⁶, R⁵ and R⁶, R⁶ and R⁷, R⁶ and R¹⁰, R¹² and R⁷, and R⁷ and R⁸ may be linked to each other to form a 4-to 7-membered ring which may contain an O atom, a substituent on the alkyl group having 1 to 18 carbon atoms which may be substituted is a hydroxyl group, a carboxyl group, an amino group represented by -NR⁴⁵R⁴⁶, a substituted or unsubstituted aryl group, a substituted or unsubstituted heteroaryl group, or a group represented by -O(CO)O-R⁴¹, -O(CO)-R⁴², -(CO)O-R⁴³, or -O-R⁴⁴, where R⁴¹, R⁴², R⁴³ R⁴⁴, R⁴⁵, and R⁴⁶ each independently represent a hydrocarbon group having 1 to 18 carbon atoms,
a substituent on the substituted or unsubstituted aryl group and on the substituted or unsubstituted heteroaryl group is an alkyl group having 1 to 18 carbon atoms, a hydroxyl group, a carboxyl group, an amino group represented by -NR⁴⁵R⁴⁶, or a group represented by -O(CO)O-R⁴¹, -O(CO)-R⁴², -(CO)O-R⁴³, or -O-R⁴⁴ , where R⁴¹, R⁴², R⁴³, R⁴⁴, R⁴⁵, and R⁴⁶ each independently represent a hydrocarbon group having 1 to 18 carbon atoms, and
a, b, c, and d each independently represent an integer of 0 to 3, provided that a + b is 1 or more, and c + d is 1 or more.

7. The method according to any one of claims 1 to 5,
wherein the ionizable lipid is a compound represented by Formula (2) or a salt thereof, 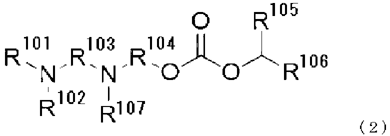
in the formula,
R¹⁰¹ and R¹⁰² each independently represent a hydrocarbon group having 1 to 18 carbon atoms, and R¹⁰³ represents a hydrocarbon group having 2 to 8 carbon atoms, where the hydrocarbon groups represented by R¹⁰¹, R¹⁰², and R¹⁰³ may be substituted with one or more substituents selected from -OH, -COOH, -NR¹⁵¹R¹⁵², -OC(O)O-R¹⁵³, -C(O)O-R¹⁵⁴, -OC(O)-R¹⁵⁵, and -O-R¹⁵⁶, and R¹⁰² and R¹⁰³ may be linked to each other to form a 4- to 7-membered ring,
R¹⁰⁴ represents a hydrocarbon group having 1 to 8 carbon atoms,
R¹⁰⁵ and R¹⁰⁶ each independently represent a hydrocarbon group having 1 to 8 carbon atoms or -R¹⁰⁸-L¹⁰¹-R¹⁰⁹, excluding a case where both R¹⁰⁵ and R¹⁰⁶ are hydrocarbon groups having 1 to 8 carbon atoms,
R¹⁰⁷ represents -R¹¹⁰-L¹⁰²-R¹¹¹-L¹⁰³-R¹¹²,
R¹⁵¹ and R¹⁵² each independently represent a hydrocarbon group having 1 to 8 carbon atoms,
R¹⁵³, R¹⁵⁴, R¹⁵⁵, and R¹⁵⁶ each independently represent a hydrocarbon group having 1 to 24 carbon atoms,
the hydrocarbon groups represented by R¹⁵³, R¹⁵⁴, R¹⁵⁵, and R¹⁵⁶ may be substituted with an aryl group having 6 to 20 carbon atoms or -S-R¹⁵⁸,
the aryl group having 6 to 20 carbon atoms may be substituted with -OH, -COOH, -NR¹⁵¹R¹⁵², -OC(O)O-R¹⁵³, -C(O)O-R¹⁵⁴, -OC(O)-R¹⁵⁵, -O-R¹⁵⁶, or -(hydrocarbon group having 1 to 12 carbon atoms)-R¹⁵⁷,
R¹⁵⁸ represents a hydrocarbon group having 1 to 12 carbon atoms,
R¹⁵⁷ represents -OH, -COOH, -NR¹⁶¹R¹⁶², -OC(O)O-R¹⁶³, -C(O)O-R¹⁶⁴, -OC(O)-R¹⁶⁵, or -O-R¹⁶⁶,
R¹⁶¹ and R¹⁶² each independently represent a hydrocarbon group having 1 to 8 carbon atoms,
R¹⁶³, R¹⁶⁴, R¹⁶⁵, and R¹⁶⁶ each independently represent a hydrocarbon group having 1 to 24 carbon atoms,
the hydrocarbon groups represented by R¹⁶³, R¹⁶⁴, R¹⁶⁵, and R¹⁶⁶ may be substituted with an aryl group having 6 to 20 carbon atoms or -S-R¹⁶⁸,
the aryl group having 6 to 20 carbon atoms may be substituted with -OH, -COOH, -NR¹⁶¹R¹⁶², -OC(O)O-R¹⁶³, -C(O)O-R¹⁶⁴, -OC(O)-R¹⁶⁵, -O-R¹⁶⁶, or a hydrocarbon group having 1 to 12 carbon atoms,
R¹⁶⁸ represents a hydrocarbon group having 1 to 12 carbon atoms,
L¹⁰¹, L¹⁰², and L¹⁰³ each independently represent -OC(O)O-, -C(O)O-, -OC(O)-, or -O-,
R¹⁰⁸ represents a hydrocarbon group having 1 to 12 carbon atoms,
R¹⁰⁹ represents a hydrocarbon group having 1 to 24 carbon atoms,
R¹¹⁰ represents a hydrocarbon group having 1 to 8 carbon atoms,
R¹¹¹ represents a hydrocarbon group having 1 to 24 carbon atoms,
R¹¹² represents a hydrocarbon group having 1 to 24 carbon atoms,
the hydrocarbon groups represented by R¹⁰⁹ and R¹¹² may be substituted with an aryl group, -OC(O)O-R¹⁵³, -C(O)O-R¹⁵⁴, -OC(O)-R¹⁵⁵, or -S-R¹⁵⁸, where definitions of R¹⁵³, R¹⁵⁴, R¹⁵⁵, and R¹⁵⁸ are as described above, and
the hydrocarbon group represented by R¹¹¹ may be substituted with -OC(O)O-R¹⁵³, -C(O)O-R¹⁵⁴, or -OC(O)-R¹⁵⁵, where the definitions of R¹⁵³, R¹⁵⁴, and R¹⁵⁵ are as described above.

8. The method according to any one of claims 1 to 5,
wherein the non-ionizable lipid contains a sterol or a derivative thereof, and a phospholipid.

9. The method according to claim 8,
wherein a molar ratio of the sterol to total lipids in a lipid composition is 30 to 70 mol%.

10. The method according to any one of claims 1 to 5,
wherein a pH of a lipid composition is 3.0 to 6.5.

11. The method according to any one of claims 1 to 5,
wherein, in the step B, a mass ratio of a lipid concentration to a nucleic acid concentration in a solution after the mixing is 5:1 to 1,000:1.

12. The method according to any one of claims 1 to 5,
wherein the immune cell is an activated cell or a non-activated cell.

13. The method according to any one of claims 1 to 5,
wherein the immune cell is derived from a primary cell or a stem cell.

14. The method according to any one of claims 1 to 5,
wherein the nucleic acid is
mRNA, or
a nucleic acid for gene editing that contains an mRNA encoding a Cas nuclease and a guide RNA.

15. A nucleic acid delivery agent for an immune cell, comprising:
a lipid composition containing an ionizable lipid that is a compound represented by Formula (1) or Formula (2) or a salt thereof, a non-ionizable lipid, a lipid having a nonionic polymer, and a nucleic acid, 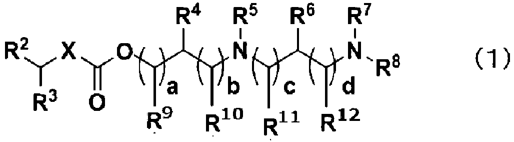
wherein in the formula, X represents -NR¹- or -O-,
R¹ represents a hydrogen atom, a hydrocarbon group having 6 to 24 carbon atoms, or a group represented by R²¹-L¹-R²²-, where R²¹ represents a hydrocarbon group having 1 to 24 carbon atoms, L¹ represents -O(CO)O-, -O(CO)-, -(CO)O-, -O-, or 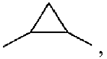 and R³² represents a divalent hydrocarbon linking group having 1 to 18 carbon atoms,
R² and R³ each independently represent a hydrogen atom, a hydrocarbon group having 3 to 24 carbon atoms, or a group represented by R³¹-L²-R³²-, where R³¹ represents a hydrocarbon group having 1 to 24 carbon atoms, L² represents -O(CO)O-, -O(CO)-, -(CO)O-, -O-, or 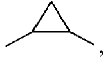 and R³² represents a divalent hydrocarbon linking group having 1 to 18 carbon atoms,
R⁴, R⁵, R⁶, R⁷, R⁸, R¹⁰, R¹¹, and R¹² each independently represent a hydrogen atom or an alkyl group having 1 to 18 carbon atoms that may be substituted,
any one or more of the pairs of R⁴ and R⁵, R¹⁰ and R⁵, R⁵ and R¹², R⁴ and R⁶, R⁵ and R⁶, R⁶ and R⁷, R⁶ and R¹⁰, R¹² and R⁷, and R⁷ and R⁸ may be linked to each other to form a 4- to 7-membered ring which may contain an O atom, a substituent on the alkyl group having 1 to 18 carbon atoms which may be substituted is a hydroxyl group, a carboxyl group, an amino group represented by -NR⁴⁵R⁴⁶, a substituted or unsubstituted aryl group, a substituted or unsubstituted heteroaryl group, or a group represented by -O(CO)O-R⁴¹, -O(CO)-R⁴², -(CO)O-R⁴³, or -O-R⁴⁴, where R⁴¹, R⁴², R⁴³ R⁴⁴, R⁴⁵, and R⁴⁶ each independently represent a hydrocarbon group having 1 to 18 carbon atoms,
a substituent on the substituted or unsubstituted aryl group and on the substituted or unsubstituted heteroaryl group is an alkyl group having 1 to 18 carbon atoms, a hydroxyl group, a carboxyl group, an amino group represented by -NR⁴⁵R⁴⁶, or a group represented by -O(CO)O-R⁴¹, -O(CO)-R⁴², -(CO)O-R⁴³, or -O-R⁴⁴, where R⁴¹, R⁴², R⁴³, R⁴⁴, R⁴⁵, and R⁴⁶ each independently represent a hydrocarbon group having 1 to 18 carbon atoms,
a, b, c, and d each independently represent an integer of 0 to 3, provided that a + b is 1 or more, and c + d is 1 or more, 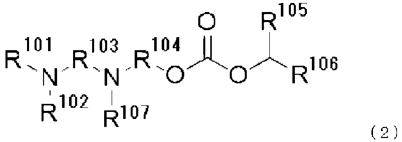
in the formula,
R¹⁰¹ and R¹⁰² each independently represent a hydrocarbon group having 1 to 18 carbon atoms, and R¹⁰³ represents a hydrocarbon group having 2 to 8 carbon atoms, where the hydrocarbon groups represented by R¹⁰¹, R¹⁰², and R¹⁰³ may be substituted with one or more substituents selected from -OH, -COOH, -NR¹⁵¹R¹⁵², -OC(O)O-R¹⁵³, -C(O)O-R¹⁵⁴, -OC(O)-R¹⁵⁵, and -O-R¹⁵⁶, and R¹⁰² and R¹⁰³ may be linked to each other to form a 4- to 7-membered ring,
R¹⁰⁴ represents a hydrocarbon group having 1 to 8 carbon atoms,
R¹⁰⁵ and R¹⁰⁶ each independently represent a hydrocarbon group having 1 to 8 carbon atoms or -R¹⁰⁸-L¹⁰¹-R¹⁰⁹, excluding a case where both R¹⁰⁵ and R¹⁰⁶ are hydrocarbon groups having 1 to 8 carbon atoms,
R¹⁰⁷ represents -R¹¹⁰-L¹⁰²-R¹¹¹-L¹⁰³-R¹¹²,
R¹⁵¹ and R¹⁵² each independently represent a hydrocarbon group having 1 to 8 carbon atoms,
R¹⁵³, R¹⁵⁴, R¹⁵⁵, and R¹⁵⁶ each independently represent a hydrocarbon group having 1 to 24 carbon atoms,
the hydrocarbon groups represented by R¹⁵³, R¹⁵⁴, R¹⁵⁵, and R¹⁵⁶ may be substituted with an aryl group having 6 to 20 carbon atoms or -S-R¹⁵⁸,
the aryl group having 6 to 20 carbon atoms may be substituted with -OH, -COOH, -NR¹⁵¹R¹⁵², -OC(O)O-R¹⁵³, -C(O)O-R¹⁵⁴, -OC(O)-R¹⁵⁵, -O-R¹⁵⁶, or -(hydrocarbon group having 1 to 12 carbon atoms)-R¹⁵⁷,
R¹⁵⁸ represents a hydrocarbon group having 1 to 12 carbon atoms,
R¹⁵⁷ represents -OH, -COOH, -NR¹⁶¹R¹⁶², -OC(O)O-R¹⁶³, -C(O)O-R¹⁶⁴, -OC(O)-R¹⁶⁵, or -O-R¹⁶⁶,
R¹⁶¹ and R¹⁶² each independently represent a hydrocarbon group having 1 to 8 carbon atoms,
R¹⁶³, R¹⁶⁴, R¹⁶⁵, and R¹⁶⁶ each independently represent a hydrocarbon group having 1 to 24 carbon atoms,
the hydrocarbon groups represented by R¹⁶³, R¹⁶⁴, R¹⁶⁵, and R¹⁶⁶ may be substituted with an aryl group having 6 to 20 carbon atoms or -S-R¹⁶⁸,
the aryl group having 6 to 20 carbon atoms may be substituted with -OH, -COOH, -NR¹⁶¹R¹⁶², -OC(O)O-R¹⁶³, -C(O)O-R¹⁶⁴, -OC(O)-R¹⁶⁵, -O-R¹⁶⁶, or a hydrocarbon group having 1 to 12 carbon atoms,
R¹⁶⁸ represents a hydrocarbon group having 1 to 12 carbon atoms, and
L¹⁰¹, L¹⁰², and L¹⁰³ each independently represent -OC(O)O-, -C(O)O-, -OC(O)-, or -O-,
R¹⁰⁸ represents a hydrocarbon group having 1 to 12 carbon atoms,
R¹⁰⁹ represents a hydrocarbon group having 1 to 24 carbon atoms,
R¹¹⁰ represents a hydrocarbon group having 1 to 8 carbon atoms,
R¹¹¹ represents a hydrocarbon group having 1 to 24 carbon atoms,
R¹¹² represents a hydrocarbon group having 1 to 24 carbon atoms,
the hydrocarbon groups represented by R¹⁰⁹ and R¹¹² may be substituted with an aryl group, -OC(O)O-R¹⁵³, -C(O)O-R¹⁵⁴, -OC(O)-R¹⁵⁵, or -S-R¹⁵⁸, where definitions of R⁵³, R⁵⁴, R⁵⁵, and R⁵⁸ are as described above, and
the hydrocarbon group represented by R¹¹¹ may be substituted with -OC(O)O-R¹⁵³, -C(O)O-R¹⁵⁴, or -OC(O)-R¹⁵⁵, where the definitions of R¹⁵³, R¹⁵⁴, and R¹⁵⁵ are as described above.

16. The nucleic acid delivery agent for an immune cell according to claim 15,
wherein the non-ionizable lipid contains a sterol or a derivative thereof, and a phospholipid.

17. The nucleic acid delivery agent for an immune cell according to claim 16,
wherein a molar ratio of the sterol to total lipids in a lipid composition is 30 to 70 mol%.

18. The nucleic acid delivery agent for an immune cell according to any one of claims 15 to 17,
wherein the nucleic acid is
mRNA, or
a nucleic acid for gene editing, that contains an mRNA encoding a Cas nuclease and a guide RNA.

19. A nucleic acid delivery agent for an immune cell, comprising:
a lipid composition containing an ionizable lipid that is a compound represented by Formula (1) or Formula (2) or a salt thereof, a non-ionizable lipid, and a lipid having a nonionic polymer, 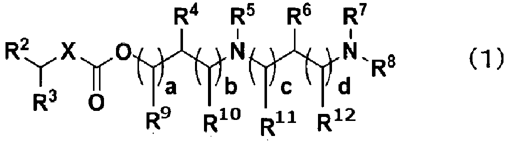
wherein in the formula, X represents -NR¹- or -O-,
R¹ represents a hydrogen atom, a hydrocarbon group having 10 to 24 carbon atoms, or a group represented by R²¹-L¹-R²²-, where R²¹ represents a hydrocarbon group having 1 to 24 carbon atoms, L¹ represents -O(CO)O-, -O(CO)-, -(CO)O-, -O-, or 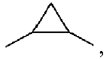 and R³² represents a divalent hydrocarbon linking group having 1 to 18 carbon atoms,
R² and R³ each independently represent a hydrogen atom, a hydrocarbon group having 3 to 24 carbon atoms, or a group represented by R³¹-L²-R³²-, where R³¹ represents a hydrocarbon group having 1 to 24 carbon atoms, L² represents -O(CO)O-, -O(CO)-, -(CO)O-, -O-, or 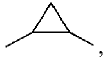 and R³² represents a divalent hydrocarbon linking group having 1 to 18 carbon atoms,
R⁴, R⁵, R⁶, R⁷, R⁸, R¹⁰, R¹¹, and R¹² each independently represent a hydrogen atom or an alkyl group having 1 to 18 carbon atoms that may be substituted,
any one or more of the pairs of R⁴ and R⁵, R¹⁰ and R⁵, R⁵ and R¹², R⁴ and R⁶, R⁵ and R⁶, R⁶ and R⁷, R⁶ and R¹⁰, R¹² and R⁷, and R⁷ and R⁸ may be linked to each other to form a 4-to 7-membered ring which may contain an O atom,
a substituent on the alkyl group having 1 to 18 carbon atoms which may be substituted is a hydroxyl group, a carboxyl group, an amino group represented by -NR⁴⁵R⁴⁶, a substituted or unsubstituted aryl group, a substituted or unsubstituted heteroaryl group, or a group represented by -O(CO)O-R⁴¹, -O(CO)-R⁴², -(CO)O-R⁴³, or -O-R⁴⁴, where R⁴¹, R⁴², R⁴³, R⁴⁴, R⁴⁵, and R⁴⁶ each independently represent a hydrocarbon group having 1 to 18 carbon atoms,
a substituent on the substituted or unsubstituted aryl group and on the substituted or unsubstituted heteroaryl group is an alkyl group having 1 to 18 carbon atoms, a hydroxyl group, a carboxyl group, an amino group represented by -NR⁴⁵R⁴⁶, or a group represented by -O(CO)O-R⁴¹, -O(CO)-R⁴², -(CO)O-R⁴³, or -O-R⁴⁴, where R⁴¹, R⁴², R⁴³, R⁴⁴, R⁴⁵, and R⁴⁶ each independently represent a hydrocarbon group having 1 to 18 carbon atoms, and
a, b, c, and d each independently represent an integer of 0 to 3, provided that a + b is 1 or more, and c + d is 1 or more, 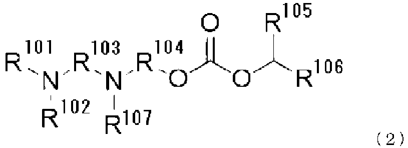
in the formula,
R¹⁰¹ and R¹⁰² each independently represent a hydrocarbon group having 1 to 18 carbon atoms, and R¹⁰³ represents a hydrocarbon group having 2 to 8 carbon atoms, where the hydrocarbon groups represented by R¹⁰¹, R¹⁰², and R¹⁰³ may be substituted with one or more substituents selected from -OH, -COOH, -NR¹⁵¹R¹⁵², -OC(O)O-R¹⁵³, -C(O)O-R¹⁵⁴, -OC(O)-R¹⁵⁵, and -O-R¹⁵⁶, and R¹⁰² and R¹⁰³ may be linked to each other to form a 4- to 7-membered ring,
R¹⁰⁴ represents a hydrocarbon group having 1 to 8 carbon atoms,
R¹⁰⁵ and R¹⁰⁶ each independently represent a hydrocarbon group having 1 to 8 carbon atoms or -R¹⁰⁸-L¹⁰¹-R¹⁰⁹, excluding a case where both R¹⁰⁵ and R¹⁰⁶ are hydrocarbon groups having 1 to 8 carbon atoms,
R¹⁰⁷ represents -R¹¹⁰-L¹⁰²-R¹¹¹-L¹⁰³-R¹¹²,
R¹⁵¹ and R¹⁵² each independently represent a hydrocarbon group having 1 to 8 carbon atoms,
R¹⁵³, R¹⁵⁴, R¹⁵⁵, and R¹⁵⁶ each independently represent a hydrocarbon group having 1 to 24 carbon atoms,
the hydrocarbon groups represented by R¹⁵³, R¹⁵⁴, R¹⁵⁵, and R¹⁵⁶ may be substituted with an aryl group having 6 to 20 carbon atoms or -S-R¹⁵⁸,
the aryl group having 6 to 20 carbon atoms may be substituted with -OH, -COOH, -NR¹⁵¹R¹⁵², -OC(O)O-R¹⁵³, -C(O)O-R¹⁵⁴, -OC(O)-R¹⁵⁵, -O-R¹⁵⁶, or -(hydrocarbon group having 1 to 12 carbon atoms)-R¹⁵⁷,
R¹⁵⁸ represents a hydrocarbon group having 1 to 12 carbon atoms,
R¹⁵⁷ represents -OH, -COOH, -NR¹⁶¹R¹⁶², -OC(O)O-R¹⁶³, -C(O)O-R¹⁶⁴, -OC(O)-R¹⁶⁵, or -O-R¹⁶⁶,
R¹⁶¹ and R¹⁶² each independently represent a hydrocarbon group having 1 to 8 carbon atoms,
R¹⁶³, R¹⁶⁴, R¹⁶⁵, and R¹⁶⁶ each independently represent a hydrocarbon group having 1 to 24 carbon atoms,
the hydrocarbon groups represented by R¹⁶³, R¹⁶⁴, R¹⁶⁵, and R¹⁶⁶ may be substituted with an aryl group having 6 to 20 carbon atoms or -S-R¹⁶⁸,
the aryl group having 6 to 20 carbon atoms may be substituted with -OH, -COOH, -NR¹⁶¹R¹⁶², -OC(O)O-R¹⁶³, -C(O)O-R¹⁶⁴, -OC(O)-R¹⁶⁵, -O-R¹⁶⁶, or a hydrocarbon group having 1 to 12 carbon atoms,
R¹⁶⁸ represents a hydrocarbon group having 1 to 12 carbon atoms, and
L¹⁰¹, L¹⁰², and L¹⁰³ each independently represent -OC(O)O-, -C(O)O-, -OC(O)-, or -O-,
R¹⁰⁸ represents a hydrocarbon group having 1 to 12 carbon atoms,
R¹⁰⁹ represents a hydrocarbon group having 1 to 24 carbon atoms,
R¹¹⁰ represents a hydrocarbon group having 1 to 8 carbon atoms,
R¹¹¹ represents a hydrocarbon group having 1 to 24 carbon atoms,
R¹¹² represents a hydrocarbon group having 1 to 24 carbon atoms,
the hydrocarbon groups represented by R¹⁰⁹ and R¹¹² may be substituted with an aryl group, -OC(O)O-R¹⁵³, -C(O)O-R¹⁵⁴, -OC(O)-R¹⁵⁵, or -S-R¹⁵⁸, where definitions of R⁵³, R⁵⁴, R⁵⁵, and R⁵⁸ are as described above, and
the hydrocarbon group represented by R¹¹¹ may be substituted with -OC(O)O-R¹⁵³, -C(O)O-R¹⁵⁴, or -OC(O)-R¹⁵⁵, where the definitions of R¹⁵³, R¹⁵⁴, and R¹⁵⁵ are as described above.

20. The nucleic acid delivery agent for an immune cell according to claim 19,
wherein the non-ionizable lipid contains a sterol or a derivative thereof, and a phospholipid.

21. The nucleic acid delivery agent for an immune cell according to claim 19,
wherein a molar ratio of the sterol to total lipids in a lipid composition is 30 to 70 mol%.

22. A kit for delivering nucleic acid to a cell, the kit comprising:
the following reagents (A) to (C),
(A) a lipid composition containing an ionizable lipid that is a compound represented by Formula (1) or Formula (2) or a salt thereof, a non-ionizable lipid, and a lipid having a nonionic polymer,
(B) a pH adjusting agent, and
(C) an apolipoprotein, 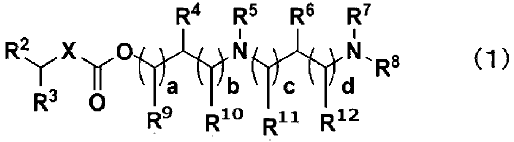
wherein in the formula, X represents -NR¹- or -O-,
R¹ represents a hydrogen atom, a hydrocarbon group having 14 to 24 carbon atoms, or a group represented by R²¹-L¹-R²²-, where R²¹ represents a hydrocarbon group having 1 to 24. carbon atoms, L¹ represents -O(CO)O-, -O(CO)-, -(CO)O-, -O-, or 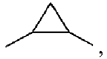 and R³² represents a divalent hydrocarbon linking group having 1 to 18 carbon atoms,
R² and R³ each independently represent a hydrogen atom, a hydrocarbon group having 3 to 24 carbon atoms, or a group represented by R³¹-L²-R³²-, where R³¹ represents a hydrocarbon group having 1 to 24 carbon atoms, L² represents -O(CO)O-, -O(CO)-, -(CO)O-, -O-, or 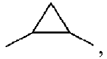 and R³² represents a divalent hydrocarbon linking group having 1 to 18 carbon atoms,
R⁴, R⁵, R⁶, R⁷, R⁸, R¹⁰, R¹¹, and R¹² each independently represent a hydrogen atom or an alkyl group having 1 to 18 carbon atoms that may be substituted,
any one or more of the pairs of R⁴ and R⁵, R¹⁰ and R⁵, R⁵ and R¹², R⁴ and R⁶, R⁵ and R⁶, R⁶ and R⁷, R⁶ and R¹⁰, R¹² and R⁷, and R⁷ and R⁸ may be linked to each other to form a 4-to 7-membered ring which may contain an O atom,
a substituent on the alkyl group having 1 to 18 carbon atoms which may be substituted is a hydroxyl group, a carboxyl group, an amino group represented by -NR⁴⁵R⁴⁶, a substituted or unsubstituted aryl group, a substituted or unsubstituted heteroaryl group, or a group represented by -O(CO)O-R⁴¹, -O(CO)-R⁴², -(CO)O-R⁴³, or -O-R⁴⁴, where R⁴¹, R⁴², R⁴³, R⁴⁴, R⁴⁵, and R⁴⁶ each independently represent a hydrocarbon group having 1 to 18 carbon atoms,
a substituent on the substituted or unsubstituted aryl group and on the substituted or unsubstituted heteroaryl group is an alkyl group having 1 to 18 carbon atoms, a hydroxyl group, a carboxyl group, an amino group represented by -NR⁴⁵R⁴⁶, or a group represented by -O(CO)O-R⁴¹, -O(CO)-R⁴², -(CO)O-R⁴³, or -O-R⁴⁴, where R⁴¹, R⁴², R⁴³, R⁴⁴, R⁴⁵, and R⁴⁶ each independently represent a hydrocarbon group having 1 to 18 carbon atoms, and
a, b, c, and d each independently represent an integer of 0 to 3, provided that a + b is 1 or more, and c + d is 1 or more, 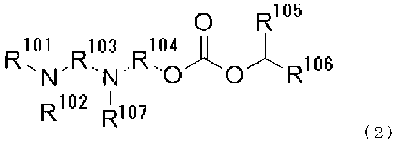
in the formula,
R¹⁰¹ and R¹⁰² each independently represent a hydrocarbon group having 1 to 18 carbon atoms, and R¹⁰³ represents a hydrocarbon group having 2 to 8 carbon atoms, where the hydrocarbon groups represented by R¹⁰¹, R¹⁰², and R¹⁰³ may be substituted with one or more substituents selected from -OH, -COOH, -NR¹⁵¹R¹⁵² , -OC(O)O-R¹⁵¹, -C(O)O-R¹⁵⁴, -OC(O)-R¹⁵⁵, and -O-R¹⁵⁶, and R¹⁰² and R¹⁰³ may be linked to each other to form a 4- to 7-membered ring,
R¹⁰⁴ represents a hydrocarbon group having 1 to 8 carbon atoms,
R¹⁰⁵ and R¹⁰⁶ each independently represent a hydrocarbon group having 1 to 8 carbon atoms or -R¹⁰⁸-L¹⁰¹-R¹⁰⁹, excluding a case where both R¹⁰⁵ and R¹⁰⁶ are hydrocarbon groups having 1 to 8 carbon atoms,
R¹⁰⁷ represents -R¹¹⁰-L¹⁰²-R¹¹¹-L¹⁰³-R¹¹²,
R¹⁵¹ and R¹⁵² each independently represent a hydrocarbon group having 1 to 8 carbon atoms,
R¹⁵³, R¹⁵⁴, R¹⁵⁵, and R¹⁵⁶ each independently represent a hydrocarbon group having 1 to 24 carbon atoms,
the hydrocarbon groups represented by R¹⁵³, R¹⁵⁴, R¹⁵⁵, and R¹⁵⁶ may be substituted with an aryl group having 6 to 20 carbon atoms or -S-R¹⁵⁸,
the aryl group having 6 to 20 carbon atoms may be substituted with -OH, -COOH, -NR¹⁵¹R¹⁵², -OC(O)O-R¹⁵³, -C(O)O-R¹⁵⁴, -OC(O)-R¹⁵⁵, -O-R¹⁵⁶, or -(hydrocarbon group having 1 to 12 carbon atoms)-R¹⁵⁷,
R¹⁵⁸ represents a hydrocarbon group having 1 to 12 carbon atoms,
R¹⁵⁷ represents -OH, -COOH, -NR¹⁶¹R¹⁶², -OC(O)O-R¹⁶³, -C(O)O-R¹⁶⁴, -OC(O)-R¹⁶⁵, or -O-R¹⁶⁶,
R¹⁶¹ and R¹⁶² each independently represent a hydrocarbon group having 1 to 8 carbon atoms,
R¹⁶³, R¹⁶⁴, R¹⁶⁵, and R¹⁶⁶ each independently represent a hydrocarbon group having 1 to 24 carbon atoms,
the hydrocarbon groups represented by R¹⁶³, R¹⁶⁴, R¹⁶⁵, and R¹⁶⁶ may be substituted with an aryl group having 6 to 20 carbon atoms or -S-R¹⁶⁸,
the aryl group having 6 to 20 carbon atoms may be substituted with -OH, -COOH, -NR¹⁶¹R¹⁶², -OC(O)O-R¹⁶³, -C(O)O-R¹⁶⁴, -OC(O)-R¹⁶⁵, -O-R¹⁶⁶, or a hydrocarbon group having 1 to 12 carbon atoms,
R¹⁶⁸ represents a hydrocarbon group having 1 to 12 carbon atoms, and
L¹⁰¹, L¹⁰², and L¹⁰³ each independently represent -OC(O)O-, -C(O)O-, -OC(O)-, or -O-,
R¹⁰⁸ represents a hydrocarbon group having 1 to 12 carbon atoms,
R¹⁰⁹ represents a hydrocarbon group having 1 to 24 carbon atoms,
R¹¹⁰ represents a hydrocarbon group having 1 to 8 carbon atoms,
R¹¹¹ represents a hydrocarbon group having 1 to 24 carbon atoms,
R¹¹² represents a hydrocarbon group having 1 to 24 carbon atoms,
the hydrocarbon groups represented by R¹⁰⁹ and R¹¹² may be substituted with an aryl group, -OC(O)O-R¹⁵³, -C(O)O-R¹⁵⁴, -OC(O)-R¹⁵⁵, or -S-R¹⁵⁸, where definitions of R⁵³, R⁵⁴, R⁵⁵, and R⁵⁸ are as described above, and
the hydrocarbon group represented by R¹¹¹ may be substituted with -OC(O)O-R¹⁵³, -C(O)O-R¹⁵⁴, or -OC(O)-R¹⁵⁵, where the definitions of R¹⁵³, R¹⁵⁴, and R¹⁵⁵ are as described above.

23. A method for delivering nucleic acid to a cell using the kit according to claim 22, the method comprising:
the following steps (1) to (4),
(1) a step of mixing the reagent (A) with a nucleic acid,
(2) a step of adjusting a pH of the mixture obtained in the step (1) with the reagent (B),
(3) a step of adding the reagent (C) to a culture medium containing a cell, and
(4) a step of adding the mixture obtained in the step (2) to the culture medium obtained in the step (3).

24. The method for delivering nucleic acid according to claim 23, wherein the cell is an immune cell.

25. At least one compound selected from the group consisting of the following compounds, or a salt thereof, 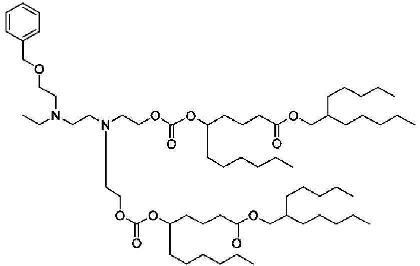 bis(2-pentylheptyl) 11-(2-((2-(benzyloxy)ethyl)(ethyl)amino)ethyl)-5,17-dihexyl-7,15-dioxo-6,8,14,16-tetraoxa-11 -azahenicosanedioate, 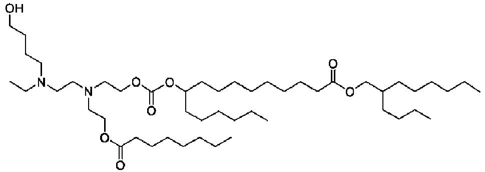 2-butyloctyl 5-ethyl-14-hexyl-1-hydroxy-8-(2-(octanoyloxy)ethyl)-12-oxo-11,13-dioxa-5,8-diazatricosan-2 3-oate, 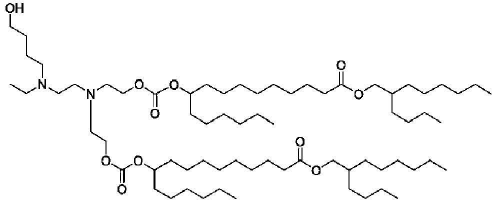 bis(2-butyloctyl) 16-(2-(ethyl(4-hydroxybutyl)amino)ethyl)-10,22-dihexyl-12,20-dioxo-11,13,19,21-tetraoxa-16 -azahentriacontanedioate, 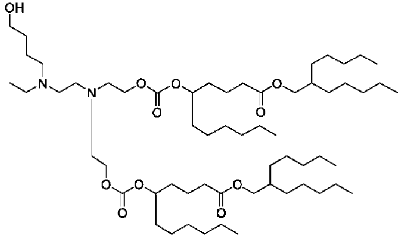 bis(2-pentylheptyl) 11-(2-(ethyl(4-hydroxybutyl)amino)ethyl)-5,17-dihexyl-7,15-dioxo-6,8,14,16-tetraoxa-11-aza henicosanedioate, 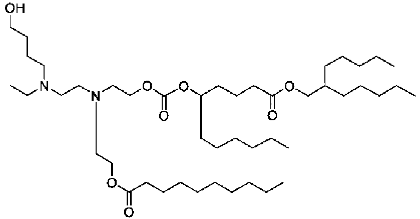 2-pentylheptyl 8-(2-(decanoyloxy)ethyl)-5-ethyl-14-hexyl-1-hydroxy-12-oxo-11,13-dioxa-5,8-diazaoctadecan -18-oate, 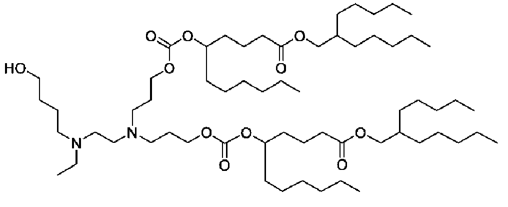 bis(2-pentylheptyl) 12-(2-(ethyl(4-hydroxybutyl)amino)ethyl)-5,19-dihexyl-7,17-dioxo-6,8,16,18-tetraoxa-12-azat ricosanedioate, 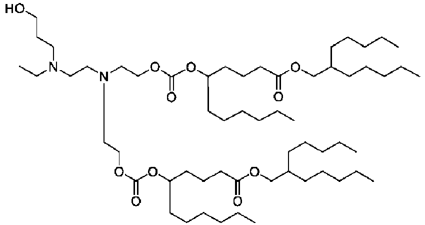 bis(2-pentylheptyl) 11-(2-(ethyl(3-hydroxypropyl)amino)ethyl)-5,17-dihexyl-7,15-dioxo-6,8,14,16-tetraoxa-11-az ahenicosanedioate, 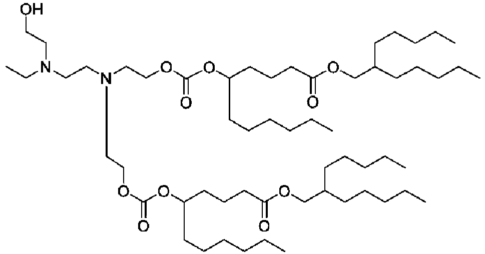 bis(2-pentylheptyl) 11-(2-(ethyl(2-hydroxyethyl)amino)ethyl)-5,17-dihexyl-7,15-dioxo-6,8,14,16-tetraoxa-11-azah enicosanedioate, 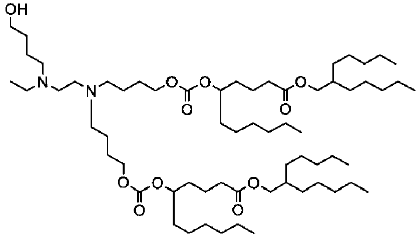 bis(2-pentylheptyl) 13-(2-(ethyl(4-hydroxybutyl)amino)ethyl)-5,21-dihexyl-7,19-dioxo-6,8,18,20-tetraoxa-13-aza pentacosanedioate, 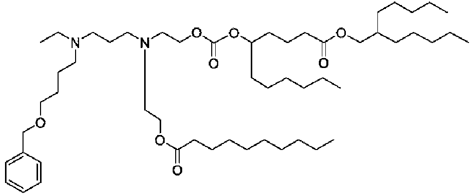 2-pentylheptyl 11-(2-(decanoyloxy)ethyl)-7-ethyl-17-hexyl-15-oxo-1-phenyl-2,14,16-trioxa-7,11-diazahenico san-21-oate, 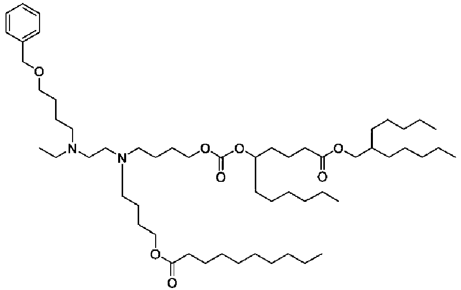 2-pentylheptyl 10-(4-(decanoyloxy)butyl)-7-ethyl-18-hexyl-16-oxo-1-phenyl-2,15,17-trioxa-7,10-diazadocos an-22-oate, 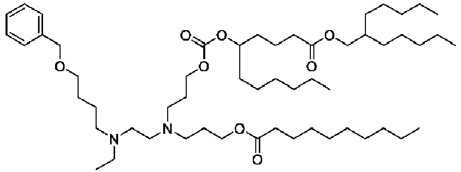 2-pentylheptyl 10-(3-(decanoyloxy)propyl)-7-ethyl-17-hexyl-15-oxo-1-phenyl-2,14,16-trioxa-7,10-diazahenic osan-21-oate, and 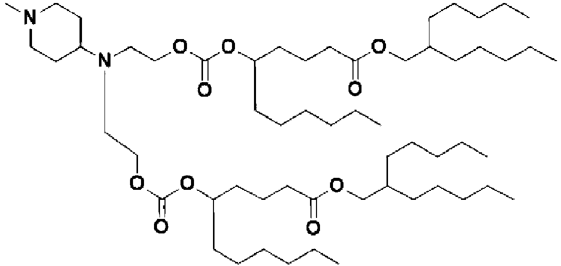 bis(2-pentylheptyl) 5,17-dihexyl-11-(1-methylpiperidin-4-yl)-7,15-dioxo-6,8,14,16-tetraoxa-11-azahenicosanedioa te.
